(19) Europäisches Patentamt
European Patent Office
Office européen des brevets

(11) EP 3 171 421 B1

(12) **EUROPEAN PATENT SPECIFICATION**

(45) Date of publication and mention of the grant of the patent:
**25.01.2023 Bulletin 2023/04**

(21) Application number: **15822630.8**

(22) Date of filing: **17.07.2015**

(51) International Patent Classification (IPC):
***H01L 51/50*** *(2006.01)* ***H01S 5/36*** *(2006.01)*
***C09K 11/06*** *(2006.01)* ***C07D 487/14*** *(2006.01)*
***C07D 405/04*** *(2006.01)* ***C07D 251/24*** *(2006.01)*
***C07D 491/147*** *(2006.01)* ***C07D 209/86*** *(2006.01)*
***C07D 213/24*** *(2006.01)* ***C07D 213/79*** *(2006.01)*
*H01S 5/04 (2006.01)*

(52) Cooperative Patent Classification (CPC):
**H01S 5/36; C07D 209/86; C07D 213/24; C07D 213/79; C07D 251/24; C07D 405/04; C07D 487/14; C07D 491/147; C09K 11/06; H01L 51/5004; H01L 51/5012;** H01S 5/041

(86) International application number:
**PCT/JP2015/070510**

(87) International publication number:
**WO 2016/010136 (21.01.2016 Gazette 2016/03)**

(54) **ORGANIC LIGHT-EMITTING ELEMENT**

ORGANISCHES LICHTEMITTIERENDES ELEMENT

ÉLÉMENT ÉLECTROLUMINESCENT ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.07.2014 JP 2014148182**

(43) Date of publication of application:
**24.05.2017 Bulletin 2017/21**

(73) Proprietors:
- **Kyushu University, National University Corporation Fukuoka-shi, Fukuoka 812-8581 (JP)**
- **KOALA Tech Inc. Fukuoka-shi, Fukuoka 819-0388 (JP)**

(72) Inventors:
- **NAKANOTANI, Hajime Fukuoka-shi Fukuoka 812-8581 (JP)**
- **FURUKAWA, Taro Fukuoka-shi Fukuoka 812-8581 (JP)**
- **ADACHI, Chihaya Fukuoka-shi Fukuoka 812-8581 (JP)**

(74) Representative: **Zimmermann & Partner Patentanwälte mbB Josephspitalstr. 15 80331 München (DE)**

(56) References cited:
**WO-A1-2012/099241 WO-A1-2012/133188**
**WO-A1-2014/013947 WO-A1-2015/002213**
**WO-A1-2015/098975 DE-A1-102012 021 174**
**JP-A- 2009 094 124 JP-B1- 5 669 163**

- **Tetsuo Nozawa: "Kyushu University Claims OLED Breakthrough", , 1 June 2014 (2014-06-01), pages 1-5, XP055447693, Retrieved from the Internet: URL:http://techon.nikkeibp.co.jp/english/NEWS_EN/20140601/355340/?ST=SP [retrieved on 2018-02-05]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 171 421 B1

## Description

### Technical Field

The present invention relates to an organic light-emitting device having a high light emission efficiency.

### Background Art

Studies are being actively performed for enhancing the light emission efficiency of an organic light-emitting device. Among these, there are seen studies relating to an organic light-emitting device (an organic semiconductor laser) using an organic light-emitting material that radiates amplified spontaneous emission (ASE) in the relaxation process from an excited state.

For example, PTL 1 discloses an organic semiconductor laser having a co-deposited film of BSB-CN (1,4-dinitrile-2,5-bis(4-(bis(4-methoxyphenyl)amino)styryl)benzene) and Ace-CBP (bis(4-carbazoylphenyl)acetylene), as an active layer. Here, BSB-CN is a fluorescent material, and Ace-CBP functions as a host material. The literature describes observation of ASE oscillation from the organic semiconductor thin film.

PTL 2 discloses an organic semiconductor laser having an organic layer that contains an organic host compound, an organic light-emitting compound and an organic dopant compound, defining that the excited triplet energy of the organic dopant compound is lower than the excited triplet energy of the organic host compound and the organic light-emitting compound and that the excited singlet energy of the organic light-emitting material is lower than the excited singlet energy of the organic host compound. Here, the literature says that the organic dopant compound functions as a "triplet manager" that traps the triplet exciton formed in the organic layer, and accordingly, accumulation of triplet excitons can be prevented and light loss and deactivation of singlet excitons caused by triplet excitons can be thereby prevented. In Tetsuo Nozawa, Kyushu University Claims OLED Breakthrough, 1 June 2014, pages 1 to 5, XP055447693, it is reported that the Center for Organic Photonics and Electronics Research (OPERA) has developed an OLED device that emits light by using a fluorescent material with an internal quantum efficiency of 100 %. The device was realized by using the "thermally activated delayed fluorescence" (TADF), which OPERA has been developing, as an assistant dopant and dispersing it in the light-emitting layer of a conventional fluorescent OLED device. Compare with previous TADF, the new technology enables to make a material and device with a more versatile and easier method, and improves the durability of the device.

From DE 10 2012 021 174 A1 a device is known which comprises an organic semiconductor laser. The organic semiconductor laser further comprises an optical resonator and an organic layer which located in the optical resonator. The organic layer comprises an organic host compound, an organic emitting compound which is capable of emitting fluorescence, and an organic doping compound. The organic doping compound is also called a "triplet-manager". The triplet energy of the organic doping compound is less than or equal to the triplet energy of the organic host compound. The triplet energy of the organic doping compound is less than or equal to the triplet energy of the organic emitting compound. The singlet energy of the organic emitting compound is less than or equal to the singlet energy of the organic host compound.

### Citation List

#### Patent Literature

PTL 1: JP-A 2006-265172
PTL 2: USP 8654806

### Summary of Invention

#### Technical Problem

However, the light emission efficiency of the organic semiconductor lasers described in PTLs 1 and 2 could not be sufficiently increased because of the following reasons.

Specifically, when external energy is given to the active layer (light-emitting layer) containing a fluorescent material that radiates ASE and a host material, the host material mainly absorbs energy and transits to an excited singlet state, and the excited singlet energy transfer to the fluorescent material. The fluorescent material having received energy and transited to an excited singlet state thereafter returns to a ground state while radiating ASE. On the other hand, in

the light-emitting layer, a triplet exciton also forms through intersystem crossing from the excited singlet state. Here, a triplet exciton relaxation process hardly occurs as compared with a singlet exciton relaxation process, and therefore a triplet exciton has a longer lifetime as compared with a singlet exciton and accumulates while energy is given to the active layer. With that, in the light-emitting layer where triplet excitons have accumulated, exciton energy absorption and singlet-triplet annihilation (STA) owing to the triplet excitons readily occur, therefore causing loss of energy capable of contributing toward ASE. For these reasons, improvement of the light emission efficiency of the binary organic semiconductor laser described in PTL 1 is limited, and the threshold level necessary for ASE oscillation becomes high.

On the other hand, the organic semiconductor laser described in PTL 2 uses an organic dopant compound that traps triplet excitons, and therefore can reduce the negative influence to be caused by accumulation of triplet excitons that may occur in a binary system of a fluorescent material and a host material. However, in the organic semiconductor laser, the triplet excitons trapped by the organic dopant compound do not contribute toward ASE radiation, and therefore the excited triplet energy after all goes to waste, and in principle, therefore, the light emission efficiency could not be 100%. In particular, in the system where excitons are formed by carrier injection into a light-emitting layer, that is, in a current excitation-type organic semiconductor laser device, the probability of forming singlet excitons is statistically 25% and that of forming triplet excitons is 75%, and accordingly the loss by no use of excited triplet energy grows at a great rate.

Technical Solution

For solving the problems, the present inventors have started various investigations relating to materials for a light-emitting layer from the viewpoint of effectively utilizing excited triplet energy and, as a result, have found for the first time that, by using a delayed fluorescent material (delayed fluorescent emitter) in addition to the light-emitting material and the host material as the materials for a light-emitting layer, excited triplet energy can become efficiently utilized for ASE radiation, and have further promoted the investigations. As mentioned above, PTL 1 describes an active layer containing BSB-CN of a fluorescent material and Ace-CBP of a host material; and PTL 2 describes an organic layer containing an organic host compound and an organic light-emitting compound and further an organic dopant compound capable of trapping triplet excitons. However, these documents say nothing about addition of a delayed fluorescent material to the active layer, and therefore no one could heretofore anticipate the light emission characteristics of an organic light-emitting device using a delayed fluorescent material.

Under the situation, the present inventors have further investigated the light emission characteristics of an organic light-emitting device that contains a host material, a delayed fluorescent material and a light-emitting material, and have still further promoted the investigations for the purpose of providing an organic light-emitting device having a high light emission efficiency.

As a result of assiduous studies, the present inventors have found that, using a host material, a delayed fluorescent material and a light-emitting material while defining the relationship of the lowest excited singlet energy level $E_{S1}$ between the materials, both the excited singlet energy and the excited triplet energy generated inside an organic light-emitting device can be efficiently contributed to light emission and therefore an organic light-emitting device having a high light emission efficiency can be provided. Based on these findings, the present inventors have provided the present invention descried below, as a means for solving the above-mentioned problems.

[1] An organic light-emitting device containing a host material, a delayed fluorescent material and a light-emitting material satisfying the following expression (1):

$$\text{Expression (1)} \quad E_{S1}(H) > E_{S1}(F) > E_{S1}(D)$$

wherein $E_{S1}(H)$ represents a lowest excited singlet energy level of the host material, $E_{S1}(F)$ represents a lowest excited singlet energy level of the delayed fluorescent material, and $E_{S1}(D)$ represents a lowest excited singlet energy level of the light-emitting material,

wherein the organic light-emitting device is an organic semiconductor laser, which is characterized in that

the delayed fluorescent material has an energy difference $\Delta E_{st}$ between a lowest excited singlet state and a lowest excited triplet state at 77 K of 0.3 eV or less,

energy from an excited singlet state of the host material transfers to the delayed fluorescent material and the light-emitting material,

a triplet excited state of the delayed fluorescent material transfers to a singlet excited state of the delayed fluorescent material through reverse intersystem crossing, and

the excited singlet energy of the delayed fluorescent material transfers to the light-emitting material.

[2] The organic light-emitting device according to [1], wherein the delayed fluorescent material has an energy dif-

ference $\Delta E_{st}$ between a lowest excited singlet state and a lowest excited triplet state at 77 K of 0.08 eV or less.

[3] The organic light-emitting device according to any one of [1] or [2], wherein the delayed fluorescent material has a rate constant $k_{RISC}$ from a lowest excited triplet state to a lowest excited singlet state of $10^5$/s or more.

[4] The organic light-emitting device according to any one of [1] to [3], wherein the light-emitting material radiates fluorescence when returning from a lowest excited singlet energy level to a ground energy level.

[5] The organic light-emitting device according to any one of [1] to [4], wherein the light-emitting material radiates amplified spontaneous emission.

[6] The organic light-emitting device according to any one of [1] to [5], wherein the content of the delayed fluorescent material is smaller than the content of the host material.

[7] The organic light-emitting device according to any one of [1] to [6], containing two or more kinds of compounds as the light-emitting material.

[8] The organic light-emitting device according to any one of [1] to [7], having a light-emitting layer that contains the host material, the delayed fluorescent material and the light-emitting material satisfying the expression (1).

[9] The organic light-emitting device according to [8], wherein the light-emitting layer has a multilayer configuration of plural layers.

[10] The organic light-emitting device according to [9], wherein the plural layers constituting the light-emitting layer each have a different content of the delayed fluorescent material therein.

[11] The organic light-emitting device according to any one of [1] to [10], which is a photoexcitation-type organic semiconductor laser.

[12] The organic light-emitting device according to [1], which is a carrier injection-type organic semiconductor laser.

Advantageous Effects of Invention

The organic light-emitting device of the present invention contains a host material, a delayed fluorescent material and a light-emitting material and is so designed that the relationship of the lowest excited singlet energy level between the materials is specifically defined, and therefore has an extremely high light emission efficiency. In particular, in the case where the light emitting material is an organic laser dye that radiates ASE, the threshold energy or the threshold current density necessary for ASE radiation can be reduced, and therefore an organic semiconductor laser excellent in ASE characteristics can be realized.

Brief Description of Drawings

[Fig. 1] This is a schematic view showing an estimated energy transfer mechanism in the organic light-emitting device of the present invention.

[Fig. 2] This is a schematic cross-sectional view showing an example of a layer configuration of a carrier injection-type organic light-emitting device.

[Fig. 3] This is a view showing a time-dependent light intensity change observed with a streak camera in the photoexcitation-type organic light-emitting device produced in Example 1.

[Fig. 4] This is a view showing a time-dependent light intensity change observed with a streak camera in the photoexcitation-type organic light-emitting device produced in Comparative Example 1.

[Fig. 5] This is the light emission spectra of the photoexcitation-type organic light-emitting device produced in Example 1.

[Fig. 6] This is a graph showing the relationship between the excitation energy and the half-value width of the emission peak FWHM of the photoexcitation-type organic light-emitting device produced in Example 1 and Comparative Example 1.

[Fig. 7] This is a graph showing the relationship between the excitation energy and the emission peak intensity of the photoexcitation-type organic light-emitting device of Example 1.

[Fig. 8] This is a graph showing the relationship between the excitation energy and the emission peak intensity of the photoexcitation-type organic light-emitting device of Comparative Example 1.

[Fig. 9] This is an emission spectrum of the photoexcitation-type organic light-emitting device of Comparative Example 2.

[Fig. 10] This is a graph showing the relationship between the excitation energy and the emission peak intensity of the photoexcitation-type organic light-emitting device of Comparative Example 2.

[Fig. 11] This is an emission spectrum of the carrier injection-type organic light-emitting device of Example 2 and Comparative Example 3.

[Fig. 12] This is a graph showing a voltage-current density characteristic of the carrier injection-type organic light-emitting device of Example 2 and Comparative Example 3.

[Fig. 13] This is a graph showing a current density-external quantum efficiency characteristic of the carrier injection-type organic light-emitting device of Example 1 and Comparative Example 3.

Description of Embodiments

The contents of the present invention are described in detail hereinunder. The constitutional elements may be described below with reference to representative embodiments and specific examples of the invention, but the invention is not limited to the embodiments and the examples. In the description, a numerical range expressed with reference to the expressions, an upper limit or less and/or a lower limit or more, means a range that includes the upper limit and/or the lower limit. In the invention, the hydrogen atom that is present in a molecule of the compound used in the invention is not particularly limited in isotope species, and for example, all the hydrogen atoms in the molecule may be $^1H$, and all or a part of them may be $^2H$ (deuterium (D)).

<Organic Light-Emitting Device>

The organic light-emitting device of the present invention contains a host material, a delayed fluorescent material and a light-emitting material satisfying the following expression (1):

$$\text{Expression (1)} \quad E_{S1}(H) > E_{S1}(F) > E_{S1}(D)$$

In the above expression, $E_{S1}(H)$ represents a lowest excited singlet energy level of the host material, $E_{S1}(F)$ represents a lowest excited singlet energy level of the delayed fluorescent material, and $E_{S1}(D)$ represents a lowest excited singlet energy level of the light-emitting material.

The "delayed fluorescent material" in the present invention means an organic compound that is capable of being transferred to the triplet excited state and then undergoing reverse intersystem crossing to the singlet excited state, and emits fluorescent light on returning from the singlet excited state to the ground state. The light formed through the reverse intersystem crossing from the triplet excited state to the singlet excited state has a lifetime that is longer than normal fluorescent light (prompt fluorescent light) and phosphorescent light, and thus is observed as fluorescent light that is delayed therefrom. Accordingly, the fluorescent light of this type is referred to as "delayed fluorescent light".

The organic light-emitting device of the type contains a host material, a delayed fluorescent material and a light-emitting material, and the lowest excited singlet energy levels $E_{S1}(H)$, $E_{S1}(F)$ and $E_{S1}(D)$ of the constituent materials satisfy the above-mentioned expression (1), and accordingly in the device, the energy given from the outside can be efficiently converted into light therefore realizing a high light emission efficiency. This may be considered because of the following reasons.

Fig. 1 shows an estimated energy transfer mechanism of the organic light-emitting device of the present invention. Fig. 1 is to schematically show the estimated energy transfer mechanism, and the lowest excited singlet energy level $E_{S1}$ and the exciton transfer pathway of each material are not limited thereto.

As shown in Fig. 1, when the organic light-emitting device is, for example, irradiated with exciting light, mainly the host material absorbs energy and changes from the ground state to an excited singlet state. Here, in this organic light-emitting device, the lowest excited singlet energy levels of the materials $E_{S1}(H)$, $E_{S1}(F)$, $E_{S1}(D)$ satisfy the above-mentioned expression (1), and therefore the excited singlet energy of the host material transfers to the delayed fluorescent material and the light-emitting material via a Forster mechanism (FRET) or the like, and further the excited singlet energy of the delayed fluorescent material transfers to the light-emitting material. The light-emitting material thus having transferred to an excited singlet state after having received the energy is thereafter returns back to the ground state while radiating fluorescent light.

At this time, in the organic light-emitting device, an excited triplet state occurs through intersystem crossing from the excited singlet state to an excited triplet state, but since the device contains a delayed fluorescent material, the triplet excited state transfers to a singlet excited state through reverse intersystem crossing in this delayed fluorescent material, and the excited singlet state energy generated through the reverse intersystem crossing also transfers to the light-emitting material. Accordingly, the excited triplet energy may indirectly contribute toward light emission, and as compared with a constitution not containing a delayed fluorescent material, the device of the type can drastically improve the light emission efficiency thereof.

In the case where carriers are injected into an organic light-emitting device for light emission, singlet excitons and triplet excitons are formed in a ratio of 1/3 through the carrier injection, and also in this case, the energy of the triplet excitons indirectly contributes toward light emission via reverse intersystem crossing with the delayed fluorescent material. Consequently, the energy of the triplet excitons to be formed in a ratio of 75% can be efficiently utilized for light

emission, and as compared with a constitution not containing a delayed fluorescent material, the device of the type can realize a drastically high light emission efficiency.

In the organic light-emitting device of the present invention, light emission occurs mainly from the light-emitting material, but a part of light emission therein may also be from the host material and the delayed fluorescent material. In addition, the light emission includes fluorescence emission, delayed fluorescence emission and amplified spontaneous emission (ASE).

The organic light-emitting device of the present invention is not specifically limited in point of the type and the combination of the host material, the delayed fluorescent material and the light-emitting material therein, so far as the materials satisfy the above-mentioned expression (1). In the following, the present invention is described further concretely with reference to preferred examples thereof, but the scope of the present invention should not be interpreted in a limited way by the description based on the following examples.

[Materials of Organic Light-Emitting Device]

(Delayed Fluorescent Material)

The delayed fluorescent material is not specifically limited but is preferably a thermal activation type delayed fluorescent material that undergoes reverse intersystem crossing from an excited singlet state to an excited triplet state through absorption of heat energy. The thermal activation type delayed fluorescent material relatively easily undergoes reverse intersystem crossing from an excited triplet state to an excited singlet state through absorption of heat that is formed by the device, and can make the excited triplet energy thereof contribute to light emission efficiently.

The delayed fluorescent material has an energy difference $\Delta E_{st}$ between the energy in the lowest excited singlet state and the energy in the lowest excited triplet state at 77K of 0.3 eV or less, preferably 0.2 eV or less, more preferably 0.1 eV or less, and further preferably 0.08 eV or less. The delayed fluorescent material that has an energy difference $\Delta E_{st}$ within the range relatively easily undergoes reverse intersystem crossing from the excited triplet state to the excited singlet state, and can make the excited triplet energy thereof contribute to light emission efficiently.

Preferably, the delayed fluorescent material has a rate constant $k_{RISC}$ from a lowest excited triplet state to a lowest excited singlet state of $10^3$/s or more, more preferably $10^4$/s or more, even more preferably $10^5$/s or more. The delayed fluorescent material whose rate constant $k_{RISC}$ falls within the range relatively easily undergoes reverse intersystem crossing from the excited triplet state to the excited singlet state, and can make the excited triplet energy thereof contribute to light emission efficiently.

The delayed fluorescent material is not specifically limited so far as it can radiate delayed fluorescence.

As a preferred delayed fluorescent material, a compound represented by the following general formula can be mentioned.

[Chem. 1]

General Formula (101)

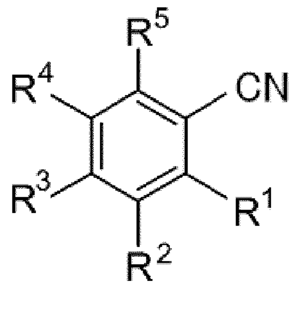

In the general formula (101), at least one of $R^1$ to $R^5$ represents a cyano group, at least one of $R^1$ to $R^5$ represents a group represented by the following general formula (111), and the balance of $R^1$ to $R^5$ each represent a hydrogen atom or a substituent.

[Chem. 2]

General Formula (111)

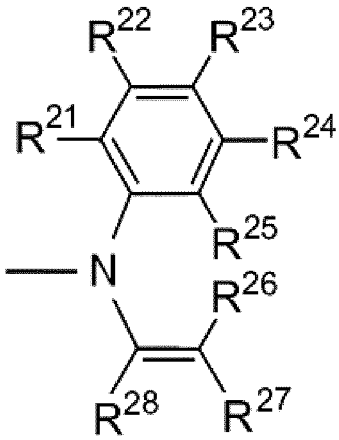

In the general formula (111), $R^{21}$ to $R^{28}$ each independently represent a hydrogen atom or a substituent, provided that at least one of the following conditions <A> and <B> is satisfied:

<A> $R^{25}$ and $R^{26}$ together form a single bond, and
<B> $R^{27}$ and $R^{28}$ each represent an atomic group necessary for forming a substituted or unsubstituted benzene ring as combined together.

Here, at least one of $R^1$ to $R^5$ preferably represents a group represented by any one of the following general formulae (112) to (115).

[Chem. 3]

General Formula (112)

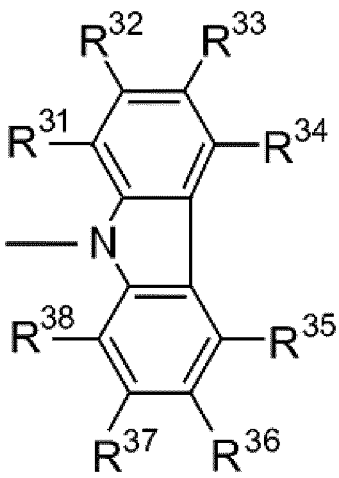

In the general formula (112), $R^{31}$ to $R^{38}$ each independently represent a hydrogen atom or a substituent.

[Chem. 4]

General Formula (113)

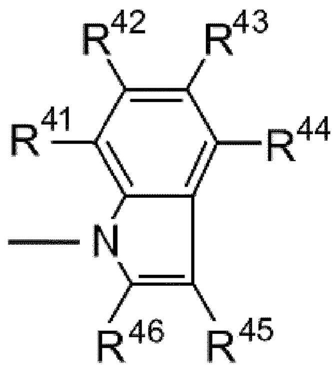

In the general formula (113), $R^{41}$ to $R^{46}$ each independently represent a hydrogen atom or a substituent.

[Chem. 5]

General Formula (114)

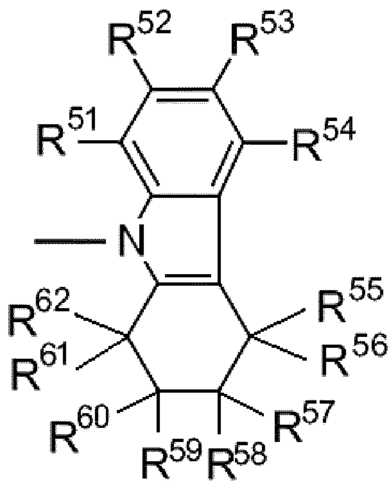

In the general formula (114), $R^{51}$ to $R^{62}$ each independently represent a hydrogen atom or a substituent.

[Chem. 6]

General Formula (115)

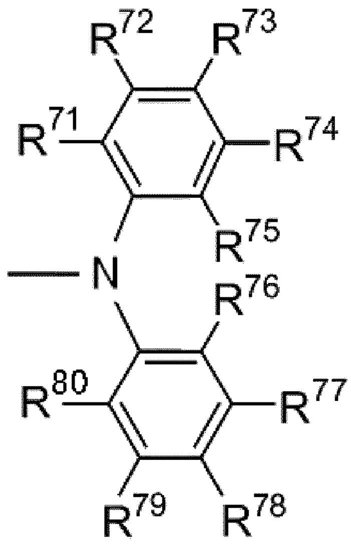

In the general formula (115), $R^{71}$ to $R^{80}$ each independently represent a hydrogen atom or a substituent.

Specific examples of the compounds include the compounds shown in the following tables. In the case where two or more groups represented by any one of the general formulae (112) to (115) are present in the molecule of the following example compounds, all the groups have the same structure. The formulae (121) to (124) in the tables represent the following formulae, respectively, and n represents the number of the repeating units.

[Chem. 7]

Formula (121)

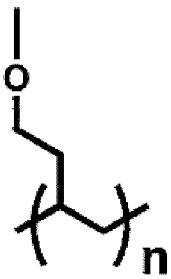

Formula (122)

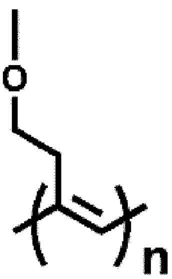

Formula (123)

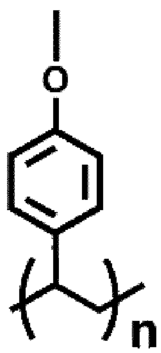

Formula (124)

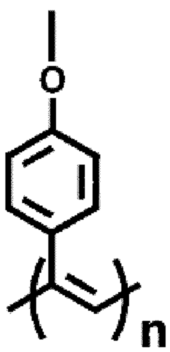

Table 1

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 1 | General formula (112) | General formula (112) | CN | General formula (112) | General formula (112) | H | H | H | H |
| 2 | General formula (112) | General formula (112) | CN | General formula (112) | General formula (112) | H | $CH_3$ | H | H |
| 3 | General formula (112) | General formula (112) | CN | General formula (112) | General formula (112) | H | $CH_3O$ | H | H |
| 4 | General formula (112) | General formula (112) | CN | General formula (112) | General formula (112) | H | H | $CH_3$ | H |
| 5 | General formula (112) | General formula (112) | CN | General formula (112) | General formula (112) | H | H | $CH_3O$ | H |
| 6 | General formula (112) | General formula (112) | CN | General formula (112) | General formula (112) | H | H | $t\text{-}C_4H_9$ | H |
| 7 | General formula (112) | General formula (112) | CN | General formula (112) | General formula (112) | H | H | Cl | H |
| 8 | General formula (112) | General formula (112) | CN | General formula (112) | General formula (112) | H | H | F | H |
| 9 | General formula (112) | General formula (112) | CN | General formula (112) | General formula (112) | H | H | H | $CH_3$ |
| 10 | General formula (112) | General formula (112) | CN | General formula (112) | General formula (112) | H | H | H | $CH_3O$ |
| 11 | General formula (112) | General formula (112) | CN | General formula (112) | H | H | H | H | H |
| 12 | General formula (112) | General formula (112) | CN | General formula (112) | H | H | $CH_3$ | H | H |
| 13 | General formula (112) | General formula (112) | CN | General formula (112) | H | H | $CH_3O$ | H | H |
| 14 | General formula (112) | General formula (112) | CN | General formula (112) | H | H | H | $CH_3$ | H |
| 15 | General formula (112) | General formula (112) | CN | General formula (112) | H | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 16 | General formula (112) | General formula (112) | CN | General formula (112) | H | H | H | t-$C_4H_9$ | H |
| 17 | General formula (112) | General formula (112) | CN | General formula (112) | H | H | H | Cl | H |
| 18 | General formula (112) | General formula (112) | CN | General formula (112) | H | H | H | F | H |
| 19 | General formula (112) | General formula (112) | CN | General formula (112) | H | H | H | H | $CH_3$ |
| 20 | General formula (112) | General formula (112) | CN | General formula (112) | H | H | H | H | $CH_3O$ |
| 21 | General formula (112) | General formula (112) | CN | H | H | H | H | H | H |
| 22 | General formula (112) | General formula (112) | CN | H | H | H | $CH_3$ | H | H |
| 23 | General formula (112) | General formula (112) | CN | H | H | H | $CH_3O$ | H | H |
| 24 | General formula (112) | General formula (112) | CN | H | H | H | H | $CH_3$ | H |
| 25 | General formula (112) | General formula (112) | CN | H | H | H | H | $CH_3O$ | H |
| 26 | General formula (112) | General formula (112) | CN | H | H | H | H | t-$C_4H_9$ | H |
| 27 | General formula (112) | General formula (112) | CN | H | H | H | H | Cl | H |
| 28 | General formula (112) | General formula (112) | CN | H | H | H | H | F | H |
| 29 | General formula (112) | General formula (112) | CN | H | H | H | H | H | $CH_3$ |
| 30 | General formula (112) | General formula (112) | CN | H | H | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 31 | General formula (112) | H | CN | General formula (112) | H | H | H | H | H |
| 32 | General formula (112) | H | CN | General formula (112) | H | H | $CH_3$ | H | H |
| 33 | General formula (112) | H | CN | General formula (112) | H | H | $CH_3O$ | H | H |
| 34 | General formula (112) | H | CN | General formula (112) | H | H | H | $CH_3$ | H |
| 35 | General formula (112) | H | CN | General formula (112) | H | H | H | $CH_3O$ | H |
| 36 | General formula (112) | H | CN | General formula (112) | H | H | H | t-$C_4H_9$ | H |
| 37 | General formula (112) | H | CN | General formula (112) | H | H | H | Cl | H |
| 38 | General formula (112) | H | CN | General formula (112) | H | H | H | F | H |
| 39 | General formula (112) | H | CN | General formula (112) | H | H | H | H | $CH_3$ |
| 40 | General formula (112) | H | CN | General formula (112) | H | H | H | H | $CH_3O$ |
| 41 | General formula (112) | H | CN | H | General formula (112) | H | H | H | H |
| 42 | General formula (112) | H | CN | H | General formula (112) | H | $CH_3$ | H | H |
| 43 | General formula (112) | H | CN | H | General formula (112) | H | $CH_3O$ | H | H |
| 44 | General formula (112) | H | CN | H | General formula (112) | H | H | $CH_3$ | H |
| 45 | General formula (112) | H | CN | H | General formula (112) | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 46 | General formula (112) | H | CN | H | General formula (112) | H | H | t-$C_4H_9$ | H |
| 47 | General formula (112) | H | CN | H | General formula (112) | H | H | Cl | H |
| 48 | General formula (112) | H | CN | H | General formula (112) | H | H | F | H |
| 49 | General formula (112) | H | CN | H | General formula (112) | H | H | H | $CH_3$ |
| 50 | General formula (112) | H | CN | H | General formula (112) | H | H | H | $CH_3O$ |
| 51 | General formula (112) | H | CN | H | H | H | H | H | H |
| 52 | General formula (112) | H | CN | H | H | H | $CH_3$ | H | H |
| 53 | General formula (112) | H | CN | H | H | H | $CH_3O$ | H | H |
| 54 | General formula (112) | H | CN | H | H | H | H | $CH_3$ | H |
| 55 | General formula (112) | H | CN | H | H | H | H | $CH_3O$ | H |
| 56 | General formula (112) | H | CN | H | H | H | H | t-$C_4H_9$ | H |
| 57 | General formula (112) | H | CN | H | H | H | H | Cl | H |
| 58 | General formula (112) | H | CN | H | H | H | H | F | H |
| 59 | General formula (112) | H | CN | H | H | H | H | H | $CH_3$ |
| 60 | General formula (112) | H | CN | H | H | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 61 | General formula (112) | General formula (112) | CN | General formula (112) | F | H | H | H | H |
| 62 | General formula (112) | General formula (112) | CN | General formula (112) | F | H | $CH_3$ | H | H |
| 63 | General formula (112) | General formula (112) | CN | General formula (112) | F | H | $CH_3O$ | H | H |
| 64 | General formula (112) | General formula (112) | CN | General formula (112) | F | H | H | $CH_3$ | H |
| 65 | General formula (112) | General formula (112) | CN | General formula (112) | F | H | H | $CH_3O$ | H |
| 66 | General formula (112) | General formula (112) | CN | General formula (112) | F | H | H | t-$C_4H_9$ | H |
| 67 | General formula (112) | General formula (112) | CN | General formula (112) | F | H | H | Cl | H |
| 68 | General formula (112) | General formula (112) | CN | General formula (112) | F | H | H | F | H |
| 69 | General formula (112) | General formula (112) | CN | General formula (112) | F | H | H | H | $CH_3$ |
| 70 | General formula (112) | General formula (112) | CN | General formula (112) | F | H | H | H | $CH_3O$ |
| 71 | General formula (112) | General formula (112) | CN | F | F | H | H | H | H |
| 72 | General formula (112) | General formula (112) | CN | F | F | H | $CH_3$ | H | H |
| 73 | General formula (112) | General formula (112) | CN | F | F | H | $CH_3O$ | H | H |
| 74 | General formula (112) | General formula (112) | CN | F | F | H | H | $CH_3$ | H |
| 75 | General formula (112) | General formula (112) | CN | F | F | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 76 | General formula (112) | General formula (112) | CN | F | F | H | H | t-$C_4H_9$ | H |
| 77 | General formula (112) | General formula (112) | CN | F | F | H | H | Cl | H |
| 78 | General formula (112) | General formula (112) | CN | F | F | H | H | F | H |
| 79 | General formula (112) | General formula (112) | CN | F | F | H | H | H | $CH_3$ |
| 80 | General formula (112) | General formula (112) | CN | F | F | H | H | H | $CH_3O$ |
| 81 | General formula (112) | F | CN | General formula (112) | F | H | H | H | H |
| 82 | General formula (112) | F | CN | General formula (112) | F | H | $CH_3$ | H | H |
| 83 | General formula (112) | F | CN | General formula (112) | F | H | $CH_3O$ | H | H |
| 84 | General formula (112) | F | CN | General formula (112) | F | H | H | $CH_3$ | H |
| 85 | General formula (112) | F | CN | General formula (112) | F | H | H | $CH_3O$ | H |
| 86 | General formula (112) | F | CN | General formula (112) | F | H | H | t-$C_4H_9$ | H |
| 87 | General formula (112) | F | CN | General formula (112) | F | H | H | Cl | H |
| 88 | General formula (112) | F | CN | General formula (112) | F | H | H | F | H |
| 89 | General formula (112) | F | CN | General formula (112) | F | H | H | H | $CH_3$ |
| 90 | General formula (112) | F | CN | General formula (112) | F | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 91 | General formula (112) | F | CN | F | General formula (112) | H | H | H | H |
| 92 | General formula (112) | F | CN | F | General formula (112) | H | $CH_3$ | H | H |
| 93 | General formula (112) | F | CN | F | General formula (112) | H | $CH_3O$ | H | H |
| 94 | General formula (112) | F | CN | F | General formula (112) | H | H | $CH_3$ | H |
| 95 | General formula (112) | F | CN | F | General formula (112) | H | H | $CH_3O$ | H |
| 96 | General formula (112) | F | CN | F | General formula (112) | H | H | t-$C_4H_9$ | H |
| 97 | General formula (112) | F | CN | F | General formula (112) | H | H | Cl | H |
| 98 | General formula (112) | F | CN | F | General formula (112) | H | H | F | H |
| 99 | General formula (112) | F | CN | F | General formula (112) | H | H | H | $CH_3$ |
| 100 | General formula (112) | F | CN | F | General formula (112) | H | H | H | $CH_3O$ |
| 101 | General formula (112) | F | CN | F | F | H | H | H | H |
| 102 | General formula (112) | F | CN | F | F | H | $CH_3$ | H | H |
| 103 | General formula (112) | F | CN | F | F | H | $CH_3O$ | H | H |
| 104 | General formula (112) | F | CN | F | F | H | H | $CH_3$ | H |
| 105 | General formula (112) | F | CN | F | F | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 106 | General formula (112) | F | CN | F | F | H | H | t-$C_4H_9$ | H |
| 107 | General formula (112) | F | CN | F | F | H | H | Cl | H |
| 108 | General formula (112) | F | CN | F | F | H | H | F | H |
| 109 | General formula (112) | F | CN | F | F | H | H | H | $CH_3$ |
| 110 | General formula (112) | F | CN | F | F | H | H | H | $CH_3O$ |
| 111 | General formula (112) | General formula (112) | CN | General formula (112) | OH | H | H | H | H |
| 112 | General formula (112) | General formula (112) | CN | General formula (112) | OH | H | $CH_3$ | H | H |
| 113 | General formula (112) | General formula (112) | CN | General formula (112) | OH | H | $CH_3O$ | H | H |
| 114 | General formula (112) | General formula (112) | CN | General formula (112) | OH | H | H | $CH_3$ | H |
| 115 | General formula (112) | General formula (112) | CN | General formula (112) | OH | H | H | $CH_3O$ | H |
| 116 | General formula (112) | General formula (112) | CN | General formula (112) | OH | H | H | t-$C_4H_9$ | H |
| 117 | General formula (112) | General formula (112) | CN | General formula (112) | OH | H | H | Cl | H |
| 118 | General formula (112) | General formula (112) | CN | General formula (112) | OH | H | H | F | H |
| 119 | General formula (112) | General formula (112) | CN | General formula (112) | OH | H | H | H | $CH_3$ |
| 120 | General formula (112) | General formula (112) | CN | General formula (112) | OH | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 121 | General formula (112) | General formula (112) | CN | OH | OH | H | H | H | H |
| 122 | General formula (112) | General formula (112) | CN | OH | OH | H | $CH_3$ | H | H |
| 123 | General formula (112) | General formula (112) | CN | OH | OH | H | $CH_3O$ | H | H |
| 124 | General formula (112) | General formula (112) | CN | OH | OH | H | H | $CH_3$ | H |
| 125 | General formula (112) | General formula (112) | CN | OH | OH | H | H | $CH_3O$ | H |
| 126 | General formula (112) | General formula (112) | CN | OH | OH | H | H | t-$C_4H_9$ | H |
| 127 | General formula (112) | General formula (112) | CN | OH | OH | H | H | Cl | H |
| 128 | General formula (112) | General formula (112) | CN | OH | OH | H | H | F | H |
| 129 | General formula (112) | General formula (112) | CN | OH | OH | H | H | H | $CH_3$ |
| 130 | General formula (112) | General formula (112) | CN | OH | OH | H | H | H | $CH_3O$ |
| 131 | General formula (112) | OH | CN | General formula (112) | OH | H | H | H | H |
| 132 | General formula (112) | OH | CN | General formula (112) | OH | H | $CH_3$ | H | H |
| 133 | General formula (112) | OH | CN | General formula (112) | OH | H | $CH_3O$ | H | H |
| 134 | General formula (112) | OH | CN | General formula (112) | OH | H | H | $CH_3$ | H |
| 135 | General formula (112) | OH | CN | General formula (112) | OH | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 136 | General formula (112) | OH | CN | General formula (112) | OH | H | H | t-$C_4H_9$ | H |
| 137 | General formula (112) | OH | CN | General formula (112) | OH | H | H | Cl | H |
| 138 | General formula (112) | OH | CN | General formula (112) | OH | H | H | F | H |
| 139 | General formula (112) | OH | CN | General formula (112) | OH | H | H | H | $CH_3$ |
| 140 | General formula (112) | OH | CN | General formula (112) | OH | H | H | H | $CH_3O$ |
| 141 | General formula (112) | OH | CN | OH | General formula (112) | H | H | H | H |
| 142 | General formula (112) | OH | CN | OH | General formula (112) | H | $CH_3$ | H | H |
| 143 | General formula (112) | OH | CN | OH | General formula (112) | H | $CH_3O$ | H | H |
| 144 | General formula (112) | OH | CN | OH | General formula (112) | H | H | $CH_3$ | H |
| 145 | General formula (112) | OH | CN | OH | General formula (112) | H | H | $CH_3O$ | H |
| 146 | General formula (112) | OH | CN | OH | General formula (112) | H | H | t-$C_4H_9$ | H |
| 147 | General formula (112) | OH | CN | OH | General formula (112) | H | H | Cl | H |
| 148 | General formula (112) | OH | CN | OH | General formula (112) | H | H | F | H |
| 149 | General formula (112) | OH | CN | OH | General formula (112) | H | H | H | $CH_3$ |
| 150 | General formula (112) | OH | CN | OH | General formula (112) | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 151 | General formula (112) | OH | CN | OH | OH | H | H | H | H |
| 152 | General formula (112) | OH | CN | OH | OH | H | $CH_3$ | H | H |
| 153 | General formula (112) | OH | CN | OH | OH | H | $CH_3O$ | H | H |
| 154 | General formula (112) | OH | CN | OH | OH | H | H | $CH_3$ | H |
| 155 | General formula (112) | OH | CN | OH | OH | H | H | $CH_3O$ | H |
| 156 | General formula (112) | OH | CN | OH | OH | H | H | t-$C_4H_9$ | H |
| 157 | General formula (112) | OH | CN | OH | OH | H | H | Cl | H |
| 158 | General formula (112) | OH | CN | OH | OH | H | H | F | H |
| 159 | General formula (112) | OH | CN | OH | OH | H | H | H | $CH_3$ |
| 160 | General formula (112) | OH | CN | OH | OH | H | H | H | $CH_3O$ |
| 161 | General formula (112) | General formula (112) | CN | General formula (112) | Cl | H | H | H | H |
| 162 | General formula (112) | General formula (112) | CN | General formula (112) | Cl | H | $CH_3$ | H | H |
| 163 | General formula (112) | General formula (112) | CN | General formula (112) | Cl | H | $CH_3O$ | H | H |
| 164 | General formula (112) | General formula (112) | CN | General formula (112) | Cl | H | H | $CH_3$ | H |
| 165 | General formula (112) | General formula (112) | CN | General formula (112) | Cl | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 166 | General formula (112) | General formula (112) | CN | General formula (112) | Cl | H | H | t-$C_4H_9$ | H |
| 167 | General formula (112) | General formula (112) | CN | General formula (112) | Cl | H | H | Cl | H |
| 168 | General formula (112) | General formula (112) | CN | General formula (112) | Cl | H | H | F | H |
| 169 | General formula (112) | General formula (112) | CN | General formula (112) | Cl | H | H | H | $CH_3$ |
| 170 | General formula (112) | General formula (112) | CN | General formula (112) | Cl | H | H | H | $CH_3O$ |
| 171 | General formula (112) | General formula (112) | CN | General formula (112) | F | H | H | H | H |
| 172 | General formula (112) | General formula (112) | CN | General formula (112) | F | H | $CH_3$ | H | H |
| 173 | General formula (112) | General formula (112) | CN | General formula (112) | F | H | $CH_3O$ | H | H |
| 174 | General formula (112) | General formula (112) | CN | General formula (112) | F | H | H | $CH_3$ | H |
| 175 | General formula (112) | General formula (112) | CN | General formula (112) | F | H | H | $CH_3O$ | H |
| 176 | General formula (112) | General formula (112) | CN | General formula (112) | F | H | H | t-$C_4H_9$ | H |
| 177 | General formula (112) | General formula (112) | CN | General formula (112) | F | H | H | Cl | H |
| 178 | General formula (112) | General formula (112) | CN | General formula (112) | F | H | H | F | H |
| 179 | General formula (112) | General formula (112) | CN | General formula (112) | F | H | H | H | $CH_3$ |
| 180 | General formula (112) | General formula (112) | CN | General formula (112) | F | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 181 | General formula (112) | General formula (112) | CN | General formula (112) | $CH_3O$ | H | H | H | H |
| 182 | General formula (112) | General formula (112) | CN | General formula (112) | $CH_3O$ | H | $CH_3$ | H | H |
| 183 | General formula (112) | General formula (112) | CN | General formula (112) | $CH_3O$ | H | $CH_3O$ | H | H |
| 184 | General formula (112) | General formula (112) | CN | General formula (112) | $CH_3O$ | H | H | $CH_3$ | H |
| 185 | General formula (112) | General formula (112) | CN | General formula (112) | $CH_3O$ | H | H | $CH_3O$ | H |
| 186 | General formula (112) | General formula (112) | CN | General formula (112) | $CH_3O$ | H | H | t-$C_4H_9$ | H |
| 187 | General formula (112) | General formula (112) | CN | General formula (112) | $CH_3O$ | H | H | Cl | H |
| 188 | General formula (112) | General formula (112) | CN | General formula (112) | $CH_3O$ | H | H | F | H |
| 189 | General formula (112) | General formula (112) | CN | General formula (112) | $C_2H_5O$ | H | H | H | $CH_3$ |
| 190 | General formula (112) | General formula (112) | CN | General formula (112) | $C_2H_5O$ | H | H | H | $CH_3O$ |
| 191 | General formula (112) | General formula (112) | CN | General formula (112) | $C_2H_5O$ | H | H | H | H |
| 192 | General formula (112) | General formula (112) | CN | General formula (112) | $C_2H_5O$ | H | $CH_3$ | H | H |
| 193 | General formula (112) | General formula (112) | CN | General formula (112) | $C_2H_5O$ | H | $CH_3O$ | H | H |
| 194 | General formula (112) | General formula (112) | CN | General formula (112) | $C_2H_5O$ | H | H | $CH_3$ | H |
| 195 | General formula (112) | General formula (112) | CN | General formula (112) | $C_2H_5O$ | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 196 | General formula (112) | General formula (112) | CN | General formula (112) | $C_2H_5O$ | H | H | t-$C_4H_9$ | H |
| 197 | General formula (112) | General formula (112) | CN | General formula (112) | $C_2H_5O$ | H | H | Cl | H |
| 198 | General formula (112) | General formula (112) | CN | General formula (112) | $C_2H_5O$ | H | H | F | H |
| 199 | General formula (112) | General formula (112) | CN | General formula (112) | $C_2H_5O$ | H | H | H | $CH_3$ |
| 200 | General formula (112) | General formula (112) | CN | General formula (112) | $C_2H_5O$ | H | H | H | $CH_3O$ |
| 201 | General formula (112) | General formula (112) | CN | General formula (112) | $C_6H_5O$ | H | H | H | H |
| 202 | General formula (112) | General formula (112) | CN | General formula (112) | $C_6H_5O$ | H | $CH_3$ | H | H |
| 203 | General formula (112) | General formula (112) | CN | General formula (112) | $C_6H_5O$ | H | $CH_3O$ | H | H |
| 204 | General formula (112) | General formula (112) | CN | General formula (112) | $C_6H_5O$ | H | H | $CH_3$ | H |
| 205 | General formula (112) | General formula (112) | CN | General formula (112) | $C_6H_5O$ | H | H | $CH_3O$ | H |
| 206 | General formula (112) | General formula (112) | CN | General formula (112) | $C_6H_5O$ | H | H | t-$C_4H_9$ | H |
| 207 | General formula (112) | General formula (112) | CN | General formula (112) | $C_6H_5O$ | H | H | Cl | H |
| 208 | General formula (112) | General formula (112) | CN | General formula (112) | $C_6H_5O$ | H | H | F | H |
| 209 | General formula (112) | General formula (112) | CN | General formula (112) | $C_6H_5O$ | H | H | H | $CH_3$ |
| 210 | General formula (112) | General formula (112) | CN | General formula (112) | $C_6H_5O$ | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 211 | General formula (112) | General formula (112) | CN | General formula (112) | Formula (121) | H | H | H | H |
| 212 | General formula (112) | General formula (112) | CN | General formula (112) | Formula (121) | H | $CH_3$ | H | H |
| 213 | General formula (112) | General formula (112) | CN | General formula (112) | Formula (121) | H | $CH_3O$ | H | H |
| 214 | General formula (112) | General formula (112) | CN | General formula (112) | Formula (121) | H | H | $CH_3$ | H |
| 215 | General formula (112) | General formula (112) | CN | General formula (112) | Formula (121) | H | H | $CH_3O$ | H |
| 216 | General formula (112) | General formula (112) | CN | General formula (112) | Formula (121) | H | H | t-$C_4H_9$ | H |
| 217 | General formula (112) | General formula (112) | CN | General formula (112) | Formula (121) | H | H | Cl | H |
| 218 | General formula (112) | General formula (112) | CN | General formula (112) | Formula (121) | H | H | F | H |
| 219 | General formula (112) | General formula (112) | CN | General formula (112) | Formula (121) | H | H | H | $CH_3$ |
| 220 | General formula (112) | General formula (112) | CN | General formula (112) | Formula (121) | H | H | H | $CH_3O$ |
| 221 | General formula (112) | General formula (112) | CN | General formula (112) | Formula (122) | H | H | H | H |
| 222 | General formula (112) | General formula (112) | CN | General formula (112) | Formula (122) | H | $CH_3$ | H | H |
| 223 | General formula (112) | General formula (112) | CN | General formula (112) | Formula (122) | H | $CH_3O$ | H | H |
| 224 | General formula (112) | General formula (112) | CN | General formula (112) | Formula (122) | H | H | $CH_3$ | H |
| 225 | General formula (112) | General formula (112) | CN | General formula (112) | Formula (122) | H | H | $CH_3O$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 226 | General formula (112) | General formula (112) | CN | General formula (112) | Formula (122) | H | H | t-$C_4H_9$ | H |
| 227 | General formula (112) | General formula (112) | CN | General formula (112) | Formula (122) | H | H | Cl | H |
| 228 | General formula (112) | General formula (112) | CN | General formula (112) | Formula (122) | H | H | F | H |
| 229 | General formula (112) | General formula (112) | CN | General formula (112) | Formula (122) | H | H | H | $CH_3$ |
| 230 | General formula (112) | General formula (112) | CN | General formula (112) | Formula (122) | H | H | H | $CH_3O$ |
| 231 | General formula (112) | General formula (112) | CN | General formula (112) | Formula (123) | H | H | H | H |
| 232 | General formula (112) | General formula (112) | CN | General formula (112) | Formula (123) | H | $CH_3$ | H | H |
| 233 | General formula (112) | General formula (112) | CN | General formula (112) | Formula (123) | H | $CH_3O$ | H | H |
| 234 | General formula (112) | General formula (112) | CN | General formula (112) | Formula (123) | H | H | $CH_3$ | H |
| 235 | General formula (112) | General formula (112) | CN | General formula (112) | Formula (123) | H | H | $CH_3O$ | H |
| 236 | General formula (112) | General formula (112) | CN | General formula (112) | Formula (123) | H | H | t-$C_4H_9$ | H |
| 237 | General formula (112) | General formula (112) | CN | General formula (112) | Formula (123) | H | H | Cl | H |
| 238 | General formula (112) | General formula (112) | CN | General formula (112) | Formula (123) | H | H | F | H |
| 239 | General formula (112) | General formula (112) | CN | General formula (112) | Formula (123) | H | H | H | $CH_3$ |
| 240 | General formula (112) | General formula (112) | CN | General formula (112) | Formula (123) | H | H | H | $CH_3O$ |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 241 | General formula (112) | General formula (112) | CN | General formula (112) | Formula (124) | H | H | H | H |
| 242 | General formula (112) | General formula (112) | CN | General formula (112) | Formula (124) | H | $CH_3$ | H | H |
| 243 | General formula (112) | General formula (112) | CN | General formula (112) | Formula (124) | H | $CH_3O$ | H | H |
| 244 | General formula (112) | General formula (112) | CN | General formula (112) | Formula (124) | H | H | $CH_3$ | H |
| 245 | General formula (112) | General formula (112) | CN | General formula (112) | Formula (124) | H | H | $CH_3O$ | H |
| 246 | General formula (112) | General formula (112) | CN | General formula (112) | Formula (124) | H | H | t-$C_4H_9$ | H |
| 247 | General formula (112) | General formula (112) | CN | General formula (112) | Formula (124) | H | H | Cl | H |
| 248 | General formula (112) | General formula (112) | CN | General formula (112) | Formula (124) | H | H | F | H |
| 249 | General formula (112) | General formula (112) | CN | General formula (112) | Formula (124) | H | H | H | $CH_3$ |
| 250 | General formula (112) | General formula (112) | CN | General formula (112) | Formula (124) | H | H | H | $CH_3O$ |
| 251 | General formula (112) | General formula (112) | CN | General formula (112) | General formula (112) | H | $C_6H_5$ | H | H |
| 252 | General formula (112) | General formula (112) | CN | General formula (112) | General formula (112) | H | H | $C_6H_5$ | H |
| 253 | General formula (112) | General formula (112) | CN | General formula (112) | H | H | $C_6H_5$ | H | H |
| 254 | General formula (112) | General formula (112) | CN | General formula (112) | H | H | H | $C_6H_5$ | H |
| 255 | General formula (112) | General formula (112) | CN | H | H | H | $C_6H_5$ | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 256 | General formula (112) | General formula (112) | CN | H | H | H | H | $C_6H_5$ | H |
| 257 | General formula (112) | H | CN | General formula (112) | H | H | $C_6H_5$ | H | H |
| 258 | General formula (112) | H | CN | General formula (112) | H | H | H | $C_6H_5$ | H |
| 259 | General formula (112) | H | CN | H | General formula (112) | H | $C_6H_5$ | H | H |
| 260 | General formula (112) | H | CN | H | General formula (112) | H | H | $C_6H_5$ | H |
| 261 | General formula (112) | H | CN | H | H | H | $C_6H_5$ | H | H |
| 262 | General formula (112) | H | CN | H | H | H | H | $C_6H_5$ | H |
| 263 | General formula (112) | General formula (112) | CN | General formula (112) | F | H | $C_6H_5$ | H | H |
| 264 | General formula (112) | General formula (112) | CN | General formula (112) | F | H | H | $C_6H_5$ | H |
| 265 | General formula (112) | General formula (112) | CN | F | F | H | $C_6H_5$ | H | H |
| 266 | General formula (112) | General formula (112) | CN | F | F | H | H | $C_6H_5$ | H |
| 267 | General formula (112) | F | CN | General formula (112) | F | H | $C_6H_5$ | H | H |
| 268 | General formula (112) | F | CN | General formula (112) | F | H | H | $C_6H_5$ | H |
| 269 | General formula (112) | F | CN | F | General formula (112) | H | $C_6H_5$ | H | H |
| 270 | General formula (112) | F | CN | F | General formula (112) | H | H | $C_6H_5$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 271 | General formula (112) | F | CN | F | F | H | $C_6H_5$ | H | H |
| 272 | General formula (112) | F | CN | F | F | H | H | $C_6H_5$ | H |
| 273 | General formula (112) | General formula (112) | CN | General formula (112) | OH | H | $C_6H_5$ | H | H |
| 274 | General formula (112) | General formula (112) | CN | General formula (112) | OH | H | H | $C_6H_5$ | H |
| 275 | General formula (112) | General formula (112) | CN | OH | OH | H | $C_6H_5$ | H | H |
| 276 | General formula (112) | General formula (112) | CN | OH | OH | H | H | $C_6H_5$ | H |
| 277 | General formula (112) | OH | CN | General formula (112) | OH | H | $C_6H_5$ | H | H |
| 278 | General formula (112) | OH | CN | General formula (112) | OH | H | H | $C_6H_5$ | H |
| 279 | General formula (112) | OH | CN | OH | General formula (112) | H | $C_6H_5$ | H | H |
| 280 | General formula (112) | OH | CN | OH | General formula (112) | H | H | $C_6H_5$ | H |
| 281 | General formula (112) | OH | CN | OH | OH | H | $C_6H_5$ | H | H |
| 282 | General formula (112) | OH | CN | OH | OH | H | H | $C_6H_5$ | H |
| 283 | General formula (112) | General formula (112) | CN | General formula (112) | Cl | H | $C_6H_5$ | H | H |
| 284 | General formula (112) | General formula (112) | CN | General formula (112) | Cl | H | H | $C_6H_5$ | H |
| 285 | General formula (112) | General formula (112) | CN | General formula (112) | F | H | $C_6H_5$ | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 286 | General formula (112) | General formula (112) | CN | General formula (112) | F | H | H | $C_6H_5$ | H |
| 287 | General formula (112) | General formula (112) | CN | General formula (112) | $CH_3O$ | H | $C_6H_5$ | H | H |
| 288 | General formula (112) | General formula (112) | CN | General formula (112) | $CH_3O$ | H | H | $C_6H_5$ | H |
| 289 | General formula (112) | General formula (112) | CN | General formula (112) | $C_2H_5O$ | H | $C_6H_5$ | H | H |
| 290 | General formula (112) | General formula (112) | CN | General formula (112) | $C_2H_5O$ | H | H | $C_6H_5$ | H |
| 291 | General formula (112) | General formula (112) | CN | General formula (112) | $C_6H_5O$ | H | $C_6H_5$ | H | H |
| 292 | General formula (112) | General formula (112) | CN | General formula (112) | $C_6H_5O$ | H | H | $C_6H_5$ | H |
| 293 | General formula (112) | General formula (112) | CN | General formula (112) | Formula (121) | H | $C_6H_5$ | H | H |
| 294 | General formula (112) | General formula (112) | CN | General formula (112) | Formula (121) | H | H | $C_6H_5$ | H |
| 295 | General formula (112) | General formula (112) | CN | General formula (112) | Formula (122) | H | $C_6H_5$ | H | H |
| 296 | General formula (112) | General formula (112) | CN | General formula (112) | Formula (122) | H | H | $C_6H_5$ | H |
| 297 | General formula (112) | General formula (112) | CN | General formula (112) | Formula (123) | H | $C_6H_5$ | H | H |
| 298 | General formula (112) | General formula (112) | CN | General formula (112) | Formula (123) | H | H | $C_6H_5$ | H |
| 299 | General formula (112) | General formula (112) | CN | General formula (112) | Formula (124) | H | $C_6H_5$ | H | H |
| 300 | General formula (112) | General formula (112) | CN | General formula (112) | Formula (124) | H | H | $C_6H_5$ | H |

Table 2

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 301 | General formula (112) | CN | General formula (112) | General formula (112) | General formula (112) | H | H | H | H |
| 302 | General formula (112) | CN | General formula (112) | General formula (112) | General formula (112) | H | $CH_3$ | H | H |
| 303 | General formula (112) | CN | General formula (112) | General formula (112) | General formula (112) | H | $CH_3O$ | H | H |
| 304 | General formula (112) | CN | General formula (112) | General formula (112) | General formula (112) | H | H | $CH_3$ | H |
| 305 | General formula (112) | CN | General formula (112) | General formula (112) | General formula (112) | H | H | $CH_3O$ | H |
| 306 | General formula (112) | CN | General formula (112) | General formula (112) | General formula (112) | H | H | t-$C_4H_9$ | H |
| 307 | General formula (112) | CN | General formula (112) | General formula (112) | General formula (112) | H | H | Cl | H |
| 308 | General formula (112) | CN | General formula (112) | General formula (112) | General formula (112) | H | H | F | H |
| 309 | General formula (112) | CN | General formula (112) | General formula (112) | General formula (112) | H | H | H | $CH_3$ |
| 310 | General formula (112) | CN | General formula (112) | General formula (112) | General formula (112) | H | H | H | $CH_3O$ |
| 311 | General formula (112) | CN | General formula (112) | General formula (112) | H | H | H | H | H |
| 312 | General formula (112) | CN | General formula (112) | General formula (112) | H | H | H | $CH_3$ | H |
| 313 | General formula (112) | CN | General formula (112) | General formula (112) | H | H | H | $CH_3O$ | H |
| 314 | General formula (112) | CN | General formula (112) | H | General formula (112) | H | H | H | H |
| 315 | General formula (112) | CN | General formula (112) | H | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 316 | General formula (112) | CN | General formula (112) | H | General formula (112) | H | H | $CH_3O$ | H |
| 317 | General formula (112) | CN | H | General formula (112) | General formula (112) | H | H | H | H |
| 318 | General formula (112) | CN | H | General formula (112) | General formula (112) | H | H | $CH_3$ | H |
| 319 | General formula (112) | CN | H | General formula (112) | General formula (112) | H | H | $CH_3O$ | H |
| 320 | H | CN | General formula (112) | General formula (112) | General formula (112) | H | H | H | H |
| 321 | H | CN | General formula (112) | General formula (112) | General formula (112) | H | H | $CH_3$ | H |
| 322 | H | CN | General formula (112) | General formula (112) | General formula (112) | H | H | $CH_3O$ | H |
| 323 | General formula (112) | CN | General formula (112) | H | H | H | H | H | H |
| 324 | General formula (112) | CN | General formula (112) | H | H | H | H | $CH_3$ | H |
| 325 | General formula (112) | CN | General formula (112) | H | H | H | H | $CH_3O$ | H |
| 326 | General formula (112) | CN | H | General formula (112) | H | H | H | H | H |
| 327 | General formula (112) | CN | H | General formula (112) | H | H | H | $CH_3$ | H |
| 328 | General formula (112) | CN | H | General formula (112) | H | H | H | $CH_3O$ | H |
| 329 | H | CN | General formula (112) | General formula (112) | H | H | H | H | H |
| 330 | H | CN | General formula (112) | General formula (112) | H | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 331 | H | CN | General formula (112) | General formula (112) | H | H | H | $CH_3O$ | H |
| 332 | General formula (112) | CN | H | H | General formula (112) | H | H | H | H |
| 333 | General formula (112) | CN | H | H | General formula (112) | H | H | $CH_3$ | H |
| 334 | General formula (112) | CN | H | H | General formula (112) | H | H | $CH_3O$ | H |
| 335 | H | CN | General formula (112) | H | General formula (112) | H | H | H | H |
| 336 | H | CN | General formula (112) | H | General formula (112) | H | H | $CH_3$ | H |
| 337 | H | CN | General formula (112) | H | General formula (112) | H | H | $CH_3O$ | H |
| 338 | H | CN | H | General formula (112) | General formula (112) | H | H | H | H |
| 339 | H | CN | H | General formula (112) | General formula (112) | H | H | $CH_3$ | H |
| 340 | H | CN | H | General formula (112) | General formula (112) | H | H | $CH_3O$ | H |
| 341 | General formula (112) | CN | H | H | H | H | H | H | H |
| 342 | General formula (112) | CN | H | H | H | H | H | $CH_3$ | H |
| 343 | General formula (112) | CN | H | H | H | H | H | $CH_3O$ | H |
| 344 | H | CN | General formula (112) | H | H | H | H | H | H |
| 345 | H | CN | General formula (112) | H | H | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 346 | H | CN | General formula (112) | H | H | H | H | $CH_3O$ | H |
| 347 | H | CN | H | General formula (112) | H | H | H | H | H |
| 348 | H | CN | H | General formula (112) | H | H | H | $CH_3$ | H |
| 349 | H | CN | H | General formula (112) | H | H | H | $CH_3O$ | H |
| 350 | General formula (112) | CN | General formula (112) | General formula (112) | F | H | H | H | H |
| 351 | General formula (112) | CN | General formula (112) | General formula (112) | F | H | H | $CH_3$ | H |
| 352 | General formula (112) | CN | General formula (112) | General formula (112) | F | H | H | $CH_3O$ | H |
| 353 | General formula (112) | CN | General formula (112) | F | General formula (112) | H | H | H | H |
| 354 | General formula (112) | CN | General formula (112) | F | General formula (112) | H | H | $CH_3$ | H |
| 355 | General formula (112) | CN | General formula (112) | F | General formula (112) | H | H | $CH_3O$ | H |
| 356 | General formula (112) | CN | F | General formula (112) | General formula (112) | H | H | H | H |
| 357 | General formula (112) | CN | F | General formula (112) | General formula (112) | H | H | $CH_3$ | H |
| 358 | General formula (112) | CN | F | General formula (112) | General formula (112) | H | H | $CH_3O$ | H |
| 359 | F | CN | General formula (112) | General formula (112) | General formula (112) | H | H | H | H |
| 360 | F | CN | General formula (112) | General formula (112) | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 361 | F | CN | General formula (112) | General formula (112) | General formula (112) | H | H | $CH_3O$ | H |
| 362 | General formula (112) | CN | General formula (112) | F | F | H | H | H | H |
| 363 | General formula (112) | CN | General formula (112) | F | F | H | H | $CH_3$ | H |
| 364 | General formula (112) | CN | General formula (112) | F | F | H | H | $CH_3O$ | H |
| 365 | General formula (112) | CN | F | General formula (112) | F | H | H | H | H |
| 366 | General formula (112) | CN | F | General formula (112) | F | H | H | $CH_3$ | H |
| 367 | General formula (112) | CN | F | General formula (112) | F | H | H | $CH_3O$ | H |
| 368 | F | CN | General formula (112) | General formula (112) | F | H | H | H | H |
| 369 | F | CN | General formula (112) | General formula (112) | F | H | H | $CH_3$ | H |
| 370 | F | CN | General formula (112) | General formula (112) | F | H | H | $CH_3O$ | H |
| 371 | General formula (112) | CN | F | F | General formula (112) | H | H | H | H |
| 372 | General formula (112) | CN | F | F | General formula (112) | H | H | $CH_3$ | H |
| 373 | General formula (112) | CN | F | F | General formula (112) | H | H | $CH_3O$ | H |
| 374 | F | CN | General formula (112) | F | General formula (112) | H | H | H | H |
| 375 | F | CN | General formula (112) | F | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 376 | F | CN | General formula (112) | F | General formula (112) | H | H | $CH_3O$ | H |
| 377 | F | CN | F | General formula (112) | General formula (112) | H | H | H | H |
| 378 | F | CN | F | General formula (112) | General formula (112) | H | H | $CH_3$ | H |
| 379 | F | CN | F | General formula (112) | General formula (112) | H | H | $CH_3O$ | H |
| 380 | General formula (112) | CN | F | F | F | H | H | H | H |
| 381 | General formula (112) | CN | F | F | F | H | H | $CH_3$ | H |
| 382 | General formula (112) | CN | F | F | F | H | H | $CH_3O$ | H |
| 383 | F | CN | General formula (112) | F | F | H | H | H | H |
| 384 | F | CN | General formula (112) | F | F | H | H | $CH_3$ | H |
| 385 | F | CN | General formula (112) | F | F | H | H | $CH_3O$ | H |
| 386 | F | CN | F | General formula (112) | F | H | H | H | H |
| 387 | F | CN | F | General formula (112) | F | H | H | $CH_3$ | H |
| 388 | F | CN | F | General formula (112) | F | H | H | $CH_3O$ | H |
| 389 | General formula (112) | CN | General formula (112) | General formula (112) | OH | H | H | H | H |
| 390 | General formula (112) | CN | General formula (112) | General formula (112) | OH | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 391 | General formula (112) | CN | General formula (112) | General formula (112) | OH | H | H | $CH_3O$ | H |
| 392 | General formula (112) | CN | General formula (112) | OH | General formula (112) | H | H | H | H |
| 393 | General formula (112) | CN | General formula (112) | OH | General formula (112) | H | H | $CH_3$ | H |
| 394 | General formula (112) | CN | General formula (112) | OH | General formula (112) | H | H | $CH_3O$ | H |
| 395 | General formula (112) | CN | General formula (112) | OH | General formula (112) | H | H | t-$C_4H_9$ | H |
| 396 | General formula (112) | CN | General formula (112) | OH | General formula (112) | H | H | Cl | H |
| 397 | General formula (112) | CN | General formula (112) | OH | General formula (112) | H | H | F | H |
| 398 | General formula (112) | CN | OH | General formula (112) | General formula (112) | H | H | H | H |
| 399 | General formula (112) | CN | OH | General formula (112) | General formula (112) | H | H | $CH_3$ | H |
| 400 | General formula (112) | CN | OH | General formula (112) | General formula (112) | H | H | $CH_3O$ | H |
| 401 | OH | CN | General formula (112) | General formula (112) | General formula (112) | H | H | H | H |
| 402 | OH | CN | General formula (112) | General formula (112) | General formula (112) | H | H | $CH_3$ | H |
| 403 | OH | CN | General formula (112) | General formula (112) | General formula (112) | H | H | $CH_3O$ | H |
| 404 | General formula (112) | CN | General formula (112) | OH | OH | H | H | H | H |
| 405 | General formula (112) | CN | General formula (112) | OH | OH | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 406 | General formula (112) | CN | General formula (112) | OH | OH | H | H | $CH_3O$ | H |
| 407 | General formula (112) | CN | OH | General formula (112) | OH | H | H | H | H |
| 408 | General formula (112) | CN | OH | General formula (112) | OH | H | H | $CH_3$ | H |
| 409 | General formula (112) | CN | OH | General formula (112) | OH | H | H | $CH_3O$ | H |
| 410 | OH | CN | General formula (112) | General formula (112) | OH | H | H | H | H |
| 411 | OH | CN | General formula (112) | General formula (112) | OH | H | H | $CH_3$ | H |
| 412 | OH | CN | General formula (112) | General formula (112) | OH | H | H | $CH_3O$ | H |
| 413 | General formula (112) | CN | OH | OH | General formula (112) | H | H | H | H |
| 414 | General formula (112) | CN | OH | OH | General formula (112) | H | H | $CH_3$ | H |
| 415 | General formula (112) | CN | OH | OH | General formula (112) | H | H | $CH_3O$ | H |
| 416 | OH | CN | General formula (112) | OH | General formula (112) | H | H | H | H |
| 417 | OH | CN | General formula (112) | OH | General formula (112) | H | H | $CH_3$ | H |
| 418 | OH | CN | General formula (112) | OH | General formula (112) | H | H | $CH_3O$ | H |
| 419 | OH | CN | OH | General formula (112) | General formula (112) | H | H | H | H |
| 420 | OH | CN | OH | General formula (112) | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 421 | OH | CN | OH | General formula (112) | General formula (112) | H | H | $CH_3O$ | H |
| 422 | General formula (112) | CN | OH | OH | OH | H | H | H | H |
| 423 | General formula (112) | CN | OH | OH | OH | H | H | $CH_3$ | H |
| 424 | General formula (112) | CN | OH | OH | OH | H | H | $CH_3O$ | H |
| 425 | OH | CN | General formula (112) | OH | OH | H | H | H | H |
| 426 | OH | CN | General formula (112) | OH | OH | H | H | $CH_3$ | H |
| 427 | OH | CN | General formula (112) | OH | OH | H | H | $CH_3O$ | H |
| 428 | OH | CN | OH | General formula (112) | OH | H | H | H | H |
| 429 | OH | CN | OH | General formula (112) | OH | H | H | $CH_3$ | H |
| 430 | OH | CN | OH | General formula (112) | OH | H | H | $CH_3O$ | H |
| 431 | OH | CN | OH | OH | General formula (112) | H | H | H | H |
| 432 | OH | CN | OH | OH | General formula (112) | H | H | $CH_3$ | H |
| 433 | OH | CN | OH | OH | General formula (112) | H | H | $CH_3O$ | H |
| 434 | General formula (112) | CN | General formula (112) | Cl | General formula (112) | H | H | H | H |
| 435 | General formula (112) | CN | General formula (112) | Cl | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 436 | General formula (112) | CN | General formula (112) | Cl | General formula (112) | H | H | $CH_3O$ | H |
| 437 | General formula (112) | CN | General formula (112) | Cl | General formula (112) | H | H | t-$C_4H_9$ | H |
| 438 | General formula (112) | CN | General formula (112) | Cl | General formula (112) | H | H | Cl | H |
| 439 | General formula (112) | CN | General formula (112) | Cl | General formula (112) | H | H | F | H |
| 440 | General formula (112) | CN | General formula (112) | F | General formula (112) | H | H | H | H |
| 441 | General formula (112) | CN | General formula (112) | F | General formula (112) | H | H | $CH_3$ | H |
| 442 | General formula (112) | CN | General formula (112) | F | General formula (112) | H | H | $CH_3O$ | H |
| 443 | General formula (112) | CN | General formula (112) | F | General formula (112) | H | H | t-$C_4H_9$ | H |
| 444 | General formula (112) | CN | General formula (112) | F | General formula (112) | H | H | Cl | H |
| 445 | General formula (112) | CN | General formula (112) | F | General formula (112) | H | H | F | H |
| 446 | General formula (112) | CN | General formula (112) | $CH_3O$ | General formula (112) | H | H | H | H |
| 447 | General formula (112) | CN | General formula (112) | $CH_3O$ | General formula (112) | H | H | $CH_3$ | H |
| 448 | General formula (112) | CN | General formula (112) | $CH_3O$ | General formula (112) | H | H | $CH_3O$ | H |
| 449 | General formula (112) | CN | General formula (112) | $CH_3O$ | General formula (112) | H | H | t-$C_4H_9$ | H |
| 450 | General formula (112) | CN | General formula (112) | $CH_3O$ | General formula (112) | H | H | Cl | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 451 | General formula (112) | CN | General formula (112) | $CH_3O$ | General formula (112) | H | H | F | H |
| 452 | General formula (112) | CN | General formula (112) | $C_2H_5O$ | General formula (112) | H | H | H | H |
| 453 | General formula (112) | CN | General formula (112) | $C_2H_5O$ | General formula (112) | H | H | $CH_3$ | H |
| 454 | General formula (112) | CN | General formula (112) | $C_2H_5O$ | General formula (112) | H | H | $CH_3O$ | H |
| 455 | General formula (112) | CN | General formula (112) | $C_2H_5O$ | General formula (112) | H | H | t-$C_4H_9$ | H |
| 456 | General formula (112) | CN | General formula (112) | $C_2H_5O$ | General formula (112) | H | H | Cl | H |
| 457 | General formula (112) | CN | General formula (112) | $C_2H_5O$ | General formula (112) | H | H | F | H |
| 458 | General formula (112) | CN | General formula (112) | $C_6H_5O$ | General formula (112) | H | H | H | H |
| 459 | General formula (112) | CN | General formula (112) | $C_6H_5O$ | General formula (112) | H | H | $CH_3$ | H |
| 460 | General formula (112) | CN | General formula (112) | $C_6H_5O$ | General formula (112) | H | H | $CH_3O$ | H |
| 461 | General formula (112) | CN | General formula (112) | $C_6H_5O$ | General formula (112) | H | H | t-$C_4H_9$ | H |
| 462 | General formula (112) | CN | General formula (112) | $C_6H_5O$ | General formula (112) | H | H | Cl | H |
| 463 | General formula (112) | CN | General formula (112) | $C_6H_5O$ | General formula (112) | H | H | F | H |
| 464 | General formula (112) | CN | General formula (112) | Formula (121) | General formula (112) | H | H | H | H |
| 465 | General formula (112) | CN | General formula (112) | Formula (121) | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 466 | General formula (112) | CN | General formula (112) | Formula (121) | General formula (112) | H | H | $CH_3O$ | H |
| 467 | General formula (112) | CN | General formula (112) | Formula (121) | General formula (112) | H | H | t-$C_4H_9$ | H |
| 468 | General formula (112) | CN | General formula (112) | Formula (121) | General formula (112) | H | H | Cl | H |
| 469 | General formula (112) | CN | General formula (112) | Formula (121) | General formula (112) | H | H | F | H |
| 470 | General formula (112) | CN | General formula (112) | Formula (122) | General formula (112) | H | H | H | H |
| 471 | General formula (112) | CN | General formula (112) | Formula (122) | General formula (112) | H | H | $CH_3$ | H |
| 472 | General formula (112) | CN | General formula (112) | Formula (122) | General formula (112) | H | H | $CH_3$ | H |
| 473 | General formula (112) | CN | General formula (112) | Formula (122) | General formula (112) | H | H | t-$C_4H_9$ | H |
| 474 | General formula (112) | CN | General formula (112) | Formula (122) | General formula (112) | H | H | Cl | H |
| 475 | General formula (112) | CN | General formula (112) | Formula (122) | General formula (112) | H | H | F | H |
| 476 | General formula (112) | CN | General formula (112) | Formula (123) | General formula (112) | H | H | H | H |
| 477 | General formula (112) | CN | General formula (112) | Formula (123) | General formula (112) | H | H | $CH_3$ | H |
| 478 | General formula (112) | CN | General formula (112) | Formula (123) | General formula (112) | H | H | $CH_3O$ | H |
| 479 | General formula (112) | CN | General formula (112) | Formula (123) | General formula (112) | H | H | t-$C_4H_9$ | H |
| 480 | General formula (112) | CN | General formula (112) | Formula (123) | General formula (112) | H | H | Cl | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 481 | General formula (112) | CN | General formula (112) | Formula (123) | General formula (112) | H | H | F | H |
| 482 | General formula (112) | CN | General formula (112) | Formula (124) | General formula (112) | H | H | H | H |
| 483 | General formula (112) | CN | General formula (112) | Formula (124) | General formula (112) | H | H | $CH_3$ | H |
| 484 | General formula (112) | CN | General formula (112) | Formula (124) | General formula (112) | H | H | $CH_3O$ | H |
| 485 | General formula (112) | CN | General formula (112) | Formula (124) | General formula (112) | H | H | t-$C_4H_9$ | H |
| 486 | General formula (112) | CN | General formula (112) | Formula (124) | General formula (112) | H | H | Cl | H |
| 487 | General formula (112) | CN | General formula (112) | Formula (124) | General formula (112) | H | H | F | H |
| 488 | General formula (112) | CN | General formula (112) | General formula (112) | General formula (112) | H | $C_6H_5$ | H | H |
| 489 | General formula (112) | CN | General formula (112) | General formula (112) | General formula (112) | H | H | $C_6H_5$ | H |
| 490 | General formula (112) | CN | General formula (112) | General formula (112) | H | H | $C_6H_5$ | H | H |
| 491 | General formula (112) | CN | General formula (112) | General formula (112) | H | H | H | $C_6H_5$ | H |
| 492 | General formula (112) | CN | General formula (112) | H | General formula (112) | H | $C_6H_5$ | H | H |
| 493 | General formula (112) | CN | General formula (112) | H | General formula (112) | H | H | $C_6H_5$ | H |
| 494 | General formula (112) | CN | H | General formula (112) | General formula (112) | H | $C_6H_5$ | H | H |
| 495 | General formula (112) | CN | H | General formula (112) | General formula (112) | H | H | $C_6H_5$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 496 | H | CN | General formula (112) | General formula (112) | General formula (112) | H | $C_6H_5$ | H | H |
| 497 | H | CN | General formula (112) | General formula (112) | General formula (112) | H | H | $C_6H_5$ | H |
| 498 | General formula (112) | CN | General formula (112) | H | H | H | $C_6H_5$ | H | H |
| 499 | General formula (112) | CN | General formula (112) | H | H | H | H | $C_6H_5$ | H |
| 500-1 | General formula (112) | CN | H | General formula (112) | H | H | $C_6H_5$ | H | H |
| 500-2 | General formula (112) | CN | H | General formula (112) | H | H | H | $C_6H_5$ | H |
| 500-3 | H | CN | General formula (112) | General formula (112) | H | H | $C_6H_5$ | H | H |
| 500-4 | H | CN | General formula (112) | General formula (112) | H | H | H | $C_6H_5$ | H |
| 500-5 | General formula (112) | CN | H | H | General formula (112) | H | $C_6H_5$ | H | H |
| 500-6 | General formula (112) | CN | H | H | General formula (112) | H | H | $C_6H_5$ | H |
| 500-7 | H | CN | General formula (112) | H | General formula (112) | H | $C_6H_5$ | H | H |
| 500-8 | H | CN | General formula (112) | H | General formula (112) | H | H | $C_6H_5$ | H |
| 500-9 | H | CN | H | General formula (112) | General formula (112) | H | $C_6H_5$ | H | H |
| 500-10 | H | CN | H | General formula (112) | General formula (112) | H | H | $C_6H_5$ | H |
| 500-11 | General formula (112) | CN | H | H | H | H | $C_6H_5$ | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 500-12 | General formula (112) | CN | H | H | H | H | H | $C_6H_5$ | H |
| 500-13 | H | CN | General formula (112) | H | H | H | $C_6H_5$ | H | H |
| 500-14 | H | CN | General formula (112) | H | H | H | H | $C_6H_5$ | H |
| 500-15 | H | CN | H | General formula (112) | H | H | $C_6H_5$ | H | H |
| 500-16 | H | CN | H | General formula (112) | H | H | H | $C_6H_5$ | H |
| 500-17 | General formula (112) | CN | General formula (112) | General formula (112) | F | H | H | $C_6H_5$ | H |
| 500-18 | General formula (112) | CN | General formula (112) | F | General formula (112) | H | H | $C_6H_5$ | H |
| 500-19 | General formula (112) | CN | F | General formula (112) | General formula (112) | H | H | $C_6H_5$ | H |
| 500-20 | F | CN | General formula (112) | General formula (112) | General formula (112) | H | H | $C_6H_5$ | H |
| 500-21 | General formula (112) | CN | General formula (112) | F | F | H | H | $C_6H_5$ | H |
| 500-22 | General formula (112) | CN | F | General formula (112) | F | H | H | $C_6H_5$ | H |
| 500-23 | F | CN | General formula (112) | General formula (112) | F | H | H | $C_6H_5$ | H |
| 500-24 | General formula (112) | CN | F | F | General formula (112) | H | H | $C_6H_5$ | H |
| 500-25 | F | CN | General formula (112) | F | General formula (112) | H | H | $C_6H_5$ | H |
| 500-26 | F | CN | F | General formula (112) | General formula (112) | H | H | $C_6H_5$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 500-27 | General formula (112) | CN | F | F | F | H | H | $C_6H_5$ | H |
| 500-28 | F | CN | General formula (112) | F | F | H | H | $C_6H_5$ | H |
| 500-29 | F | CN | F | General formula (112) | F | H | H | $C_6H_5$ | H |
| 500-30 | General formula (112) | CN | General formula (112) | General formula (112) | OH | H | H | $C_6H_5$ | H |
| 500-31 | General formula (112) | CN | General formula (112) | OH | General formula (112) | H | H | $C_6H_5$ | H |
| 500-32 | General formula (112) | CN | OH | General formula (112) | General formula (112) | H | H | $C_6H_5$ | H |
| 500-33 | OH | CN | General formula (112) | General formula (112) | General formula (112) | H | H | $C_6H_5$ | H |
| 500-34 | General formula (112) | CN | General formula (112) | OH | OH | H | H | $C_6H_5$ | H |
| 500-35 | General formula (112) | CN | OH | General formula (112) | OH | H | H | $C_6H_5$ | H |
| 500-36 | OH | CN | General formula (112) | General formula (112) | OH | H | H | $C_6H_5$ | H |
| 500-37 | General formula (112) | CN | OH | OH | General formula (112) | H | H | $C_6H_5$ | H |
| 500-38 | OH | CN | General formula (112) | OH | General formula (112) | H | H | $C_6H_5$ | H |
| 500-39 | OH | CN | OH | General formula (112) | General formula (112) | H | H | $C_6H_5$ | H |
| 500-40 | General formula (112) | CN | OH | OH | OH | H | H | $C_6H_5$ | H |
| 500-41 | OH | CN | General formula (112) | OH | OH | H | H | $C_6H_5$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 500-42 | OH | CN | OH | General formula (112) | OH | H | H | $C_6H_5$ | H |
| 500-43 | OH | CN | OH | OH | General formula (112) | H | H | $C_6H_5$ | H |
| 500-44 | General formula (112) | CN | General formula (112) | Cl | General formula (112) | H | H | $C_6H_5$ | H |
| 500-45 | General formula (112) | CN | General formula (112) | F | General formula (112) | H | H | $C_6H_5$ | H |
| 500-46 | General formula (112) | CN | General formula (112) | $CH_3O$ | General formula (112) | H | H | $C_6H_5$ | H |
| 500-47 | General formula (112) | CN | General formula (112) | $C_2H_5O$ | General formula (112) | H | H | $C_6H_5$ | H |
| 500-48 | General formula (112) | CN | General formula (112) | $C_6H_5O$ | General formula (112) | H | H | $C_6H_5$ | H |
| 500-49 | General formula (112) | CN | General formula (112) | Formula (121) | General formula (112) | H | H | $C_6H_5$ | H |
| 500-50 | General formula (112) | CN | General formula (112) | Formula (122) | General formula (112) | H | H | $C_6H_5$ | H |
| 500-51 | General formula (112) | CN | General formula (112) | Formula (123) | General formula (112) | H | H | $C_6H_5$ | H |
| 500-52 | General formula (112) | CN | General formula (112) | Formula (124) | General formula (112) | H | H | $C_6H_5$ | H |

Table 3

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 501 | CN | General formula (112) | General formula (112) | General formula (112) | General formula (112) | H | H | H | H |
| 502 | CN | General formula (112) | General formula (112) | General formula (112) | General formula (112) | H | $CH_3$ | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 503 | CN | General formula (112) | General formula (112) | General formula (112) | General formula (112) | H | $CH_3O$ | H | H |
| 504 | CN | General formula (112) | General formula (112) | General formula (112) | General formula (112) | H | H | $CH_3$ | H |
| 505 | CN | General formula (112) | General formula (112) | General formula (112) | General formula (112) | H | H | $CH_3O$ | H |
| 506 | CN | General formula (112) | General formula (112) | General formula (112) | General formula (112) | H | H | t-$C_4H_9$ | H |
| 507 | CN | General formula (112) | General formula (112) | General formula (112) | General formula (112) | H | H | Cl | H |
| 508 | CN | General formula (112) | General formula (112) | General formula (112) | General formula (112) | H | H | F | H |
| 509 | CN | General formula (112) | General formula (112) | General formula (112) | General formula (112) | H | H | H | $CH_3$ |
| 510 | CN | General formula (112) | General formula (112) | General formula (112) | General formula (112) | H | H | H | $CH_3O$ |
| 511 | CN | General formula (112) | General formula (112) | General formula (112) | H | H | H | H | H |
| 512 | CN | General formula (112) | General formula (112) | General formula (112) | H | H | H | $CH_3$ | H |
| 513 | CN | General formula (112) | General formula (112) | General formula (112) | H | H | H | $CH_3O$ | H |
| 514 | CN | General formula (112) | General formula (112) | H | General formula (112) | H | H | H | H |
| 515 | CN | General formula (112) | General formula (112) | H | General formula (112) | H | H | $CH_3$ | H |
| 516 | CN | General formula (112) | General formula (112) | H | General formula (112) | H | H | $CH_3O$ | H |
| 517 | CN | General formula (112) | General formula (112) | H | H | H | H | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 518 | CN | General formula (112) | General formula (112) | H | H | H | H | $CH_3$ | H |
| 519 | CN | General formula (112) | General formula (112) | H | H | H | H | $CH_3O$ | H |
| 520 | CN | General formula (112) | H | General formula (112) | H | H | H | H | H |
| 521 | CN | General formula (112) | H | General formula (112) | H | H | H | $CH_3$ | H |
| 522 | CN | General formula (112) | H | General formula (112) | H | H | H | $CH_3O$ | H |
| 523 | CN | H | General formula (112) | General formula (112) | H | H | H | H | H |
| 524 | CN | H | General formula (112) | General formula (112) | H | H | H | $CH_3$ | H |
| 525 | CN | H | General formula (112) | General formula (112) | H | H | H | $CH_3O$ | H |
| 526 | CN | General formula (112) | H | H | General formula (112) | H | H | H | H |
| 527 | CN | General formula (112) | H | H | General formula (112) | H | H | $CH_3$ | H |
| 528 | CN | General formula (112) | H | H | General formula (112) | H | H | $CH_3O$ | H |
| 529 | CN | General formula (112) | H | H | H | H | H | H | H |
| 530 | CN | General formula (112) | H | H | H | H | H | $CH_3$ | H |
| 531 | CN | General formula (112) | H | H | H | H | H | $CH_3O$ | H |
| 532 | CN | H | General formula (112) | H | H | H | H | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 533 | CN | H | General formula (112) | H | H | H | H | $CH_3$ | H |
| 534 | CN | H | General formula (112) | H | H | H | H | $CH_3O$ | H |
| 535 | CN | General formula (112) | General formula (112) | General formula (112) | F | H | H | H | H |
| 536 | CN | General formula (112) | General formula (112) | General formula (112) | F | H | H | $CH_3$ | H |
| 537 | CN | General formula (112) | General formula (112) | General formula (112) | F | H | H | $CH_3O$ | H |
| 538 | CN | General formula (112) | General formula (112) | F | General formula (112) | H | H | H | H |
| 539 | CN | General formula (112) | General formula (112) | F | General formula (112) | H | H | $CH_3$ | H |
| 540 | CN | General formula (112) | General formula (112) | F | General formula (112) | H | H | $CH_3O$ | H |
| 541 | CN | General formula (112) | General formula (112) | F | F | H | H | H | H |
| 542 | CN | General formula (112) | General formula (112) | F | F | H | H | $CH_3$ | H |
| 543 | CN | General formula (112) | General formula (112) | F | F | H | H | $CH_3O$ | H |
| 544 | CN | General formula (112) | F | General formula (112) | F | H | H | H | H |
| 545 | CN | General formula (112) | F | General formula (112) | F | H | H | $CH_3$ | H |
| 546 | CN | General formula (112) | F | General formula (112) | F | H | H | $CH_3O$ | H |
| 547 | CN | F | General formula (112) | General formula (112) | F | H | H | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 548 | CN | F | General formula (112) | General formula (112) | F | H | H | $CH_3$ | H |
| 549 | CN | F | General formula (112) | General formula (112) | F | H | H | $CH_3O$ | H |
| 550 | CN | General formula (112) | F | F | General formula (112) | H | H | H | H |
| 551 | CN | General formula (112) | F | F | General formula (112) | H | H | $CH_3$ | H |
| 552 | CN | General formula (112) | F | F | General formula (112) | H | H | $CH_3O$ | H |
| 553 | CN | General formula (112) | F | F | F | H | H | H | H |
| 554 | CN | General formula (112) | F | F | F | H | H | $CH_3$ | H |
| 555 | CN | General formula (112) | F | F | F | H | H | $CH_3O$ | H |
| 556 | CN | F | General formula (112) | F | F | H | H | H | H |
| 557 | CN | F | General formula (112) | F | F | H | H | $CH_3$ | H |
| 558 | CN | F | General formula (112) | F | F | H | H | $CH_3O$ | H |
| 559 | CN | General formula (112) | General formula (112) | General formula (112) | OH | H | H | H | H |
| 560 | CN | General formula (112) | General formula (112) | General formula (112) | OH | H | H | $CH_3$ | H |
| 561 | CN | General formula (112) | General formula (112) | General formula (112) | OH | H | H | $CH_3O$ | H |
| 562 | CN | General formula (112) | General formula (112) | OH | General formula (112) | H | H | H | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 563 | CN | General formula (112) | General formula (112) | OH | General formula (112) | H | H | $CH_3$ | H |
| 654 | CN | General formula (112) | General formula (112) | OH | General formula (112) | H | H | $CH_3O$ | H |
| 565 | CN | General formula (112) | General formula (112) | OH | General formula (112) | H | H | Cl | H |
| 566 | CN | General formula (112) | General formula (112) | OH | General formula (112) | H | H | F | H |
| 567 | CN | General formula (112) | General formula (112) | OH | OH | H | H | H | H |
| 568 | CN | General formula (112) | General formula (112) | OH | OH | H | H | $CH_3$ | H |
| 569 | CN | General formula (112) | General formula (112) | OH | OH | H | H | $CH_3O$ | H |
| 570 | CN | General formula (112) | OH | General formula (112) | OH | H | H | H | H |
| 571 | CN | General formula (112) | OH | General formula (112) | OH | H | H | $CH_3$ | H |
| 572 | CN | General formula (112) | OH | General formula (112) | OH | H | H | $CH_3O$ | H |
| 573 | CN | OH | General formula (112) | General formula (112) | OH | H | H | H | H |
| 574 | CN | OH | General formula (112) | General formula (112) | OH | H | H | $CH_3$ | H |
| 575 | CN | OH | General formula (112) | General formula (112) | OH | H | H | $CH_3O$ | H |
| 576 | CN | General formula (112) | OH | OH | General formula (112) | H | H | H | H |
| 577 | CN | General formula (112) | OH | OH | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 578 | CN | General formula (112) | OH | OH | General formula (112) | H | H | $CH_3O$ | H |
| 579 | CN | General formula (112) | OH | OH | OH | H | H | H | H |
| 580 | CN | General formula (112) | OH | OH | OH | H | H | $CH_3$ | H |
| 581 | CN | General formula (112) | OH | OH | OH | H | H | $CH_3O$ | H |
| 582 | CN | OH | General formula (112) | OH | OH | H | H | H | H |
| 583 | CN | OH | General formula (112) | OH | OH | H | H | $CH_3$ | H |
| 584 | CN | OH | General formula (112) | OH | OH | H | H | $CH_3O$ | H |
| 585 | CN | General formula (112) | General formula (112) | Cl | General formula (112) | H | H | H | H |
| 586 | CN | General formula (112) | General formula (112) | Cl | General formula (112) | H | H | $CH_3$ | H |
| 587 | CN | General formula (112) | General formula (112) | Cl | General formula (112) | H | H | $CH_3O$ | H |
| 588 | CN | General formula (112) | General formula (112) | Cl | General formula (112) | H | H | t-$C_4H_9$ | H |
| 589 | CN | General formula (112) | General formula (112) | Cl | General formula (112) | H | H | Cl | H |
| 590 | CN | General formula (112) | General formula (112) | Cl | General formula (112) | H | H | F | H |
| 591 | CN | General formula (112) | General formula (112) | F | General formula (112) | H | H | H | H |
| 592 | CN | General formula (112) | General formula (112) | F | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 593 | CN | General formula (112) | General formula (112) | F | General formula (112) | H | H | $CH_3O$ | H |
| 594 | CN | General formula (112) | General formula (112) | F | General formula (112) | H | H | t-$C_4H_9$ | H |
| 595 | CN | General formula (112) | General formula (112) | F | General formula (112) | H | H | Cl | H |
| 596 | CN | General formula (112) | General formula (112) | F | General formula (112) | H | H | F | H |
| 597 | CN | General formula (112) | General formula (112) | $CH_3O$ | General formula (112) | H | H | H | H |
| 598 | CN | General formula (112) | General formula (112) | $CH_3O$ | General formula (112) | H | H | $CH_3$ | H |
| 599 | CN | General formula (112) | General formula (112) | $CH_3O$ | General formula (112) | H | H | $CH_3O$ | H |
| 600 | CN | General formula (112) | General formula (112) | $CH_3O$ | General formula (112) | H | H | t-$C_4H_9$ | H |
| 601 | CN | General formula (112) | General formula (112) | $CH_3O$ | General formula (112) | H | H | Cl | H |
| 602 | CN | General formula (112) | General formula (112) | $CH_3O$ | General formula (112) | H | H | F | H |
| 603 | CN | General formula (112) | General formula (112) | $C_2H_5O$ | General formula (112) | H | H | H | H |
| 604 | CN | General formula (112) | General formula (112) | $C_2H_5O$ | General formula (112) | H | H | $CH_3$ | H |
| 605 | CN | General formula (112) | General formula (112) | $C_2H_5O$ | General formula (112) | H | H | $CH_3O$ | H |
| 606 | CN | General formula (112) | General formula (112) | $C_2H_5O$ | General formula (112) | H | H | t-$C_4H_9$ | H |
| 607 | CN | General formula (112) | General formula (112) | $C_2H_5O$ | General formula (112) | H | H | Cl | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 608 | CN | General formula (112) | General formula (112) | $C_2H_5O$ | General formula (112) | H | H | F | H |
| 609 | CN | General formula (112) | General formula (112) | $C_6H_5O$ | General formula (112) | H | H | H | H |
| 610 | CN | General formula (112) | General formula (112) | $C_6H_5O$ | General formula (112) | H | H | $CH_3$ | H |
| 611 | CN | General formula (112) | General formula (112) | $C_6H_5O$ | General formula (112) | H | H | $CH_3O$ | H |
| 612 | CN | General formula (112) | General formula (112) | $C_6H_5O$ | General formula (112) | H | H | t-$C_4H_9$ | H |
| 613 | CN | General formula (112) | General formula (112) | $C_6H_5O$ | General formula (112) | H | H | Cl | H |
| 614 | CN | General formula (112) | General formula (112) | $C_6H_5O$ | General formula (112) | H | H | F | H |
| 615 | CN | General formula (112) | General formula (112) | Formula (121) | General formula (112) | H | H | H | H |
| 616 | CN | General formula (112) | General formula (112) | Formula (121) | General formula (112) | H | H | $CH_3$ | H |
| 617 | CN | General formula (112) | General formula (112) | Formula (121) | General formula (112) | H | H | $CH_3O$ | H |
| 618 | CN | General formula (112) | General formula (112) | Formula (121) | General formula (112) | H | H | t-$C_4H_9$ | H |
| 619 | CN | General formula (112) | General formula (112) | Formula (121) | General formula (112) | H | H | Cl | H |
| 620 | CN | General formula (112) | General formula (112) | Formula (121) | General formula (112) | H | H | F | H |
| 621 | CN | General formula (112) | General formula (112) | Formula (122) | General formula (112) | H | H | H | H |
| 622 | CN | General formula (112) | General formula (112) | Formula (122) | General formula (112) | H | H | $CH_3$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 623 | CN | General formula (112) | General formula (112) | Formula (122) | General formula (112) | H | H | $CH_3O$ | H |
| 624 | CN | General formula (112) | General formula (112) | Formula (122) | General formula (112) | H | H | t-$C_4H_9$ | H |
| 625 | CN | General formula (112) | General formula (112) | Formula (122) | General formula (112) | H | H | Cl | H |
| 626 | CN | General formula (112) | General formula (112) | Formula (122) | General formula (112) | H | H | F | H |
| 627 | CN | General formula (112) | General formula (112) | Formula (123) | General formula (112) | H | H | H | H |
| 628 | CN | General formula (112) | General formula (112) | Formula (123) | General formula (112) | H | H | $CH_3$ | H |
| 629 | CN | General formula (112) | General formula (112) | Formula (123) | General formula (112) | H | H | $CH_3O$ | H |
| 630 | CN | General formula (112) | General formula (112) | Formula (123) | General formula (112) | H | H | t-$C_4H_9$ | H |
| 631 | CN | General formula (112) | General formula (112) | Formula (123) | General formula (112) | H | H | Cl | H |
| 632 | CN | General formula (112) | General formula (112) | Formula (123) | General formula (112) | H | H | F | H |
| 633 | CN | General formula (112) | General formula (112) | Formula (124) | General formula (112) | H | H | H | H |
| 634 | CN | General formula (112) | General formula (112) | Formula (124) | General formula (112) | H | H | $CH_3$ | H |
| 635 | CN | General formula (112) | General formula (112) | Formula (124) | General formula (112) | H | H | $CH_3O$ | H |
| 636 | CN | General formula (112) | General formula (112) | Formula (124) | General formula (112) | H | H | t-$C_4H_9$ | H |
| 637 | CN | General formula (112) | General formula (112) | Formula (124) | General formula (112) | H | H | Cl | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 638 | CN | General formula (112) | General formula (112) | Formula (124) | General formula (112) | H | H | F | H |
| 639 | CN | General formula (112) | General formula (112) | General formula (112) | General formula (112) | H | $C_6H_5$ | H | H |
| 640 | CN | General formula (112) | General formula (112) | General formula (112) | General formula (112) | H | H | $C_6H_5$ | H |
| 641 | CN | General formula (112) | General formula (112) | General formula (112) | H | H | $C_6H_5$ | H | H |
| 642 | CN | General formula (112) | General formula (112) | General formula (112) | H | H | H | $C_6H_5$ | H |
| 643 | CN | General formula (112) | General formula (112) | H | General formula (112) | H | $C_6H_5$ | H | H |
| 644 | CN | General formula (112) | General formula (112) | H | General formula (112) | H | H | $C_6H_5$ | H |
| 645 | CN | General formula (112) | General formula (112) | H | H | H | $C_6H_5$ | H | H |
| 646 | CN | General formula (112) | General formula (112) | H | H | H | H | $C_6H_5$ | H |
| 647 | CN | General formula (112) | H | General formula (112) | H | H | $C_6H_5$ | H | H |
| 648 | CN | General formula (112) | H | General formula (112) | H | H | H | $C_6H_5$ | H |
| 649 | CN | H | General formula (112) | General formula (112) | H | H | $C_6H_5$ | H | H |
| 650 | CN | H | General formula (112) | General formula (112) | H | H | H | $C_6H_5$ | H |
| 651 | CN | H | H | General formula (112) | General formula (112) | H | $C_6H_5$ | H | H |
| 652 | CN | H | H | General formula (112) | General formula (112) | H | H | $C_6H_5$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 653 | CN | General formula (112) | H | H | H | H | $C_6H_5$ | H | H |
| 654 | CN | General formula (112) | H | H | H | H | H | $C_6H_5$ | H |
| 655 | CN | H | General formula (112) | H | H | H | $C_6H_5$ | H | H |
| 656 | CN | H | General formula (112) | H | H | H | H | $C_6H_5$ | H |
| 657 | CN | General formula (112) | General formula (112) | General formula (112) | F | H | H | $C_6H_5$ | H |
| 658 | CN | General formula (112) | General formula (112) | F | General formula (112) | H | H | $C_6H_5$ | H |
| 659 | CN | General formula (112) | General formula (112) | F | F | H | H | $C_6H_5$ | H |
| 660 | CN | General formula (112) | F | General formula (112) | F | H | H | $C_6H_5$ | H |
| 661 | CN | F | General formula (112) | General formula (112) | F | H | H | $C_6H_5$ | H |
| 662 | CN | F | F | General formula (112) | General formula (112) | H | H | $C_6H_5$ | H |
| 663 | CN | General formula (112) | F | F | F | H | H | $C_6H_5$ | H |
| 664 | CN | F | General formula (112) | F | F | H | H | $C_6H_5$ | H |
| 665 | CN | General formula (112) | General formula (112) | General formula (112) | OH | H | H | $C_6H_5$ | H |
| 666 | CN | General formula (112) | General formula (112) | OH | General formula (112) | H | H | $C_6H_5$ | H |
| 667 | CN | General formula (112) | General formula (112) | OH | OH | H | H | $C_6H_5$ | H |

(continued)

| Compound No. | General formula (1) | | | | | General formula (112) | | | |
|---|---|---|---|---|---|---|---|---|---|
| | $R^1$ | $R^2$ | $R^3$ | $R^4$ | $R^5$ | $R^{31}$, $R^{38}$ | $R^{32}$, $R^{37}$ | $R^{33}$, $R^{36}$ | $R^{34}$, $R^{35}$ |
| 668 | CN | General formula (112) | OH | General formula (112) | OH | H | H | $C_6H_5$ | H |
| 669 | CN | OH | General formula (112) | General formula (112) | OH | H | H | $C_6H_5$ | H |
| 670 | CN | OH | OH | General formula (112) | General formula (112) | H | H | $C_6H_5$ | H |
| 671 | CN | General formula (112) | OH | OH | OH | H | H | $C_6H_5$ | H |
| 672 | CN | OH | General formula (112) | OH | OH | H | H | $C_6H_5$ | H |
| 673 | CN | General formula (112) | General formula (112) | Cl | General formula (112) | H | H | $C_6H_5$ | H |
| 674 | CN | General formula (112) | General formula (112) | F | General formula (112) | H | H | $C_6H_5$ | H |
| 675 | CN | General formula (112) | General formula (112) | $CH_3O$ | General formula (112) | H | H | $C_6H_5$ | H |
| 676 | CN | General formula (112) | General formula (112) | $C_2H_5O$ | General formula (112) | H | H | $C_6H_5$ | H |
| 677 | CN | General formula (112) | General formula (112) | $C_6H_5O$ | General formula (112) | H | H | $C_6H_5$ | H |
| 678 | CN | General formula (112) | General formula (112) | Formula (121) | General formula (112) | H | H | $C_6H_5$ | H |
| 679 | CN | General formula (112) | General formula (112) | Formula (122) | General formula (112) | H | H | $C_6H_5$ | H |
| 680 | CN | General formula (112) | General formula (112) | Formula (123) | General formula (112) | H | H | $C_6H_5$ | H |
| 681 | CN | General formula (112) | General formula (112) | Formula (124) | General formula (112) | H | H | $C_6H_5$ | H |

Table 4

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 701 | General formula (113) | General formula (113) | CN | General formula (113) | General formula (113) | H | H | H | H | H | H |
| 702 | General formula (113) | General formula (113) | CN | General formula (113) | General formula (113) | H | $CH_3$ | H | H | H | H |
| 703 | General formula (113) | General formula (113) | CN | General formula (113) | General formula (113) | H | $CH_3O$ | H | H | H | H |
| 704 | General formula (113) | General formula (113) | CN | General formula (113) | General formula (113) | H | H | $CH_3$ | H | H | H |
| 705 | General formula (113) | General formula (113) | CN | General formula (113) | General formula (113) | H | H | $CH_3O$ | H | H | H |
| 706 | General formula (113) | General formula (113) | CN | General formula (113) | General formula (113) | H | H | t-$C_4H_9$ | H | H | H |
| 707 | General formula (113) | General formula (113) | CN | General formula (113) | General formula (113) | H | H | Cl | H | H | H |
| 708 | General formula (113) | General formula (113) | CN | General formula (113) | General formula (113) | H | H | F | H | H | H |
| 709 | General formula (113) | General formula (113) | CN | General formula (113) | General formula (113) | H | H | H | $CH_3$ | H | H |
| 710 | General formula (113) | General formula (113) | CN | General formula (113) | General formula (113) | H | H | H | $CH_3O$ | H | H |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 711 | General formula (113) | General formula (113) | CN | General formula (113) | General formula (113) | H | H | H | H | $CH_3$ | H |
| 712 | General formula (113) | General formula (113) | CN | General formula (113) | General formula (113) | H | H | H | H | $CH_3O$ | H |
| 713 | General formula (113) | General formula (113) | CN | General formula (113) | General formula (113) | H | H | H | H | t-$C_4H_9$ | H |
| 714 | General formula (113) | General formula (113) | CN | General formula (113) | General formula (113) | H | H | H | H | Cl | H |
| 715 | General formula (113) | General formula (113) | CN | General formula (113) | General formula (113) | H | H | H | H | F | H |
| 716 | General formula (113) | General formula (113) | CN | General formula (113) | General formula (113) | H | H | H | H | $C_6H_5$ | H |
| 717 | General formula (113) | General formula (113) | CN | General formula (113) | General formula (113) | H | H | H | H | p-$CH_3C_6H_4$ | H |
| 718 | General formula (113) | General formula (113) | CN | General formula (113) | General formula (113) | H | H | H | H | 2,4,6-$(CH_3)_3C_6H_2$ | H |
| 719 | General formula (113) | General formula (113) | CN | General formula (113) | General formula (113) | H | H | H | H | p-$CH_3OC_6H_4$ | H |
| 720 | General formula (113) | General formula (113) | CN | General formula (113) | General formula (113) | H | H | H | H | p-$(CH_3)_2NC_6H_4$ | H |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 721 | General formula (113) | General formula (113) | CN | General formula (113) | General formula (113) | H | H | H | H | p-$FC_6H_4$ | H |
| 722 | General formula (113) | General formula (113) | CN | General formula (113) | General formula (113) | H | H | H | H | p-$CNC_6H_4$ | H |
| 723 | General formula (113) | General formula (113) | CN | General formula (113) | General formula (113) | H | H | H | H | H | $CH_3$ |
| 724 | General formula (113) | General formula (113) | CN | General formula (113) | General formula (113) | H | H | H | H | H | $CH_3O$ |
| 725 | General formula (113) | General formula (113) | CN | General formula (113) | General formula (113) | H | H | H | H | H | t-$C_4H_9$ |
| 726 | General formula (113) | General formula (113) | CN | General formula (113) | General formula (113) | H | H | H | H | H | Cl |
| 727 | General formula (113) | General formula (113) | CN | General formula (113) | General formula (113) | H | H | H | H | H | F |
| 728 | General formula (113) | General formula (113) | CN | General formula (113) | General formula (113) | H | H | H | H | H | $C_6H_5$ |
| 729 | General formula (113) | General formula (113) | CN | General formula (113) | General formula (113) | H | H | H | H | H | p-$CH_3C_6H_4$ |
| 730 | General formula (113) | General formula (113) | CN | General formula (113) | General formula (113) | H | H | H | H | H | 2,4,6-$(CH_3)_3C_6H_2$ |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 731 | General formula (113) | General formula (113) | CN | General formula (113) | General formula (113) | H | H | H | H | H | p-$CH_3OC_6H_4$ |
| 732 | General formula (113) | General formula (113) | CN | General formula (113) | General formula (113) | H | H | H | H | H | p-$(CH_3)_2NC_6H_4$ |
| 733 | General formula (113) | General formula (113) | CN | General formula (113) | General formula (113) | H | H | H | H | H | p-$FC_6H_4$ |
| 734 | General formula (113) | General formula (113) | CN | General formula (113) | General formula (113) | H | H | H | H | H | p-$CNC_6H_4$ |
| 735 | General formula (113) | General formula (113) | CN | General formula (113) | H | H | H | H | H | H | H |
| 736 | General formula (113) | General formula (113) | CN | H | General formula (113) | H | H | H | H | H | H |
| 737 | General formula (113) | General formula (113) | CN | H | H | H | H | H | H | H | H |
| 738 | General formula (113) | H | CN | General formula (113) | H | H | H | H | H | H | H |
| 739 | H | General formula (113) | CN | General formula (113) | H | H | H | H | H | H | H |
| 740 | General formula (113) | H | CN | H | H | H | H | H | H | H | H |
| 741 | General formula (113) | General formula (113) | CN | General formula (113) | F | H | H | H | H | H | H |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 742 | General formula (113) | General formula (113) | CN | F | General formula (113) | H | H | H | H | H | H |
| 743 | General formula (113) | General formula (113) | CN | F | F | H | H | H | H | H | H |
| 744 | General formula (113) | F | CN | General formula (113) | F | H | H | H | H | H | H |
| 745 | F | General formula (113) | CN | General formula (113) | F | H | H | H | H | H | H |
| 746 | General formula (113) | F | CN | F | F | H | H | H | H | H | H |
| 747 | General formula (113) | General formula (113) | CN | General formula (113) | OH | H | H | H | H | H | H |
| 748 | General formula (113) | General formula (113) | CN | OH | General formula (113) | H | H | H | H | H | H |
| 749 | General formula (113) | General formula (113) | CN | OH | OH | H | H | H | H | H | H |
| 750 | General formula (113) | OH | CN | General formula (113) | OH | H | H | H | H | H | H |
| 751 | OH | General formula (113) | CN | General formula (113) | OH | H | H | H | H | H | H |
| 752 | General formula (113) | OH | CN | OH | OH | H | H | H | H | H | H |
| 753 | General formula (113) | General formula (113) | CN | Cl | General formula (113) | H | H | H | H | H | H |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 754 | General formula (113) | General formula (113) | CN | Cl | General formula (113) | H | H | $CH_3$ | H | H | H |
| 755 | General formula (113) | General formula (113) | CN | Cl | General formula (113) | H | H | $CH_3O$ | H | H | H |
| 756 | General formula (113) | General formula (113) | CN | Cl | General formula (113) | H | H | t-$C_4H_9$ | H | H | H |
| 757 | General formula (113) | General formula (113) | CN | Cl | General formula (113) | H | H | Cl | H | H | H |
| 758 | General formula (113) | General formula (113) | CN | Cl | General formula (113) | H | H | F | H | H | H |
| 759 | General formula (113) | General formula (113) | CN | F | General formula (113) | H | H | H | H | H | H |
| 760 | General formula (113) | General formula (113) | CN | F | General formula (113) | H | H | $CH_3$ | H | H | H |
| 761 | General formula (113) | General formula (113) | CN | F | General formula (113) | H | H | $CH_3O$ | H | H | H |
| 762 | General formula (113) | General formula (113) | CN | F | General formula (113) | H | H | t-$C_4H_9$ | H | H | H |
| 763 | General formula (113) | General formula (113) | CN | F | General formula (113) | H | H | Cl | H | H | H |
| 764 | General formula (113) | General formula (113) | CN | F | General formula (113) | H | H | F | H | H | H |
| 765 | General formula (113) | General formula (113) | CN | $CH_3O$ | General formula (113) | H | H | H | H | H | H |
| 766 | General formula (113) | General formula (113) | CN | $CH_3O$ | General formula (113) | H | H | $CH_3$ | H | H | H |
| 767 | General formula (113) | General formula (113) | CN | $CH_3O$ | General formula (113) | H | H | $CH_3O$ | H | H | H |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 768 | General formula (113) | General formula (113) | CN | $CH_3O$ | General formula (113) | H | H | t-$C_4H_9$ | H | H | H |
| 769 | General formula (113) | General formula (113) | CN | $CH_3O$ | General formula (113) | H | H | Cl | H | H | H |
| 770 | General formula (113) | General formula (113) | CN | $CH_3O$ | General formula (113) | H | H | F | H | H | H |
| 771 | General formula (113) | General formula (113) | CN | $C_2H_5O$ | General formula (113) | H | H | H | H | H | H |
| 772 | General formula (113) | General formula (113) | CN | $C_2H_5O$ | General formula (113) | H | H | $CH_3$ | H | H | H |
| 773 | General formula (113) | General formula (113) | CN | $C_2H_5O$ | General formula (113) | H | H | $CH_3O$ | H | H | H |
| 774 | General formula (113) | General formula (113) | CN | $C_2H_5O$ | General formula (113) | H | H | t-$C_4H_9$ | H | H | H |
| 775 | General formula (113) | General formula (113) | CN | $C_2H_5O$ | General formula (113) | H | H | Cl | H | H | H |
| 776 | General formula (113) | General formula (113) | CN | $C_2H_5O$ | General formula (113) | H | H | F | H | H | H |
| 777 | General formula (113) | General formula (113) | CN | $C_6H_5O$ | General formula (113) | H | H | H | H | H | H |
| 778 | General formula (113) | General formula (113) | CN | $C_6H_5O$ | General formula (113) | H | H | $CH_3$ | H | H | H |
| 779 | General formula (113) | General formula (113) | CN | $C_6H_5O$ | General formula (113) | H | H | $CH_3O$ | H | H | H |
| 780 | General formula (113) | General formula (113) | CN | $C_6H_5O$ | General formula (113) | H | H | t-$C_4H_9$ | H | H | H |
| 781 | General formula (113) | General formula (113) | CN | $C_6H_5O$ | General formula (113) | H | H | Cl | H | H | H |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 782 | General formula (113) | General formula (113) | CN | CeHsO | General formula (113) | H | H | F | H | H | H |
| 783 | General formula (113) | General formula (113) | CN | Formula (121) | General formula (113) | H | H | H | H | H | H |
| 784 | General formula (113) | General formula (113) | CN | Formula (121) | General formula (113) | H | H | $CH_3$ | H | H | H |
| 785 | General formula (113) | General formula (113) | CN | Formula (121) | General formula (113) | H | H | $CH_3O$ | H | H | H |
| 786 | General formula (113) | General formula (113) | CN | Formula (121) | General formula (113) | H | H | t-$C_4H_9$ | H | H | H |
| 787 | General formula (113) | General formula (113) | CN | Formula (121) | General formula (113) | H | H | Cl | H | H | H |
| 788 | General formula (113) | General formula (113) | CN | Formula (121) | General formula (113) | H | H | F | H | H | H |
| 789 | General formula (113) | General formula (113) | CN | Formula (122) | General formula (113) | H | H | H | H | H | H |
| 790 | General formula (113) | General formula (113) | CN | Formula (122) | General formula (113) | H | H | $CH_3$ | H | H | H |
| 791 | General formula (113) | General formula (113) | CN | Formula (122) | General formula (113) | H | H | $CH_3O$ | H | H | H |
| 792 | General formula (113) | General formula (113) | CN | Formula (122) | General formula (113) | H | H | t-$C_4H_9$ | H | H | H |
| 793 | General formula (113) | General formula (113) | CN | Formula (122) | General formula (113) | H | H | Cl | H | H | H |
| 794 | General formula (113) | General formula (113) | CN | Formula (122) | General formula (113) | H | H | F | H | H | H |
| 795 | General formula (113) | General formula (113) | CN | Formula (123) | General formula (113) | H | H | H | H | H | H |

(continued)

| Compound No. | General formula (1) | | | | | General formula (112) | | | | | |
|---|---|---|---|---|---|---|---|---|---|---|---|
| | $R^1$ | $R^2$ | $R^3$ | $R^4$ | $R^5$ | $R^{41}$ | $R^{42}$ | $R^{43}$ | $R^{44}$ | $R^{45}$ | $R^{46}$ |
| 796 | General formula (113) | General formula (113) | CN | Formula (123) | General formula (113) | H | H | $CH_3$ | H | H | H |
| 797 | General formula (113) | General formula (113) | CN | Formula (123) | General formula (113) | H | H | $CH_3O$ | H | H | H |
| 798 | General formula (113) | General formula (113) | CN | Formula (123) | General formula (113) | H | H | t-$C_4H_9$ | H | H | H |
| 799 | General formula (113) | General formula (113) | CN | Formula (123) | General formula (113) | H | H | Cl | H | H | H |
| 800 | General formula (113) | General formula (113) | CN | Formula (123) | General formula (113) | H | H | F | H | H | H |
| 801 | General formula (113) | General formula (113) | CN | Formula (124) | General formula (113) | H | H | H | H | H | H |
| 802 | General formula (113) | General formula (113) | CN | Formula (124) | General formula (113) | H | H | $CH_3$ | H | H | H |
| 803 | General formula (113) | General formula (113) | CN | Formula (124) | General formula (113) | H | H | $CH_3O$ | H | H | H |
| 804 | General formula (113) | General formula (113) | CN | Formula (124) | General formula (113) | H | H | t-$C_4H_9$ | H | H | H |
| 805 | General formula (113) | General formula (113) | CN | Formula (124) | General formula (113) | H | H | Cl | H | H | H |
| 806 | General formula (113) | General formula (113) | CN | Formula (124) | General formula (113) | H | H | F | H | H | H |

Table 5

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 901 | General formula (114) | General formula (114) | CN | General formula (114) | General formula (114) | H | H | H | H | H | H | H | H |
| 902 | General formula (114) | General formula (114) | CN | General formula (114) | General formula (114) | $CH_3$ | H | H | H | H | H | H | H |
| 903 | General formula (114) | General formula (114) | CN | General formula (114) | General formula (114) | $CH_3O$ | H | H | H | H | H | H | H |
| 904 | General formula (114) | General formula (114) | CN | General formula (114) | General formula (114) | H | $CH_3$ | H | H | H | H | H | H |
| 905 | General formula (114) | General formula (114) | CN | General formula (114) | General formula (114) | H | $CH_3O$ | H | H | H | H | H | H |
| 906 | General formula (114) | General formula (114) | CN | General formula (114) | General formula (114) | H | t-$C_4H_9$ | H | H | H | H | H | H |
| 907 | General formula (114) | General formula (114) | CN | General formula (114) | General formula (114) | H | Cl | H | H | H | H | H | H |
| 908 | General formula (114) | General formula (114) | CN | General formula (114) | General formula (114) | H | F | H | H | H | H | H | H |
| 909 | General formula (114) | General formula (114) | CN | General formula (114) | General formula (114) | H | H | $CH_3$ | H | H | H | H | H |

(continued)

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 910 | General formula (114) | General formula (114) | CN | General formula (114) | General formula (114) | H | H | $CH_3O$ | H | H | H | H | H |
| 911 | General formula (114) | General formula (114) | CN | General formula (114) | General formula (114) | H | H | H | $CH_3$ | H | H | H | H |
| 912 | General formula (114) | General formula (114) | CN | General formula (114) | General formula (114) | H | H | H | $CH_3O$ | H | H | H | H |
| 913 | General formula (114) | General formula (114) | CN | General formula (114) | General formula (114) | H | H | H | H | $CH_3$ | H | H | H |
| 914 | General formula (114) | General formula (114) | CN | General formula (114) | General formula (114) | H | H | H | H | $CH_3O$ | H | H | H |
| 915 | General formula (114) | General formula (114) | CN | General formula (114) | General formula (114) | H | H | H | H | H | $CH_3$ | H | H |
| 916 | General formula (114) | General formula (114) | CN | General formula (114) | General formula (114) | H | H | H | H | H | $CH_3O$ | H | H |
| 917 | General formula (114) | General formula (114) | CN | General formula (114) | General formula (114) | H | H | H | H | H | H | $CH_3$ | H |
| 918 | General formula (114) | General formula (114) | CN | General formula (114) | General formula (114) | H | H | H | H | H | H | $CH_3O$ | H |

(continued)

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 919 | General formula (114) | General formula (114) | CN | General formula (114) | H | H | H | H | H | H | H | H | H |
| 920 | General formula (114) | General formula (114) | CN | H | General formula (114) | H | H | H | H | H | H | H | H |
| 921 | General formula (114) | General formula (114) | CN | H | H | H | H | H | H | H | H | H | H |
| 922 | General formula (114) | H | CN | General formula (114) | H | H | H | H | H | H | H | H | H |
| 923 | H | General formula (114) | CN | General formula (114) | H | H | H | H | H | H | H | H | H |
| 924 | General formula (114) | H | CN | H | H | H | H | H | H | H | H | H | H |
| 925 | General formula (114) | General formula (114) | CN | General formula (114) | F | H | H | H | H | H | H | H | H |
| 926 | General formula (114) | General formula (114) | CN | F | General formula (114) | H | H | H | H | H | H | H | H |
| 927 | General formula (114) | General formula (114) | CN | F | F | H | H | H | H | H | H | H | H |
| 928 | General formula (114) | F | CN | General formula (114) | F | H | H | H | H | H | H | H | H |
| 929 | F | General formula (114) | CN | General formula (114) | F | H | H | H | H | H | H | H | H |

(continued)

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 930 | General formula (114) | F | CN | F | F | H | H | H | H | H | H | H | H |
| 931 | General formula (114) | General formula (114) | CN | General formula (114) | OH | H | H | H | H | H | H | H | H |
| 932 | General formula (114) | General formula (114) | CN | OH | General formula (114) | H | H | H | H | H | H | H | H |
| 933 | General formula (114) | General formula (114) | CN | OH | OH | H | H | H | H | H | H | H | H |
| 934 | General formula (114) | OH | CN | General formula (114) | OH | H | H | H | H | H | H | H | H |
| 935 | OH | General formula (114) | CN | General formula (114) | OH | H | H | H | H | H | H | H | H |
| 936 | General formula (114) | OH | CN | OH | OH | H | H | H | H | H | H | H | H |
| 937 | General formula (114) | General formula (114) | CN | Cl | General formula (114) | H | H | H | H | H | H | H | H |
| 938 | General formula (114) | General formula (114) | CN | Cl | General formula (114) | H | $CH_3$ | H | H | H | H | H | H |
| 939 | General formula (114) | General formula (114) | CN | Cl | General formula (114) | H | $CH_3O$ | H | H | H | H | H | H |
| 940 | General formula (114) | General formula (114) | CN | Cl | General formula (114) | H | t-$C_4H_9$ | H | H | H | H | H | H |
| 941 | General formula (114) | General formula (114) | CN | Cl | General formula (114) | H | Cl | H | H | H | H | H | H |

(continued)

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 942 | General formula (114) | General formula (114) | CN | Cl | General formula (114) | H | F | H | H | H | H | H | H |
| 943 | General formula (114) | General formula (114) | CN | F | General formula (114) | H | H | H | H | H | H | H | H |
| 944 | General formula (114) | General formula (114) | CN | F | General formula (114) | H | $CH_3$ | H | H | H | H | H | H |
| 945 | General formula (114) | General formula (114) | CN | F | General formula (114) | H | $CH_3O$ | H | H | H | H | H | H |
| 946 | General formula (114) | General formula (114) | CN | F | General formula (114) | H | t-$C_4H_9$ | H | H | H | H | H | H |
| 947 | General formula (114) | General formula (114) | CN | F | General formula (114) | H | Cl | H | H | H | H | H | H |
| 948 | General formula (114) | General formula (114) | CN | F | General formula (114) | H | F | H | H | H | H | H | H |
| 949 | General formula (114) | General formula (114) | CN | $CH_3O$ | General formula (114) | H | H | H | H | H | H | H | H |
| 950 | General formula (114) | General formula (114) | CN | $CH_3O$ | General formula (114) | H | $CH_3$ | H | H | H | H | H | H |
| 951 | General formula (114) | General formula (114) | CN | $CH_3O$ | General formula (114) | H | $CH_3O$ | H | H | H | H | H | H |
| 952 | General formula (114) | General formula (114) | CN | $CH_3O$ | General formula (114) | H | t-$C_4H_9$ | H | H | H | H | H | H |
| 953 | General formula (114) | General formula (114) | CN | $CH_3O$ | General formula (114) | H | Cl | H | H | H | H | H | H |
| 954 | General formula (114) | General formula (114) | CN | $CH_3O$ | General formula (114) | H | F | H | H | H | H | H | H |

(continued)

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 955 | General formula (114) | General formula (114) | CN | $C_2H_5O$ | General formula (114) | H | H | H | H | H | H | H | H |
| 956 | General formula (114) | General formula (114) | CN | $C_2H_5O$ | General formula (114) | H | $CH_3$ | H | H | H | H | H | H |
| 957 | General formula (114) | General formula (114) | CN | $C_2H_5O$ | General formula (114) | H | $CH_3O$ | H | H | H | H | H | H |
| 958 | General formula (114) | General formula (114) | CN | $C_2H_5O$ | General formula (114) | H | t-$C_4H_9$ | H | H | H | H | H | H |
| 959 | General formula (114) | General formula (114) | CN | $C_2H_5O$ | General formula (114) | H | Cl | H | H | H | H | H | H |
| 960 | General formula (114) | General formula (114) | CN | $C_2H_5O$ | General formula (114) | H | F | H | H | H | H | H | H |
| 961 | General formula (114) | General formula (114) | CN | $C_6H_5O$ | General formula (114) | H | H | H | H | H | H | H | H |
| 962 | General formula (114) | General formula (114) | CN | $C_6H_5O$ | General formula (114) | H | $CH_3$ | H | H | H | H | H | H |
| 963 | General formula (114) | General formula (114) | CN | $C_6H_5O$ | General formula (114) | H | $CH_3O$ | H | H | H | H | H | H |
| 964 | General formula (114) | General formula (114) | CN | $C_6H_5O$ | General formula (114) | H | t-$C_4H_9$ | H | H | H | H | H | H |
| 965 | General formula (114) | General formula (114) | CN | $C_6H_5O$ | General formula (114) | H | Cl | H | H | H | H | H | H |
| 966 | General formula (114) | General formula (114) | CN | $C_6H_5O$ | General formula (114) | H | F | H | H | H | H | H | H |
| 967 | General formula (114) | General formula (114) | CN | Formula (121) | General formula (114) | H | H | H | H | H | H | H | H |

(continued)

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 968 | General formula (114) | General formula (114) | CN | Formula (121) | General formula (114) | H | $CH_3$ | H | H | H | H | H | H |
| 969 | General formula (114) | General formula (114) | CN | Formula (121) | General formula (114) | H | $CH_3O$ | H | H | H | H | H | H |
| 970 | General formula (114) | General formula (114) | CN | Formula (121) | General formula (114) | H | t-$C_4H_9$ | H | H | H | H | H | H |
| 971 | General formula (114) | General formula (114) | CN | Formula (121) | General formula (114) | H | Cl | H | H | H | H | H | H |
| 972 | General formula (114) | General formula (114) | CN | Formula (121) | General formula (114) | H | F | H | H | H | H | H | H |
| 973 | General formula (114) | General formula (114) | CN | Formula (122) | General formula (114) | H | H | H | H | H | H | H | H |
| 974 | General formula (114) | General formula (114) | CN | Formula (122) | General formula (114) | H | $CH_3$ | H | H | H | H | H | H |
| 975 | General formula (114) | General formula (114) | CN | Formula (122) | General formula (114) | H | $CH_3O$ | H | H | H | H | H | H |
| 976 | General formula (114) | General formula (114) | CN | Formula (122) | General formula (114) | H | t-$C_4H_9$ | H | H | H | H | H | H |
| 977 | General formula (114) | General formula (114) | CN | Formula (122) | General formula (114) | H | Cl | H | H | H | H | H | H |
| 978 | General formula (114) | General formula (114) | CN | Formula (122) | General formula (114) | H | F | H | H | H | H | H | H |
| 989 | General formula (114) | General formula (114) | CN | Formula (123) | General formula (114) | H | H | H | H | H | H | H | H |
| 980 | General formula (114) | General formula (114) | CN | Formula (123) | General formula (114) | H | $CH_3$ | H | H | H | H | H | H |

(continued)

| Compound No. | General formula (1) | | | | | General formula (114) | | | | | | | |
|---|---|---|---|---|---|---|---|---|---|---|---|---|---|
| | $R^1$ | $R^2$ | $R^3$ | $R^4$ | $R^5$ | $R^{52}$ | $R^{53}$ | $R^{54}$ | $R^{55}$ | $R^{57}$ | $R^{59}$ | $R^{61}$ | $R^{51}$, $R^{56}$, $R^{58}$, $R^{60}$, $R^{62}$ |
| 981 | General formula (114) | General formula (114) | CN | Formula (123) | General formula (114) | H | $CH_3O$ | H | H | H | H | H | H |
| 982 | General formula (114) | General formula (114) | CN | Formula (123) | General formula (114) | H | t-$C_4H_9$ | H | H | H | H | H | H |
| 983 | General formula (114) | General formula (114) | CN | Formula (123) | General formula (114) | H | Cl | H | H | H | H | H | H |
| 984 | General formula (114) | General formula (114) | CN | Formula (123) | General formula (114) | H | F | H | H | H | H | H | H |
| 985 | General formula (114) | General formula (114) | CN | Formula (124) | General formula (114) | H | H | H | H | H | H | H | H |
| 986 | General formula (114) | General formula (114) | CN | Formula (124) | General formula (114) | H | $CH_3$ | H | H | H | H | H | H |
| 987 | General formula (114) | General formula (114) | CN | Formula (124) | General formula (114) | H | $CH_3O$ | H | H | H | H | H | H |
| 988 | General formula (114) | General formula (114) | CN | Formula (124) | General formula (114) | H | t-$C_4H_9$ | H | H | H | H | H | H |
| 989 | General formula (114) | General formula (114) | CN | Formula (124) | General formula (114) | H | Cl | H | H | H | H | H | H |
| 990 | General formula (114) | General formula (114) | CN | Formula (124) | General formula (114) | H | F | H | H | H | H | H | H |

Table 6

| Compound No. | General formula (1) | | | | | General formula (115) | | | | |
|---|---|---|---|---|---|---|---|---|---|---|
| | $R^1$ | $R^2$ | $R^3$ | $R^4$ | $R^5$ | $R^{71}$, $R^{80}$ | $R^{72}$, $R^{79}$ | $R^{73}$, $R^{78}$ | $R^{74}$, $R^{77}$ | $R^{75}$, $R^{76}$ |
| 1001 | General formula (115) | General formula (115) | CN | General formula (115) | General formula (115) | H | H | H | H | H |
| 1002 | General formula (115) | General formula (115) | CN | General formula (115) | General formula (115) | H | $CH_3$ | H | H | H |
| 1003 | General formula (115) | General formula (115) | CN | General formula (115) | General formula (115) | H | $CH_3O$ | H | H | H |
| 1004 | General formula (115) | General formula (115) | CN | General formula (115) | General formula (115) | H | $C_6H_5$ | H | H | H |
| 1005 | General formula (115) | General formula (115) | CN | General formula (115) | General formula (115) | H | $CH_3$ | H | $CH_3$ | H |
| 1006 | General formula (115) | General formula (115) | CN | General formula (115) | General formula (115) | H | $CH_3O$ | H | $CH_3O$ | H |
| 1007 | General formula (115) | General formula (115) | CN | General formula (115) | General formula (115) | H | $C_6H_5$ | H | $C_6H_5$ | H |
| 1008 | General formula (115) | General formula (115) | CN | General formula (115) | General formula (115) | H | H | $CH_3$ | H | H |
| 1009 | General formula (115) | General formula (115) | CN | General formula (115) | General formula (115) | H | H | $CH_3O$ | H | H |
| 1010 | General formula (115) | General formula (115) | CN | General formula (115) | General formula (115) | H | H | t-$C_4H_9$ | H | H |
| 1011 | General formula (115) | General formula (115) | CN | General formula (115) | General formula (115) | H | H | Cl | H | H |
| 1012 | General formula (115) | General formula (115) | CN | General formula (115) | General formula (115) | H | H | F | H | H |
| 1013 | General formula (115) | General formula (115) | CN | General formula (115) | General formula (115) | H | H | $C_6H_5$ | H | H |
| 1014 | General formula (115) | General formula (115) | CN | General formula (115) | General formula (115) | H | H | p-$C_6H_5$-$C_6H_4$ | H | H |

(continued)

| Compound No. | General formula (1) | | | | | General formula (115) | | | | |
|---|---|---|---|---|---|---|---|---|---|---|
| | $R^1$ | $R^2$ | $R^3$ | $R^4$ | $R^5$ | $R^{71}$, $R^{80}$ | $R^{72}$, $R^{79}$ | $R^{73}$, $R^{78}$ | $R^{74}$, $R^{77}$ | $R^{75}$, $R^{76}$ |
| 1015 | General formula (115) | General formula (115) | CN | General formula (115) | H | H | H | H | H | H |
| 1016 | General formula (115) | General formula (115) | CN | H | General formula (115) | H | H | H | H | H |
| 1017 | General formula (115) | General formula (115) | CN | H | H | H | H | H | H | H |
| 1018 | General formula (115) | H | CN | General formula (115) | H | H | H | H | H | H |
| 1019 | H | General formula (115) | CN | General formula (115) | H | H | H | H | H | H |
| 1020 | General formula (115) | H | CN | H | H | H | H | H | H | H |
| 1021 | General formula (115) | General formula (115) | CN | General formula (115) | F | H | H | H | H | H |
| 1022 | General formula (115) | General formula (115) | CN | F | General formula (115) | H | H | H | H | H |
| 1023 | General formula (115) | General formula (115) | CN | F | F | H | H | H | H | H |
| 1024 | General formula (115) | F | CN | General formula (115) | F | H | H | H | H | H |
| 1025 | F | General formula (115) | CN | General formula (115) | F | H | H | H | H | H |
| 1026 | General formula (115) | F | CN | F | F | H | H | H | H | H |
| 1027 | General formula (115) | General formula (115) | CN | General formula (115) | OH | H | H | H | H | H |
| 1028 | General formula (115) | General formula (115) | CN | OH | General formula (115) | H | H | H | H | H |

(continued)

| Compound No. | General formula (1) | | | | | General formula (115) | | | | |
|---|---|---|---|---|---|---|---|---|---|---|
| | $R^1$ | $R^2$ | $R^3$ | $R^4$ | $R^5$ | $R^{71}$, $R^{80}$ | $R^{72}$, $R^{79}$ | $R^{73}$, $R^{78}$ | $R^{74}$, $R^{77}$ | $R^{75}$, $R^{76}$ |
| 1029 | General formula (115) | General formula (115) | CN | OH | OH | H | H | H | H | H |
| 1030 | General formula (115) | OH | CN | General formula (115) | OH | H | H | H | H | H |
| 1031 | OH | General formula (115) | CN | General formula (115) | OH | H | H | H | H | H |
| 1032 | General formula (115) | OH | CN | OH | OH | H | H | H | H | H |
| 1033 | General formula (115) | General formula (115) | CN | Cl | General formula (115) | H | H | H | H | H |
| 1034 | General formula (115) | General formula (115) | CN | Cl | General formula (115) | H | H | $CH_3$ | H | H |
| 1035 | General formula (115) | General formula (115) | CN | Cl | General formula (115) | H | H | $CH_3O$ | H | H |
| 1036 | General formula (115) | General formula (115) | CN | Cl | General formula (115) | H | H | t-$C_4H_9$ | H | H |
| 1037 | General formula (115) | General formula (115) | CN | Cl | General formula (115) | H | H | Cl | H | H |
| 1038 | General formula (115) | General formula (115) | CN | Cl | General formula (115) | H | H | F | H | H |
| 1039 | General formula (115) | General formula (115) | CN | F | General formula (115) | H | H | H | H | H |
| 1040 | General formula (115) | General formula (115) | CN | F | General formula (115) | H | H | $CH_3$ | H | H |
| 1041 | General formula (115) | General formula (115) | CN | F | General formula (115) | H | H | $CH_3O$ | H | H |
| 1042 | General formula (115) | General formula (115) | CN | F | General formula (115) | H | H | t-$C_4H_9$ | H | H |

(continued)

| Compound No. | General formula (1) | | | | | General formula (115) | | | | |
|---|---|---|---|---|---|---|---|---|---|---|
| | $R^1$ | $R^2$ | $R^3$ | $R^4$ | $R^5$ | $R^{71}$, $R^{80}$ | $R^{72}$, $R^{79}$ | $R^{73}$, $R^{78}$ | $R^{74}$, $R^{77}$ | $R^{75}$, $R^{76}$ |
| 1043 | General formula (115) | General formula (115) | CN | F | General formula (115) | H | H | Cl | H | H |
| 1044 | General formula (115) | General formula (115) | CN | F | General formula (115) | H | H | F | H | H |
| 1045 | General formula (115) | General formula (115) | CN | $CH_3O$ | General formula (115) | H | H | H | H | H |
| 1046 | General formula (115) | General formula (115) | CN | $CH_3O$ | General formula (115) | H | H | $CH_3$ | H | H |
| 1047 | General formula (115) | General formula (115) | CN | $CH_3O$ | General formula (115) | H | H | $CH_3O$ | H | H |
| 1048 | General formula (115) | General formula (115) | CN | $CH_3O$ | General formula (115) | H | H | t-$C_4H_9$ | H | H |
| 1049 | General formula (115) | General formula (115) | CN | $CH_3O$ | General formula (115) | H | H | Cl | H | H |
| 1050 | General formula (115) | General formula (115) | CN | $CH_3O$ | General formula (115) | H | H | F | H | H |
| 1051 | General formula (115) | General formula (115) | CN | $C_2H_5O$ | General formula (115) | H | H | H | H | H |
| 1052 | General formula (115) | General formula (115) | CN | $C_2H_5O$ | General formula (115) | H | H | $CH_3$ | H | H |
| 1053 | General formula (115) | General formula (115) | CN | $C_2H_5O$ | General formula (115) | H | H | $CH_3O$ | H | H |
| 1054 | General formula (115) | General formula (115) | CN | $C_2H_5O$ | General formula (115) | H | H | t-$C_4H_9$ | H | H |
| 1055 | General formula (115) | General formula (115) | CN | $C_2H_5O$ | General formula (115) | H | H | Cl | H | H |
| 1056 | General formula (115) | General formula (115) | CN | $C_2H_5O$ | General formula (115) | H | H | F | H | H |

(continued)

| Compound No. | General formula (1) | | | | | General formula (115) | | | | |
|---|---|---|---|---|---|---|---|---|---|---|
| | $R^1$ | $R^2$ | $R^3$ | $R^4$ | $R^5$ | $R^{71}$, $R^{80}$ | $R^{72}$, $R^{79}$ | $R^{73}$, $R^{78}$ | $R^{74}$, $R^{77}$ | $R^{75}$, $R^{76}$ |
| 1057 | General formula (115) | General formula (115) | CN | $C_6H_5O$ | General formula (115) | H | H | H | H | H |
| 1058 | General formula (115) | General formula (115) | CN | $C_6H_5O$ | General formula (115) | H | H | $CH_3$ | H | H |
| 1059 | General formula (115) | General formula (115) | CN | $C_6H_5O$ | General formula (115) | H | H | $CH_3O$ | H | H |
| 1060 | General formula (115) | General formula (115) | CN | $C_6H_5O$ | General formula (115) | H | H | t-$C_4H_9$ | H | H |
| 1061 | General formula (115) | General formula (115) | CN | $C_6H_5O$ | General formula (115) | H | H | Cl | H | H |
| 1062 | General formula (115) | General formula (115) | CN | $C_6H_5O$ | General formula (115) | H | H | F | H | H |
| 1063 | General formula (115) | General formula (115) | CN | Formula (121) | General formula (115) | H | H | H | H | H |
| 1064 | General formula (115) | General formula (115) | CN | Formula (121) | General formula (115) | H | H | $CH_3$ | H | H |
| 1065 | General formula (115) | General formula (115) | CN | Formula (121) | General formula (115) | H | H | $CH_3O$ | H | H |
| 1066 | General formula (115) | General formula (115) | CN | Formula (121) | General formula (115) | H | H | t-$C_4H_9$ | H | H |
| 1067 | General formula (115) | General formula (115) | CN | Formula (121) | General formula (115) | H | H | Cl | H | H |
| 1068 | General formula (115) | General formula (115) | CN | Formula (121) | General formula (115) | H | H | F | H | H |
| 1069 | General formula (115) | General formula (115) | CN | Formula (122) | General formula (115) | H | H | H | H | H |
| 1070 | General formula (115) | General formula (115) | CN | Formula (122) | General formula (115) | H | H | $CH_3$ | H | H |

(continued)

| Compound No. | General formula (1) | | | | | General formula (115) | | | | |
|---|---|---|---|---|---|---|---|---|---|---|
| | $R^1$ | $R^2$ | $R^3$ | $R^4$ | $R^5$ | $R^{71}$, $R^{80}$ | $R^{72}$, $R^{79}$ | $R^{73}$, $R^{78}$ | $R^{74}$, $R^{77}$ | $R^{75}$, $R^{76}$ |
| 1071 | General formula (115) | General formula (115) | CN | Formula (122) | General formula (115) | H | H | $CH_3O$ | H | H |
| 1072 | General formula (115) | General formula (115) | CN | Formula (122) | General formula (115) | H | H | $t\text{-}C_4H_9$ | H | H |
| 1073 | General formula (115) | General formula (115) | CN | Formula (122) | General formula (115) | H | H | Cl | H | H |
| 1074 | General formula (115) | General formula (115) | CN | Formula (122) | General formula (115) | H | H | F | H | H |
| 1075 | General formula (115) | General formula (115) | CN | Formula (123) | General formula (115) | H | H | H | H | H |
| 1076 | General formula (115) | General formula (115) | CN | Formula (123) | General formula (115) | H | H | $CH_3$ | H | H |
| 1077 | General formula (115) | General formula (115) | CN | Formula (123) | General formula (115) | H | H | $CH_3O$ | H | H |
| 1078 | General formula (115) | General formula (115) | CN | Formula (123) | General formula (115) | H | H | $t\text{-}C_4H_9$ | H | H |
| 1079 | General formula (115) | General formula (115) | CN | Formula (123) | General formula (115) | H | H | Cl | H | H |
| 1080 | General formula (115) | General formula (115) | CN | Formula (123) | General formula (115) | H | H | F | H | H |
| 1081 | General formula (115) | General formula (115) | CN | Formula (124) | General formula (115) | H | H | H | H | H |
| 1082 | General formula (115) | General formula (115) | CN | Formula (124) | General formula (115) | H | H | $CH_3$ | H | H |
| 1083 | General formula (115) | General formula (115) | CN | Formula (124) | General formula (115) | H | H | $CH_3O$ | H | H |
| 1084 | General formula (115) | General formula (115) | CN | Formula (124) | General formula (115) | H | H | $t\text{-}C_4H_9$ | H | H |

(continued)

| Compound No. | General formula (1) | | | | | General formula (115) | | | | |
|---|---|---|---|---|---|---|---|---|---|---|
| | $R^1$ | $R^2$ | $R^3$ | $R^4$ | $R^5$ | $R^{71}$, $R^{80}$ | $R^{72}$, $R^{79}$ | $R^{73}$, $R^{78}$ | $R^{74}$, $R^{77}$ | $R^{75}$, $R^{76}$ |
| 1085 | General formula (115) | General formula (115) | CN | Formula (124) | General formula (115) | H | H | Cl | H | H |
| 1086 | General formula (115) | General formula (115) | CN | Formula (124) | General formula (115) | H | H | F | H | H |

Preferred delayed fluorescent materials include the following compounds.

[1] A compound represented by the following general formula (131):

[Chem. 8]

General Formula (131)

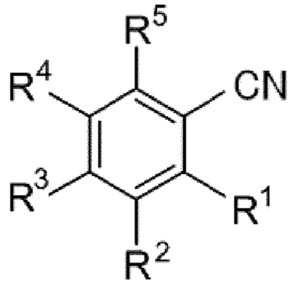

In the general formula (131), from 0 to 1 of $R^1$ to $R^5$ represents a cyano group, from 1 to 5 of $R^1$ to $R^5$ each represent a group represented by the following general formula (132), and the balance of $R^1$ to $R^5$ each represent a hydrogen atom or a substituent other than the above.

[Chem. 9]

General Formula (132)

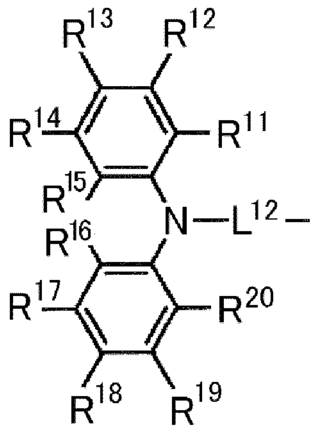

In the general formula (132), $R^{11}$ to $R^{20}$ each independently represent a hydrogen atom or a substituent. $R^{11}$ and $R^{12}$, $R^{12}$ and $R^{13}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, and $R^{19}$ and $R^{20}$ each may be bonded to each other to form a cyclic structure. $L^{12}$ represents a substituted or unsubstituted arylene group or a substituted or unsubstituted heteroarylene group.

[2] The compound according to [1], wherein the group represented by the general formula (132) is a group represented by any one of the following general formulae (133) to (138):

[Chem. 10]

General Formula (133)

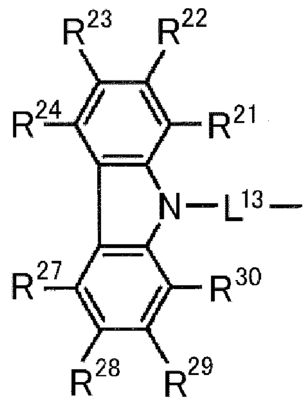

General Formula (134)

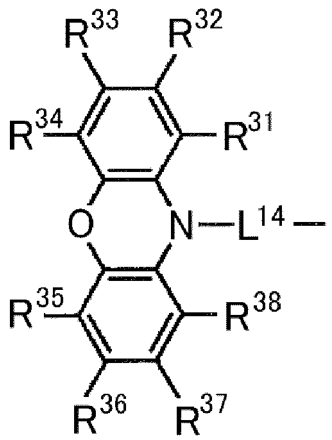

General Formula (135)

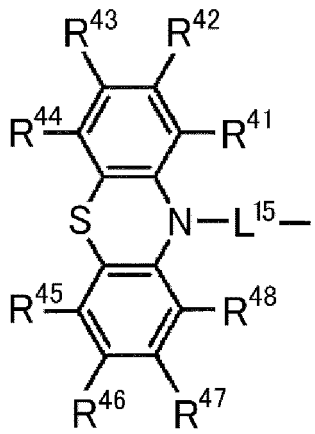

General Formula (136)

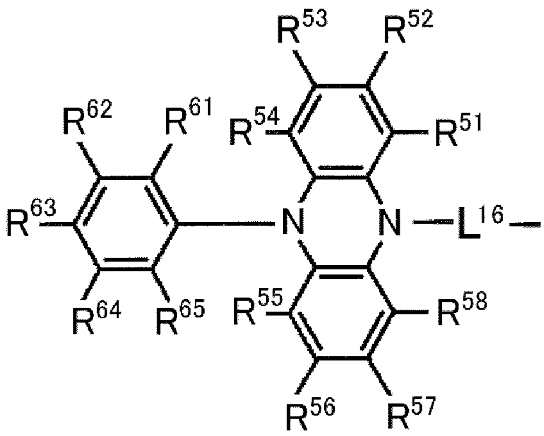

General Formula (137)

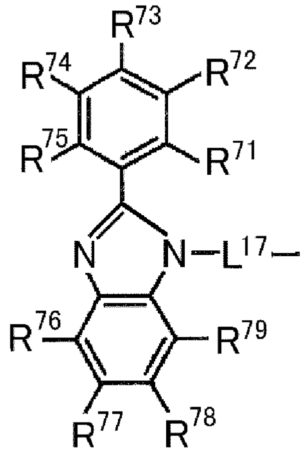

General Formula (138)

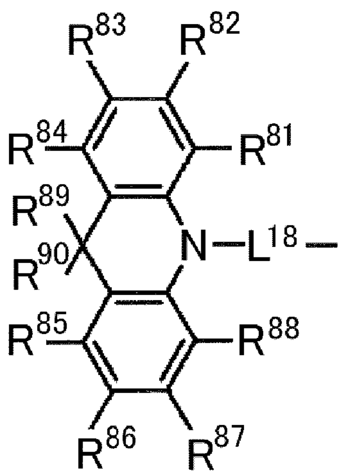

In the general formulae (133) to (138), $R^{21}$ to $R^{24}$, $R^{27}$ to $R^{38}$, $R^{41}$ to $R^{48}$, $R^{51}$ to $R^{58}$, $R^{61}$ to $R^{65}$, $R^{71}$ to $R^{79}$, $R^{81}$ to $R^{90}$ each independently represent a hydrogen atom or a substituent. $R^{21}$ and $R^{22}$, $R^{22}$ and $R^{23}$, $R^{23}$ and $R^{24}$, $R^{27}$ and $R^{28}$, $R^{28}$ and $R^{29}$, $R^{29}$ and $R^{30}$, $R^{31}$ and $R^{32}$, $R^{32}$ and $R^{33}$, $R^{33}$ and $R^{34}$, $R^{35}$ and $R^{36}$, $R^{36}$ and $R^{37}$, $R^{37}$ and $R^{38}$, $R^{41}$ and $R^{42}$, $R^{42}$ and $R^{43}$, $R^{43}$ and $R^{44}$, $R^{45}$ and $R^{46}$, $R^{46}$ and $R^{47}$, $R^{47}$ and $R^{48}$, $R^{51}$ and $R^{52}$, $R^{52}$ and $R^{53}$, $R^{53}$ and $R^{54}$, $R^{55}$ and $R^{56}$, $R^{56}$ and $R^{57}$, $R^{57}$ and $R^{58}$, $R^{61}$ and $R^{62}$, $R^{62}$ and $R^{63}$, $R^{63}$ and $R^{64}$, $R^{64}$ and $R^{65}$, $R^{54}$ and $R^{61}$, $R^{55}$ and $R^{65}$, $R^{71}$ and $R^{72}$, $R^{72}$ and $R^{73}$, $R^{73}$ and $R^{74}$, $R^{74}$ and $R^{75}$, $R^{76}$ and $R^{77}$, $R^{77}$ and $R^{78}$, $R^{78}$ and

$R^{79}$, $R^{81}$ and $R^{82}$, $R^{82}$ and $R^{83}$, $R^{83}$ and $R^{84}$, $R^{85}$ and $R^{86}$, $R^{86}$ and $R^{87}$, $R^{87}$ and $R^{88}$, and $R^{89}$ and $R^{90}$ each may be bonded to each other to form a cyclic structure. $L^{13}$ to $L^{18}$ each independently represent a substituted or unsubstituted arylene group or a substituted or unsubstituted heteroarylene group.
[3] The compound according to [1] or [2], wherein in the general formula (131), $R^3$ represents a cyano group.
[4] The compound according to any one of [1] to [3], wherein in the general formula (131), $R^1$ and $R^4$ each represent a group represented by the general formula (132).
[5] The compound according to any one of [1] to [4], wherein in the general formula (132), $L^{12}$ represents a phenylene group.
[6] The compound according to any one of [1] to [5], wherein the group represented by the general formula (132) is a group represented by the general formula (133).
[7] The compound according to [6], wherein in the general formula (133), $L^{13}$ represents a 1,3-phenylene group.
[8] The compound according to any one of [1] to [5], wherein the group represented by the general formula (132) is a group represented by the general formula (134).
[9] The compound according to [8], wherein in the general formula (134), $L^{14}$ represents a 1,4-phenylene group.
[10] The compound according to any one of [1] to [5], wherein the group represented by the general formula (132) is a group represented by the general formula (138).
[11] The compound according to [10], wherein in the general formula (132), $L^{18}$ represents a 1,4-phenylene group.

Examples of the compound include the following compounds.

[Chem. 11]

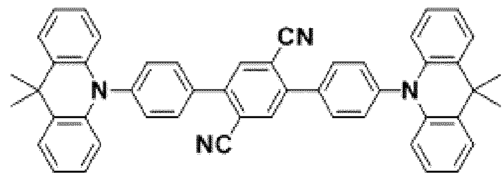

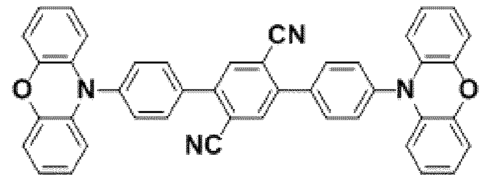

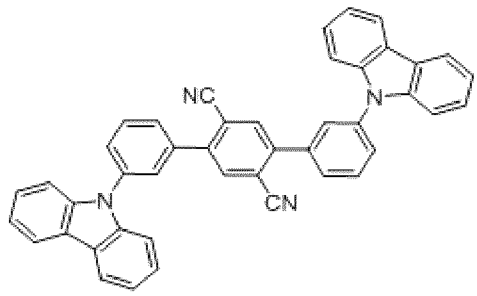

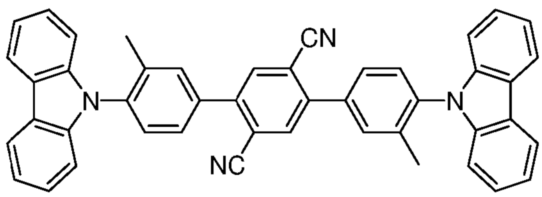

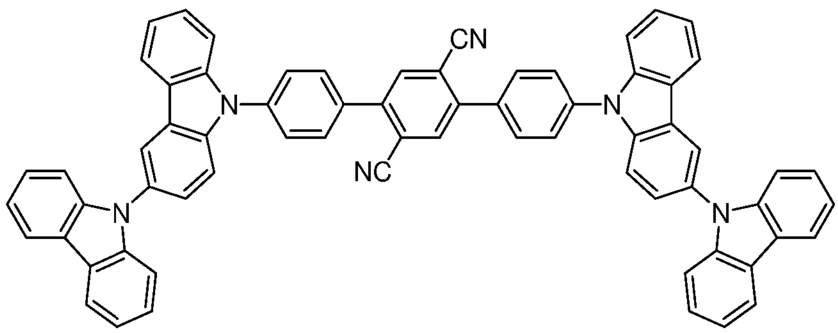

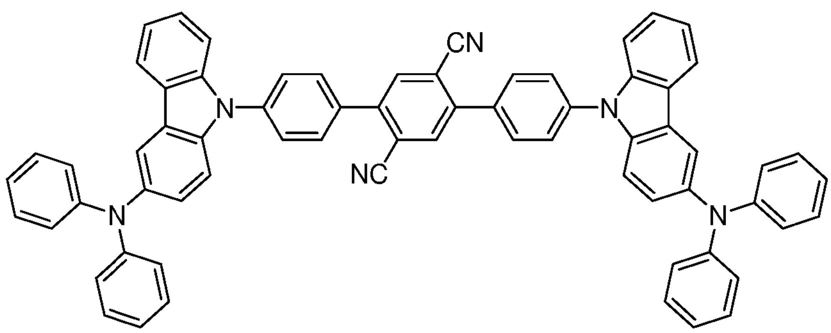

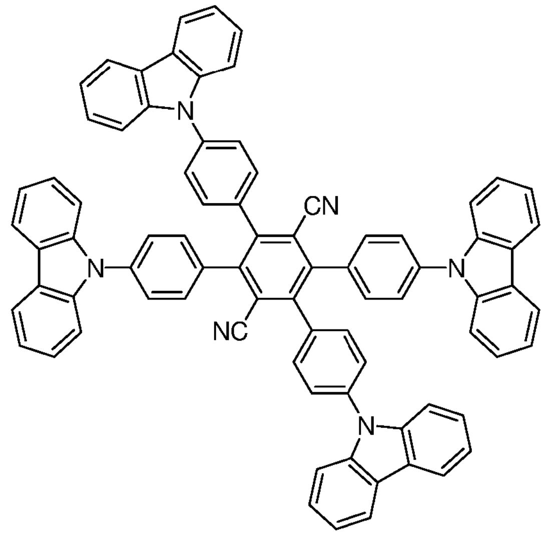

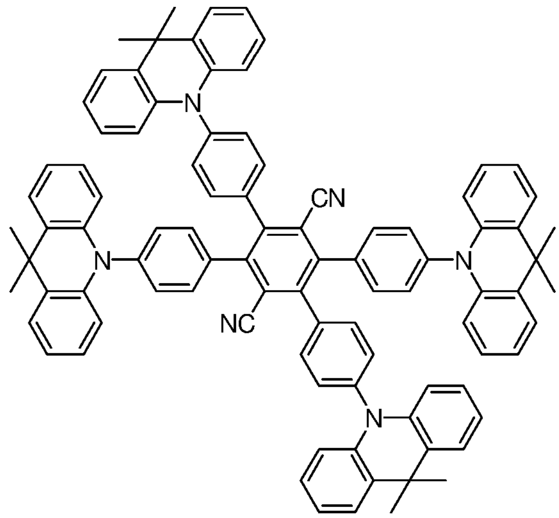

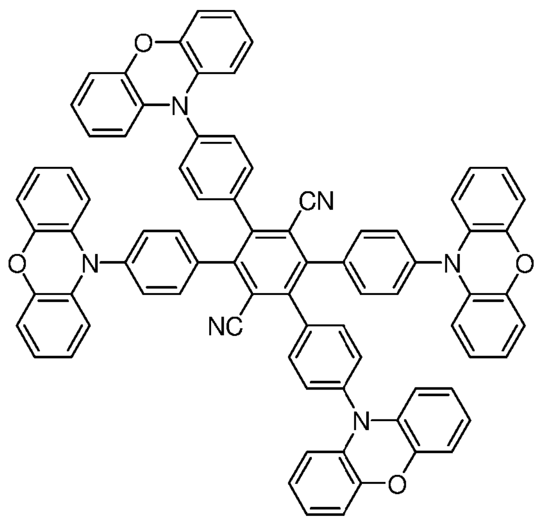

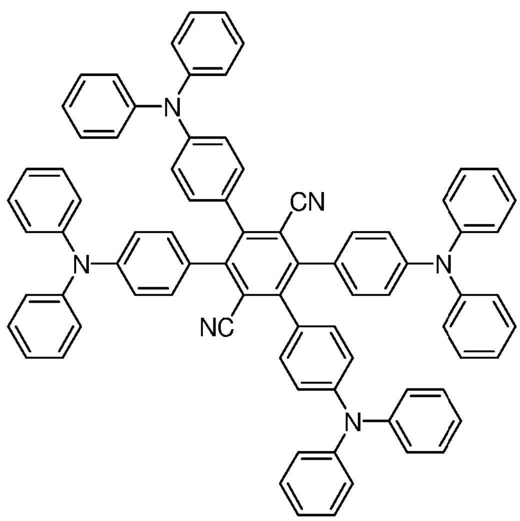

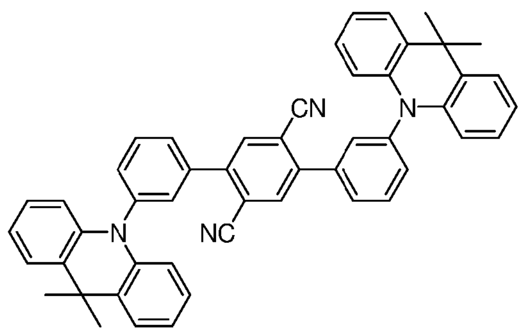

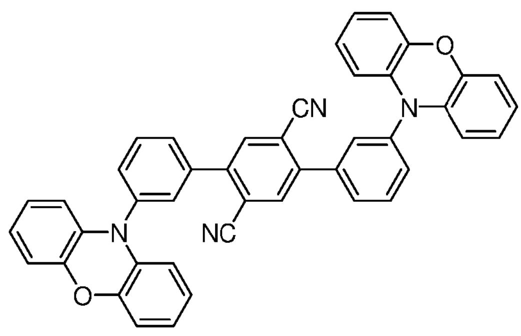

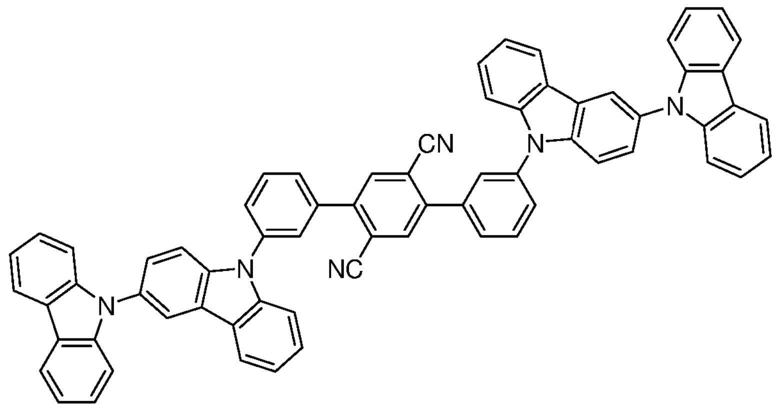

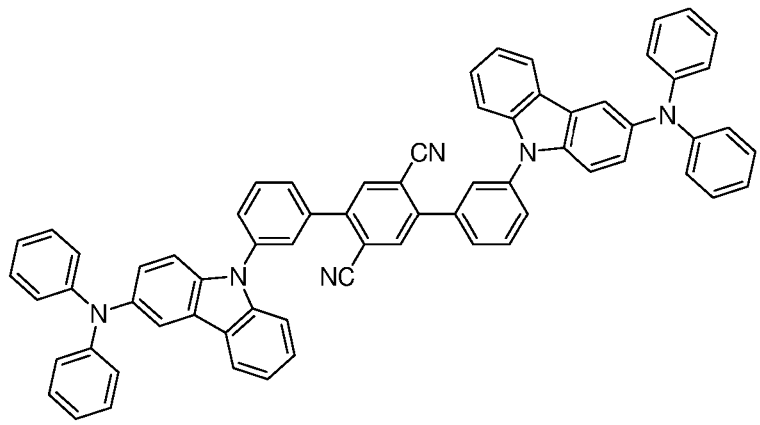

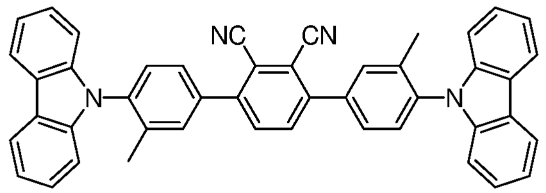

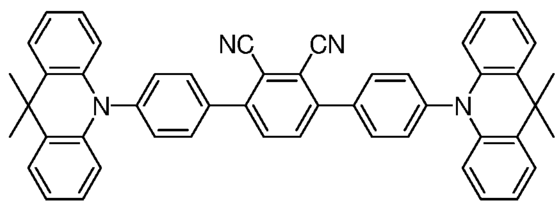

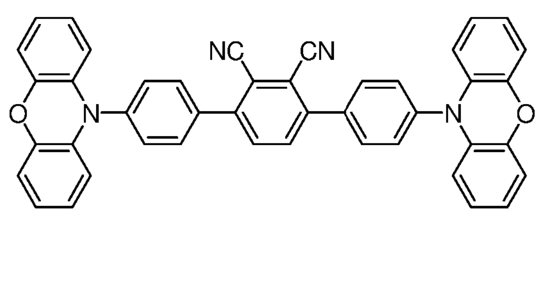

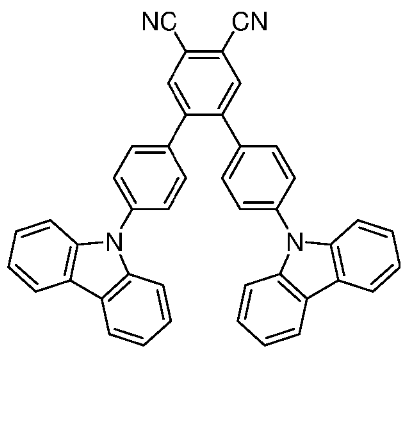

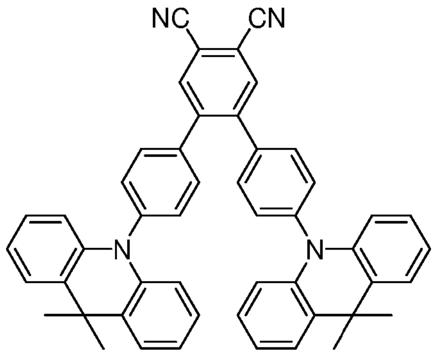

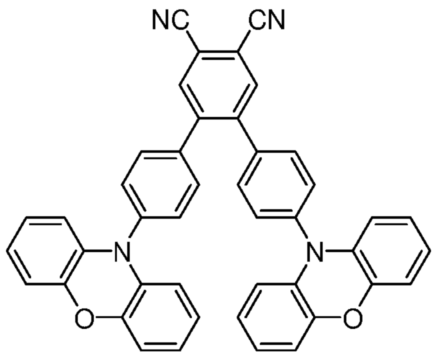

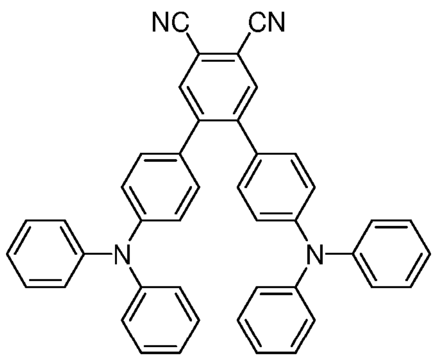

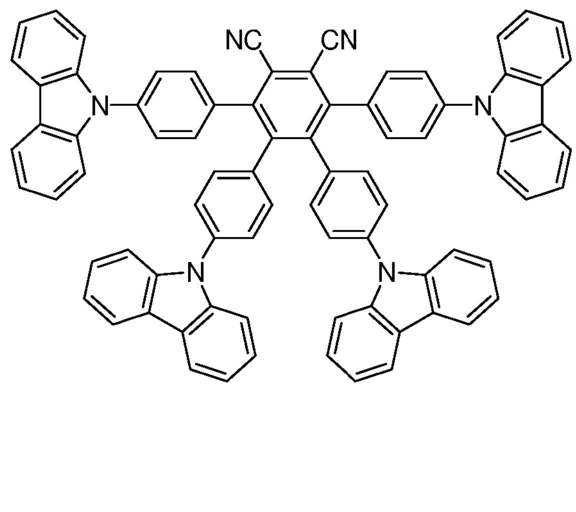

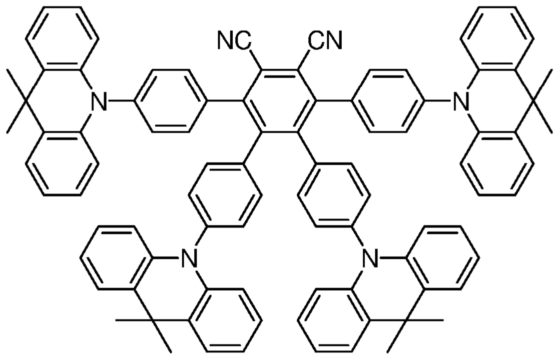

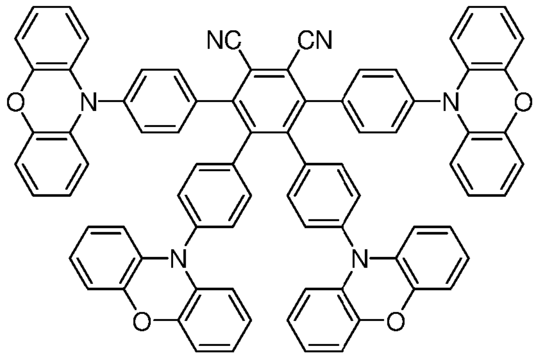

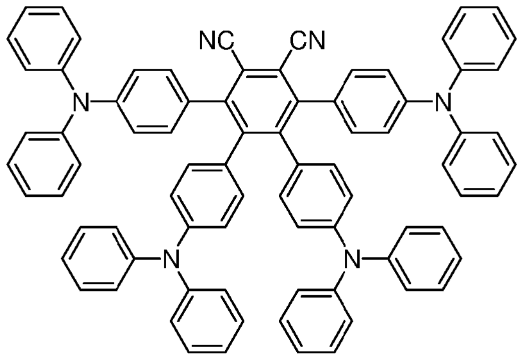

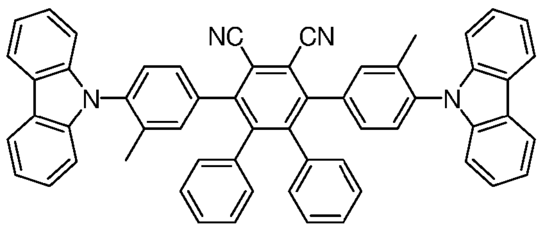

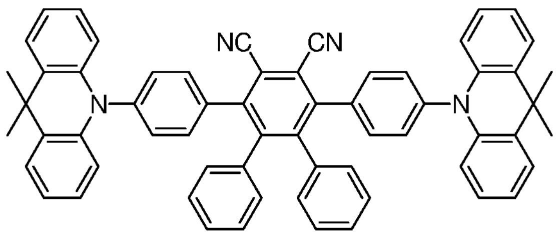

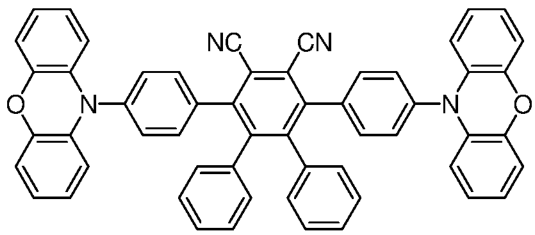

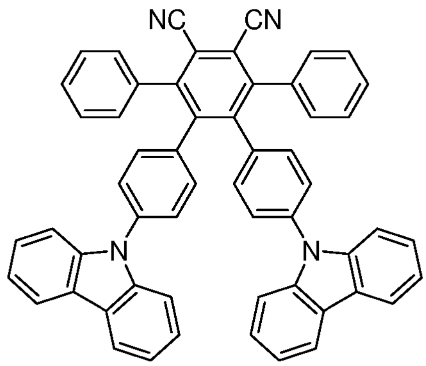
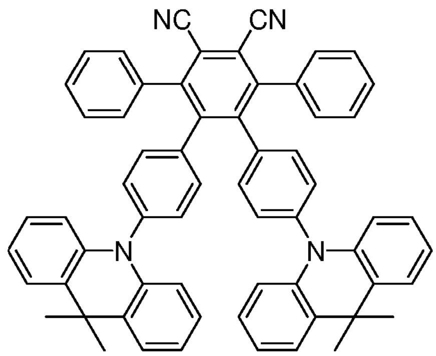
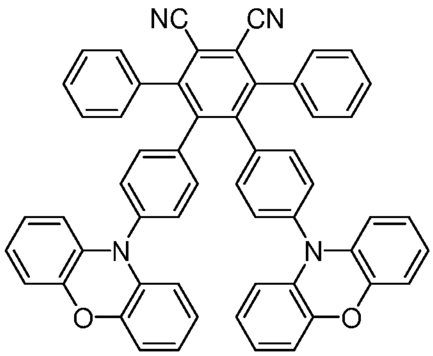
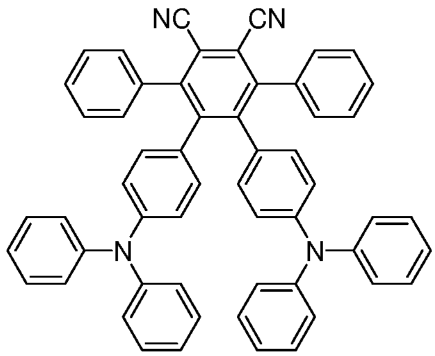

Examples of the preferred delayed fluorescent material include compounds represented by the following general formula

[Chem. 12]

General Formula (141)

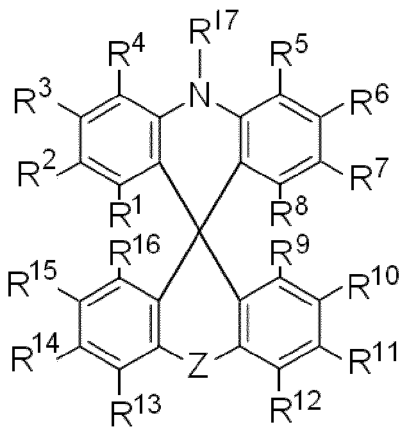

In the general formula (141), $R^1$, $R^2$, $R^3$, $R^4$, $R^5$, $R^6$, $R^7$, $R^8$ and $R^{17}$ each independently represent a hydrogen atom or an electron donating group, provided that at least one thereof represents an electron donating group. $R^9$, $R^{10}$, $R^{11}$, $R^{12}$, $R^{13}$, $R^{14}$, $R^{15}$ and $R^{16}$ each independently represent a hydrogen atom or an electron withdrawing group having no unshared electron pair at the $\alpha$-position. Z represents a single bond or >C=Y, wherein Y represents O, S, $C(CN)_2$ or $C(COOH)_2$, provided that when Z represents a single bond, at least one of $R^9$, $R^{10}$, $R^{11}$, $R^{12}$, $R^{13}$, $R^{14}$, $R^{15}$ and $R^{16}$ represents an electron withdrawing group having no unshared electron pair at the $\alpha$-position.

Specific examples of the compounds include the compounds shown in the following tables. In the tables, D1 to D3 represent the following aryl groups substituted by an electron donating group, respectively; A1 to A5 represent the following electron withdrawing groups, respectively; H represents a hydrogen atom; and Ph represents a phenyl group.

[Chem. 13]

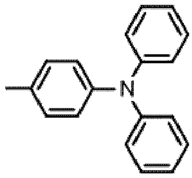

D1

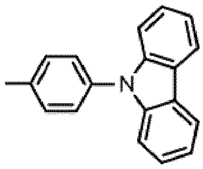

D2

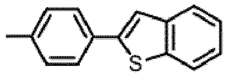

D3

—CN

A1

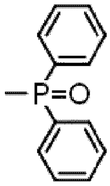

A2

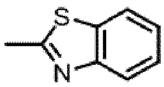

A3

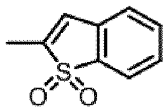

A4

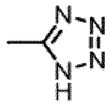

A5

Table 7

| Compound No. | $R^2$ | R7 | $R^{10}$ | $R^{15}$ | $R^{17}$ | Z | Other Rs |
|---|---|---|---|---|---|---|---|
| 2001 | H | H | A1 | A1 | Ph | single bond | H |
| 2002 | H | D1 | A1 | A1 | Ph | single bond | H |
| 2003 | H | D2 | A1 | A1 | Ph | single bond | H |
| 2004 | H | D3 | A1 | A1 | Ph | single bond | H |
| 2005 | H | H | A2 | A2 | Ph | single bond | H |
| 2006 | H | D1 | A2 | A2 | Ph | single bond | H |
| 2007 | H | D2 | A2 | A2 | Ph | single bond | H |
| 2008 | H | D3 | A2 | A2 | Ph | single bond | H |
| 2009 | H | H | A3 | A3 | Ph | single bond | H |
| 2010 | H | D1 | A3 | A3 | Ph | single bond | H |
| 2011 | H | D2 | A3 | A3 | Ph | single bond | H |
| 2012 | H | D3 | A3 | A3 | Ph | single bond | H |
| 2013 | H | H | A4 | A4 | Ph | single bond | H |
| 2014 | H | D1 | A4 | A4 | Ph | single bond | H |
| 2015 | H | D2 | A4 | A4 | Ph | single bond | H |
| 2016 | H | D3 | A4 | A4 | Ph | single bond | H |
| 2017 | H | H | A5 | A5 | Ph | single bond | H |
| 2018 | H | D1 | A5 | A5 | Ph | single bond | H |
| 2019 | H | D2 | A5 | A5 | Ph | single bond | H |
| 2020 | H | D3 | A5 | A5 | Ph | single bond | H |
| 2021 | D1 | D1 | A1 | A1 | Ph | single bond | H |

(continued)

| Compound No. | $R^2$ | R7 | $R^{10}$ | $R^{15}$ | $R^{17}$ | Z | Other Rs |
|---|---|---|---|---|---|---|---|
| 2022 | D2 | D2 | A1 | A1 | Ph | single bond | H |
| 2023 | D3 | D3 | A1 | A1 | Ph | single bond | H |
| 2024 | D1 | D1 | A2 | A2 | Ph | single bond | H |
| 2025 | D2 | D2 | A2 | A2 | Ph | single bond | H |
| 2026 | D3 | D3 | A2 | A2 | Ph | single bond | H |
| 2027 | D1 | D1 | A3 | A3 | Ph | single bond | H |
| 2028 | D2 | D2 | A3 | A3 | Ph | single bond | H |
| 2029 | D3 | D3 | A3 | A3 | Ph | single bond | H |
| 2030 | D1 | D1 | A4 | A4 | Ph | single bond | H |
| 2031 | D2 | D2 | A4 | A4 | Ph | single bond | H |
| 2032 | D3 | D3 | A4 | A4 | Ph | single bond | H |
| 2033 | D1 | D1 | A5 | A5 | Ph | single bond | H |
| 2034 | D2 | D2 | A5 | A5 | Ph | single bond | H |
| 2035 | D3 | D3 | A5 | A5 | Ph | single bond | H |

Table 8

| Compound No. | $R^3$ | $R^6$ | $R^{11}$ | $R^{14}$ | $R^{17}$ | Z | Other Rs |
|---|---|---|---|---|---|---|---|
| 2036 | H | H | H | A1 | Ph | single bond | H |
| 2037 | H | D1 | H | A1 | Ph | single bond | H |
| 2038 | H | D2 | H | A1 | Ph | single bond | H |
| 2039 | H | D3 | H | A1 | Ph | single bond | H |
| 2040 | H | H | H | A2 | Ph | single bond | H |
| 2041 | H | D1 | H | A2 | Ph | single bond | H |
| 2042 | H | D2 | H | A2 | Ph | single bond | H |
| 2043 | H | D3 | H | A2 | Ph | single bond | H |
| 2044 | H | H | H | A3 | Ph | single bond | H |
| 2045 | H | D1 | H | A3 | Ph | single bond | H |
| 2046 | H | D2 | H | A3 | Ph | single bond | H |
| 2047 | H | D3 | H | A3 | Ph | single bond | H |
| 2048 | H | H | H | A4 | Ph | single bond | H |
| 2049 | H | D1 | H | A4 | Ph | single bond | H |
| 2050 | H | D2 | H | A4 | Ph | single bond | H |
| 2051 | H | D3 | H | A4 | Ph | single bond | H |
| 2052 | H | H | H | A5 | Ph | single bond | H |
| 2053 | H | D1 | H | A5 | Ph | single bond | H |
| 2054 | H | D2 | H | A5 | Ph | single bond | H |
| 2055 | H | D3 | H | A5 | Ph | single bond | H |

(continued)

| Compound No. | $R^3$ | $R^6$ | $R^{11}$ | $R^{14}$ | $R^{17}$ | Z | Other Rs |
|---|---|---|---|---|---|---|---|
| 2056 | D1 | D1 | H | A1 | Ph | single bond | H |
| 2057 | D2 | D2 | H | A1 | Ph | single bond | H |
| 2058 | D3 | D3 | H | A1 | Ph | single bond | H |
| 2059 | D1 | D1 | H | A2 | Ph | single bond | H |
| 2060 | D2 | D2 | H | A2 | Ph | single bond | H |
| 2061 | D3 | D3 | H | A2 | Ph | single bond | H |
| 2062 | D1 | D1 | H | A3 | Ph | single bond | H |
| 2063 | D2 | D2 | H | A3 | Ph | single bond | H |
| 2064 | D3 | D3 | H | A3 | Ph | single bond | H |
| 2065 | D1 | D1 | H | A4 | Ph | single bond | H |
| 2066 | D2 | D2 | H | A4 | Ph | single bond | H |
| 2067 | D3 | D3 | H | A4 | Ph | single bond | H |
| 2068 | D1 | D1 | H | A5 | Ph | single bond | H |
| 2069 | D2 | D2 | H | A5 | Ph | single bond | H |
| 2070 | D3 | D3 | H | A5 | Ph | single bond | H |

Table 9

| Compound No. | $R^2$ | $R^7$ | $R^{10}$ | $R^{15}$ | $R^{17}$ | Z | Other Rs |
|---|---|---|---|---|---|---|---|
| 2071 | H | H | A1 | A1 | Ph | C=O | H |
| 2072 | H | D1 | A1 | A1 | Ph | C=O | H |
| 2073 | H | D2 | A1 | A1 | Ph | C=O | H |
| 2074 | H | D3 | A1 | A1 | Ph | C=O | H |
| 2075 | H | H | A2 | A2 | Ph | C=O | H |
| 2076 | H | D1 | A2 | A2 | Ph | C=O | H |
| 2077 | H | D2 | A2 | A2 | Ph | C=O | H |
| 2078 | H | D3 | A2 | A2 | Ph | C=O | H |
| 2079 | H | H | A3 | A3 | Ph | C=O | H |
| 2080 | H | D1 | A3 | A3 | Ph | C=O | H |
| 2081 | H | D2 | A3 | A3 | Ph | C=O | H |
| 2082 | H | D3 | A3 | A3 | Ph | C=O | H |
| 2083 | H | H | A4 | A4 | Ph | C=O | H |
| 2084 | H | D1 | A4 | A4 | Ph | C=O | H |
| 2085 | H | D2 | A4 | A4 | Ph | C=O | H |
| 2086 | H | D3 | A4 | A4 | Ph | C=O | H |
| 2087 | H | H | A5 | A5 | Ph | C=O | H |
| 2088 | H | D1 | A5 | A5 | Ph | C=O | H |
| 2089 | H | D2 | A5 | A5 | Ph | C=O | H |

(continued)

| Compound No. | $R^2$ | $R^7$ | $R^{10}$ | $R^{15}$ | $R^{17}$ | Z | Other Rs |
|---|---|---|---|---|---|---|---|
| 2090 | H | D3 | A5 | A5 | Ph | C=O | H |
| 2091 | D1 | D1 | A1 | A1 | Ph | C=O | H |
| 2092 | D2 | D2 | A1 | A1 | Ph | C=O | H |
| 2093 | D3 | D3 | A1 | A1 | Ph | C=O | H |
| 2094 | D1 | D1 | A2 | A2 | Ph | C=O | H |
| 2095 | D2 | D2 | A2 | A2 | Ph | C=O | H |
| 2096 | D3 | D3 | A2 | A2 | Ph | C=O | H |
| 2097 | D1 | D1 | A3 | A3 | Ph | C=O | H |
| 2098 | D2 | D2 | A3 | A3 | Ph | C=O | H |
| 2099 | D3 | D3 | A3 | A3 | Ph | C=O | H |
| 2100 | D1 | D1 | A4 | A4 | Ph | C=O | H |
| 2101 | D2 | D2 | A4 | A4 | Ph | C=O | H |
| 2102 | D3 | D3 | A4 | A4 | Ph | C=O | H |
| 2103 | D1 | D1 | A5 | A5 | Ph | C=O | H |
| 2104 | D2 | D2 | A5 | A5 | Ph | C=O | H |
| 2105 | D3 | D3 | A5 | A5 | Ph | C=O | H |

Table 10

| Compound No. | $R^3$ | $R^6$ | $R^{11}$ | $R^{14}$ | $R^{17}$ | Z | Other Rs |
|---|---|---|---|---|---|---|---|
| 2106 | H | H | H | A1 | Ph | C=O | H |
| 2107 | H | D1 | H | A1 | Ph | C=O | H |
| 2108 | H | D2 | H | A1 | Ph | C=O | H |
| 2109 | H | D3 | H | A1 | Ph | C=O | H |
| 2110 | H | H | H | A2 | Ph | C=O | H |
| 2111 | H | D1 | H | A2 | Ph | C=O | H |
| 2112 | H | D2 | H | A2 | Ph | C=O | H |
| 2113 | H | D3 | H | A2 | Ph | C=O | H |
| 2114 | H | H | H | A3 | Ph | C=O | H |
| 2115 | H | D1 | H | A3 | Ph | C=O | H |
| 2116 | H | D2 | H | A3 | Ph | C=O | H |
| 2117 | H | D3 | H | A3 | Ph | C=O | H |
| 2118 | H | H | H | A4 | Ph | C=O | H |
| 2119 | H | D1 | H | A4 | Ph | C=O | H |
| 2120 | H | D2 | H | A4 | Ph | C=O | H |
| 2121 | H | D3 | H | A4 | Ph | C=O | H |
| 2122 | H | H | H | A5 | Ph | C=O | H |
| 2123 | H | D1 | H | A5 | Ph | C=O | H |

(continued)

| Compound No. | $R^3$ | $R^6$ | $R^{11}$ | $R^{14}$ | $R^{17}$ | Z | Other Rs |
|---|---|---|---|---|---|---|---|
| 2124 | H | D2 | H | A5 | Ph | C=O | H |
| 2125 | H | D3 | H | A5 | Ph | C=O | H |
| 2126 | D1 | D1 | H | A1 | Ph | C=O | H |
| 2127 | D2 | D2 | H | A1 | Ph | C=O | H |
| 2128 | D3 | D3 | H | A1 | Ph | C=O | H |
| 2129 | D1 | D1 | H | A2 | Ph | C=O | H |
| 2130 | D2 | D2 | H | A2 | Ph | C=O | H |
| 2131 | D3 | D3 | H | A2 | Ph | C=O | H |
| 2132 | D1 | D1 | H | A3 | Ph | C=O | H |
| 2133 | D2 | D2 | H | A3 | Ph | C=O | H |
| 2134 | D3 | D3 | H | A3 | Ph | C=O | H |
| 2135 | D1 | D1 | H | A4 | Ph | C=O | H |
| 2136 | D2 | D2 | H | A4 | Ph | C=O | H |
| 2137 | D3 | D3 | H | A4 | Ph | C=O | H |
| 2138 | D1 | D1 | H | A5 | Ph | C=O | H |
| 2139 | D2 | D2 | H | A5 | Ph | C=O | H |
| 2140 | D3 | D3 | H | A5 | Ph | C=O | H |
| 2141 | H | H | H | H | Ph | C=O | H |

Table 11

| Compound No. | $R^2$ | $R^7$ | $R^{10}$ | $R^{15}$ | $R^{17}$ | Z | Other Rs |
|---|---|---|---|---|---|---|---|
| 2142 | H | H | A1 | A1 | Ph | C=S | H |
| 2143 | H | D1 | A1 | A1 | Ph | C=S | H |
| 2144 | H | D2 | A1 | A1 | Ph | C=S | H |
| 2145 | H | D3 | A1 | A1 | Ph | C=S | H |
| 2146 | H | H | A2 | A2 | Ph | C=S | H |
| 2147 | H | D1 | A2 | A2 | Ph | C=S | H |
| 2148 | H | D2 | A2 | A2 | Ph | C=S | H |
| 2149 | H | D3 | A2 | A2 | Ph | C=S | H |
| 2150 | H | H | A3 | A3 | Ph | C=S | H |
| 2151 | H | D1 | A3 | A3 | Ph | C=S | H |
| 2152 | H | D2 | A3 | A3 | Ph | C=S | H |
| 2153 | H | D3 | A3 | A3 | Ph | C=S | H |
| 2154 | H | H | A4 | A4 | Ph | C=S | H |
| 2155 | H | D1 | A4 | A4 | Ph | C=S | H |
| 2156 | H | D2 | A4 | A4 | Ph | C=S | H |
| 2157 | H | D3 | A4 | A4 | Ph | C=S | H |

(continued)

| Compound No. | $R^2$ | $R^7$ | $R^{10}$ | $R^{15}$ | $R^{17}$ | Z | Other Rs |
|---|---|---|---|---|---|---|---|
| 2158 | H | H | A5 | A5 | Ph | C=S | H |
| 2159 | H | D1 | A5 | A5 | Ph | C=S | H |
| 2160 | H | D2 | A5 | A5 | Ph | C=S | H |
| 2161 | H | D3 | A5 | A5 | Ph | C=S | H |
| 2162 | D1 | D1 | A1 | A1 | Ph | C=S | H |
| 2163 | D2 | D2 | A1 | A1 | Ph | C=S | H |
| 2164 | D3 | D3 | A1 | A1 | Ph | C=S | H |
| 2165 | D1 | D1 | A2 | A2 | Ph | C=S | H |
| 2166 | D2 | D2 | A2 | A2 | Ph | C=S | H |
| 2167 | D3 | D3 | A2 | A2 | Ph | C=S | H |
| 2168 | D1 | D1 | A3 | A3 | Ph | C=S | H |
| 2169 | D2 | D2 | A3 | A3 | Ph | C=S | H |
| 2170 | D3 | D3 | A3 | A3 | Ph | C=S | H |
| 2171 | D1 | D1 | A4 | A4 | Ph | C=S | H |
| 2172 | D2 | D2 | A4 | A4 | Ph | C=S | H |
| 2173 | D3 | D3 | A4 | A4 | Ph | C=S | H |
| 2174 | D1 | D1 | A5 | A5 | Ph | C=S | H |
| 2175 | D2 | D2 | A5 | A5 | Ph | C=S | H |
| 2176 | D3 | D3 | A5 | A5 | Ph | C=S | H |

Table 12

| Compound No. | $R^3$ | $R^6$ | $R^{11}$ | $R^{14}$ | $R^{17}$ | Z | Other Rs |
|---|---|---|---|---|---|---|---|
| 2177 | H | H | H | A1 | Ph | C=S | H |
| 2178 | H | D1 | H | A1 | Ph | C=S | H |
| 2179 | H | D2 | H | A1 | Ph | C=S | H |
| 2180 | H | D3 | H | A1 | Ph | C=S | H |
| 2181 | H | H | H | A2 | Ph | C=S | H |
| 2182 | H | D1 | H | A2 | Ph | C=S | H |
| 2183 | H | D2 | H | A2 | Ph | C=S | H |
| 2184 | H | D3 | H | A2 | Ph | C=S | H |
| 2185 | H | H | H | A3 | Ph | C=S | H |
| 2186 | H | D1 | H | A3 | Ph | C=S | H |
| 2187 | H | D2 | H | A3 | Ph | C=S | H |
| 2188 | H | D3 | H | A3 | Ph | C=S | H |
| 2189 | H | H | H | A4 | Ph | C=S | H |
| 2190 | H | D1 | H | A4 | Ph | C=S | H |
| 2191 | H | D2 | H | A4 | Ph | C=S | H |

(continued)

| Compound No. | $R^3$ | $R^6$ | $R^{11}$ | $R^{14}$ | $R^{17}$ | Z | Other Rs |
|---|---|---|---|---|---|---|---|
| 2192 | H | D3 | H | A4 | Ph | C=S | H |
| 2193 | H | H | H | A5 | Ph | C=S | H |
| 2194 | H | D1 | H | A5 | Ph | C=S | H |
| 2195 | H | D2 | H | A5 | Ph | C=S | H |
| 2196 | H | D3 | H | A5 | Ph | C=S | H |
| 2197 | D1 | D1 | H | A1 | Ph | C=S | H |
| 2198 | D2 | D2 | H | A1 | Ph | C=S | H |
| 2199 | D3 | D3 | H | A1 | Ph | C=S | H |
| 2200 | D1 | D1 | H | A2 | Ph | C=S | H |
| 2201 | D2 | D2 | H | A2 | Ph | C=S | H |
| 2202 | D3 | D3 | H | A2 | Ph | C=S | H |
| 2203 | D1 | D1 | H | A3 | Ph | C=S | H |
| 2204 | D2 | D2 | H | A3 | Ph | C=S | H |
| 2205 | D3 | D3 | H | A3 | Ph | C=S | H |
| 2206 | D1 | D1 | H | A4 | Ph | C=S | H |
| 2207 | D2 | D2 | H | A4 | Ph | C=S | H |
| 2208 | D3 | D3 | H | A4 | Ph | C=S | H |
| 2209 | D1 | D1 | H | A5 | Ph | C=S | H |
| 2210 | D2 | D2 | H | A5 | Ph | C=S | H |
| 2211 | D3 | D3 | H | A5 | Ph | C=S | H |
| 2212 | H | H | H | H | Ph | C=S | H |

Table 13

| Compound No. | $R^2$ | $R^7$ | $R^{10}$ | $R^{15}$ | $R^{17}$ | Z | Other Rs |
|---|---|---|---|---|---|---|---|
| 2213 | H | H | A1 | A1 | Ph | $C=C(CN)_2$ | H |
| 2214 | H | D1 | A1 | A1 | Ph | $C=C(CN)_2$ | H |
| 2215 | H | D2 | A1 | A1 | Ph | $C=C(CN)_2$ | H |
| 2216 | H | D3 | A1 | A1 | Ph | $C=C(CN)_2$ | H |
| 2217 | H | H | A2 | A2 | Ph | $C=C(CN)_2$ | H |
| 2218 | H | D1 | A2 | A2 | Ph | $C=C(CN)_2$ | H |
| 2219 | H | D2 | A2 | A2 | Ph | $C=C(CN)_2$ | H |
| 2220 | H | D3 | A2 | A2 | Ph | $C=C(CN)_2$ | H |
| 2221 | H | H | A3 | A3 | Ph | $C=C(CN)_2$ | H |
| 2222 | H | D1 | A3 | A3 | Ph | $C=C(CN)_2$ | H |
| 2223 | H | D2 | A3 | A3 | Ph | $C=C(CN)_2$ | H |
| 2224 | H | D3 | A3 | A3 | Ph | $C=C(CN)_2$ | H |
| 2225 | H | H | A4 | A4 | Ph | $C=C(CN)_2$ | H |

(continued)

| Compound No. | $R^2$ | $R^7$ | $R^{10}$ | $R^{15}$ | $R^{17}$ | Z | Other Rs |
|---|---|---|---|---|---|---|---|
| 2226 | H | D1 | A4 | A4 | Ph | $C=C(CN)_2$ | H |
| 2227 | H | D2 | A4 | A4 | Ph | $C=C(CN)_2$ | H |
| 2228 | H | D3 | A4 | A4 | Ph | $C=C(CN)_2$ | H |
| 2229 | H | H | A5 | A5 | Ph | $C=C(CN)_2$ | H |
| 2230 | H | D1 | A5 | A5 | Ph | $C=C(CN)_2$ | H |
| 2231 | H | D2 | A5 | A5 | Ph | $C=C(CN)_2$ | H |
| 2232 | H | D3 | A5 | A5 | Ph | $C=C(CN)_2$ | H |
| 2233 | D1 | D1 | A1 | A1 | Ph | $C=C(CN)_2$ | H |
| 2234 | D2 | D2 | A1 | A1 | Ph | $C=C(CN)_2$ | H |
| 2235 | D3 | D3 | A1 | A1 | Ph | $C=C(CN)_2$ | H |
| 2236 | D1 | D1 | A2 | A2 | Ph | $C=C(CN)_2$ | H |
| 2237 | D2 | D2 | A2 | A2 | Ph | $C=C(CN)_2$ | H |
| 2238 | D3 | D3 | A2 | A2 | Ph | $C=C(CN)_2$ | H |
| 2239 | D1 | D1 | A3 | A3 | Ph | $C=C(CN)_2$ | H |
| 2240 | D2 | D2 | A3 | A3 | Ph | $C=C(CN)_2$ | H |
| 2241 | D3 | D3 | A3 | A3 | Ph | $C=C(CN)_2$ | H |
| 2242 | D1 | D1 | A4 | A4 | Ph | $C=C(CN)_2$ | H |
| 2243 | D2 | D2 | A4 | A4 | Ph | $C=C(CN)_2$ | H |
| 2244 | D3 | D3 | A4 | A4 | Ph | $C=C(CN)_2$ | H |
| 2245 | D1 | D1 | A5 | A5 | Ph | $C=C(CN)_2$ | H |
| 2246 | D2 | D2 | A5 | A5 | Ph | $C=C(CN)_2$ | H |
| 2247 | D3 | D3 | A5 | A5 | Ph | $C=C(CN)_2$ | H |

Table 14

| Compound No. | $R^3$ | R6 | $R^{11}$ | $R^{14}$ | $R^{17}$ | Z | Other Rs |
|---|---|---|---|---|---|---|---|
| 2248 | H | H | H | A1 | Ph | $C=C(CN)_2$ | H |
| 2249 | H | D1 | H | A1 | Ph | $C=C(CN)_2$ | H |
| 2250 | H | D2 | H | A1 | Ph | $C=C(CN)_2$ | H |
| 2251 | H | D3 | H | A1 | Ph | $C=C(CN)_2$ | H |
| 2252 | H | H | H | A2 | Ph | $C=C(CN)_2$ | H |
| 2253 | H | D1 | H | A2 | Ph | $C=C(CN)_2$ | H |
| 2254 | H | D2 | H | A2 | Ph | $C=C(CN)_2$ | H |
| 2255 | H | D3 | H | A2 | Ph | $C=C(CN)_2$ | H |
| 2256 | H | H | H | A3 | Ph | $C=C(CN)_2$ | H |
| 2257 | H | D1 | H | A3 | Ph | $C=C(CN)_2$ | H |
| 2258 | H | D2 | H | A3 | Ph | $C=C(CN)_2$ | H |
| 2259 | H | D3 | H | A3 | Ph | $C=C(CN)_2$ | H |

(continued)

| Compound No. | $R^3$ | R6 | $R^{11}$ | $R^{14}$ | $R^{17}$ | Z | Other Rs |
|---|---|---|---|---|---|---|---|
| 2260 | H | H | H | A4 | Ph | $C=C(CN)_2$ | H |
| 2261 | H | D1 | H | A4 | Ph | $C=C(CN)_2$ | H |
| 2262 | H | D2 | H | A4 | Ph | $C=C(CN)_2$ | H |
| 2263 | H | D3 | H | A4 | Ph | $C=C(CN)_2$ | H |
| 2264 | H | H | H | A5 | Ph | $C=C(CN)_2$ | H |
| 2265 | H | D1 | H | A5 | Ph | $C=C(CN)_2$ | H |
| 2266 | H | D2 | H | A5 | Ph | $C=C(CN)_2$ | H |
| 2267 | H | D3 | H | A5 | Ph | $C=C(CN)_2$ | H |
| 2268 | D1 | D1 | H | A1 | Ph | $C=C(CN)_2$ | H |
| 2269 | D2 | D2 | H | A1 | Ph | $C=C(CN)_2$ | H |
| 2270 | D3 | D3 | H | A1 | Ph | $C=C(CN)_2$ | H |
| 2271 | D1 | D1 | H | A2 | Ph | $C=C(CN)_2$ | H |
| 2272 | D2 | D2 | H | A2 | Ph | $C=C(CN)_2$ | H |
| 2273 | D3 | D3 | H | A2 | Ph | $C=C(CN)_2$ | H |
| 2274 | D1 | D1 | H | A3 | Ph | $C=C(CN)_2$ | H |
| 2275 | D2 | D2 | H | A3 | Ph | $C=C(CN)_2$ | H |
| 2276 | D3 | D3 | H | A3 | Ph | $C=C(CN)_2$ | H |
| 2277 | D1 | D1 | H | A4 | Ph | $C=C(CN)_2$ | H |
| 2278 | D2 | D2 | H | A4 | Ph | $C=C(CN)_2$ | H |
| 2279 | D3 | D3 | H | A4 | Ph | $C=C(CN)_2$ | H |
| 2280 | D1 | D1 | H | A5 | Ph | $C=C(CN)_2$ | H |
| 2281 | D2 | D2 | H | A5 | Ph | $C=C(CN)_2$ | H |
| 2282 | D3 | D3 | H | A5 | Ph | $C=C(CN)_2$ | H |
| 2283 | H | H | H | H | Ph | $C=C(CN)_2$ | H |

Table 15

| Compound No. | $R^2$ | $R^7$ | $R^{10}$ | $R^{15}$ | $R^{17}$ | Z | Other Rs |
|---|---|---|---|---|---|---|---|
| 2284 | H | H | A1 | A1 | Ph | $C=C(COOH)_2$ | H |
| 2285 | H | D1 | A1 | A1 | Ph | $C=C(COOH)_2$ | H |
| 2286 | H | D2 | A1 | A1 | Ph | $C=C(COOH)_2$ | H |
| 2287 | H | D3 | A1 | A1 | Ph | $C=C(COOH)_2$ | H |
| 2288 | H | H | A2 | A2 | Ph | $C=C(COOH)_2$ | H |
| 2289 | H | D1 | A2 | A2 | Ph | $C=C(COOH)_2$ | H |
| 2290 | H | D2 | A2 | A2 | Ph | $C=C(COOH)_2$ | H |
| 2291 | H | D3 | A2 | A2 | Ph | $C=C(COOH)_2$ | H |
| 2292 | H | H | A3 | A3 | Ph | $C=C(COOH)_2$ | H |
| 2293 | H | D1 | A3 | A3 | Ph | $C=C(COOH)_2$ | H |

(continued)

| Compound No. | $R^2$ | $R^7$ | $R^{10}$ | $R^{15}$ | $R^{17}$ | Z | Other Rs |
|---|---|---|---|---|---|---|---|
| 2294 | H | D2 | A3 | A3 | Ph | $C=C(COOH)_2$ | H |
| 2295 | H | D3 | A3 | A3 | Ph | $C=C(COOH)_2$ | H |
| 2296 | H | H | A4 | A4 | Ph | $C=C(COOH)_2$ | H |
| 2297 | H | D1 | A4 | A4 | Ph | $C=C(COOH)_2$ | H |
| 2298 | H | D2 | A4 | A4 | Ph | $C=C(COOH)_2$ | H |
| 2299 | H | D3 | A4 | A4 | Ph | $C=C(COOH)_2$ | H |
| 2300 | H | H | A5 | A5 | Ph | $C=C(COOH)_2$ | H |
| 2301 | H | D1 | A5 | A5 | Ph | $C=C(COOH)_2$ | H |
| 2302 | H | D2 | A5 | A5 | Ph | $C=C(COOH)_2$ | H |
| 2303 | H | D3 | A5 | A5 | Ph | $C=C(COOH)_2$ | H |
| 2304 | D1 | D1 | A1 | A1 | Ph | $C=C(COOH)_2$ | H |
| 2305 | D2 | D2 | A1 | A1 | Ph | $C=C(COOH)_2$ | H |
| 2306 | D3 | D3 | A1 | A1 | Ph | $C=C(COOH)_2$ | H |
| 2307 | D1 | D1 | A2 | A2 | Ph | $C=C(COOH)_2$ | H |
| 2308 | D2 | D2 | A2 | A2 | Ph | $C=C(COOH)_2$ | H |
| 2309 | D3 | D3 | A2 | A2 | Ph | $C=C(COOH)_2$ | H |
| 2310 | D1 | D1 | A3 | A3 | Ph | $C=C(COOH)_2$ | H |
| 2311 | D2 | D2 | A3 | A3 | Ph | $C=C(COOH)_2$ | H |
| 2312 | D3 | D3 | A3 | A3 | Ph | $C=C(COOH)_2$ | H |
| 2313 | D1 | D1 | A4 | A4 | Ph | $C=C(COOH)_2$ | H |
| 2314 | D2 | D2 | A4 | A4 | Ph | $C=C(COOH)_2$ | H |
| 2315 | D3 | D3 | A4 | A4 | Ph | $C=C(COOH)_2$ | H |
| 2316 | D1 | D1 | A5 | A5 | Ph | $C=C(COOH)_2$ | H |
| 2317 | D2 | D2 | A5 | A5 | Ph | $C=C(COOH)_2$ | H |
| 2318 | D3 | D3 | A5 | A5 | Ph | $C=C(COOH)_2$ | H |

Table 16

| Compound No. | $R^3$ | $R^6$ | $R^{11}$ | $R^{14}$ | $R^{17}$ | Z | Other Rs |
|---|---|---|---|---|---|---|---|
| 2319 | H | H | H | A1 | Ph | $C=C(COOH)_2$ | H |
| 2320 | H | D1 | H | A1 | Ph | $C=C(COOH)_2$ | H |
| 2321 | H | D2 | H | A1 | Ph | $C=C(COOH)_2$ | H |
| 2322 | H | D3 | H | A1 | Ph | $C=C(COOH)_2$ | H |
| 2323 | H | H | H | A2 | Ph | $C=C(COOH)_2$ | H |
| 2324 | H | D1 | H | A2 | Ph | $C=C(COOH)_2$ | H |
| 2325 | H | D2 | H | A2 | Ph | $C=C(COOH)_2$ | H |
| 2326 | H | D3 | H | A2 | Ph | $C=C(COOH)_2$ | H |
| 2327 | H | H | H | A3 | Ph | $C=C(COOH)_2$ | H |

(continued)

| Compound No. | $R^3$ | $R^6$ | $R^{11}$ | $R^{14}$ | $R^{17}$ | Z | Other Rs |
|---|---|---|---|---|---|---|---|
| 2328 | H | D1 | H | A3 | Ph | $C=C(COOH)_2$ | H |
| 2329 | H | D2 | H | A3 | Ph | $C=C(COOH)_2$ | H |
| 2330 | H | D3 | H | A3 | Ph | $C=C(COOH)_2$ | H |
| 2331 | H | H | H | A4 | Ph | $C=C(COOH)_2$ | H |
| 2332 | H | D1 | H | A4 | Ph | $C=C(COOH)_2$ | H |
| 2333 | H | D2 | H | A4 | Ph | $C=C(COOH)_2$ | H |
| 2334 | H | D3 | H | A4 | Ph | $C=C(COOH)_2$ | H |
| 2335 | H | H | H | A5 | Ph | $C=C(COOH)_2$ | H |
| 2336 | H | D1 | H | A5 | Ph | $C=C(COOH)_2$ | H |
| 2337 | H | D2 | H | A5 | Ph | $C=C(COOH)_2$ | H |
| 2338 | H | D3 | H | A5 | Ph | $C=C(COOH)_2$ | H |
| 2339 | D1 | D1 | H | A1 | Ph | $C=C(COOH)_2$ | H |
| 2340 | D2 | D2 | H | A1 | Ph | $C=C(COOH)_2$ | H |
| 2341 | D3 | D3 | H | A1 | Ph | $C=C(COOH)_2$ | H |
| 2342 | D1 | D1 | H | A2 | Ph | $C=C(COOH)_2$ | H |
| 2343 | D2 | D2 | H | A2 | Ph | $C=C(COOH)_2$ | H |
| 2344 | D3 | D3 | H | A2 | Ph | $C=C(COOH)_2$ | H |
| 2345 | D1 | D1 | H | A3 | Ph | $C=C(COOH)_2$ | H |
| 2346 | D2 | D2 | H | A3 | Ph | $C=C(COOH)_2$ | H |
| 2347 | D3 | D3 | H | A3 | Ph | $C=C(COOH)_2$ | H |
| 2348 | D1 | D1 | H | A4 | Ph | $C=C(COOH)_2$ | H |
| 2349 | D2 | D2 | H | A4 | Ph | $C=C(COOH)_2$ | H |
| 2350 | D3 | D3 | H | A4 | Ph | $C=C(COOH)_2$ | H |
| 2351 | D1 | D1 | H | A5 | Ph | $C=C(COOH)_2$ | H |
| 2352 | D2 | D2 | H | A5 | Ph | $C=C(COOH)_2$ | H |
| 2353 | D3 | D3 | H | A5 | Ph | $C=C(COOH)_2$ | H |
| 2354 | H | H | H | H | Ph | $C=C(COOH)_2$ | H |

Examples of the preferred delayed fluorescent material include compounds represented by the following general formula

[Chem. 14]

General Formula (151)

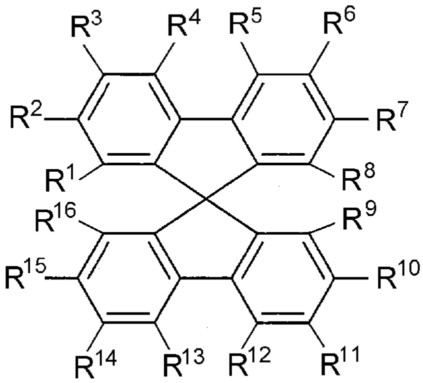

In the general formula (151), $R^1$, $R^2$, $R^3$, $R^4$, $R^5$, $R^6$, $R^7$ and $R^8$ each independently represent a hydrogen atom or an electron donating group, provided that at least one thereof represents an electron donating group. $R^9$, $R^{10}$, $R^{11}$, $R^{12}$, $R^{13}$, $R^{14}$, $R^{15}$ and $R^{16}$ each independently represent a hydrogen atom or an electron withdrawing group, provided that at least one thereof represents an electron withdrawing group.

Specific examples of the compounds include the compounds shown in the following tables. In the tables, D1 to D10 represent the unsubstituted electron donating groups having the following structures, respectively.

[Chem. 15]

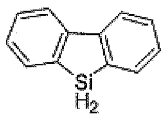
D1

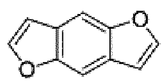
D2

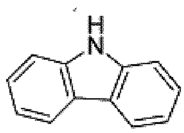
D3

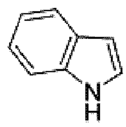
D4

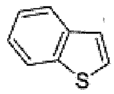
D5

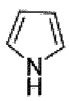
D6

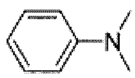
D7

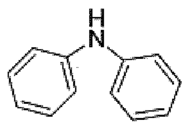
D8

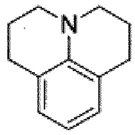
D9

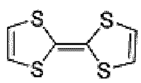
D10

Table 17

| |
|---|
| 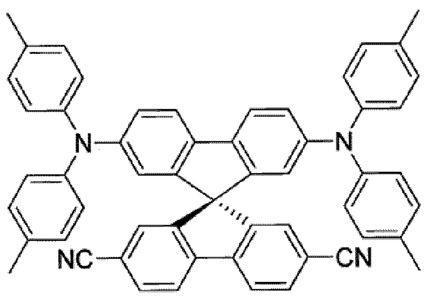 Compound 3001 |

(continued)

| Compound No. | $R^2$ | $R^7$ | $R^{10}$ | $R^{15}$ | Other Rs |
|---|---|---|---|---|---|
| 3002 | D1 | D1 | CN | CN | H |
| 3003 | D2 | D2 | CN | CN | H |
| 3004 | D3 | D3 | CN | CN | H |
| 3005 | D4 | D4 | CN | CN | H |
| 3006 | D5 | D5 | CN | CN | H |
| 3007 | D6 | D6 | CN | CN | H |
| 3008 | D7 | D7 | CN | CN | H |
| 3009 | D8 | D8 | CN | CN | H |
| 3010 | D9 | D9 | CN | CN | H |
| 3011 | D10 | D10 | CN | CN | H |
| 3012 | H | D1 | H | CN | H |
| 3013 | H | D2 | H | CN | H |
| 3014 | H | D3 | H | CN | H |
| 3015 | H | D4 | H | CN | H |
| 3016 | H | D5 | H | CN | H |
| 3017 | H | D6 | H | CN | H |
| 3018 | H | D7 | H | CN | H |
| 3019 | H | D8 | H | CN | H |
| 3020 | H | D9 | H | CN | H |
| 3021 | H | D10 | H | CN | H |

Table 18

| Compound No. | R3 | R6 | $R^{11}$ | $R^{14}$ | Other Rs |
|---|---|---|---|---|---|
| 3022 | D1 | D1 | CN | CN | H |
| 3023 | D2 | D2 | CN | CN | H |
| 3024 | D3 | D3 | CN | CN | H |
| 3025 | D4 | D4 | CN | CN | H |
| 3026 | D5 | D5 | CN | CN | H |
| 3027 | D6 | D6 | CN | CN | H |
| 3028 | D7 | D7 | CN | CN | H |
| 3029 | D8 | D8 | CN | CN | H |
| 3030 | D9 | D9 | CN | CN | H |
| 3031 | D10 | D10 | CN | CN | H |
| 3032 | H | D1 | H | CN | H |
| 3033 | H | D2 | H | CN | H |
| 3034 | H | D3 | H | CN | H |
| 3035 | H | D4 | H | CN | H |

(continued)

| Compound No. | R3 | R6 | $R^{11}$ | $R^{14}$ | Other Rs |
|---|---|---|---|---|---|
| 3036 | H | D5 | H | CN | H |
| 3037 | H | D6 | H | CN | H |
| 3038 | H | D7 | H | CN | H |
| 3039 | H | D8 | H | CN | H |
| 3040 | H | D9 | H | CN | H |
| 3041 | H | D10 | H | CN | H |

Table 19

| Compound No. | $R^2$, $R^7$ | $R^3$, $R^6$ | $R^{10}$, $R^{15}$ | $R^{11}$, $R^{14}$ | Other Rs |
|---|---|---|---|---|---|
| 3042 | diphenylamino group | H | CN | H | H |
| 3043 | bis(2-methylphenyl)amino group | H | CN | H | H |
| 3044 | bis(3-methylphenyl)amino group | H | CN | H | H |
| 3045 | bis(2,4-dimethylphenyl)amino group | H | CN | H | H |
| 3046 | bis(2,6-dimethylphenyl)amino group | H | CN | H | H |
| 3047 | bis(3,5-dimethylphenyl)amino group | H | CN | H | H |
| 3048 | bis(2,4,6-trimethylphenyl)amino group | H | CN | H | H |
| 3049 | bis(4-ethylphenyl)amino group | H | CN | H | H |
| 3050 | bis(4-propylphenyl)amino group | H | CN | H | H |
| 3051 | diphenylamino group | H | H | CN | H |
| 3052 | bis(2-methylphenyl)amino group | H | H | CN | H |
| 3053 | bis(3-methylphenyl)amino group | H | H | CN | H |
| 3054 | bis(4-methylphenyl)amino group | H | H | CN | H |
| 3055 | bis(2,4-dimethylphenyl)amino group | H | H | CN | H |
| 3056 | bis(2,6-dimethylphenyl)amino group | H | H | CN | H |
| 3057 | bis(3,5-dimethylphenyl)amino group | H | H | CN | H |
| 3058 | bis(2,4,6-trimethylphenyl)amino group | H | H | CN | H |
| 3059 | bis(4-ethylphenyl)amino group | H | H | CN | H |
| 3060 | bis(4-propylphenyl)amino group | H | H | CN | H |

Table 20

| Compound No. | $R^2$, $R^7$ | $R^3$, $R^6$ | $R^{10}$, $R^{15}$ | $R^{11}$, $R^{14}$ | Other Rs |
|---|---|---|---|---|---|
| 3061 | H | diphenylamino group | CN | H | H |
| 3062 | H | bis(2-methylphenyl)amino group | CN | H | H |
| 3063 | H | bis(3-methylphenyl)amino group | CN | H | H |
| 3064 | H | bis(4-methylphenyl)amino group | CN | H | H |
| 3065 | H | bis(2,4-dimethylphenyl)amino group | CN | H | H |
| 3066 | H | bis(2,6-dimethylphenyl)amino group | CN | H | H |

(continued)

| Compound No. | $R^2$, $R^7$ | $R^3$, $R^6$ | $R^{10}$, $R^{15}$ | $R^{11}$, $R^{14}$ | Other Rs |
|---|---|---|---|---|---|
| 3067 | H | bis(3,5-dimethylphenyl)amino group | CN | H | H |
| 3068 | H | bis(2,4,6-trimethylphenyl)amino group | CN | H | H |
| 3069 | H | bis(4-ethylphenyl)amino group | CN | H | H |
| 3070 | H | bis(4-propylphenyl)amino group | CN | H | H |
| 3071 | H | diphenylamino group | H | CN | H |
| 3072 | H | bis(2-methylphenyl)amino group | H | CN | H |
| 3073 | H | bis(3-methylphenyl)amino group | H | CN | H |
| 3074 | H | bis(4-methylphenyl)amino group | H | CN | H |
| 3075 | H | bis(2,4-dimethylphenyl)amino group | H | CN | H |
| 3076 | H | bis(2,6-dimethylphenyl)amino group | H | CN | H |
| 3077 | H | bis(3,5-dimethylphenyl)amino group | H | CN | H |
| 3078 | H | bis(2,4,6-trimethylphenyl)amino group | H | CN | H |
| 3079 | H | bis(4-ethylphenyl)amino group | H | CN | H |
| 3080 | H | bis(4-propylphenyl)amino group | H | CN | H |

Examples of the preferred delayed fluorescent material include compounds represented by the following general formula.

[Chem. 16]

General Formula (161)

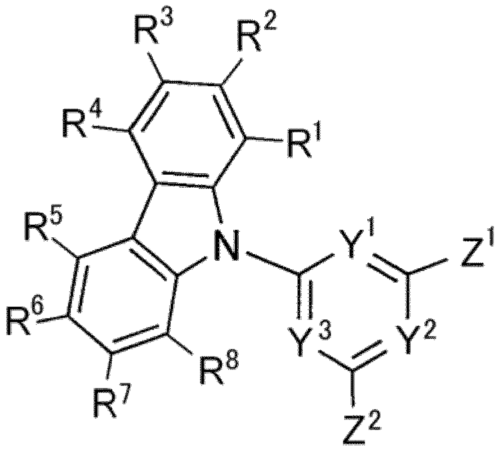

In the general formula (161), any two of $Y^1$, $Y^2$ and $Y^3$ each represent a nitrogen atom, and the balance thereof represents a methine group, of all $Y^1$, $Y^2$ and $Y^3$ each represent a nitrogen atom. $Z^1$ and $Z^2$ each independently represent a hydrogen atom or a substituent. $R^1$ to $R^8$ each independently represent a hydrogen atom or a substituent, provided that at least one of $R^1$ to $R^8$ represents a substituted or unsubstituted diarylamino group or a substituted or unsubstituted carbazolyl group. The compound represented by the general formula (161) has at least two carbazole structures in the molecule thereof.

Examples of the compound include the following compounds.

[Chem. 17]

1

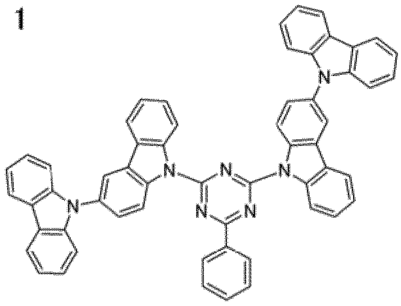

2

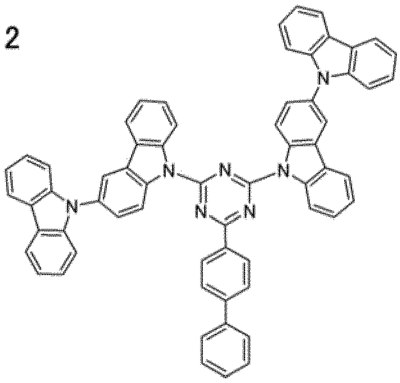

3

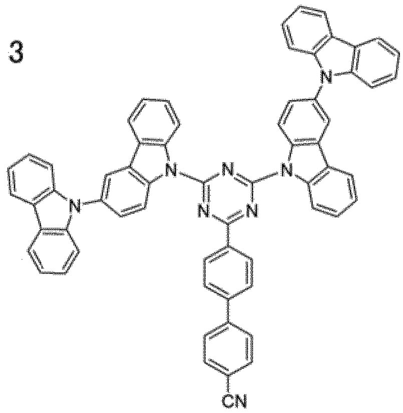

4

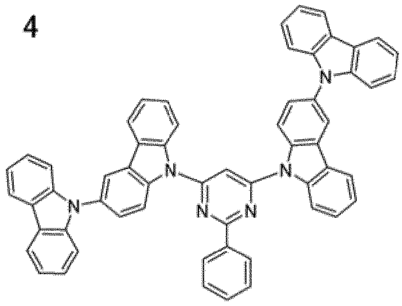

5

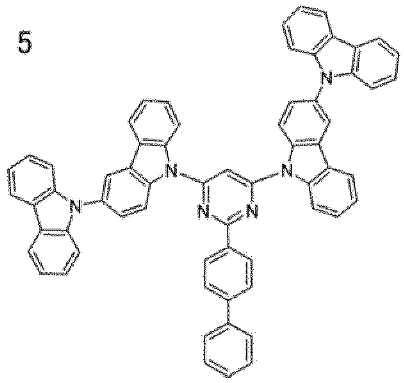

6

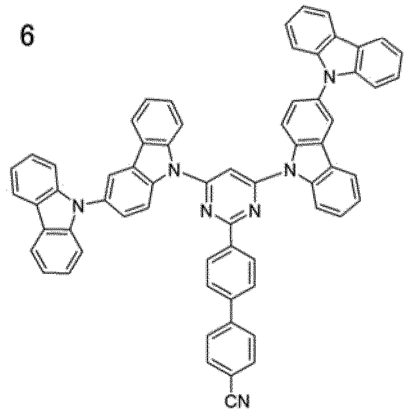

[Chem. 18]

7

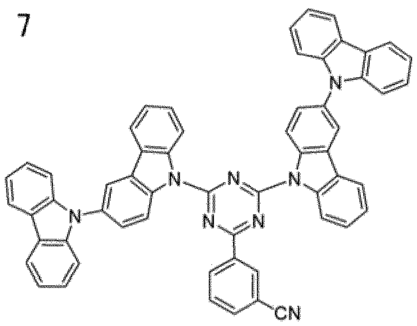

11

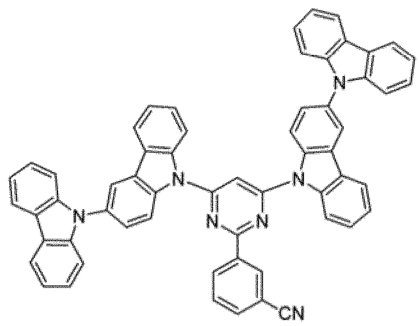

8

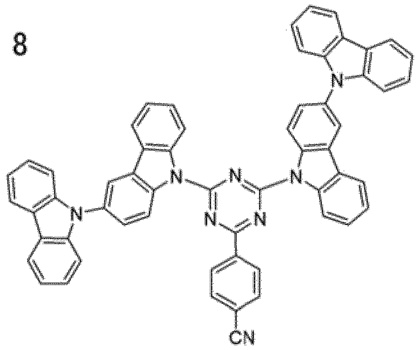

12

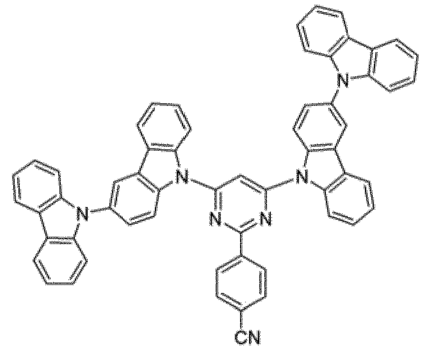

9

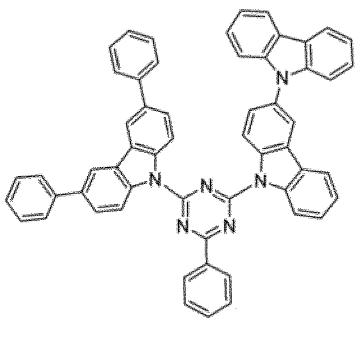

13

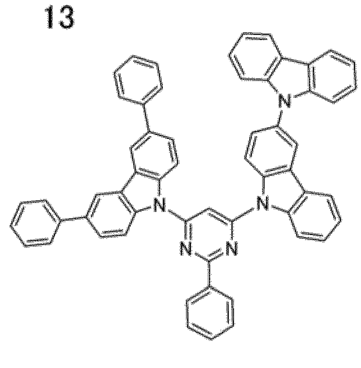

10

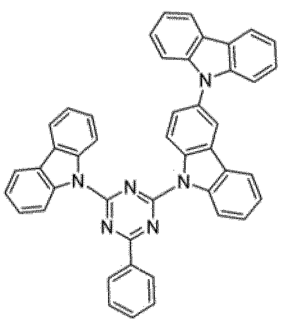

14

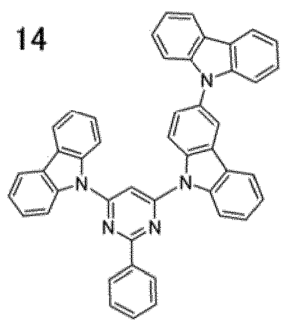

[Chem. 19]

15

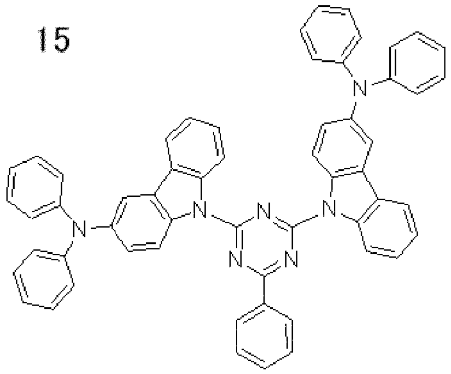

18

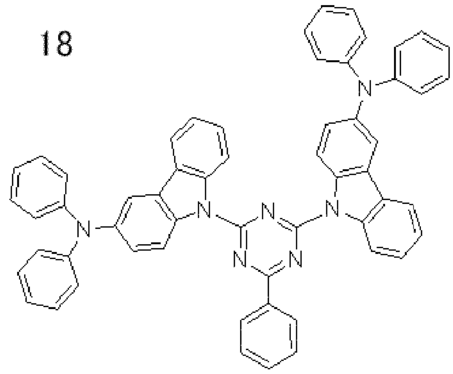

16

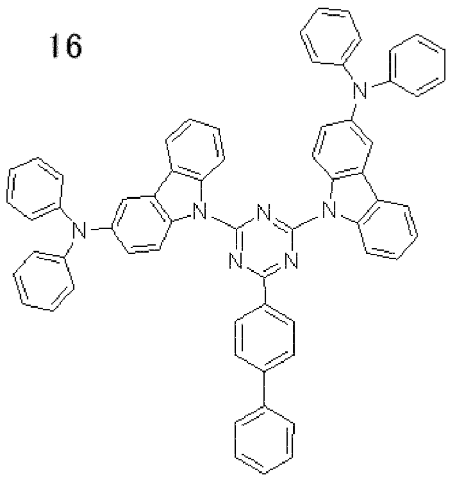

19

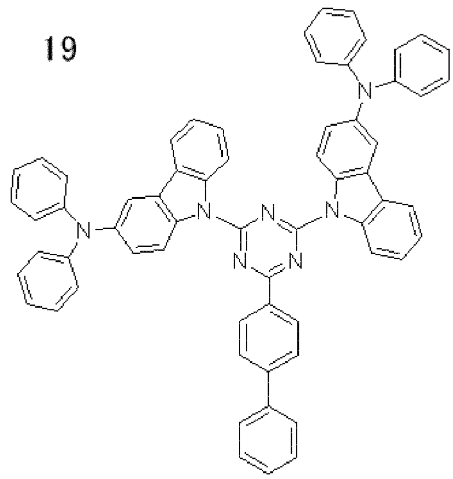

17

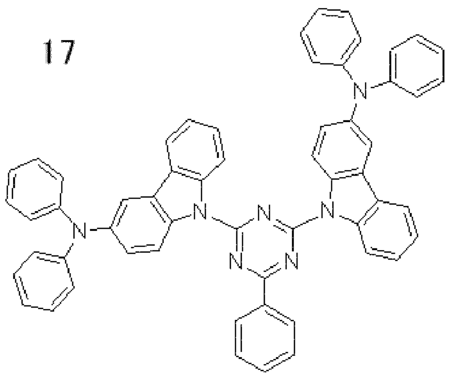

20

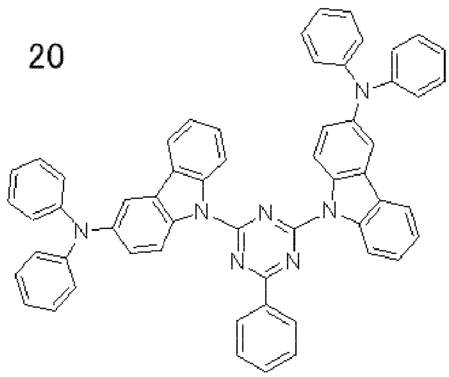

[Chem. 20]

21

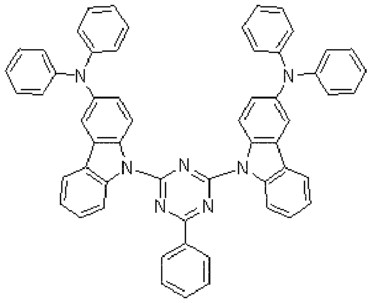

24

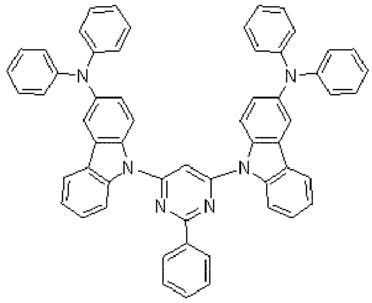

22

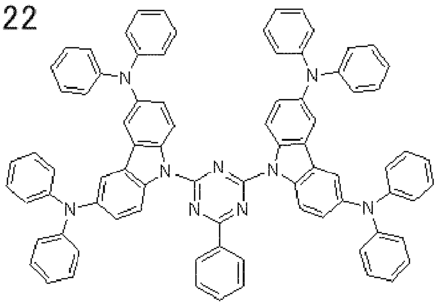

25

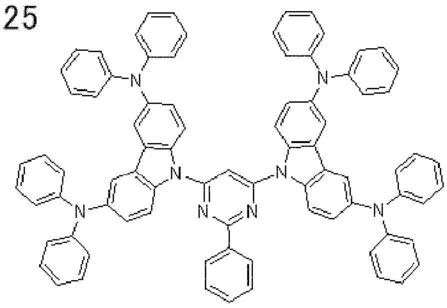

23

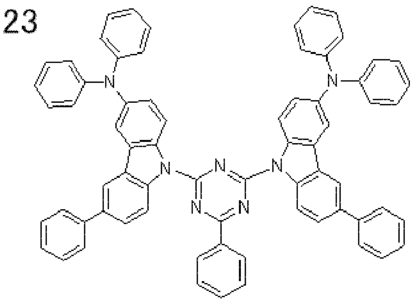

26

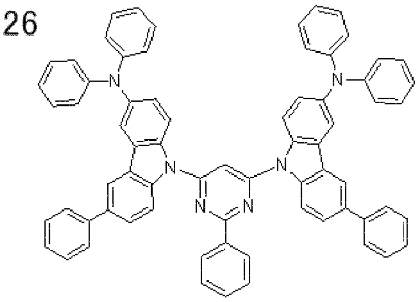

[Chem. 21]

27

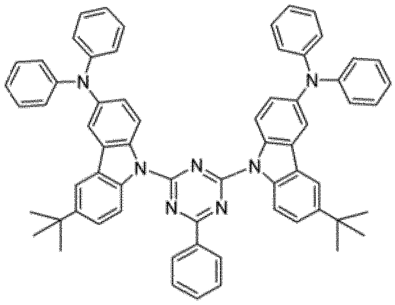

30

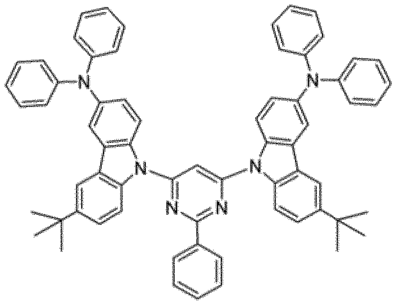

28

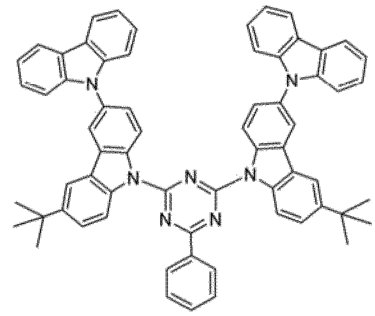

31

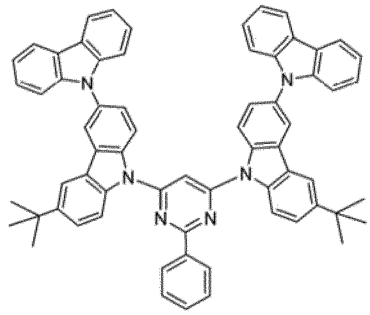

29

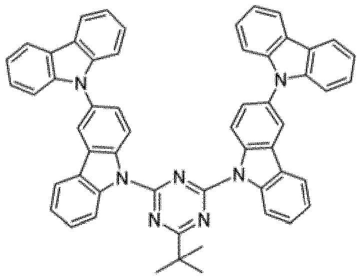

32

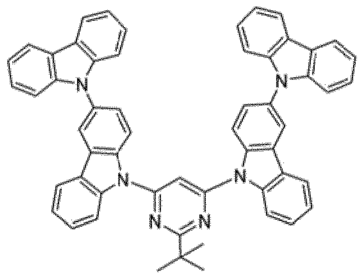

[Chem. 22]

33

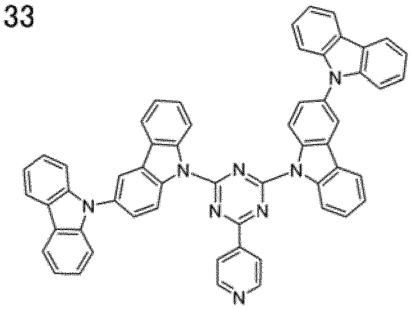

37

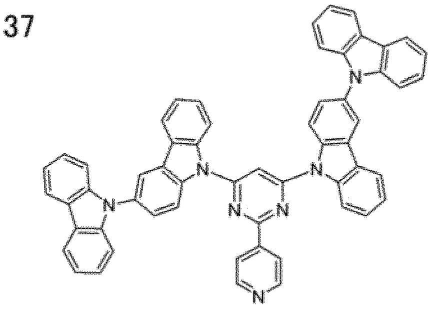

34

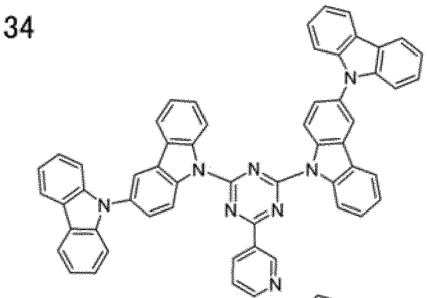

38

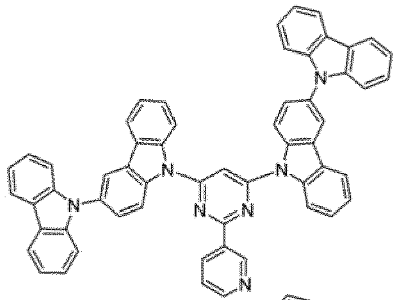

35

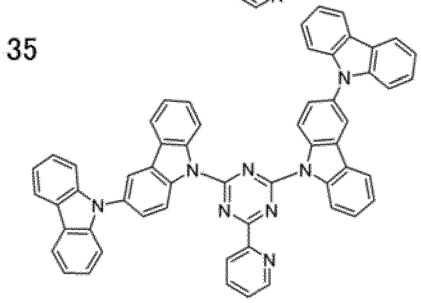

39

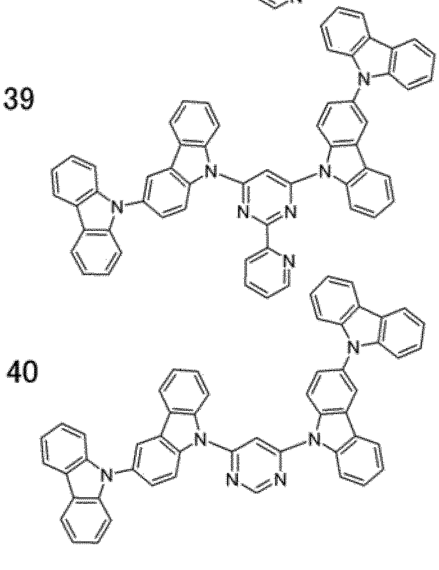

36

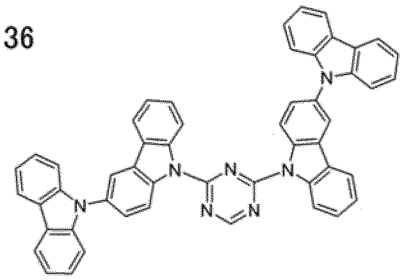

40

[Chem. 23]

41

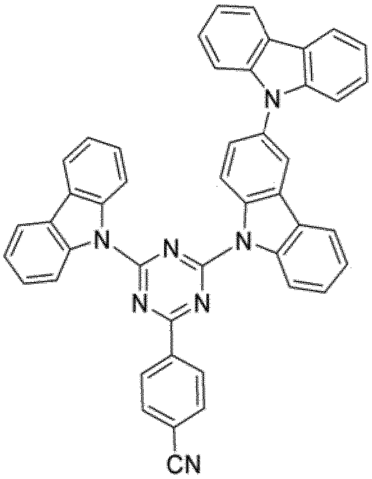

42

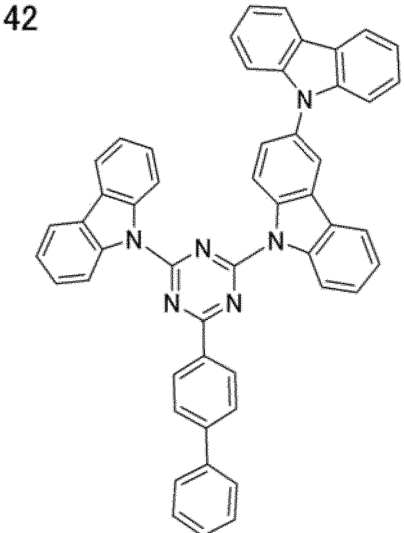

43

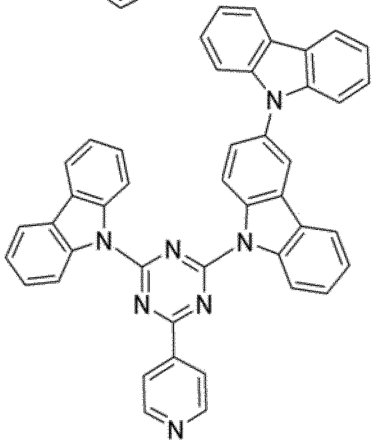

44

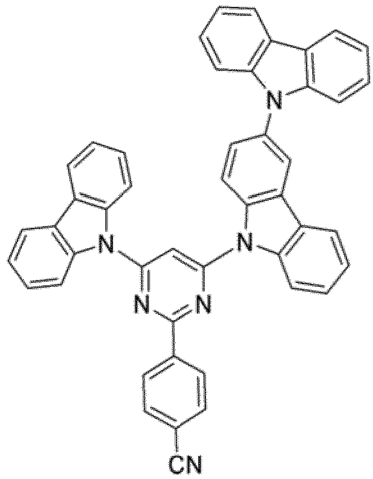

45

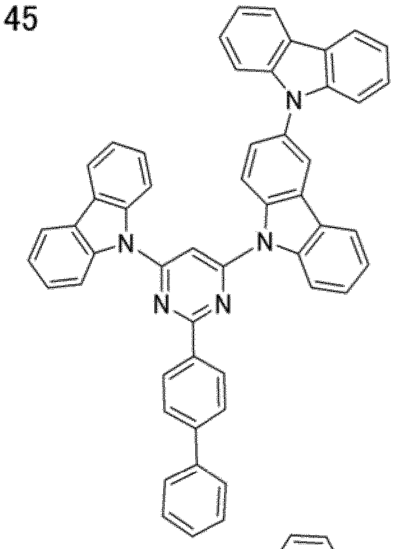

46

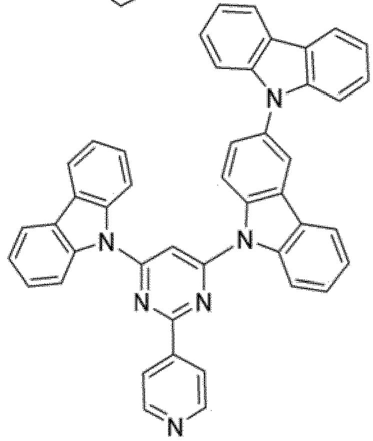

[Chem. 24]

47

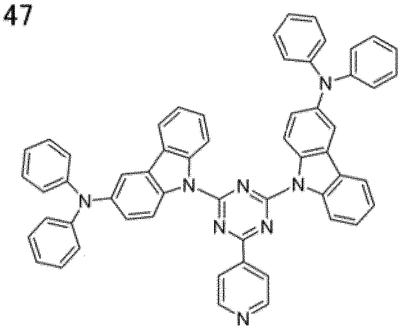

49

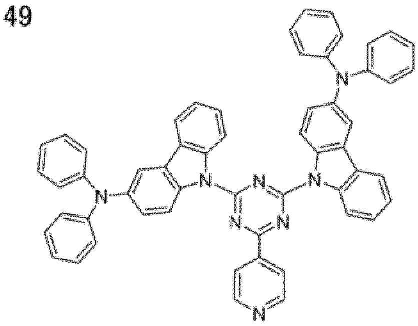

48

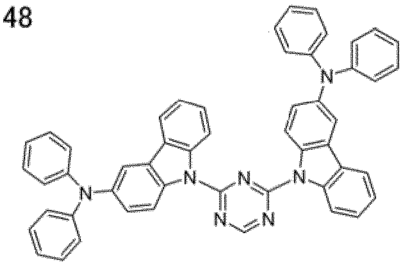

50

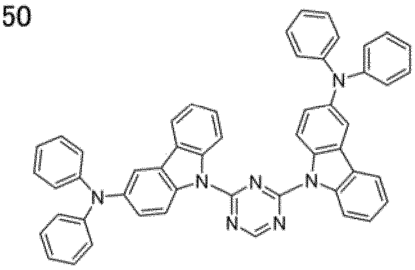

[Chem. 25]

51

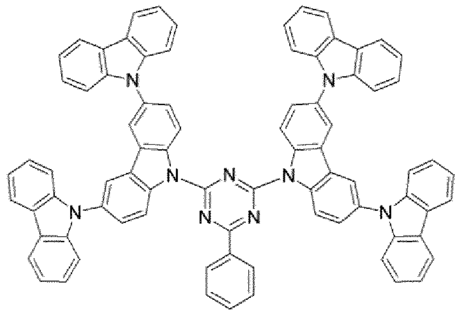

53

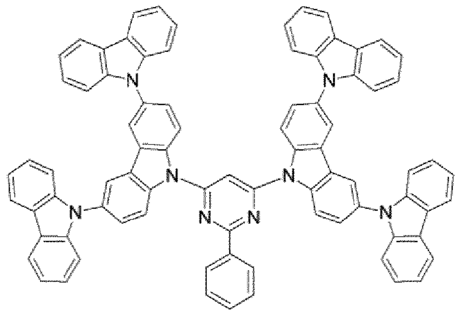

52

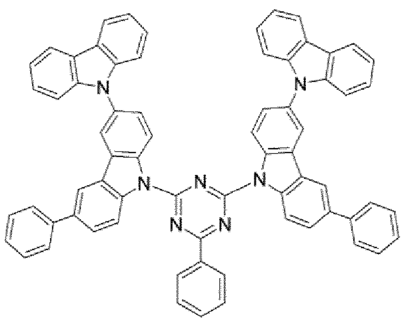

54

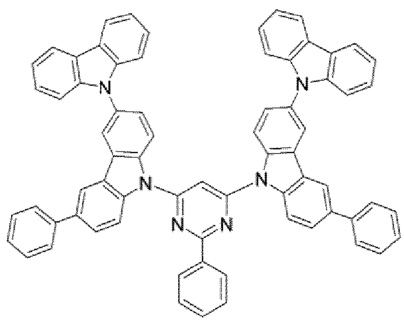

[Chem. 26]

55 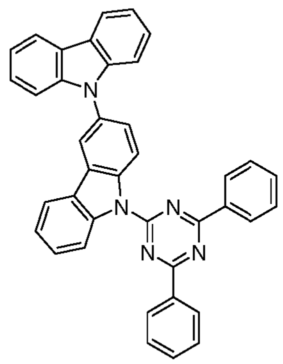

59 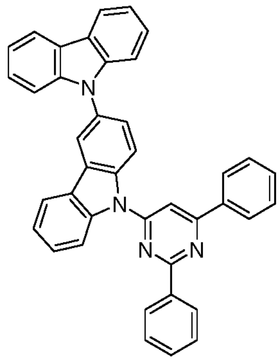

56 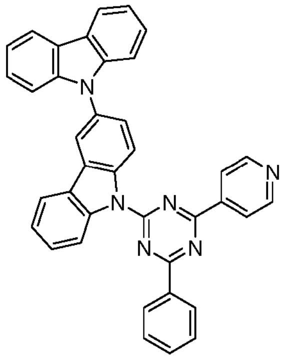

60 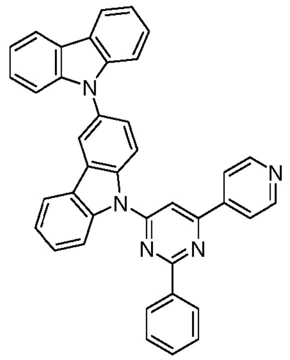

57 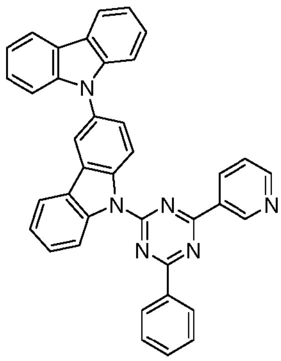

61 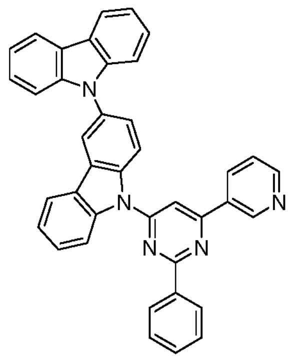

58 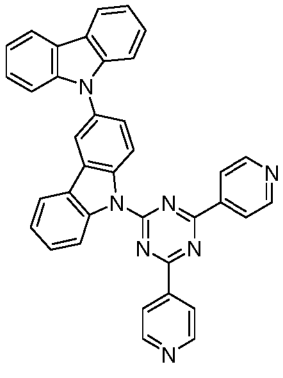

62 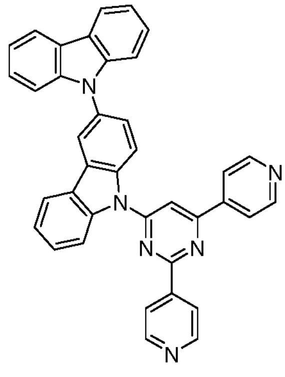

[Chem. 27]

63 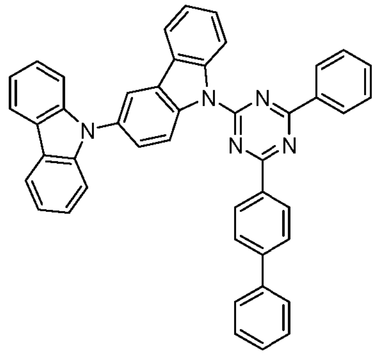

67 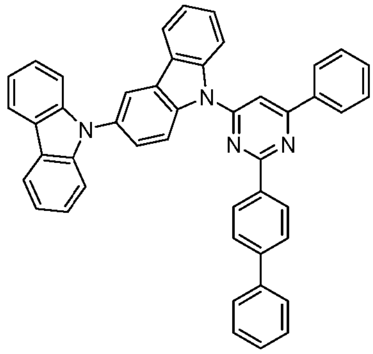

64 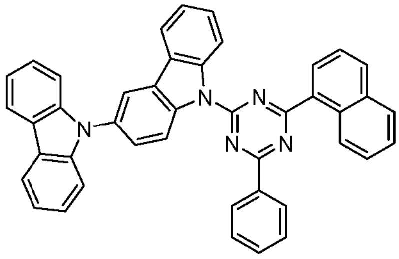

68 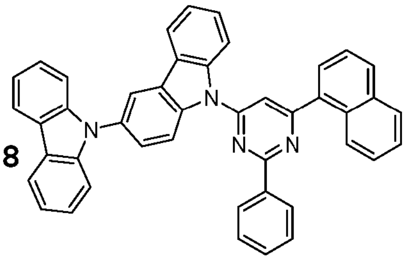

65 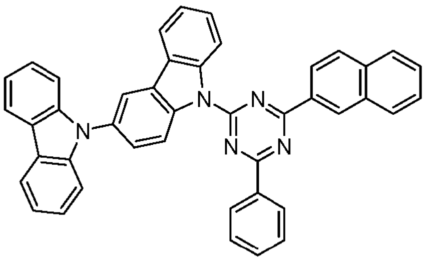

69 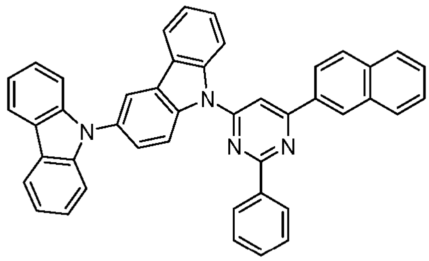

66 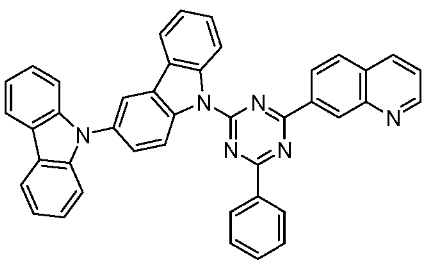

70 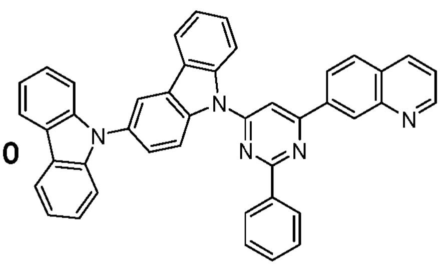

[Chem. 28]

71

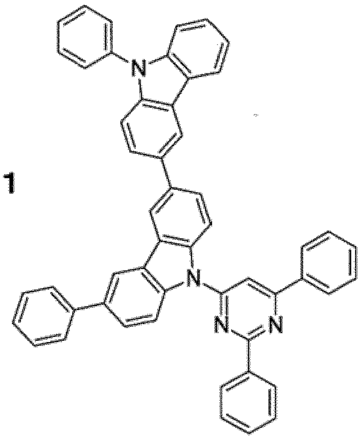

74

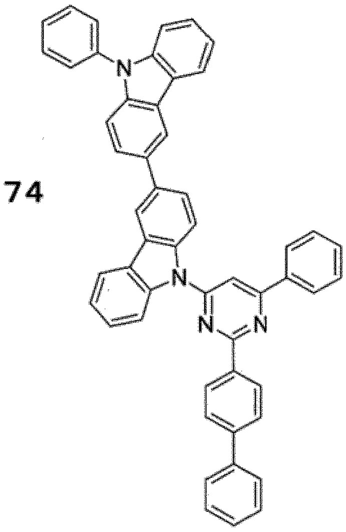

72

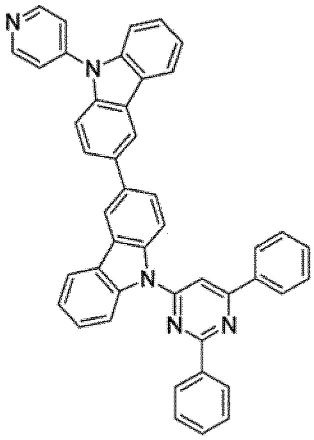

75

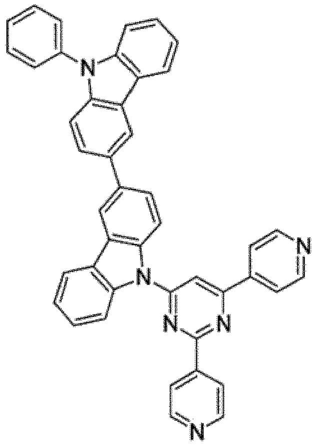

73

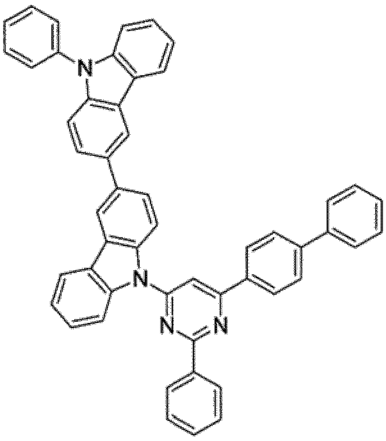

[Chem. 29]

76

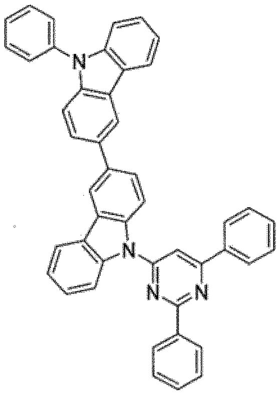

78

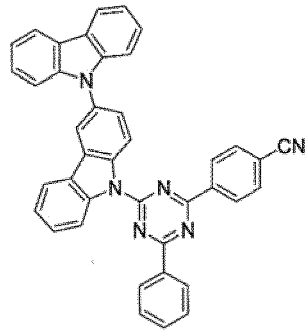

77

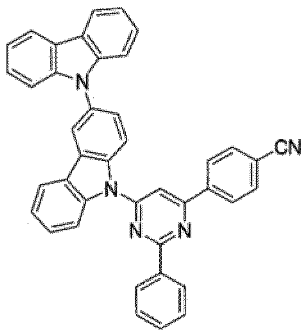

79

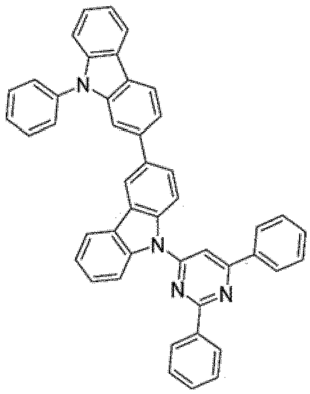

[Chem. 30]

80

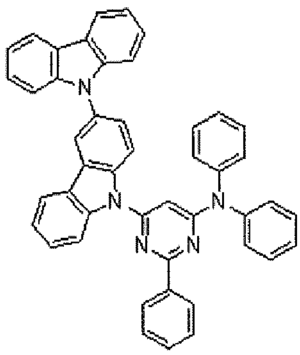

81

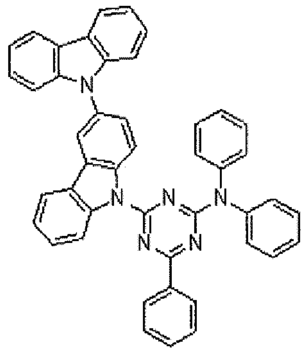

82

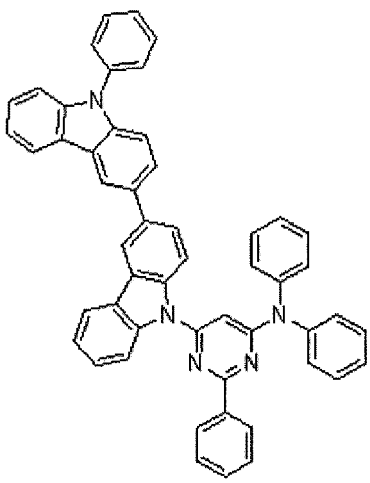

83

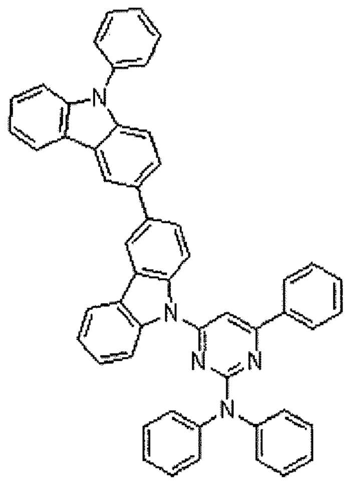

[Chem. 31]

84

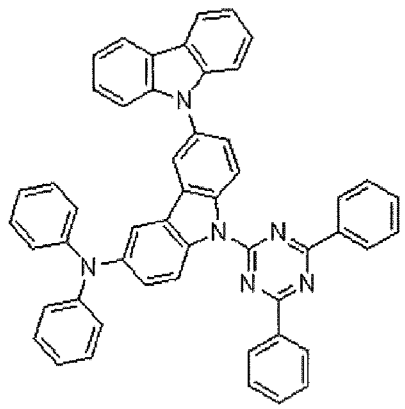

86

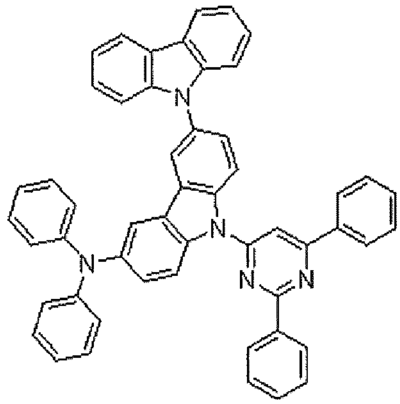

85

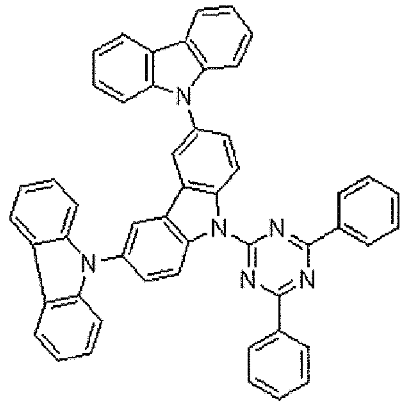

87

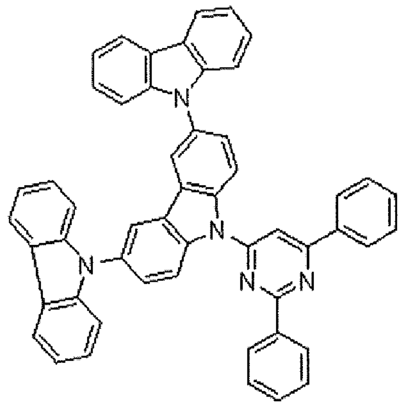

[Chem. 32]

88

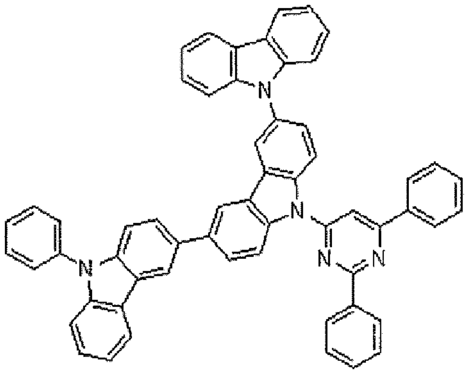

89

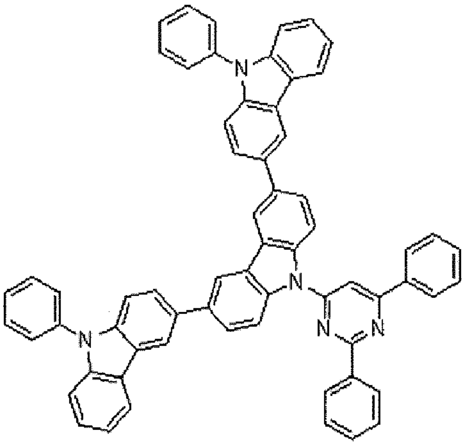

Examples of the preferred delayed fluorescent material include compounds represented by the following general formula.

[Chem. 33]

General Formula (171)

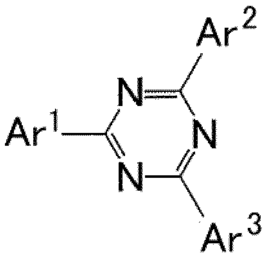

In the general formula (171), $Ar^1$ to $Ar^3$ each independently represent a substituted or unsubstituted aryl group, and at least one of them represents a substituted aryl group represented by the following general formula (172).

[Chem. 34]

General Formula (172)

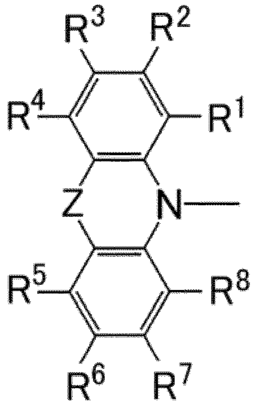

In the general formula (172), $R^1$ to $R^8$ each independently represent a hydrogen atom or a substituent. Z represents O, S, O=C or $Ar^4$-N, $Ar^4$ represents a substituted or unsubstituted aryl group. $R^1$ and $R^2$, $R^2$ and $R^3$, $R^3$ and $R^4$, $R^5$ and $R^6$, $R^6$ and $R^7$, and $R^7$ and $R^8$ each independently may be bonded to each other to form a cyclic structure.

Examples of the compound include the following compounds.

[Chem. 35]

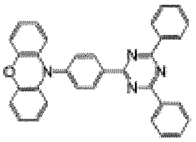
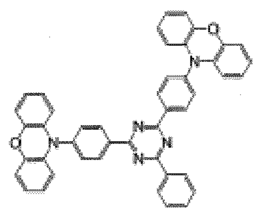
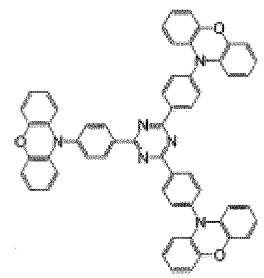

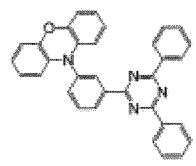
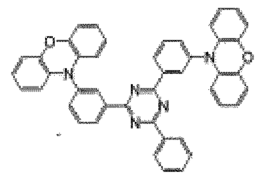
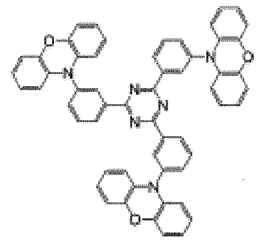

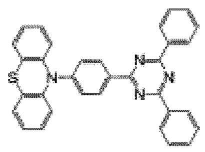
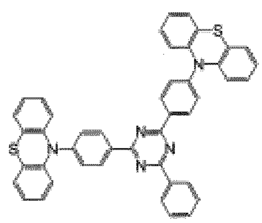
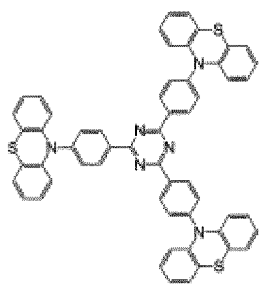

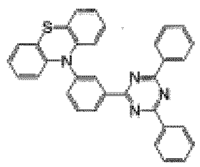
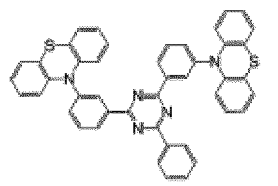
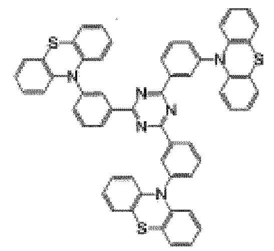

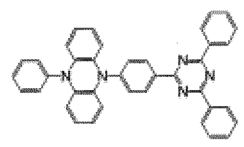
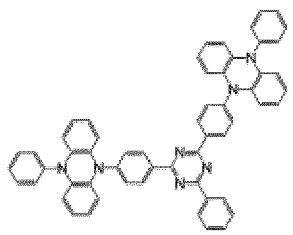
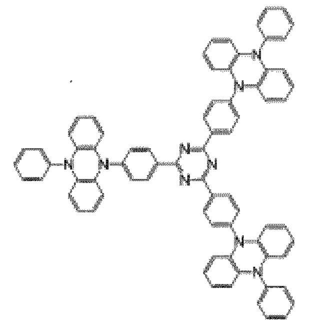

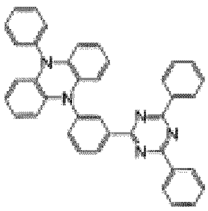

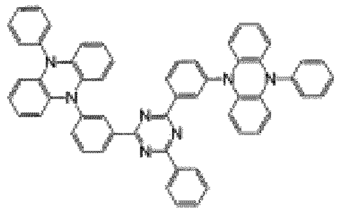

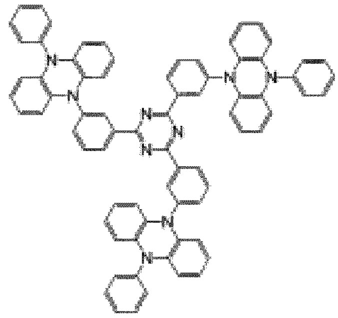

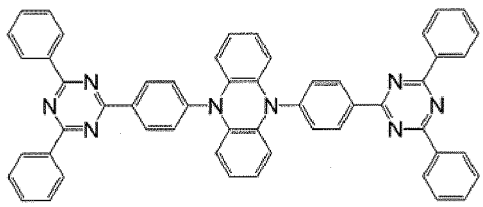

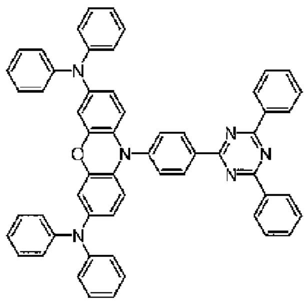

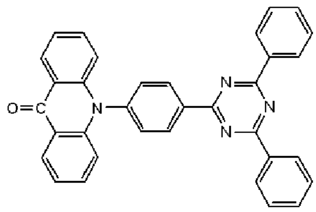

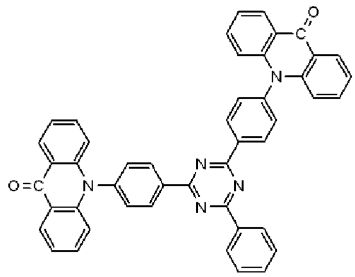

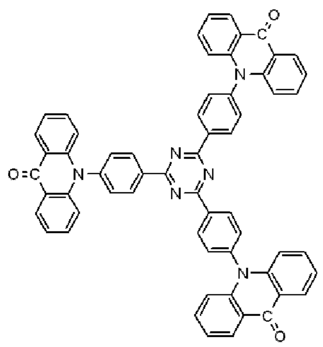

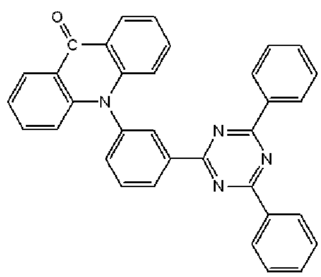

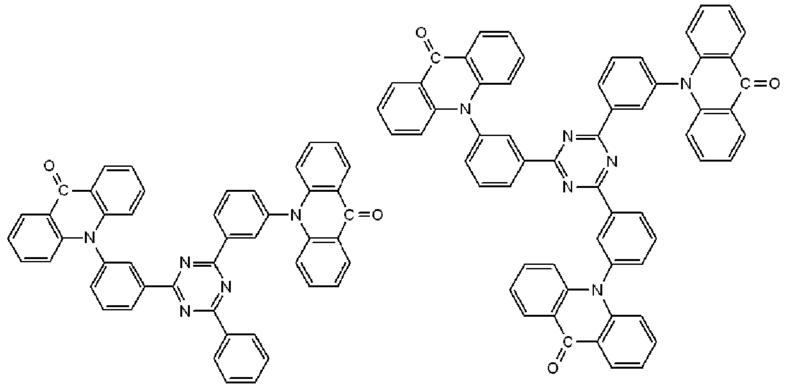

Examples of the preferred delayed fluorescent material include compounds represented by the following general formula.

[Chem. 36]

General Formula (181)

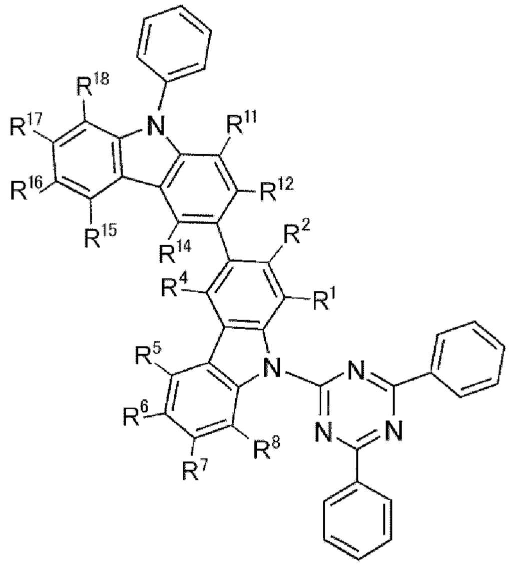

In the general formula (181), $R^1$, $R^2$, $R^4$ to $R^8$, $R^{11}$, $R^{12}$ and $R^{14}$ to $R^{18}$ each independently represent a hydrogen atom or a substituent.

Examples of the compound include the following compound.

[Chem. 37]

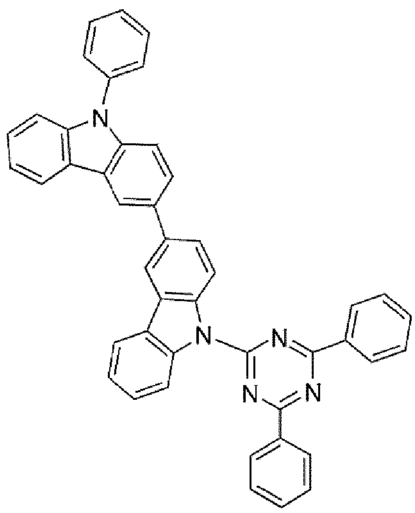

Examples of the preferred delayed fluorescent material include the following compounds.

[1] A compound represented by the following general formula (191):

General Formula (191)

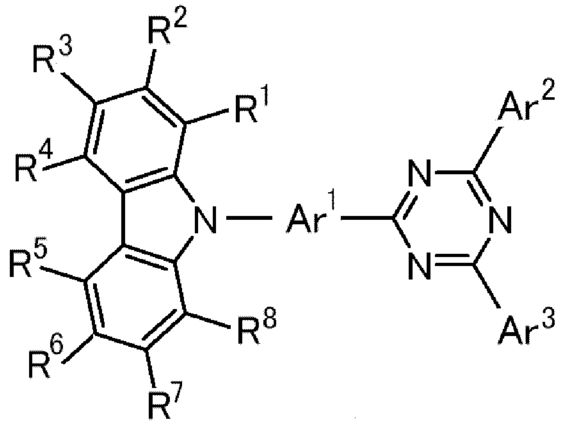

In the general formula (191), $Ar^1$ represents a substituted or unsubstituted arylene group; $Ar^2$ and $Ar^3$ each independently represent a substituted or unsubstituted aryl group. $R^1$ to $R^8$ each independently represent a hydrogen atom or a substituent, provided that at least one of $R^1$ to $R^8$ represents a substituted or unsubstituted diarylamino group, and $R^1$ and $R^2$, $R^2$ and $R^3$, $R^3$ and $R^4$, $R^5$ and $R^6$, $R^6$ and $R^7$, and $R^7$ and $R^8$ each may be bonded to each other to form a cyclic structure.

[2] The compound according to [1], wherein in the general formula (191), at least one of $R^1$ to $R^4$ represents a substituted or unsubstituted diarylamino group, and at least one of $R^5$ to $R^8$ represents a substituted or unsubstituted diarylamino group.

[3] The compound according to [2], wherein in the general formula (191), $R^3$ and $R^6$ each represent a substituted or unsubstituted diarylamino group.

[4] The compound according to any one of [1] to [3], wherein in the general formula (191), at least one of $R^1$ to $R^8$ represents a substituted or unsubstituted diphenylamino group.

[5] The compound according to any one of [1] to [4], wherein in the general formula (191), $Ar^2$ and $Ar^3$ each independently represent a substituted or unsubstituted phenyl group.

[6] The compound according to any one of [1] to [5], wherein in the general formula (191), $Ar^1$ each independently represents a substituted or unsubstituted phenylene group, a substituted or unsubstituted naphthylene group or a substituted or unsubstituted anthracenylene group.

[7] The compound according to [1], wherein the compound has a structure represented by the following general formula (192):

[Chem. 39]

General Formula (192)

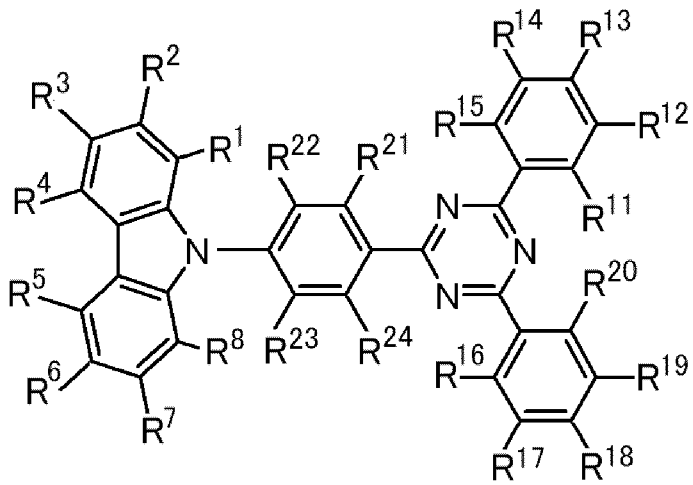

In the general formula (192), $R^1$ to $R^8$ and $R^{11}$ to $R^{24}$ each independently represent a hydrogen atom or a substituent,

provided that at least one of $R^1$ to $R^8$ represents a substituted or unsubstituted diarylamino group, and $R^1$ and $R^2$, $R^2$ and $R^3$, $R^3$ and $R^4$, $R^5$ and $R^6$, $R^6$ and $R^7$, $R^7$ and $R^8$, $R^{11}$ and $R^{12}$, $R^{12}$ and $R^{13}$, $R^{13}$ and $R^{14}$, $R^{14}$ and $R^{15}$, $R^{16}$ and $R^{17}$, $R^{17}$ and $R^{18}$, $R^{18}$ and $R^{19}$, $R^{19}$ and $R^{20}$, $R^{21}$ and $R^{22}$, and $R^{23}$ and $R^{24}$ each may be bonded to each other to form a cyclic structure.

[8] The compound according to [7], wherein in the general formula (192), at least one of $R^1$ to $R^4$ represents a substituted or unsubstituted diarylamino group, and at least one of $R^5$ to $R^8$ represents a substituted or unsubstituted diarylamino group.

[9] The compound according to [8], wherein in the general formula (192), $R^3$ and $R^6$ each represent a substituted or unsubstituted diarylamino group.

Specific examples of the compound include the following compounds. Ph represents a phenyl group.

[Chem. 40]

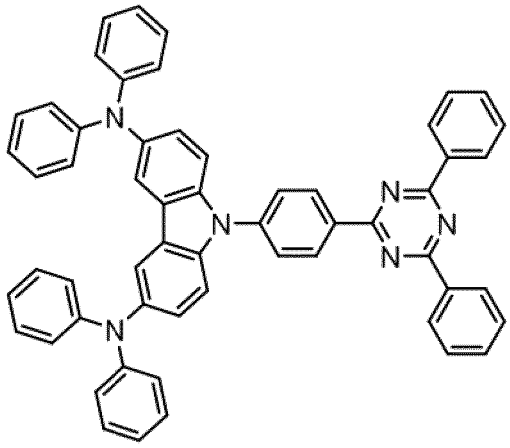

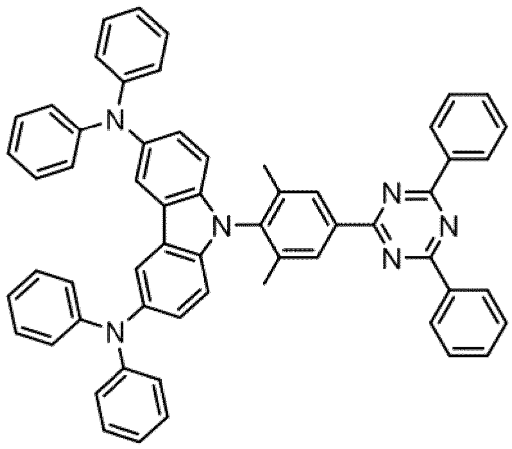

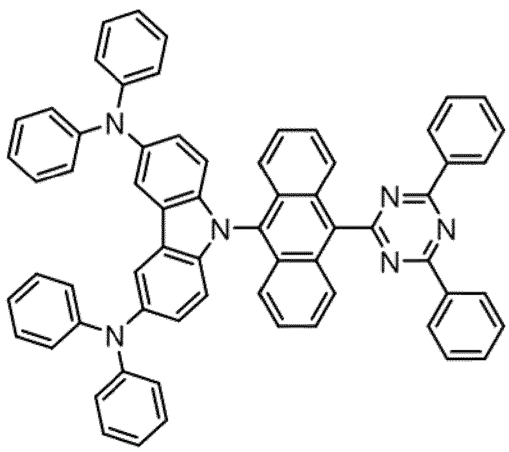

[Chem. 41]

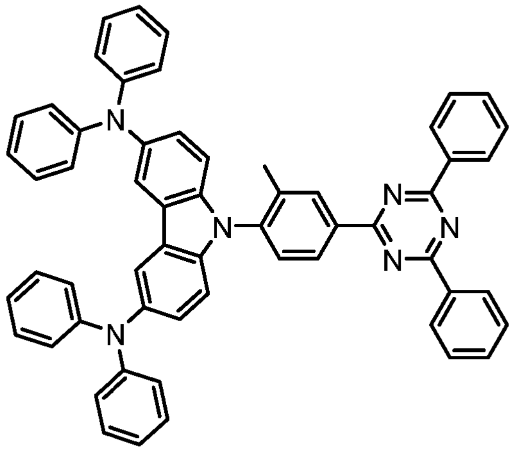

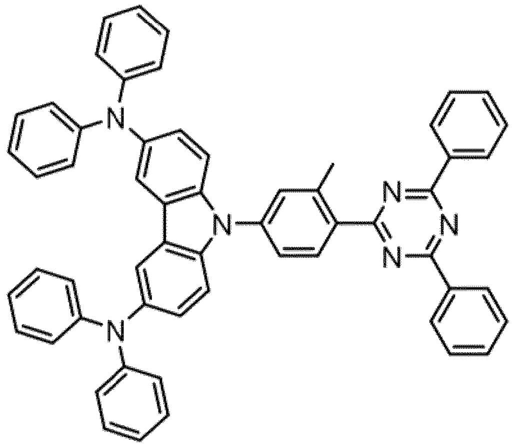

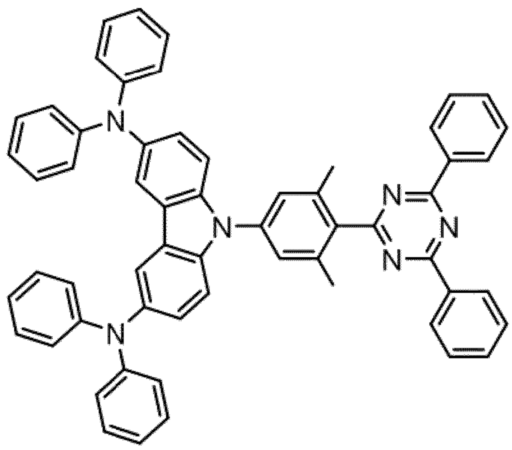

[Chem. 42]

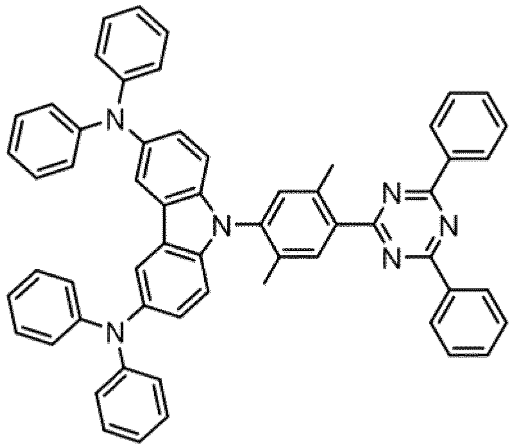

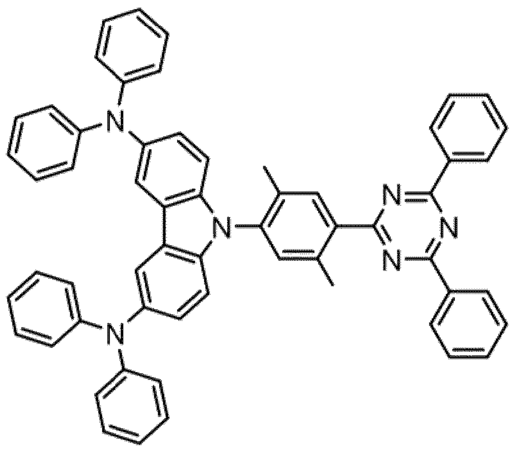

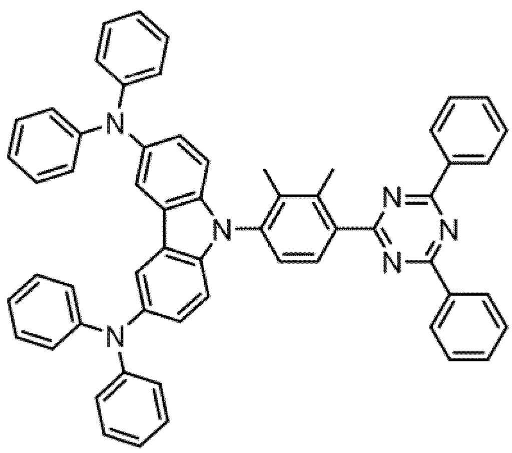

[Chem. 43]

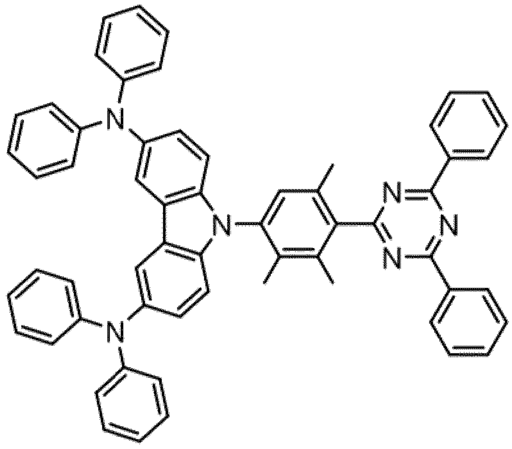

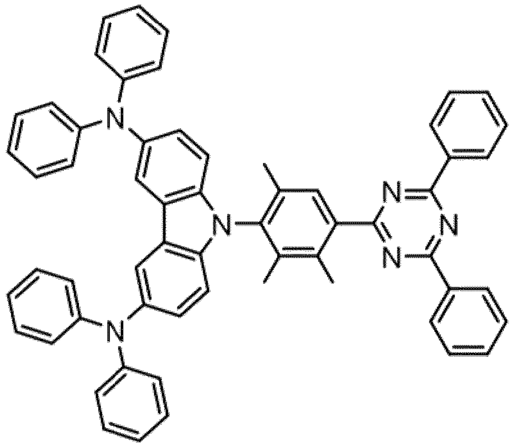

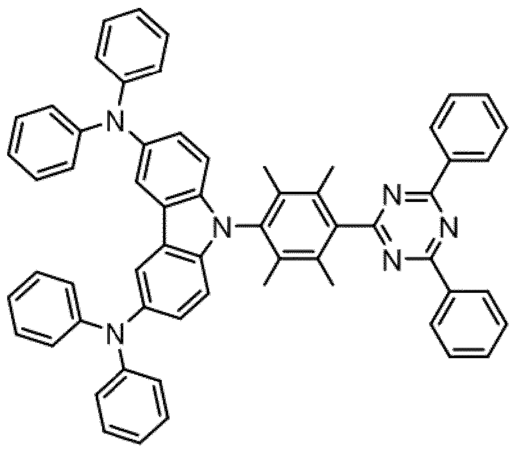

Examples of the preferred delayed fluorescent material include the following compounds.

[1] A compound represented by the following general formula (201):

[Chem. 44]

General Formula (201)

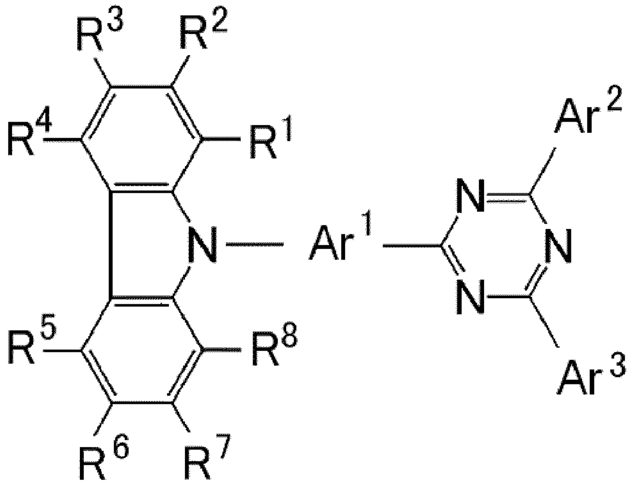

(In the above formula, $R^1$ to $R^8$ each independently represent a hydrogen atom or a substituent, provided that at least one of $R^1$ to $R^8$ represents a substituted or unsubstituted carbazolyl group. $Ar^1$ to $Ar^3$ each independently represent a substituted or unsubstituted aromatic ring or a heteroaromatic ring.

[2] The compound according to [1], wherein in the general formula (201), at least one of $R^3$ and $R^6$ represents a substituted or unsubstituted carbazolyl group.

[3] The compound according to [1] or [2], wherein the carbazolyl group is a 1-carbazolyl group, a 2-carbazolyl group, a 3-carbazolyl group or a 4-carbazolyl group.

[4] The compound according to any one of [1] to [3], wherein the carbazolyl group has a substituent on the nitrogen atom in the carbazole cyclic structure.

[5] The compound according to any one of [1] to [4], wherein in the general formula (201), at least one of $Ar^1$, $Ar^2$ and $Ar^3$ represents a benzene ring or a naphthalene ring. [0077]

[6] The compound according to any one of [1] to [5], wherein in the general formula (201), $Ar^1$, $Ar^2$ and $Ar^3$ each represent the same aromatic ring or the same heteroaromatic ring.

[7] The compound according to any one of [1] to [6], wherein in the general formula (201), $Ar^1$, $Ar^2$ and $Ar^3$ each represent a benzene ring.

Examples of the compound include the following compounds.

[Chem. 45]

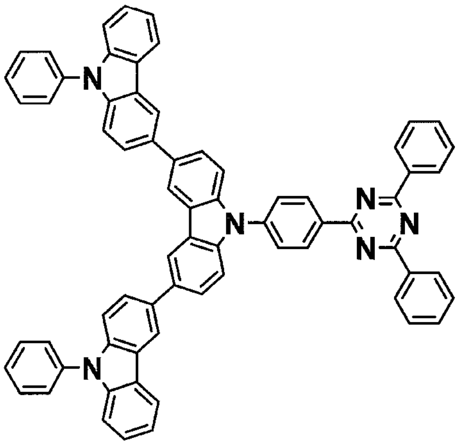

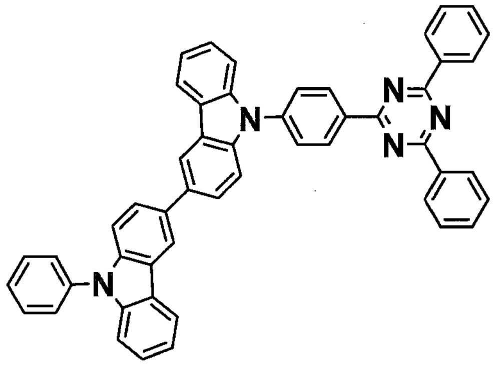

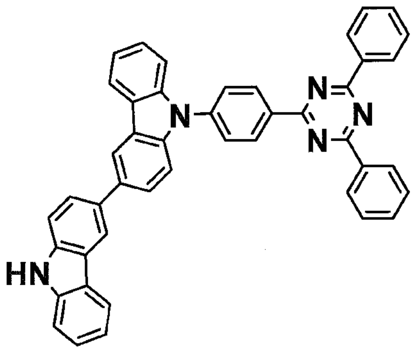

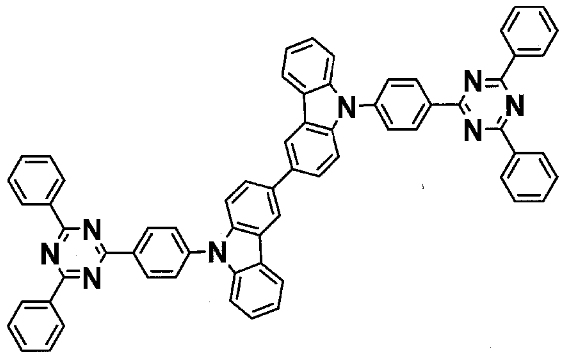

[Chem. 46]

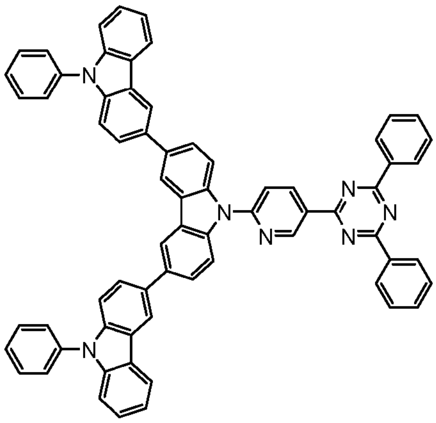

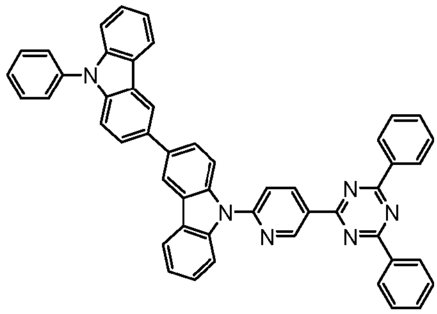

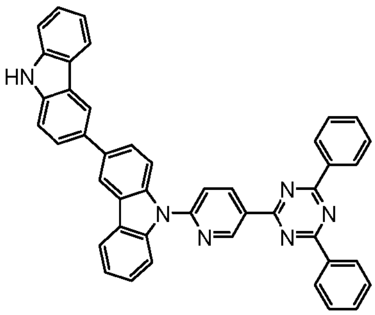

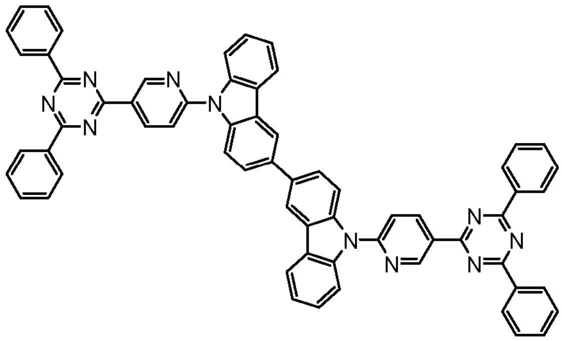

[Chem. 47]

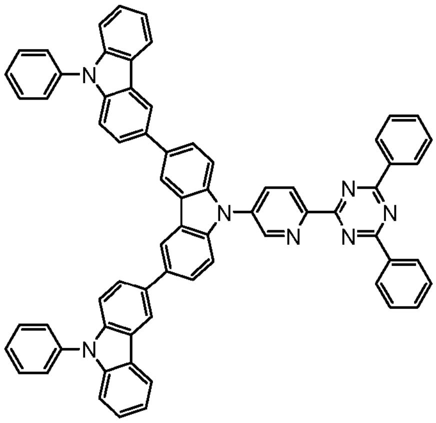

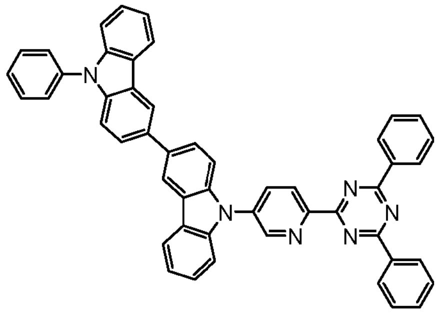

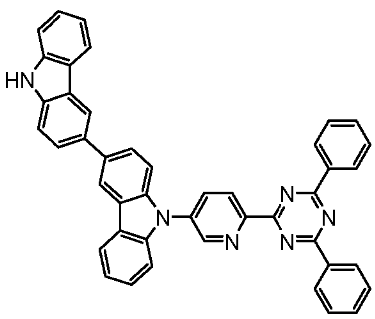

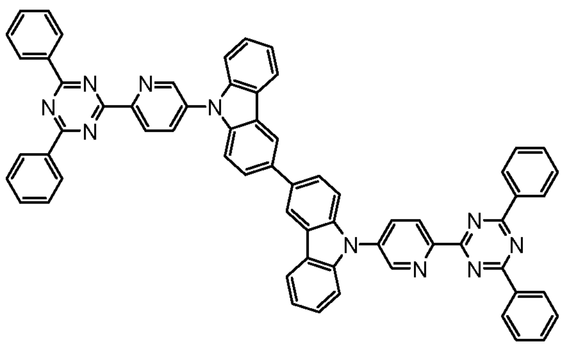

[Chem. 48]

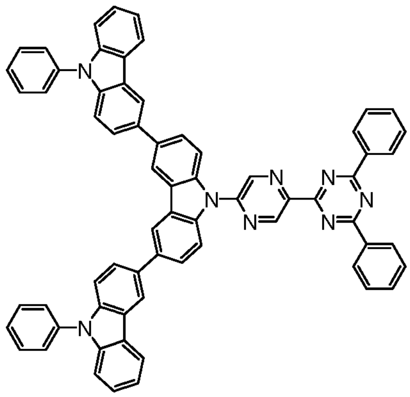

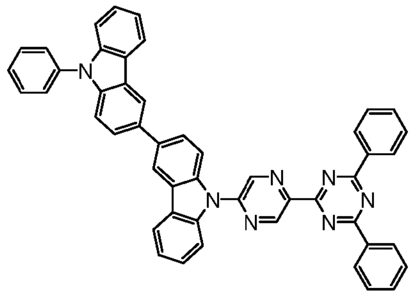

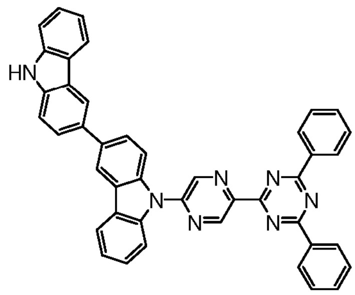

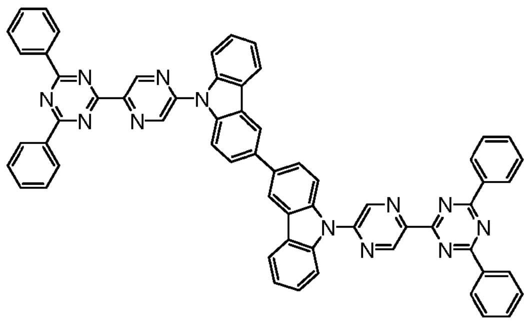

[Chem. 49]

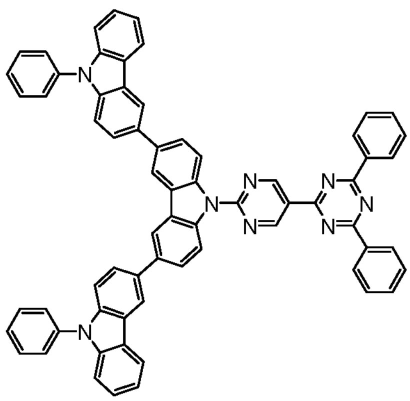

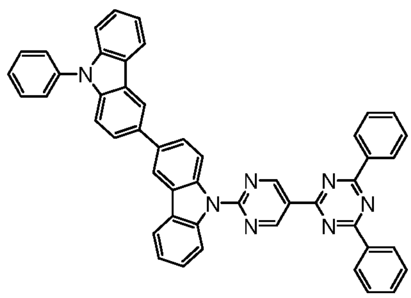

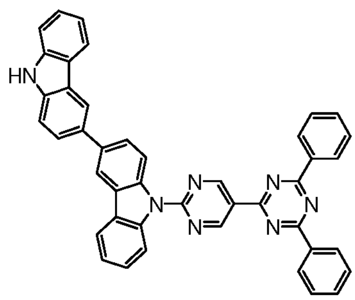

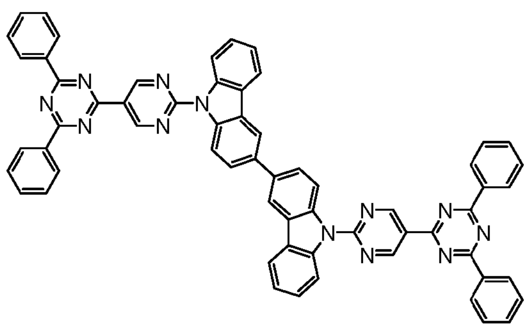

[Chem. 50]

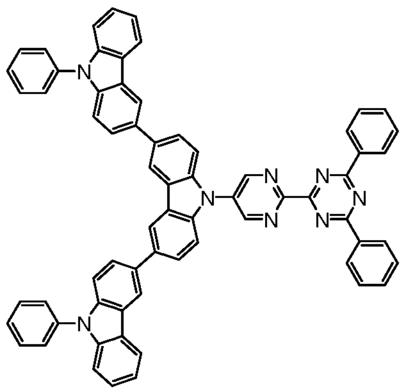

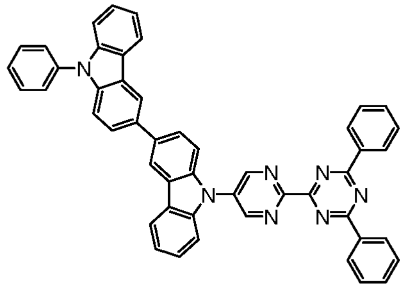

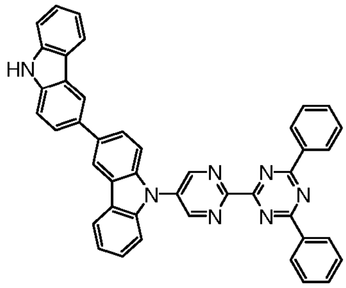

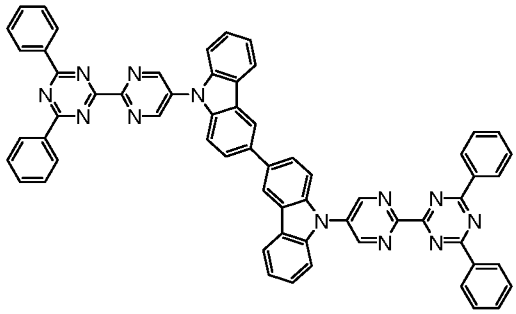

[Chem. 51]

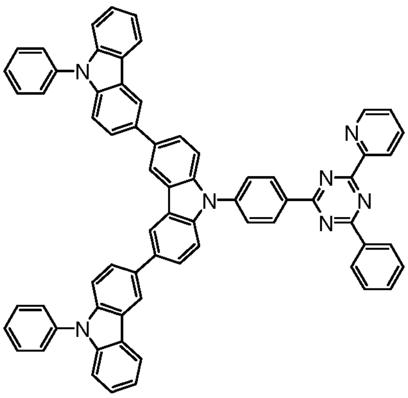

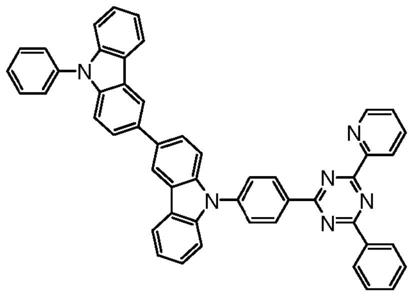

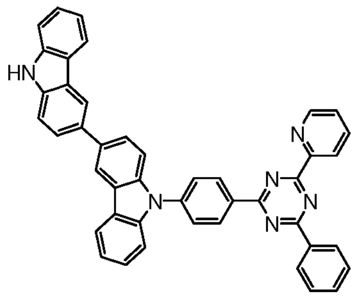

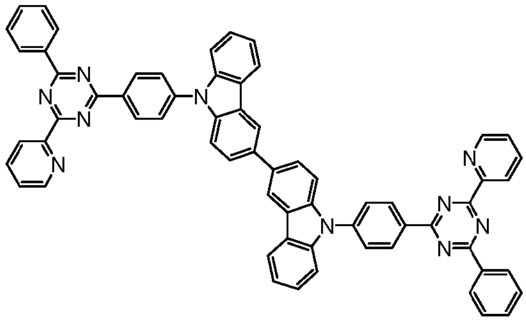

[Chem. 52]

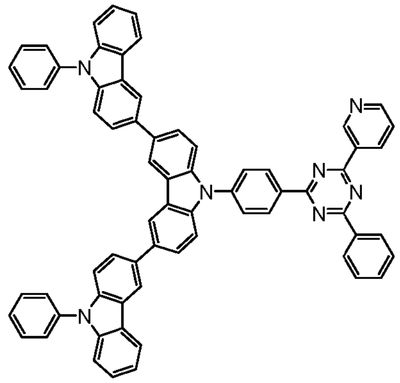

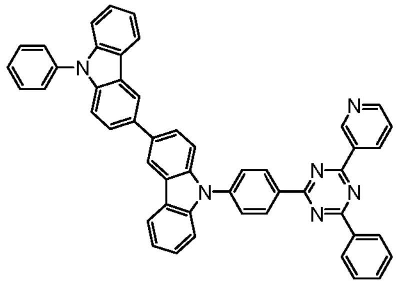

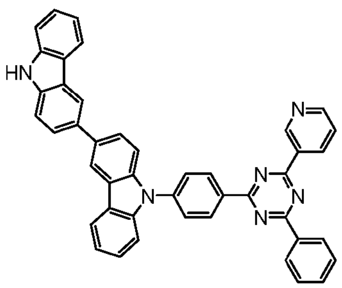

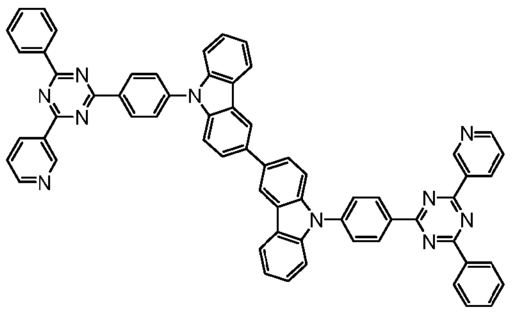

[Chem. 53]

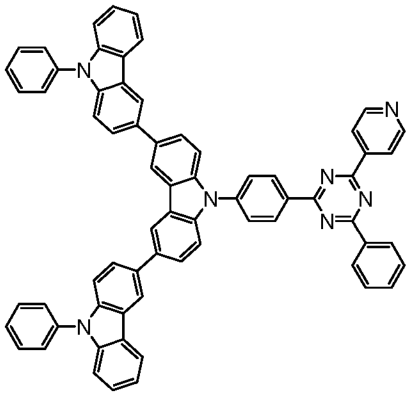

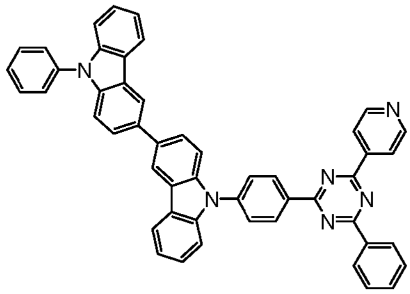

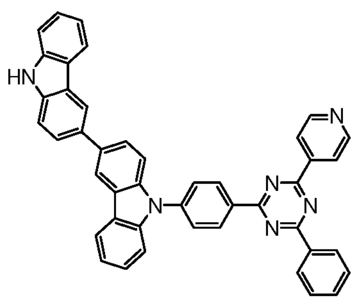

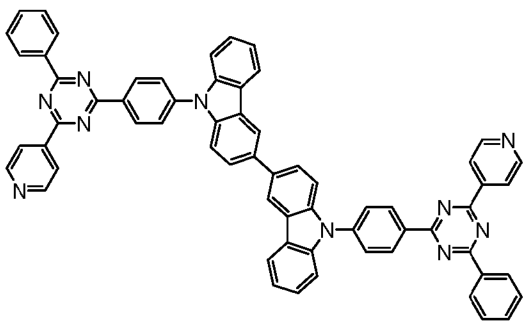

[Chem. 54]

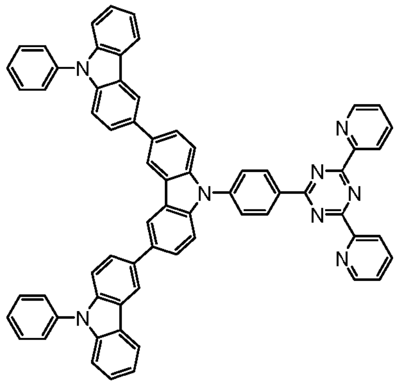

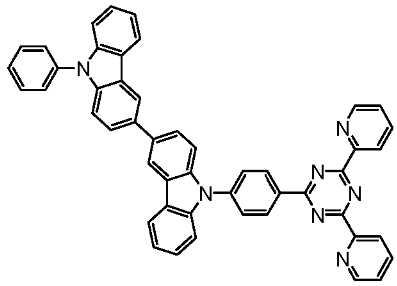

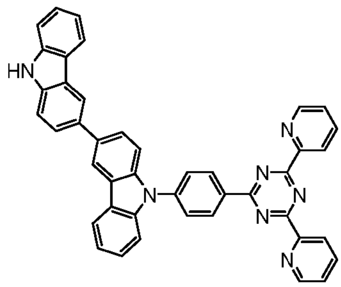

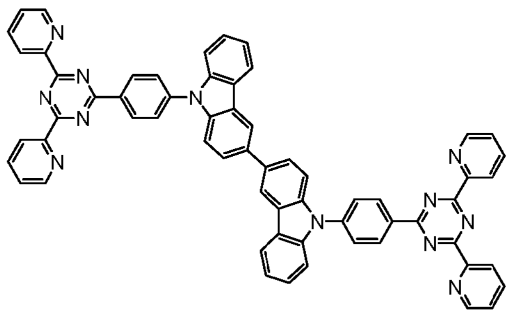

[Chem. 55]

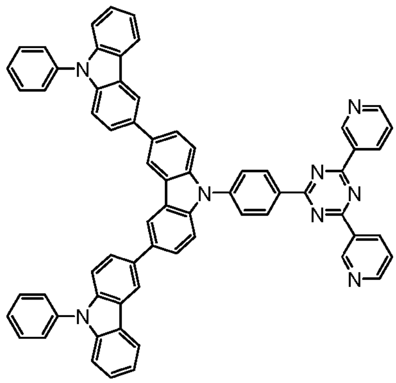

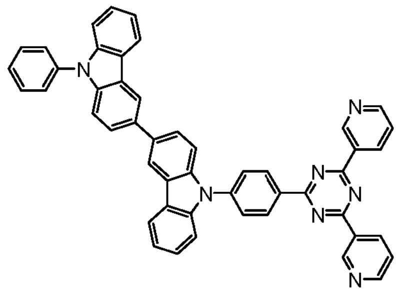

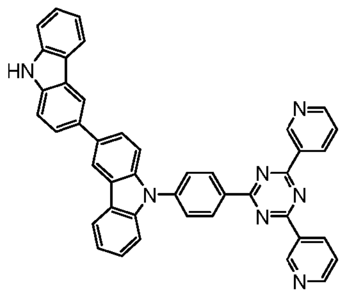

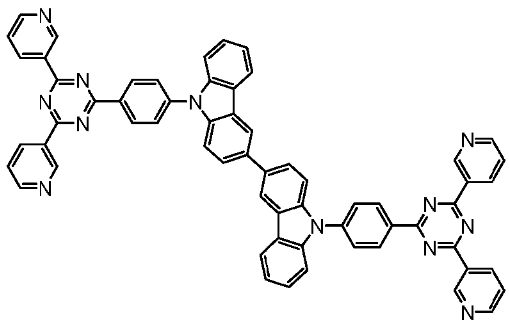

[Chem. 56]

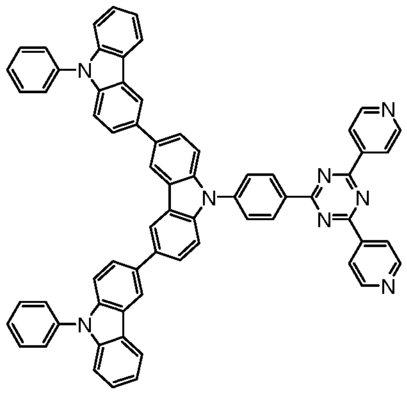

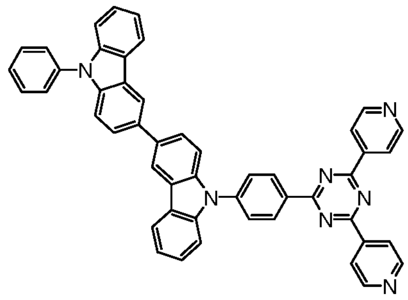

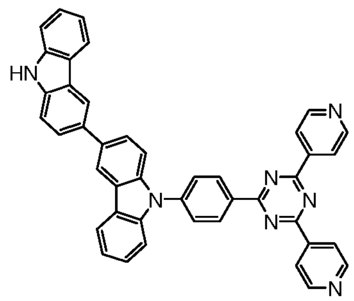

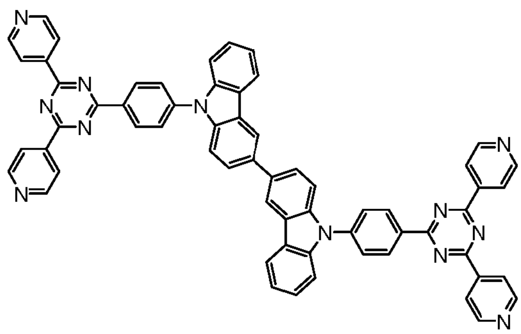

[Chem. 87]

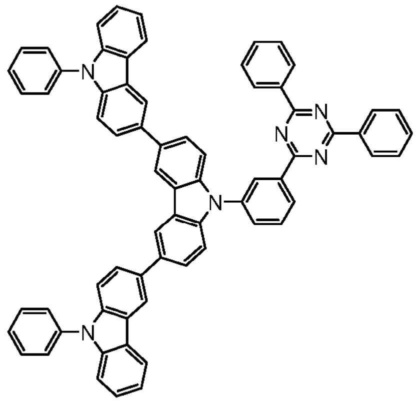

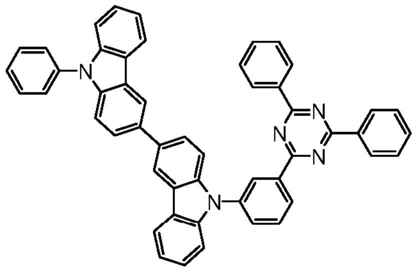

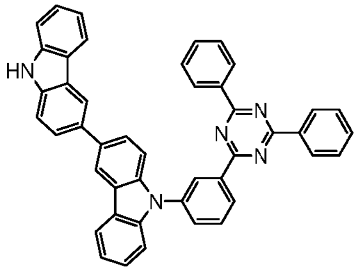

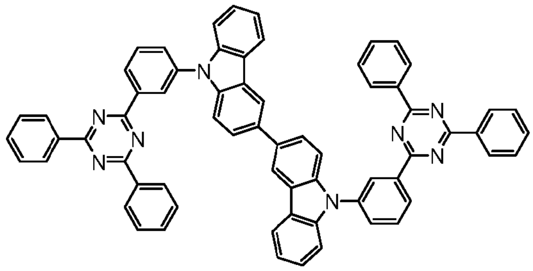

[Chem. 58]

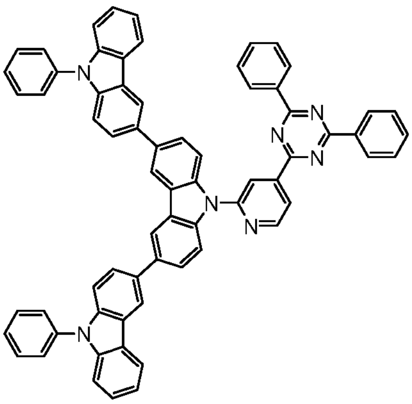

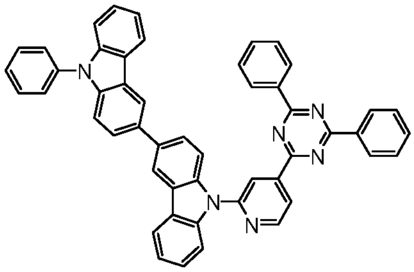

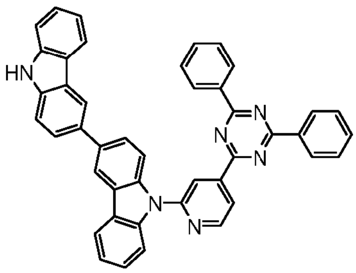

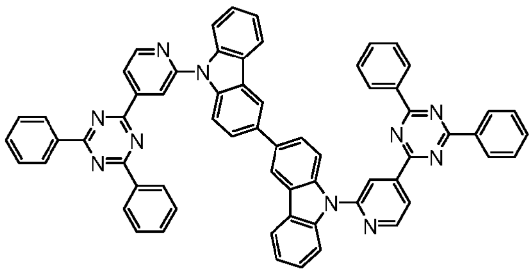

[Chem. 59]

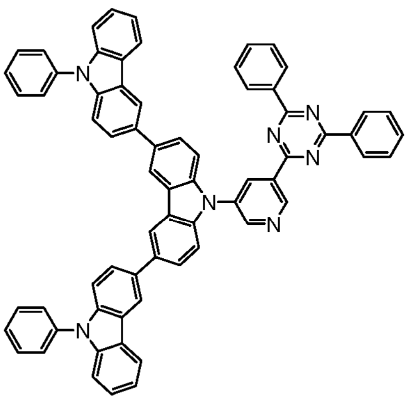

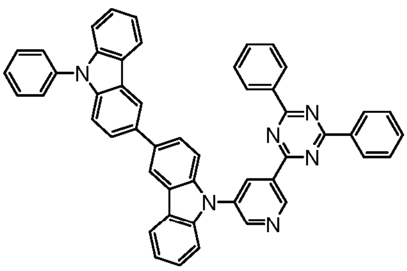

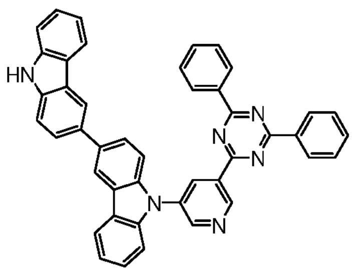

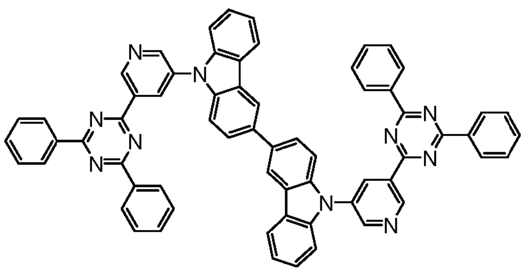

[Chem. 60]

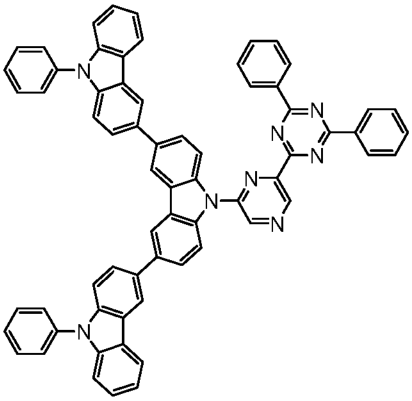

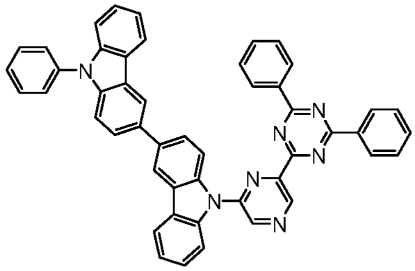

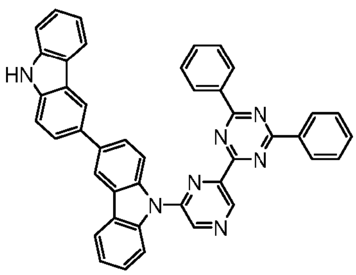

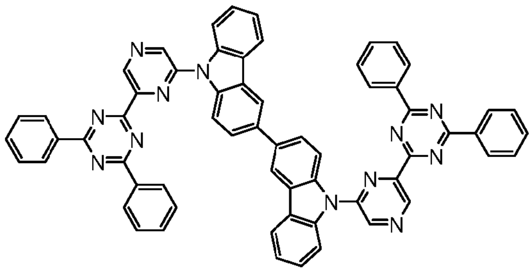

[Chem. 61]

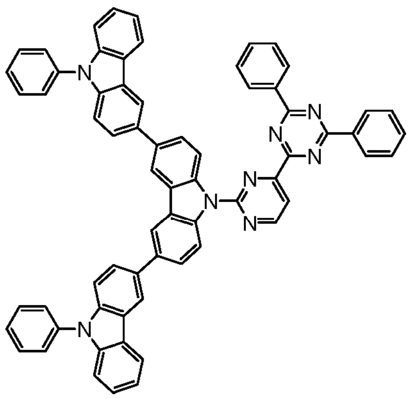

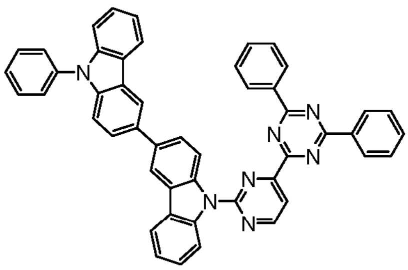

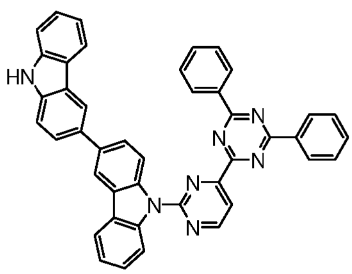

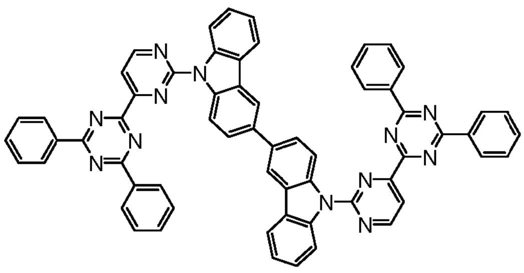

[Chem. 62]

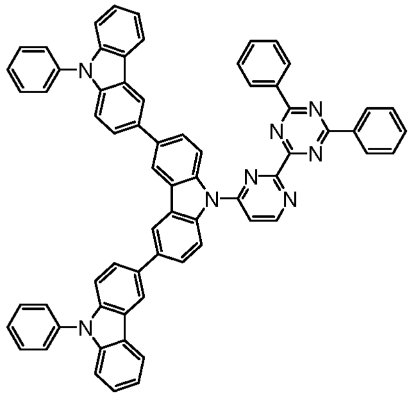

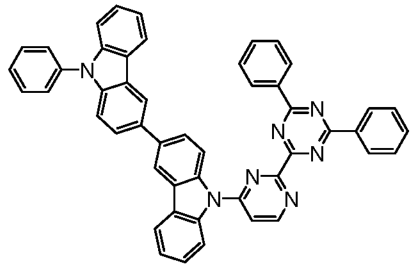

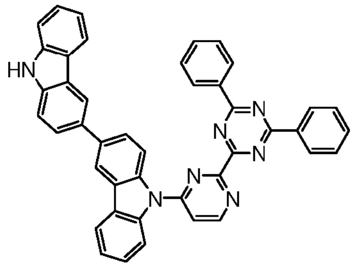

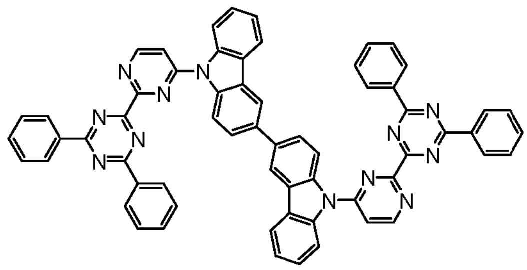

[Chem. 63]

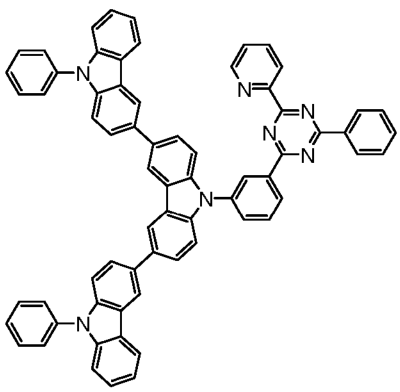

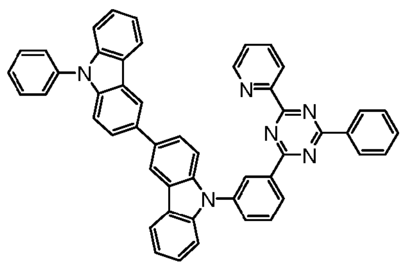

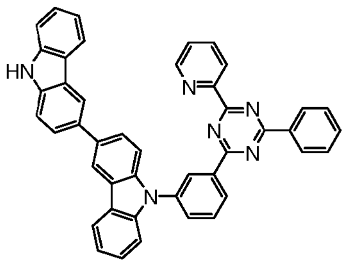

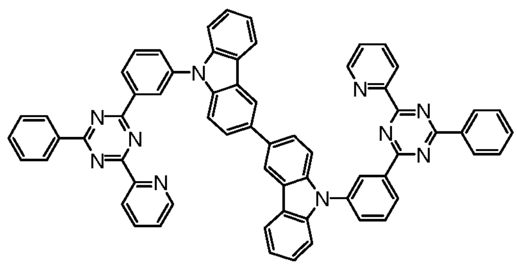

[Chem. 64]

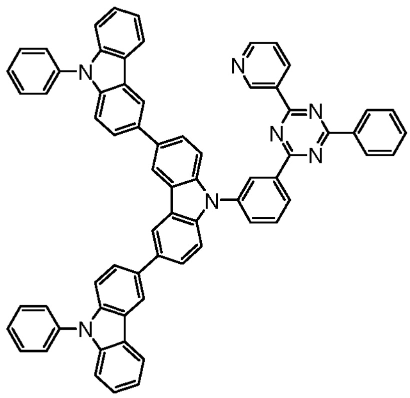
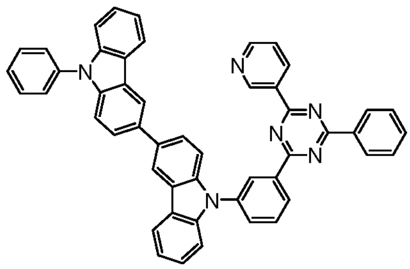
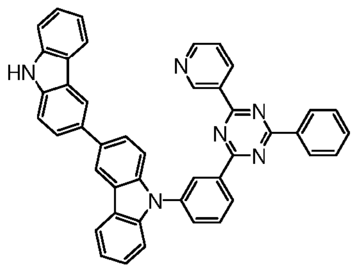
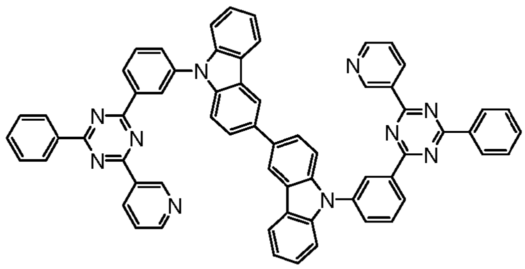

[Chem. 65]

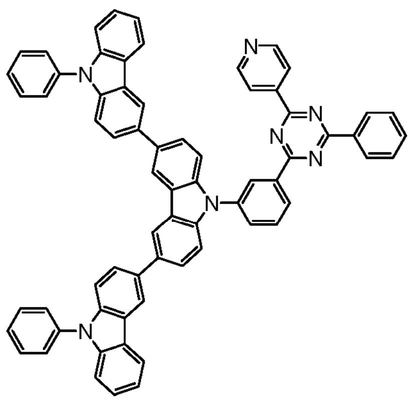
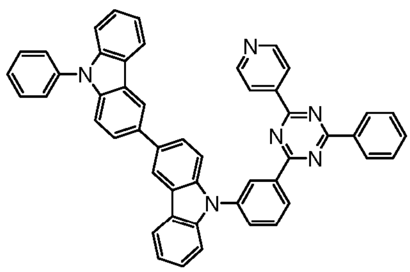
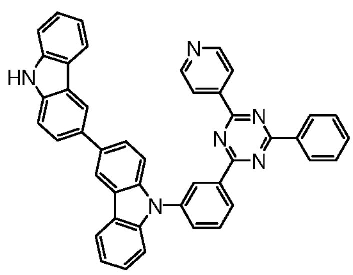
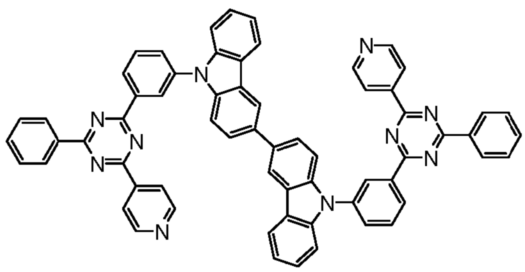

[Chem. 66]

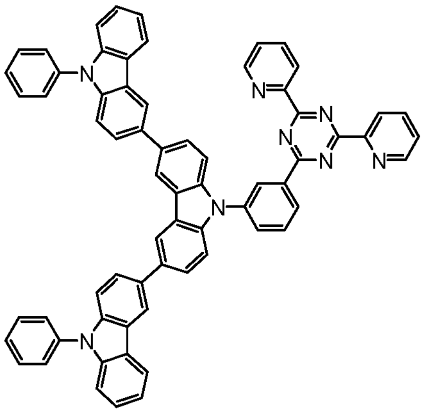

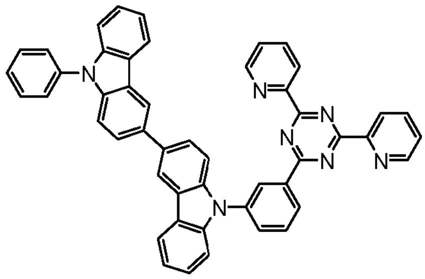

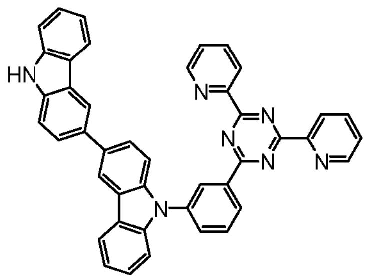

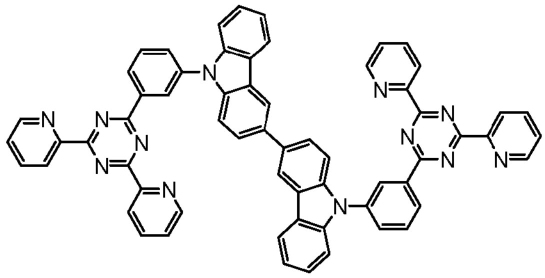

[Chem. 67]

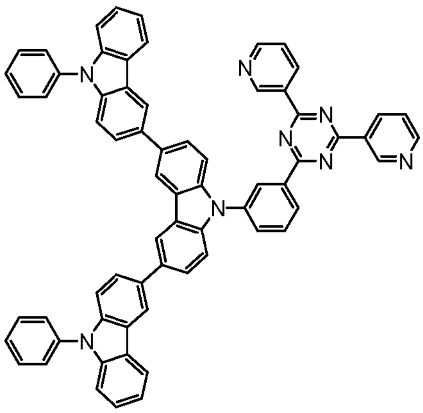

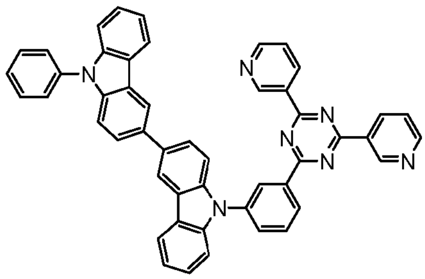

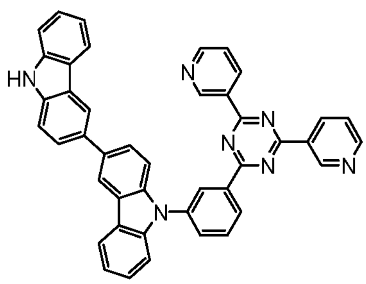

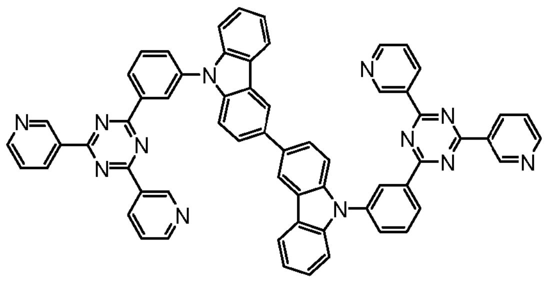

[Chem. 68]

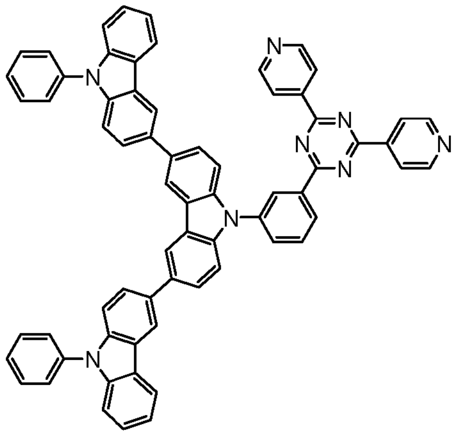

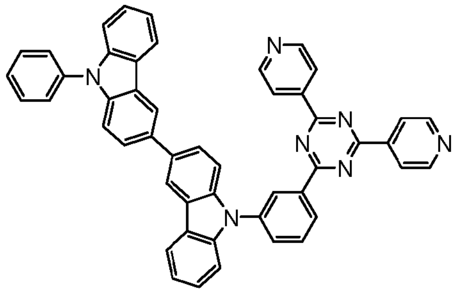

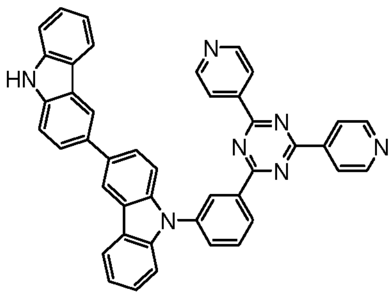

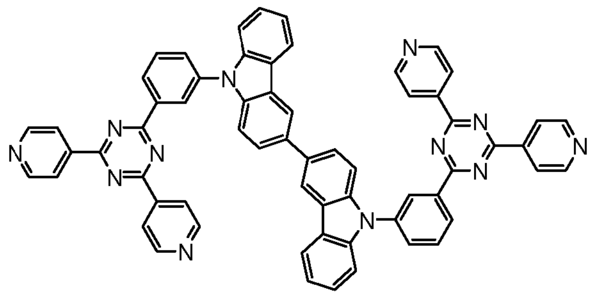

Examples of the preferred delayed fluorescent material include compounds represented by the following general formulae.

[Chem. 69]

General Formula (211)

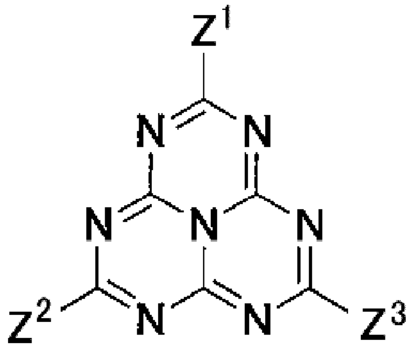

In the general formula (211), $Z^1$, $Z^2$ and $Z^3$ each independently represent a substituent.

[Chem. 70]

General Formula (212)

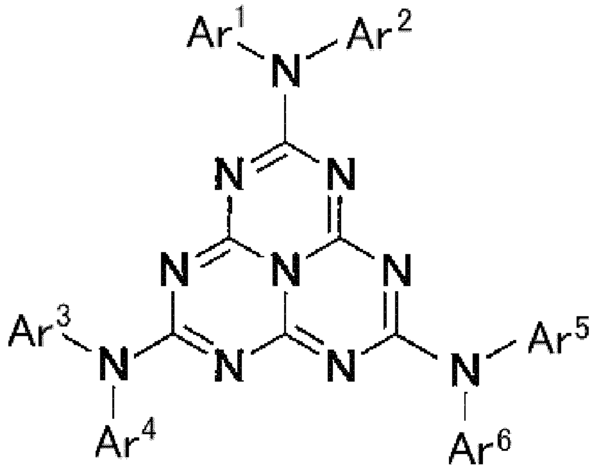

In the general formula (212), $Ar^1$, $Ar^2$, $Ar^3$, $Ar^4$, $Ar^5$ and $Ar^6$ each independently represent a substituted or unsubstituted aryl group.

Specific examples of the compound represented by the general formula (212) include the compound represented by the following structural formula.

[Chem. 71]

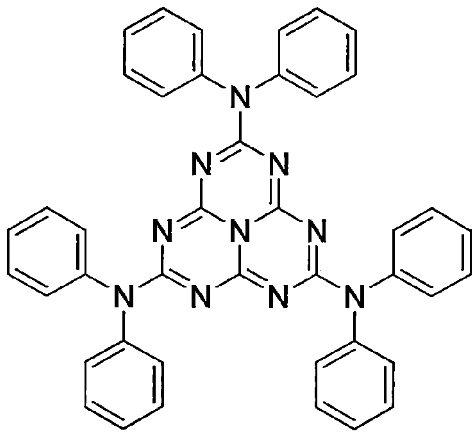

Compound 4001

Specific examples of the compound represented by the general formula (212) include the compounds shown in the following table. Here, $Ar^1$, $Ar^2$, $Ar^3$, $Ar^4$, $Ar^5$ and $Ar^6$ are the same as each other, and are expressed by Ar.

Table 21

| Compound No. | Ar |
|---|---|
| 4002 | 4-fluorophenyl |
| 4003 | 3-fluorophenyl |
| 4004 | 2-fluorophenyl |
| 4005 | 3,5-difluorophenyl |
| 4006 | 2,4,6-trifluorophenyl |
| 4007 | 4-methylphenyl |

(continued)

| Compound No. | Ar |
|---|---|
| 4008 | 3-methylphenyl |
| 4009 | 2-methylphenyl |
| 4010 | 3,5-dimethylphenyl |
| 4011 | 2,4,6-trimethylphenyl |
| 4012 | 4-ethylphenyl |
| 4013 | 3-ethylphenyl |
| 4014 | 2-ethylphenyl |
| 4015 | 3,5-diethylphenyl |
| 4016 | 4-propylphenyl |
| 4017 | 3-propylphenyl |
| 4018 | 3,5-dipropylphenyl |
| 4019 | 4-tert-butylphenyl |
| 4020 | 3-tert-butylphenyl |
| 4021 | 3,5-di-tert-butylphenyl |
| 4022 | 1-naphthyl |
| 4023 | 2-naphthyl |

Examples of the preferred delayed fluorescent material include compounds represented by the following general formula.

[Chem. 72]

General Formula (221)

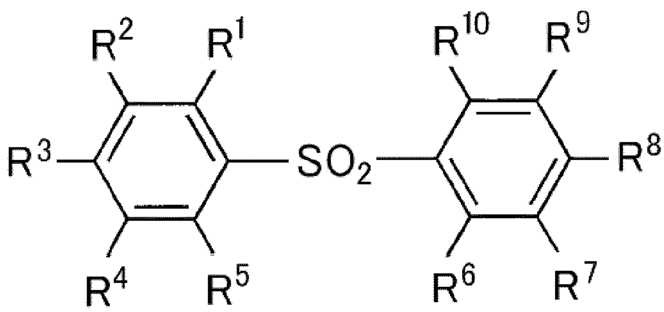

In the general formula (221), $R^1$ to $R^{10}$ each independently represent a hydrogen atom or a substituent, provided that at least one of $R^1$ to $R^{10}$ represents a substituted or unsubstituted aryl group, a substituted or unsubstituted diarylamino group or a substituted or unsubstituted 9-carbazolyl group. $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, and $R^9$ and $R^{10}$ each may be bonded to each other to form a cyclic structure.

Specific examples of the compound include the following compounds.

[Chem. 73]

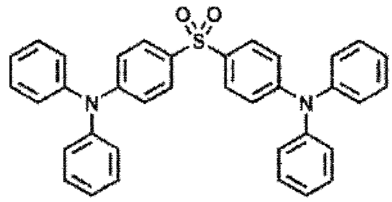

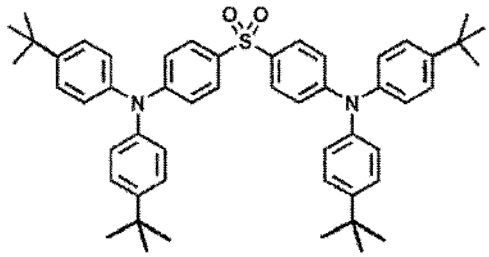

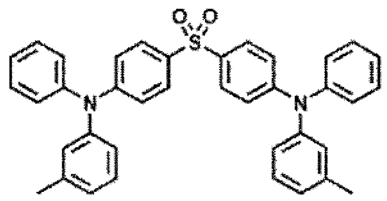

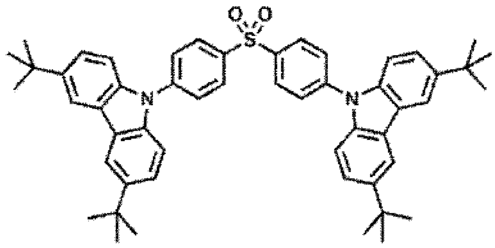

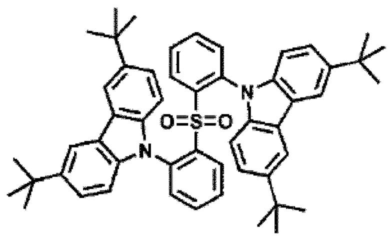

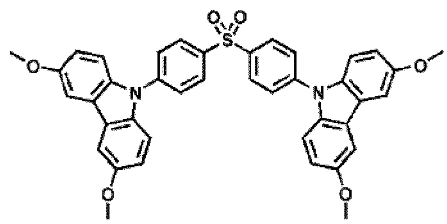

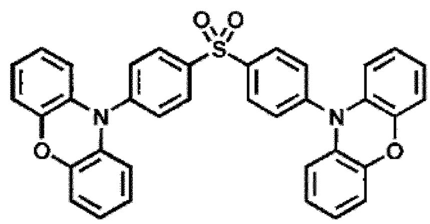

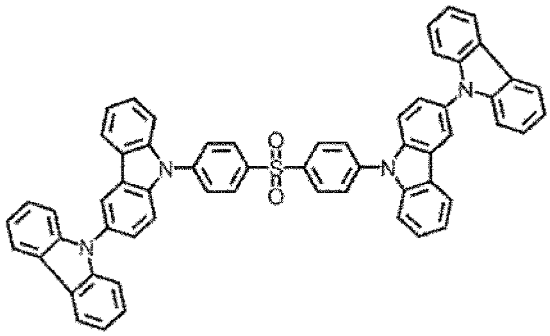

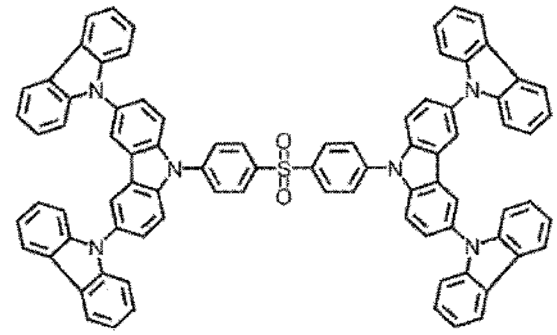

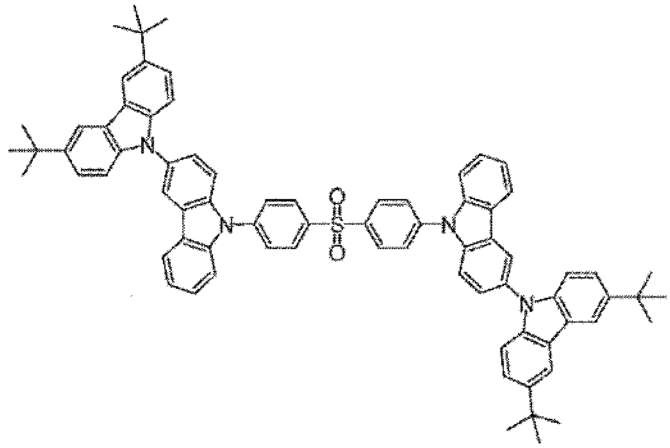

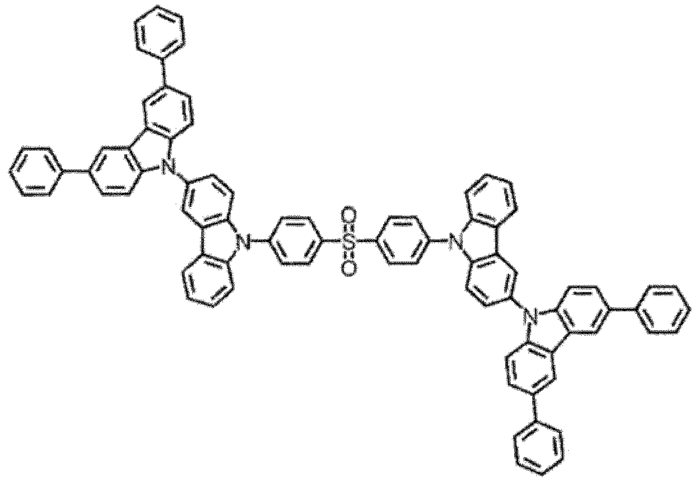

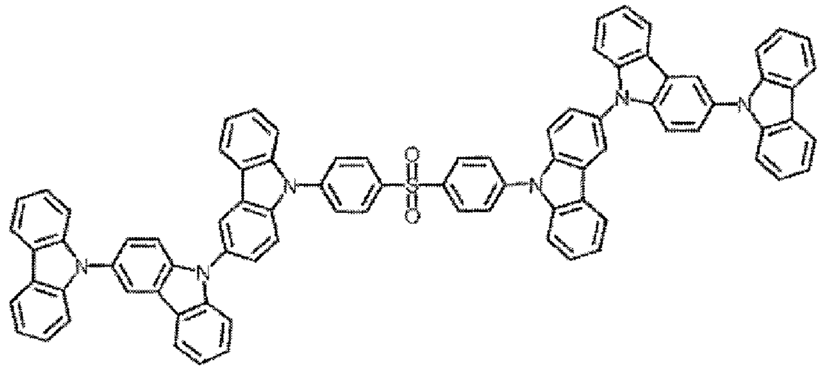

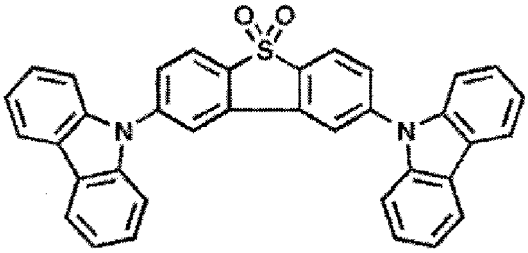

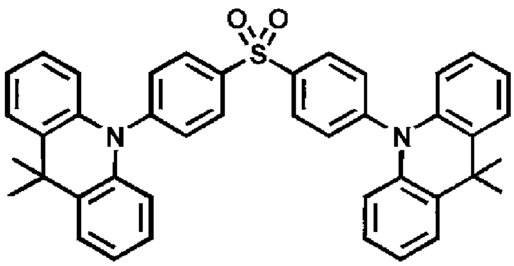

Examples of the preferred delayed fluorescent material include compounds represented by the following general formula.

[Chem. 74]

General Formula (231)

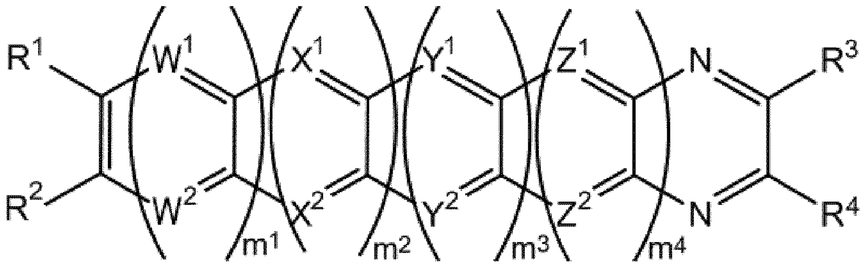

In the general formula (231), $R^1$ to $R^4$ each independently represent a hydrogen atom or a substituted or unsubstituted (N,N-diarylamino)aryl group, provided that at least one of $R^1$ to $R^4$ represents a substituted or unsubstituted (N,N-diarylamino)aryl group. Two aryl groups constituting the diarylamino moiety of the (N,N-diarylamino)aryl group may be bonded to each other. $W^1$, $W^2$, $X^1$, $X^2$, $Y^1$, $Y^2$, $Z^1$ and $Z^2$ each independently represent a carbon atom or a nitrogen atom. $m^1$ to $m^4$ each independently represent 0, 1 or 2.

Examples of the compound include the following compounds.

[Chem. 75]

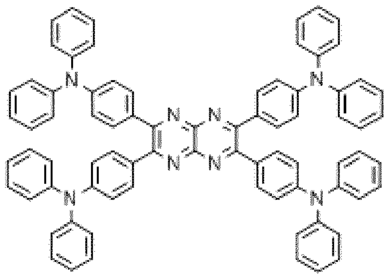

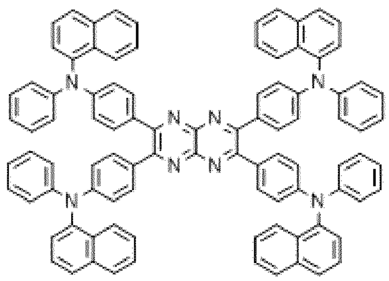

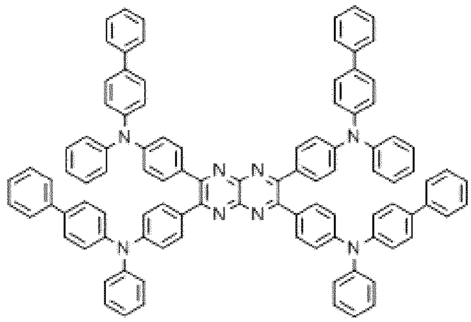

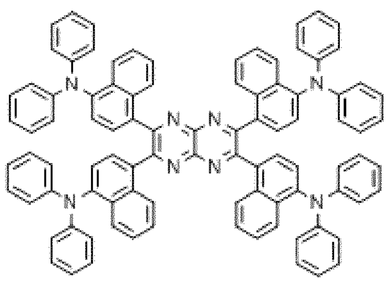

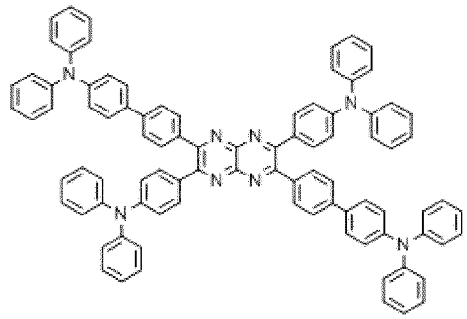

[Chem. 76]

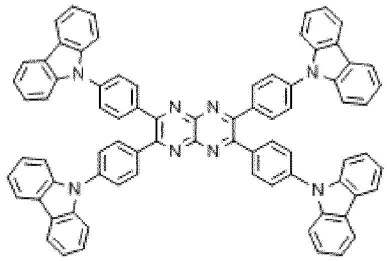

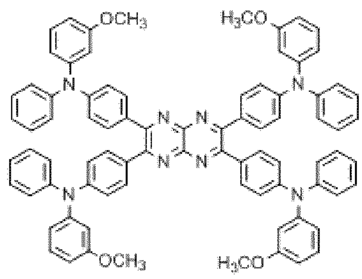

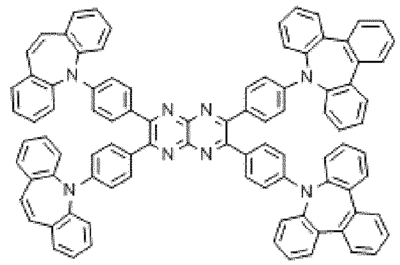

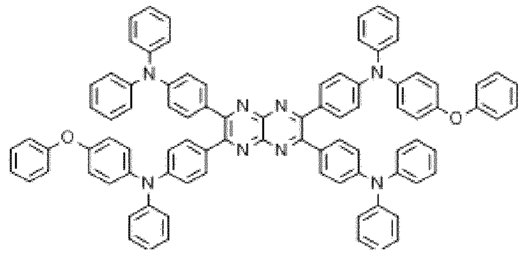

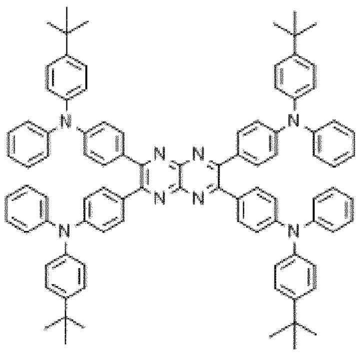

[Chem. 77]

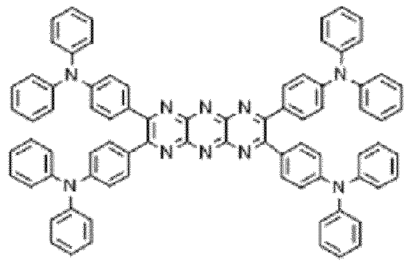

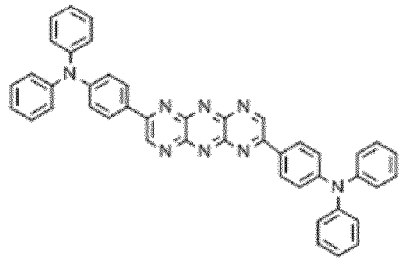

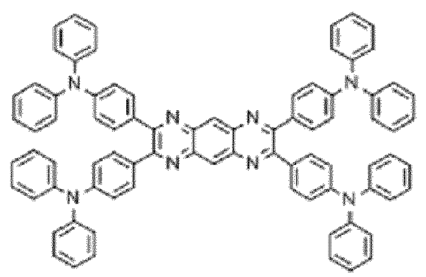

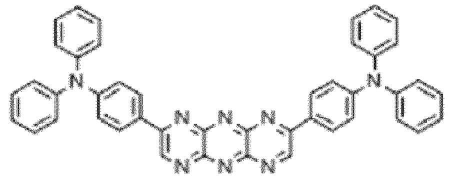

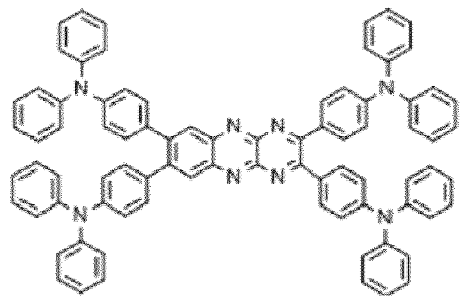

[Chem. 78]

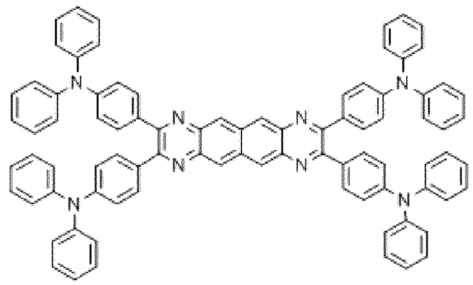

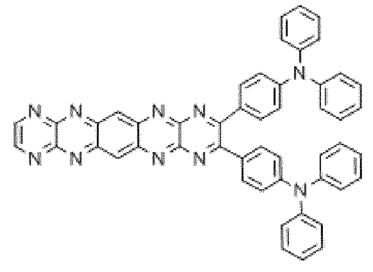

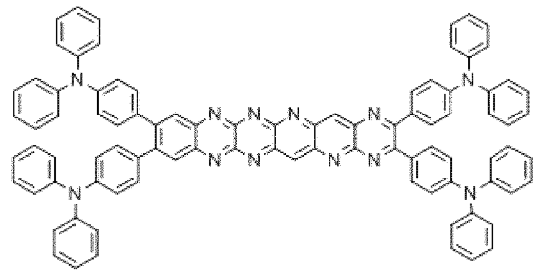

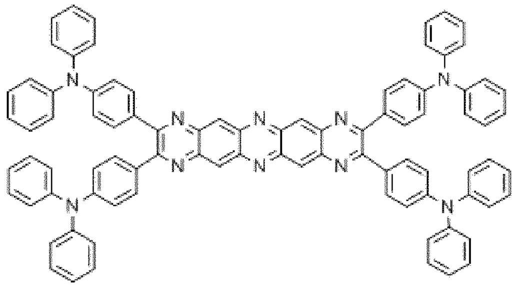

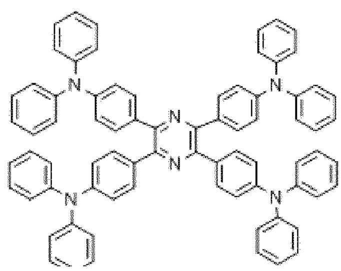

Examples of the preferred delayed fluorescent material include compounds represented by the following general formula.

[Chem. 79]

General Formula (241)

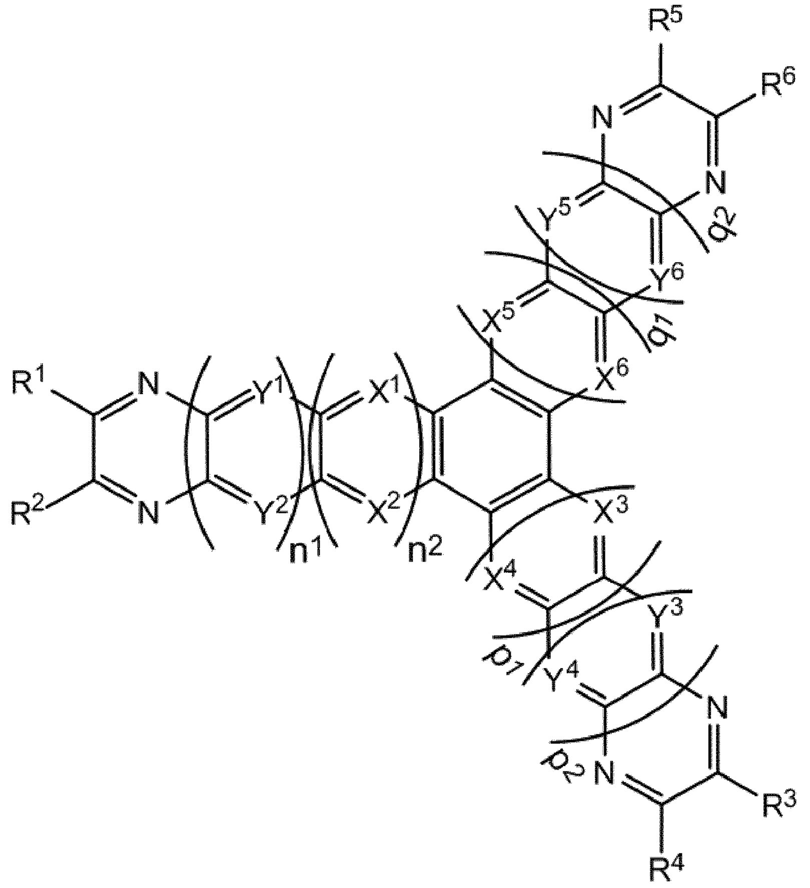

In the general formula (241), $R^1$ to $R^6$ each independently represent a hydrogen atom or a substituent, provided that at least one of $R^1$ to $R^6$ represents a substituted or unsubstituted (N,N-diarylamino)aryl group, and two aryl groups constituting the diarylamino moiety of the (N,N-diarylamino)aryl group may be bonded to each other. $X^1$ to $X^6$ and $Y^1$ to $Y^6$ each independently represent a carbon atom or a nitrogen atom. $n^1$, $n^2$, $p^1$, $p^2$, $q^1$ and $q^2$ each independently represent 0, 1 or 2.

Examples of the compound include the following compounds.

[Chem. 80]

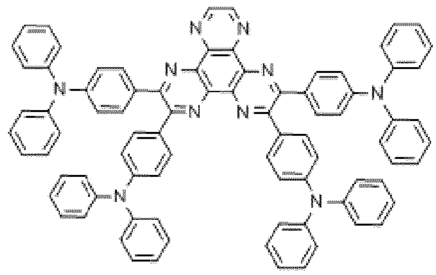

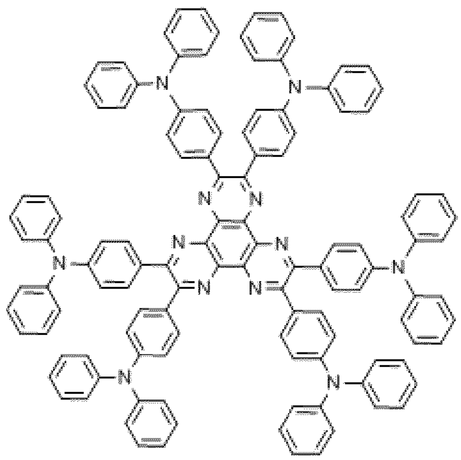

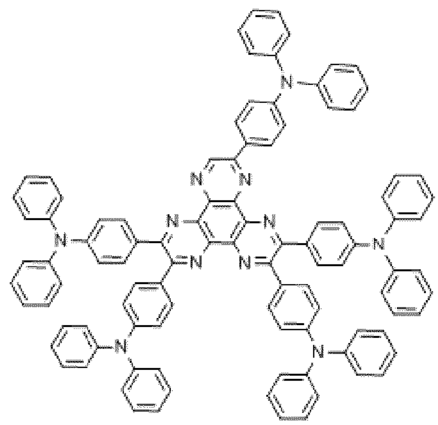

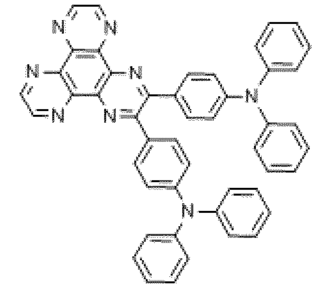

[Chem. 81]

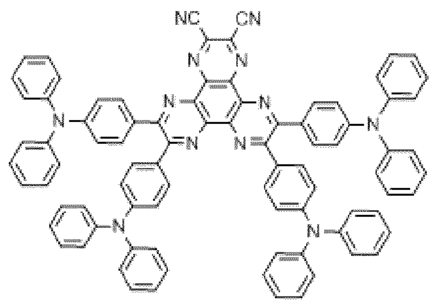

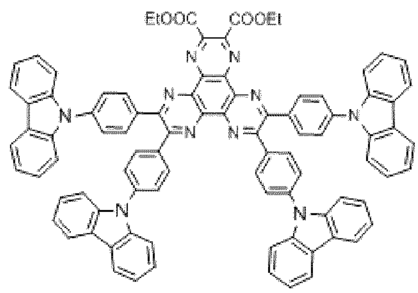

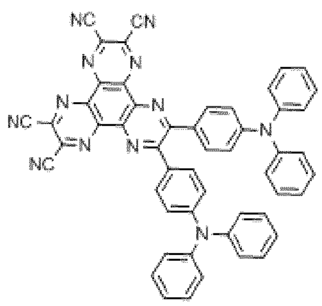

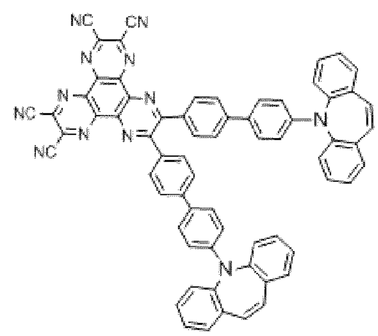

[Chem. 82]

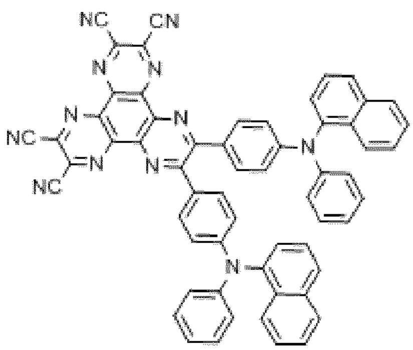

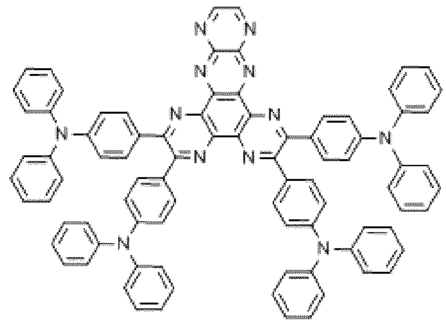

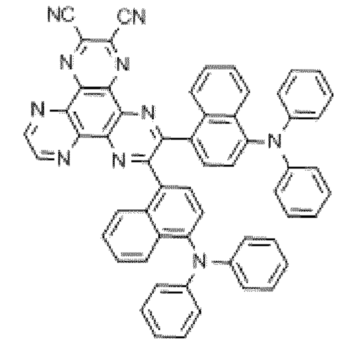

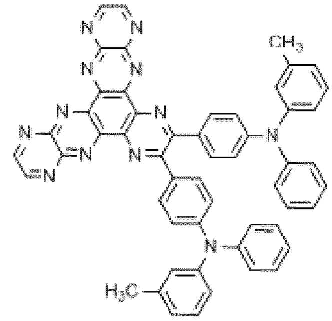

[Chem. 83]

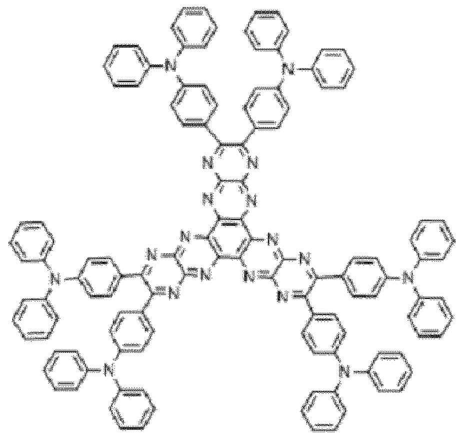

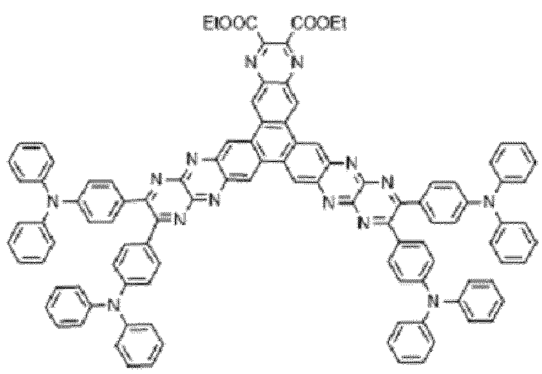

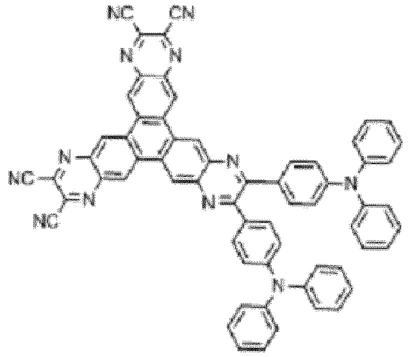

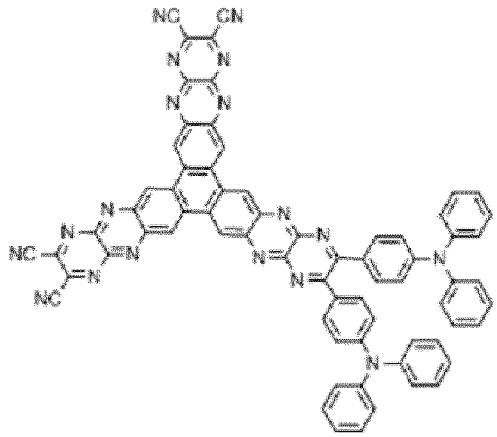

Examples of the preferred delayed fluorescent material include the following compounds.

[1] A compound represented by the following general formula (251):

[Chem. 84]

General Formula (251)

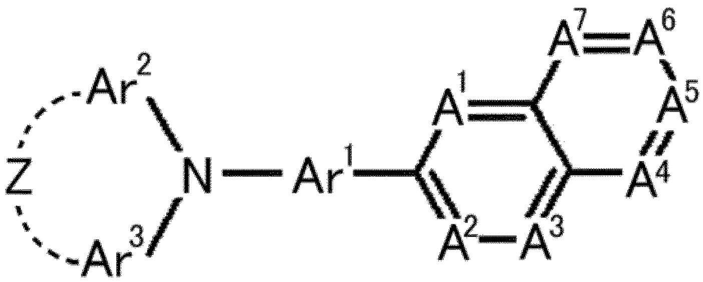

In the general formula (251), 1 to 4 of $A^1$ to $A^7$ represents N, and the balance each independently represent C-R. R represents a non-aromatic group. $Ar^1$ to $Ar^3$ each independently represent a substituted or unsubstituted arylene group. Z represents a single bond or a linking group.

[2] The compound according to [1], wherein the compound represented by the general formula (251) has a structure represented by the following general formula (252):

[Chem. 85]

General Formula (252)

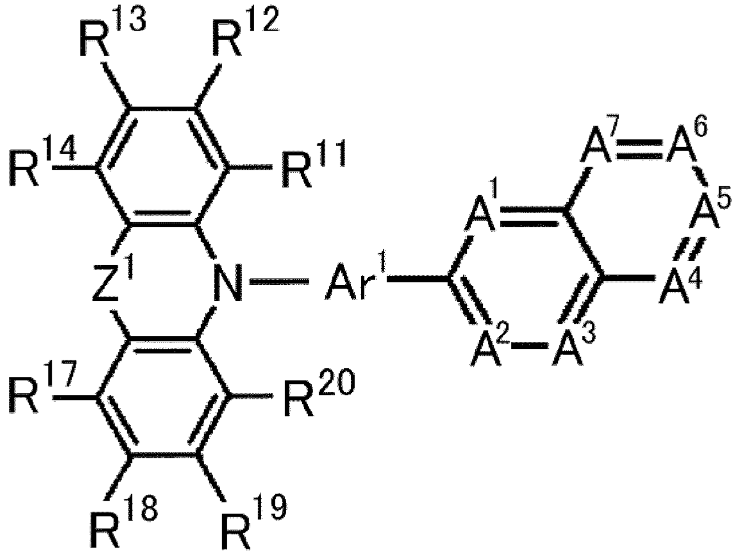

In the general formula (252), 1 to 4 of $A^1$ to $A^7$ represents N, and the balance each independently represent C-R. R represents a non-aromatic group. $Ar^1$ represents a substituted or unsubstituted arylene group. $R^{11}$ to $R^{14}$ and $R^{17}$ to $R^{20}$ each independently represent a hydrogen atom or a substituent. $R^{11}$ and $R^{12}$, $R^{12}$ and $R^{13}$, $R^{13}$ and $R^{14}$, $R^{17}$ and $R^{18}$, $R^{18}$ and $R^{19}$, and $R^{19}$ and $R^{20}$ each may be bonded to each other to form a cyclic structure. $Z^1$ represents a single bond or a linking group having 1 or 2 linking chain atoms.

[3] The compound according to [1], wherein the compound represented by the general formula (251) has a structure represented by the following general formula (253):

[Chem. 86]

General Formula (253)

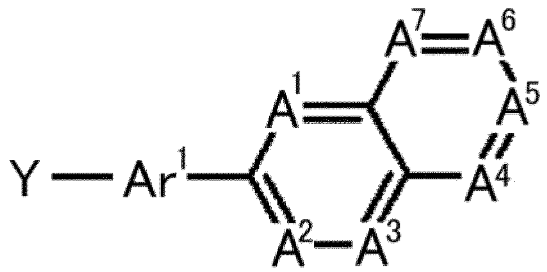

In the general formula (253), from 2 to 4 of $A^1$ to $A^7$ represent N, and the balance represent C-R. R represents a non-aromatic group. $Ar^1$ represents a substituted or unsubstituted arylene group. Y represents a substituted or unsubstituted carbazol-9-yl group, a substituted or unsubstituted 10H-phenoxazin-10-yl group, a substituted or unsubstituted 10H-phenothiazin-10-yl group, or a substituted or unsubstituted 10H-phenazin-5-yl group.

[4] The compound according to [3], wherein in the general formula (253), Y represents a group represented by any one of the following general formulae (254) to (257):

[Chem. 87]

General Formula (254)

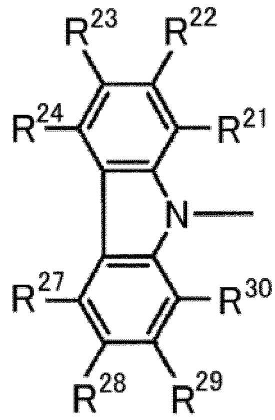

General Formula (255)

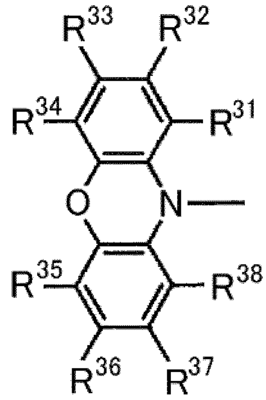

General Formula (256)

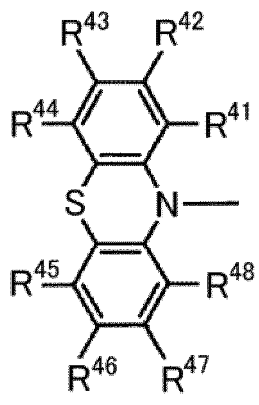

General Formula (257)

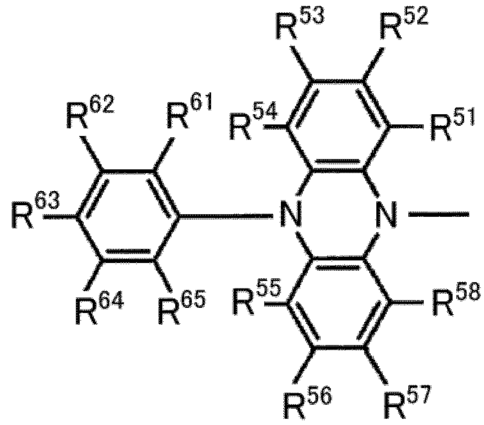

In the general formulae (254) to (257), $R^{21}$ to $R^{24}$, $R^{27}$ to $R^{38}$, $R^{41}$ to $R^{48}$, $R^{51}$ to $R^{58}$, and $R^{61}$ to $R^{65}$ each independently represent a hydrogen atom or a substituent. $R^{21}$ and $R^{22}$, $R^{22}$ and $R^{23}$, $R^{23}$ and $R^{24}$, $R^{27}$ and $R^{28}$, $R^{28}$ and $R^{29}$, $R^{29}$ and $R^{30}$, $R^{31}$ and $R^{32}$, $R^{32}$ and $R^{33}$, $R^{33}$ and $R^{34}$, $R^{35}$ and $R^{36}$, $R^{36}$ and $R^{37}$, $R^{37}$ and $R^{38}$, $R^{41}$ and $R^{42}$, $R^{42}$ and $R^{43}$, $R^{43}$ and $R^{44}$, $R^{45}$ and $R^{46}$, $R^{46}$ and $R^{47}$, $R^{47}$ and $R^{48}$, $R^{51}$ and $R^{52}$, $R^{52}$ and $R^{53}$, $R^{53}$ and $R^{54}$, $R^{55}$ and $R^{56}$, $R^{56}$ and $R^{57}$, $R^{57}$ and $R^{58}$, $R^{61}$ and $R^{62}$, $R^{62}$ and $R^{63}$, $R^{63}$ and $R^{64}$, $R^{64}$ and $R^{65}$, $R^{54}$ and $R^{61}$, and $R^{55}$ and $R^{65}$ each may be bonded to each other to form a cyclic structure.

[5] The compound according to [3], wherein in the general formula (253), Y represents a group represented by the following general formula (258):

[Chem. 88]

General Formula (258)

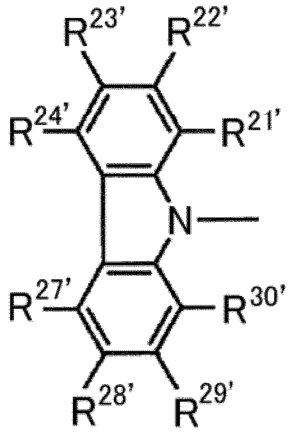

In the general formula (258), $R^{21'}$ to $R^{24'}$ and $R^{27'}$ to $R^{30'}$ each independently represent a hydrogen atom or a substituent, provided that at least one of $R^{23'}$ and $R^{28'}$ represents a substituent, and $R^{21}$ and $R^{22'}$, $R^{22'}$ and $R^{23'}$, $R^{23'}$ and $R^{24'}$, $R^{27'}$ and $R^{28'}$, $R^{28'}$ and $R^{29'}$, and $R^{29'}$ and $R^{30'}$ each may be bonded to each other to form a cyclic structure.

[6] The compound according to [5], wherein in the general formula (258), at least one of $R^{23'}$ and $R^{28'}$ represents a substituted or unsubstituted diarylamino group or a substituted or unsubstituted carbazol-9-yl group.

[7] The compound according to [4], wherein in the general formula (253), Y represents a group represented by the general formula (255).

Examples of the compound include the following compounds.

[Chem. 89]

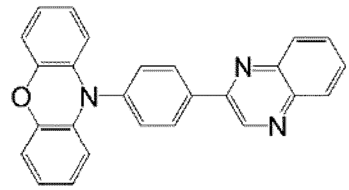
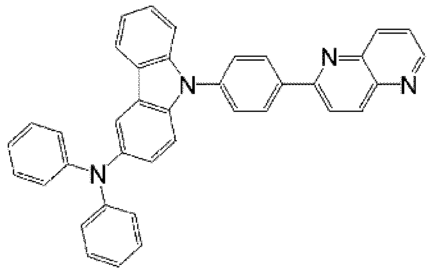
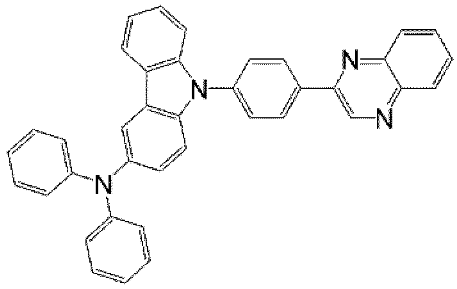
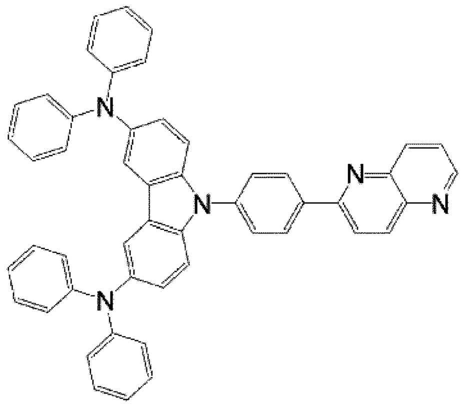
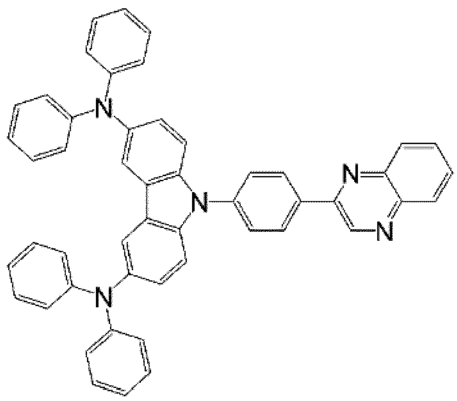
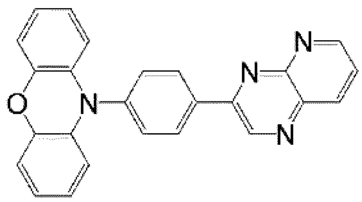
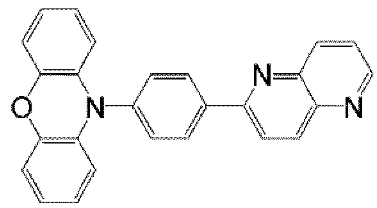
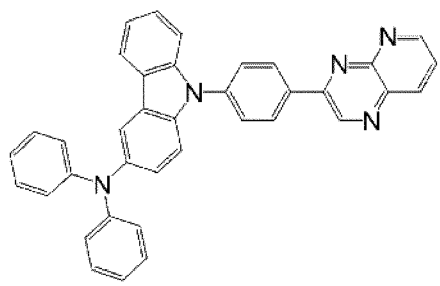

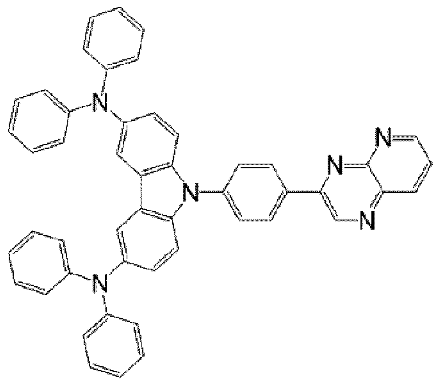

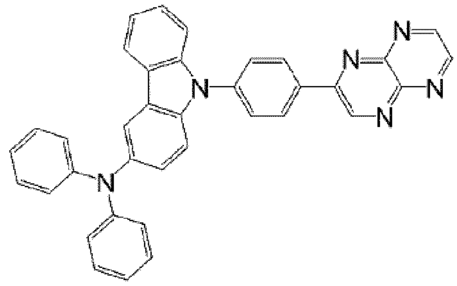

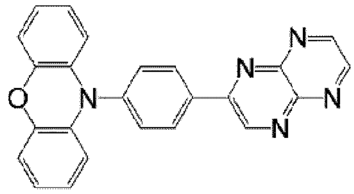

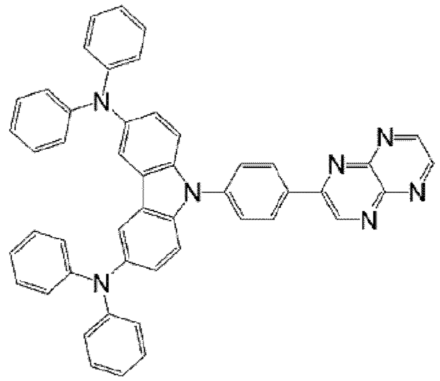

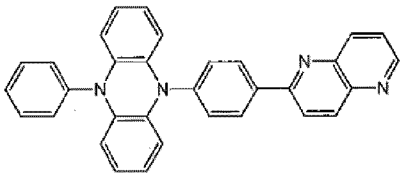

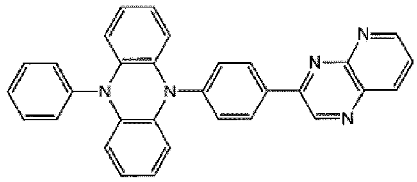

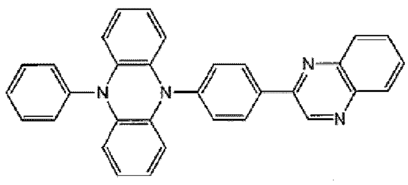

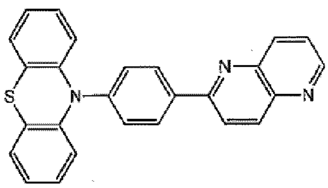

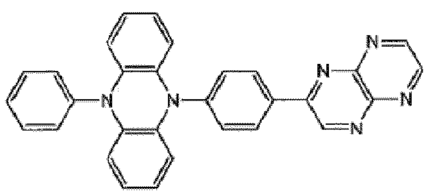

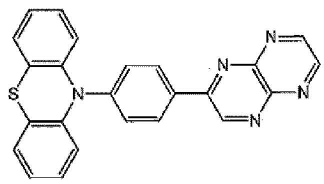

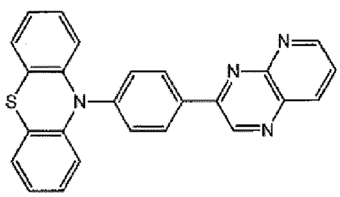

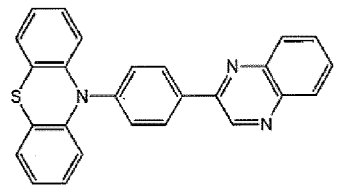

Examples of the preferred delayed fluorescent material include the following compounds.

[1] A compound represented by the following general formula (261):

[Chem. 91]

General Formula (261)

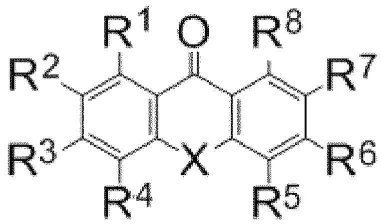

In the general formula (261), X represents an oxygen atom or a sulfur atom, $R^1$ to $R^8$ each independently represent a hydrogen atom or a substituent, provided that at least one of $R^1$ to $R^8$ each independently represents a group represented by any of the following general formulae (262) to (266), and $R^1$ and $R^2$, $R^2$ and $R^3$, $R^3$ and $R^4$, $R^5$ and $R^6$, $R^6$ and $R^7$, and $R^7$ and $R^8$ each may be bonded to each other to form a cyclic structure.

[Chem. 92]

General Formula (262)

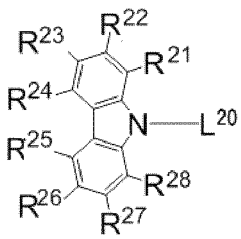

General Formula (263)

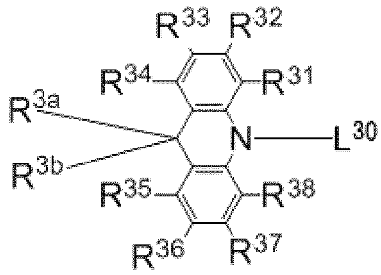

General Formula (264)

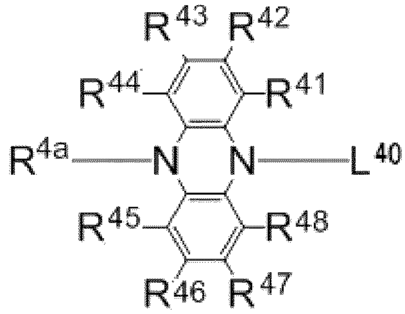

General Formula (265)

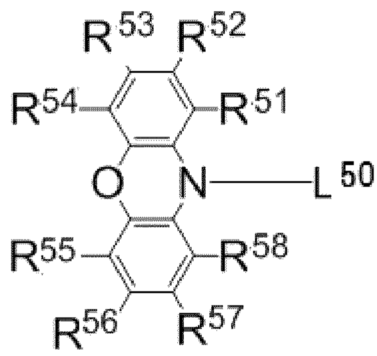

General Formula (266)

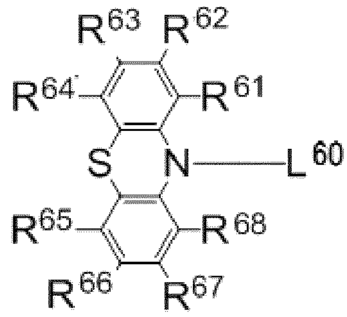

In the general formulae (262) to (266), $L^{20}$, $L^{30}$, $L^{40}$, $L^{50}$ and $L^{60}$ each independently represent a single bond or a divalent linking group, and bond to the ring skeleton of the general formula (261) via $L^{20}$, $L^{30}$, $L^{40}$, $L^{50}$ or $L^{60}$. $R^{21}$ to $R^{28}$, $R^{31}$ to $R^{38}$, $R^{3a}$, $R^{3b}$, $R^{41}$ to $R^{48}$, $R^{4a}$, $R^{51}$ to $R^{58}$, and $R^{61}$ to $R^{68}$ each independently represent a hydrogen atom or a substituent. $R^{21}$ and $R^{22}$, $R^{22}$ and $R^{23}$, $R^{23}$ and $R^{24}$, $R^{24}$ and $R^{25}$, $R^{25}$ and $R^{26}$, $R^{26}$ and $R^{27}$, $R^{27}$ and $R^{28}$, $R^{31}$ and $R^{32}$, $R^{32}$ and $R^{33}$, $R^{33}$ and $R^{34}$, $R^{35}$ and $R^{36}$, $R^{36}$ and $R^{37}$, $R^{37}$ and $R^{38}$, $R^{3a}$ and $R^{3b}$, $R^{41}$ and $R^{42}$, $R^{42}$ and $R^{43}$, $R^{43}$ and $R^{44}$, $R^{45}$ and $R^{46}$, $R^{46}$ and $R^{47}$, $R^{47}$ and $R^{48}$, $R^{51}$ and $R^{52}$, $R^{52}$ and $R^{53}$, $R^{53}$ and $R^{54}$, $R^{55}$ and $R^{56}$, $R^{56}$ and $R^{57}$, $R^{57}$ and $R^{58}$, $R^{61}$ and $R^{62}$, $R^{62}$ and $R^{63}$, $R^{63}$ and $R^{64}$, $R^{65}$ and $R^{66}$, $R^{66}$ and $R^{67}$, and $R^{67}$ and $R^{68}$ each may be bonded to each other to form a cyclic structure.

[2] The compound according to [1], wherein at least one of $R^3$ or $R^6$ in the general formula (261) is a group represented by any of the general formulae (262) to (266).

[3] The compound according to [2], wherein $R^3$ and $R^6$ in the general formula (261) each are a group represented by any of the general formulae (262) to (266).

[4] The compound according to [2], wherein at least one of $R^3$ and $R^6$ in the general formula (261) is a group represented by the general formula (263).

[5] The compound according to [2], wherein at least one of $R^3$ and $R^6$ in the general formula (261) is a group represented by the general formula (262).

[6] The compound according to any one of [1] to [5], wherein at least one of $R^{21}$ to $R^{28}$, $R^{31}$ to $R^{38}$, $R^{41}$ to $R^{48}$, $R^{51}$ to $R^{58}$, and $R^{61}$ to $R^{68}$ represents a substituent.

[7] The compound according to [6], wherein at least one of $R^{23}$, $R^{26}$, $R^{33}$, $R^{36}$, $R^{43}$, $R^{46}$, $R^{53}$, $R^{56}$, $R^{63}$ and $R^{66}$ represents a substituent.

[8] The compound according to [7], wherein the substituent is a group represented by any of the general formulae (262) to (266).

[9] The compound according to any one of [1] to [8], wherein L in the general formulae (262) to (266) is a single bond.

[10] The compound according to any of [1] to [9], wherein X in the general formula (261) is an oxygen atom.

Examples of the compound include the following compounds.

[Chem. 93]

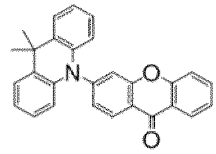

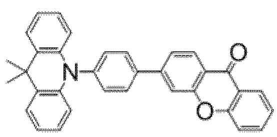

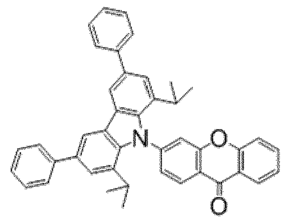

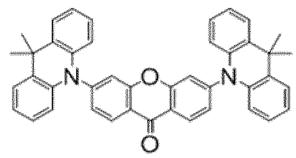

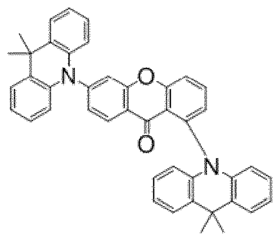

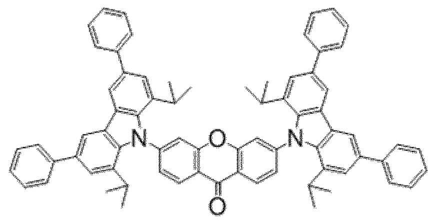

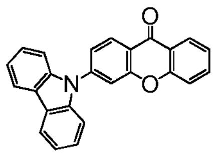

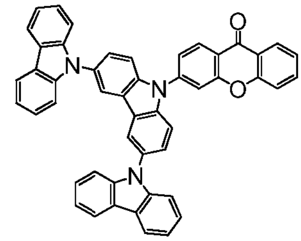

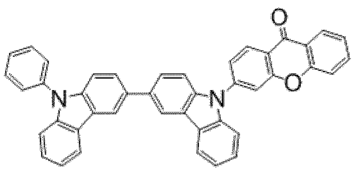

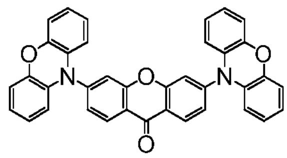

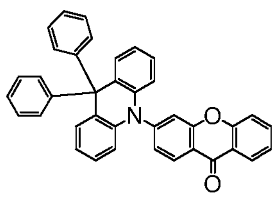

[Chem. 94]

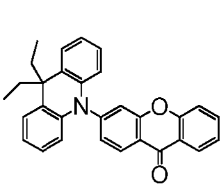
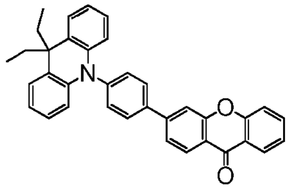
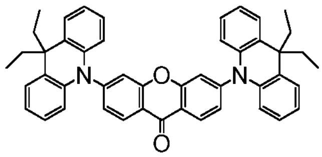
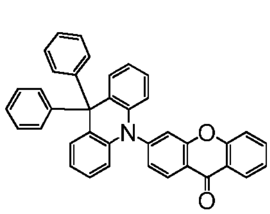
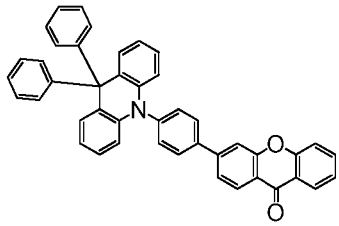
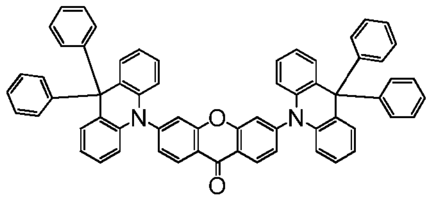
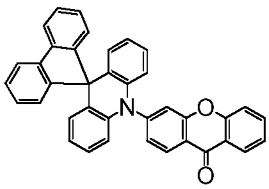
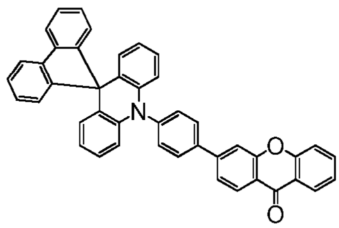
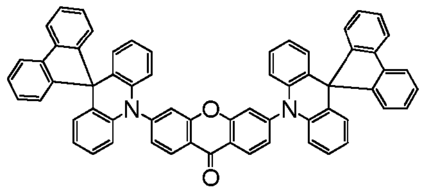
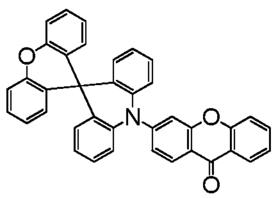
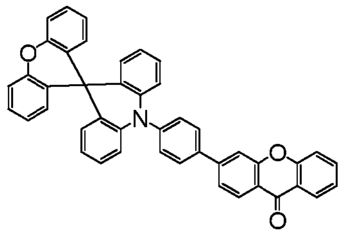
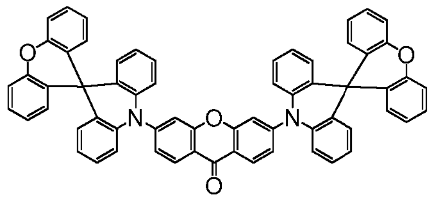
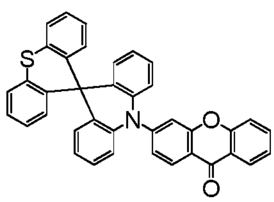
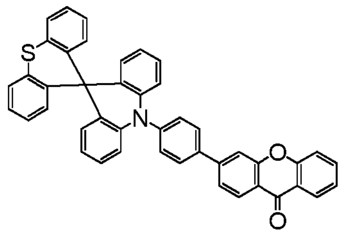
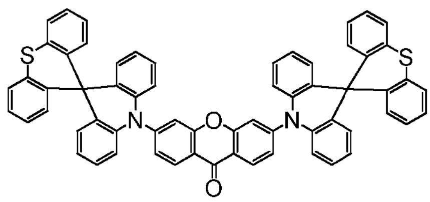
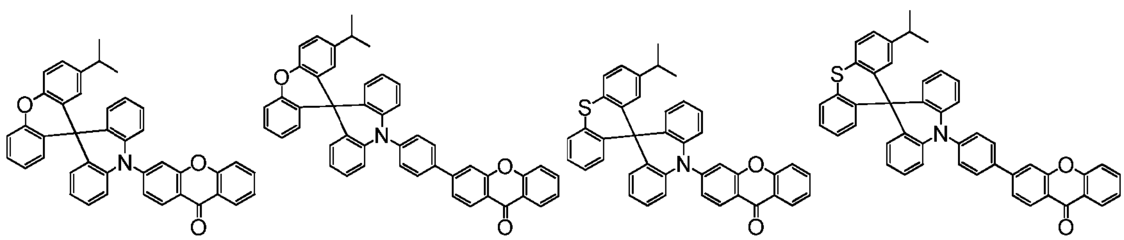

[Chem. 95]

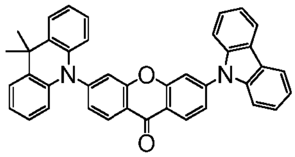

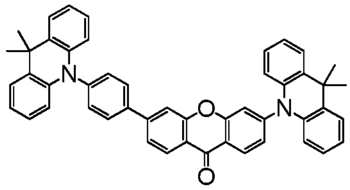

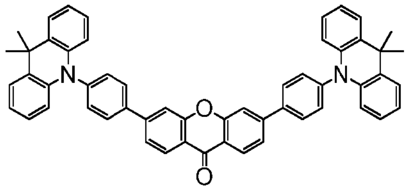

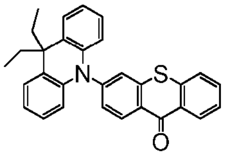
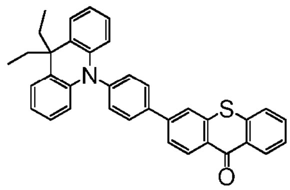
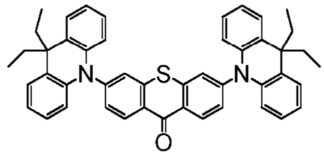

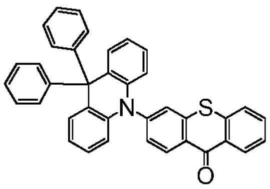
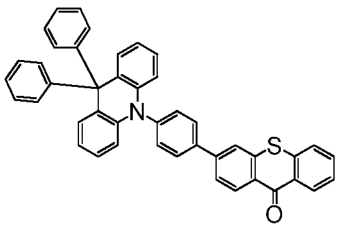

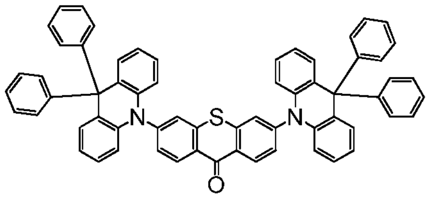

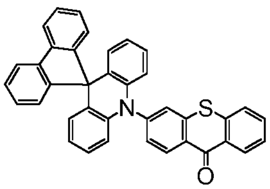
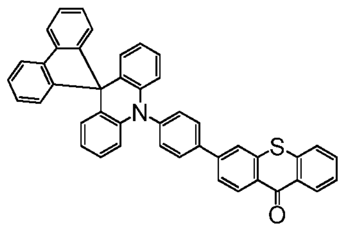

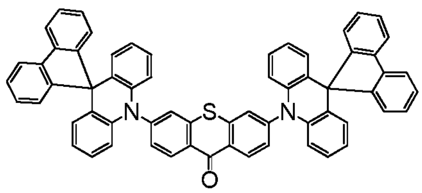

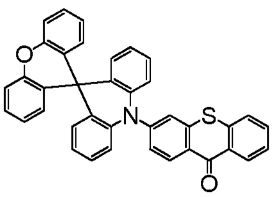
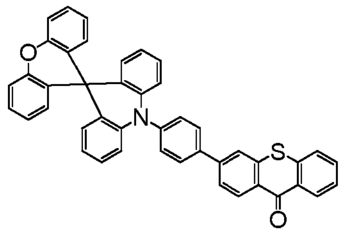

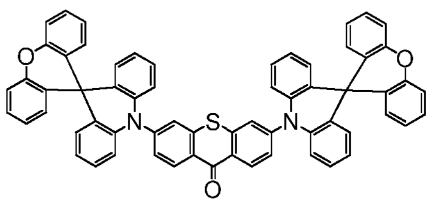

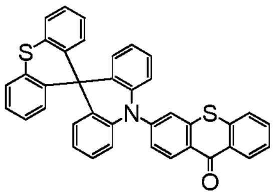

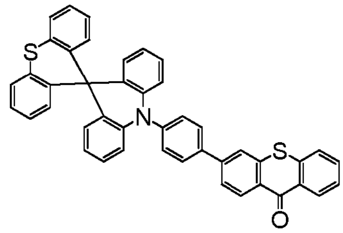

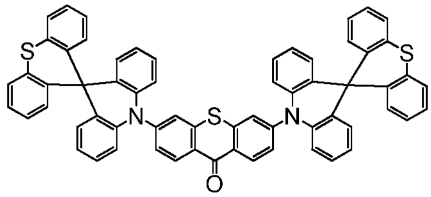

[Chem. 96]

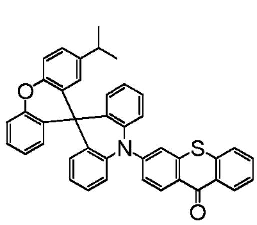

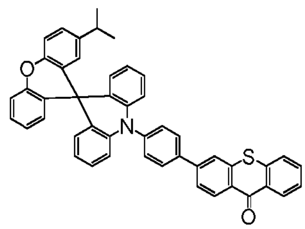

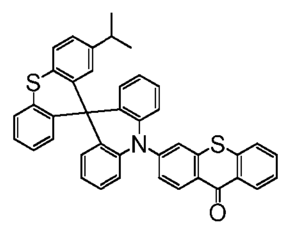

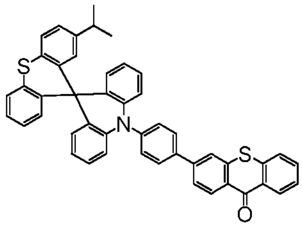

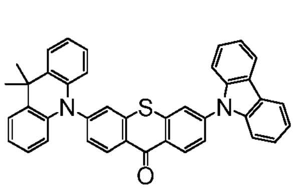

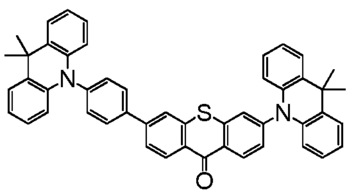

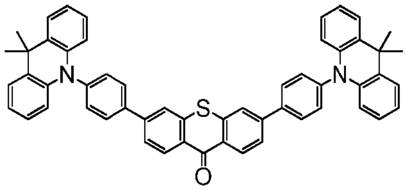

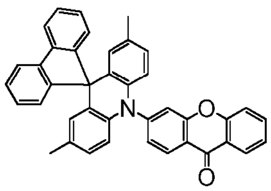

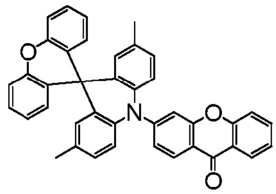

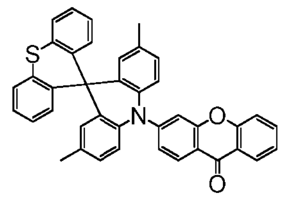

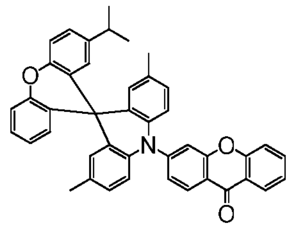

Examples of the preferred delayed fluorescent material include the following compounds.

[1] A compound represented by the following general formula (271):

[Chem. 97]

General Formula (271)

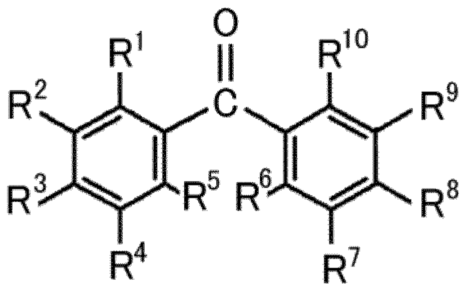

In the general formula (271), $R^1$ to $R^{10}$ each independently represent a hydrogen atom or a substituent, provided that at least one of $R^1$ to $R^{10}$ each independently represent a group represented by the following general formula (272), and $R^1$ and $R^2$, $R^2$ and $R^3$, $R^3$ and $R^4$, $R^4$ and $R^5$, $R^6$ and $R^7$, $R^7$ and $R^8$, $R^8$ and $R^9$, and $R^9$ and $R^{10}$ each may be bonded to each other to form a cyclic structure.

[Chem. 98]

General Formula (272)

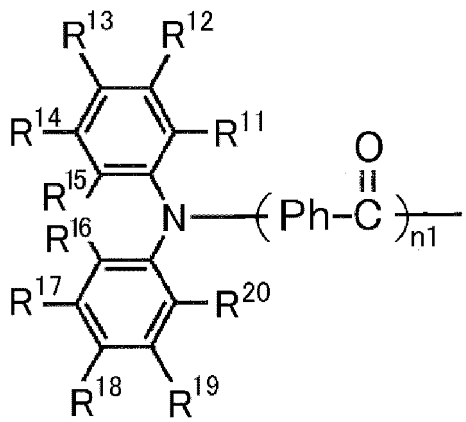

In the general formula (272), $R^{11}$ to $R^{20}$ each independently represent a hydrogen atom or a substituent. $R^{11}$ and $R^{12}$, $R^{12}$ and $R^{13}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, and $R^{19}$ and $R^{20}$ each may be bonded to each other to form a cyclic structure. Ph represents a substituted or unsubstituted phenylene group. n1 represents 0 or 1.

[2] The compound according to [1], wherein the group represented by the general formula (272) is a group represented by any one of the following general formulae (273) to (278):

[Chem. 99]

General Formula (273)

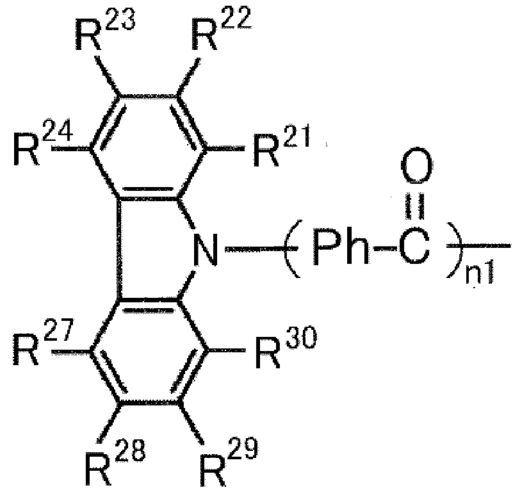

General Formula (274)

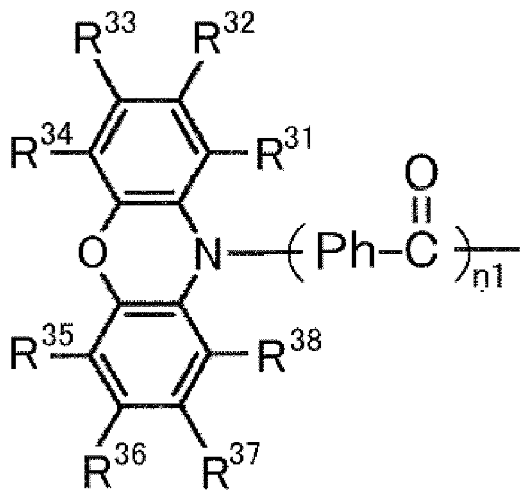

General Formula (275)

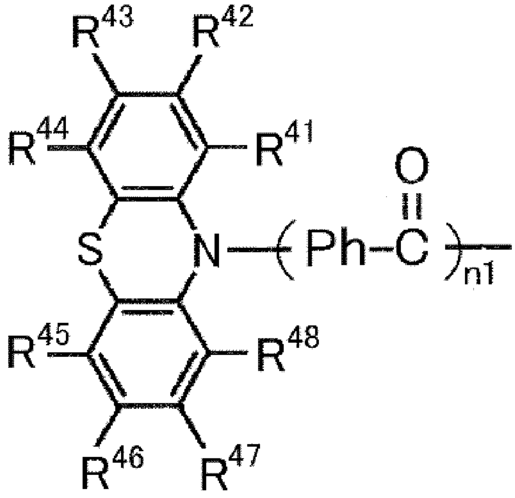

General Formula (276)

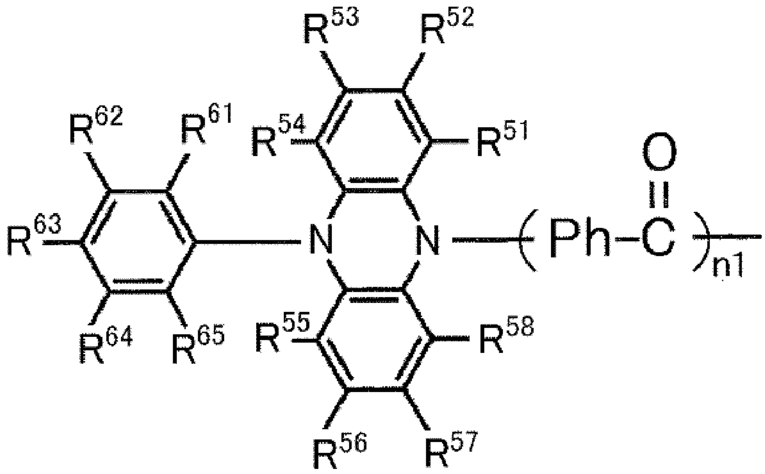

General Formula (277)

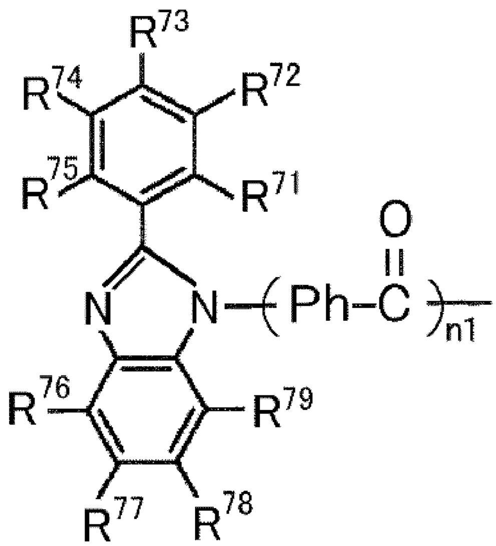

General Formula (278)

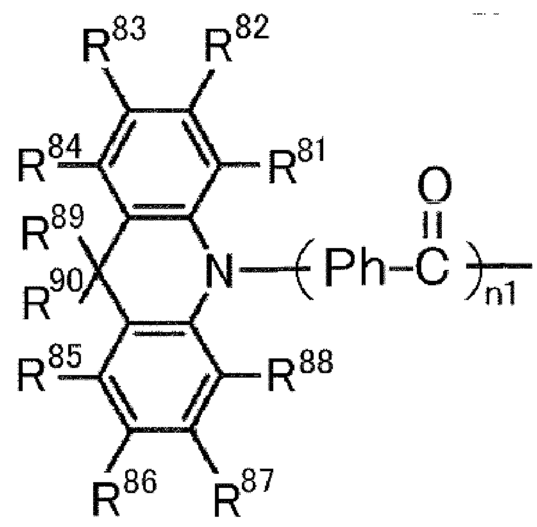

In the general formulae (273) to (278), $R^{21}$ to $R^{24}$, $R^{27}$ to $R^{38}$, $R^{41}$ to $R^{48}$, $R^{51}$ to $R^{58}$, $R^{61}$ to $R^{65}$, $R^{71}$ to $R^{79}$, and $R^{81}$ to $R^{90}$ each independently represent a hydrogen atom or a substituent. $R^{21}$ and $R^{22}$, $R^{22}$ and $R^{23}$, $R^{23}$ and $R^{24}$, $R^{27}$ and $R^{28}$, $R^{28}$ and $R^{29}$, $R^{29}$ and $R^{30}$, $R^{31}$ and $R^{32}$, $R^{32}$ and $R^{33}$, $R^{33}$ and $R^{34}$, $R^{35}$ and $R^{36}$, $R^{36}$ and $R^{37}$, $R^{37}$ and $R^{38}$, $R^{41}$ and $R^{42}$, $R^{42}$ and $R^{43}$, $R^{43}$ and $R^{44}$, $R^{45}$ and $R^{46}$, $R^{46}$ and $R^{47}$, $R^{47}$ and $R^{48}$, $R^{51}$ and $R^{52}$, $R^{52}$ and $R^{53}$, $R^{53}$ and $R^{54}$, $R^{55}$ and $R^{56}$, $R^{56}$ and $R^{57}$, $R^{57}$ and $R^{58}$, $R^{61}$ and $R^{62}$, $R^{62}$ and $R^{63}$, $R^{63}$ and $R^{64}$, $R^{64}$ and $R^{65}$, $R^{54}$ and $R^{61}$, $R^{55}$ and $R^{65}$, $R^{71}$ and $R^{72}$, $R^{72}$ and $R^{73}$, $R^{73}$ and $R^{74}$, $R^{74}$ and $R^{75}$, $R^{76}$ and $R^{77}$, $R^{77}$ and $R^{78}$, $R^{78}$ and $R^{79}$, $R^{81}$ and $R^{82}$, $R^{82}$ and $R^{83}$, $R^{83}$ and $R^{84}$, $R^{85}$ and $R^{16}$, $R^{86}$ and $R^{87}$, $R^{17}$ and $R^{88}$, and $R^{89}$ and $R^{90}$ each may be bonded to each other to form a cyclic structure. Ph represents a substituted or unsubstituted phenylene group. n1 represents 0 or 1.

[3] The compound according to [1] or [2], wherein in the general formula (271), at least one of $R^1$ to $R^5$ and at least one of $R^6$ to $R^{10}$ each represent a group represented by the general formula (272).

[4] The compound according to [3], wherein in the general formula (271), $R^3$ and $R^8$ each represent a group represented by the general formula (272).

[5] The compound according to any one of [1] to [4], wherein the group represented by the general formula (272) is a group represented by the general formula (274).

[6] The compound according to any one of [1] to [4], wherein the group represented by the general formula (272) is a group represented by the general formula (273).

[7] The compound according to [6], wherein in the general formula (273), at least one of $R^{21}$ to $R^{24}$ and $R^{27}$ to $R^{30}$ represents a substituent.

[8] The compound according to [7], wherein the substituent is a group represented by any one of the general formulae (273) to (278).

[9] The compound according to [8], wherein in the general formula (273), at least one of $R^{23}$ and $R^{28}$ represents the substituent.

Examples of the compound include the following compounds.

[Chem. 100]

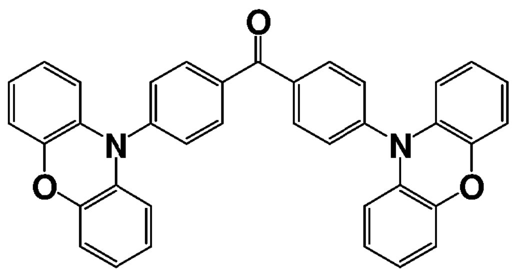

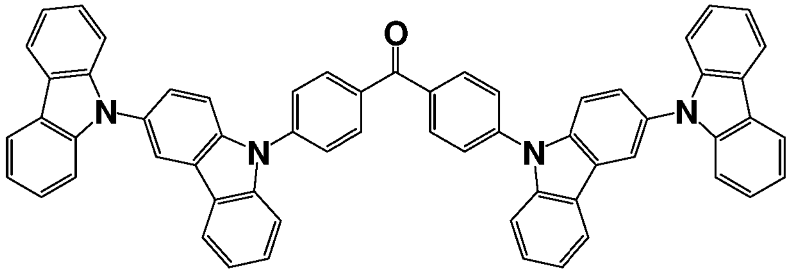

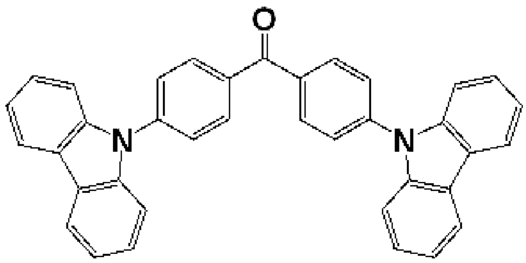

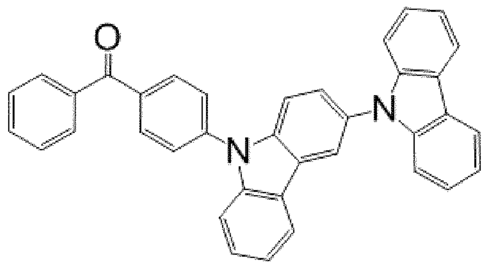

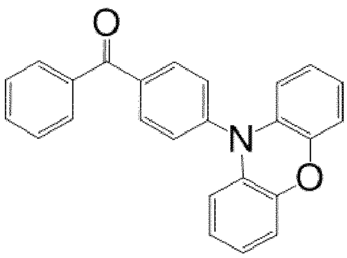

[Chem. 101]

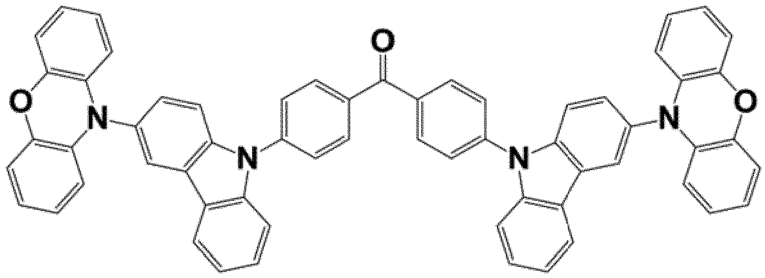

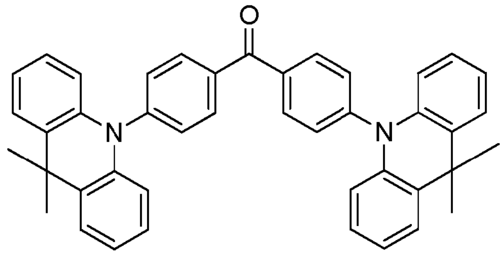

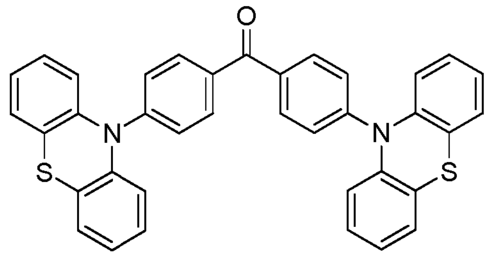

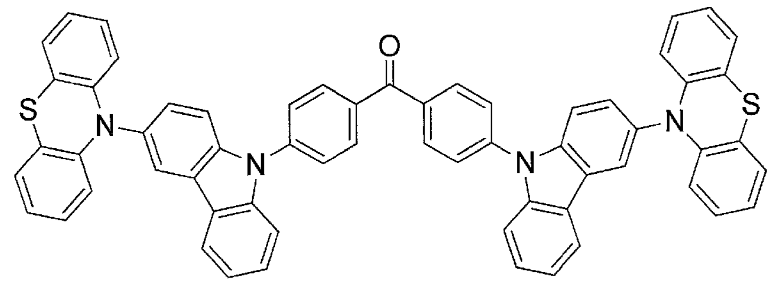

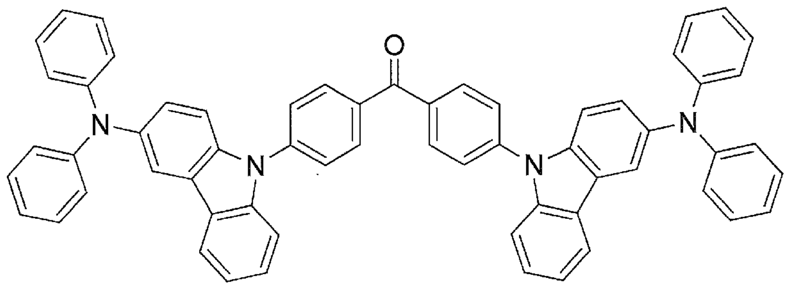

[Chem. 102]

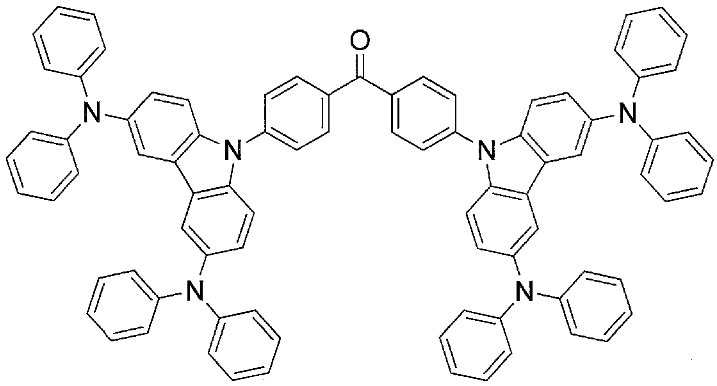

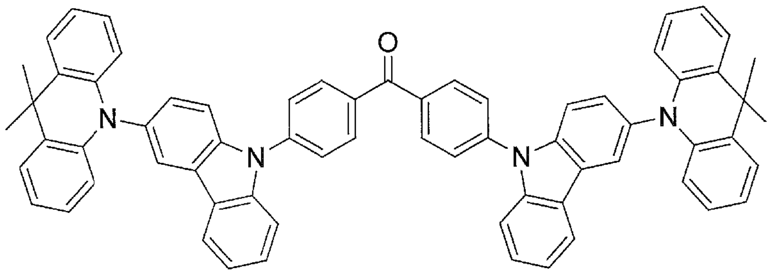

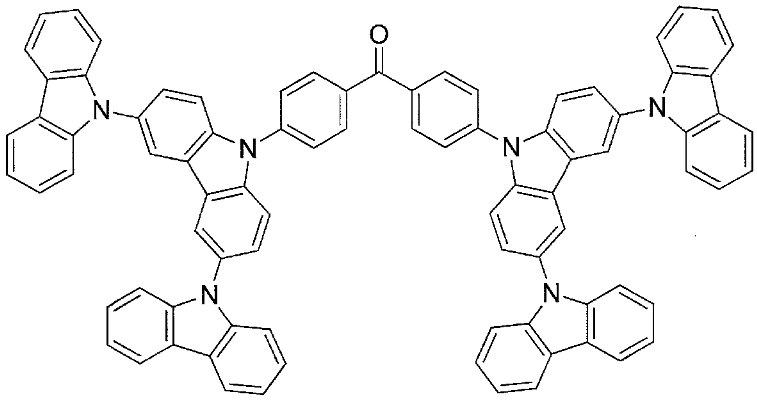

[Chem. 103]

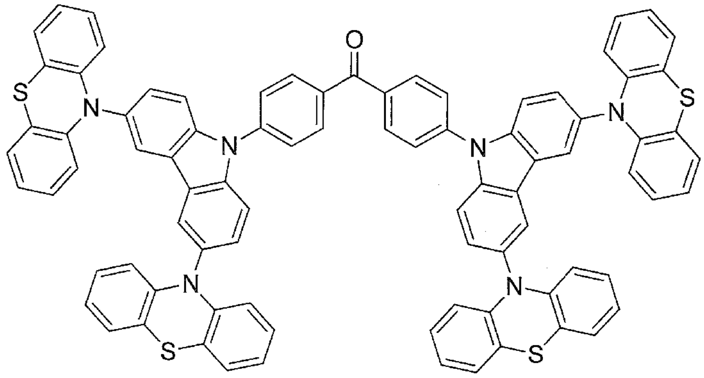

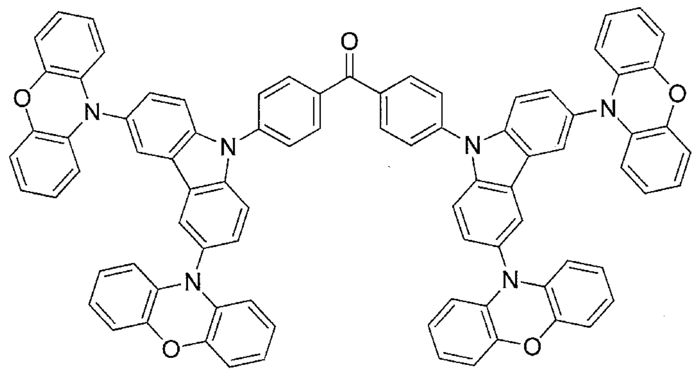

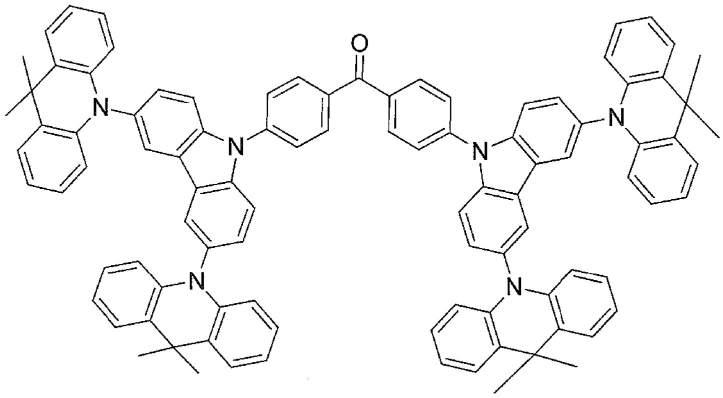

[Chem. 104]

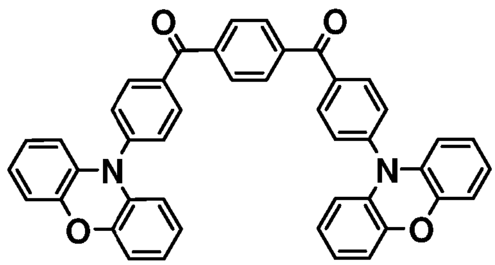

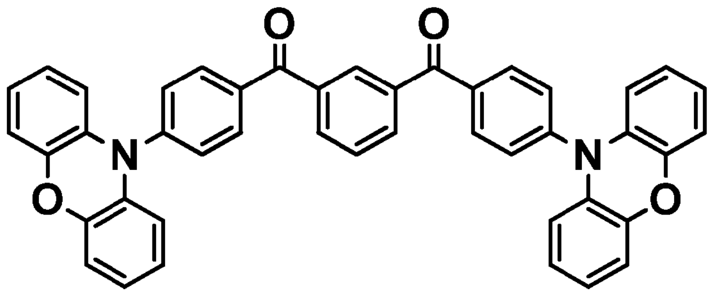

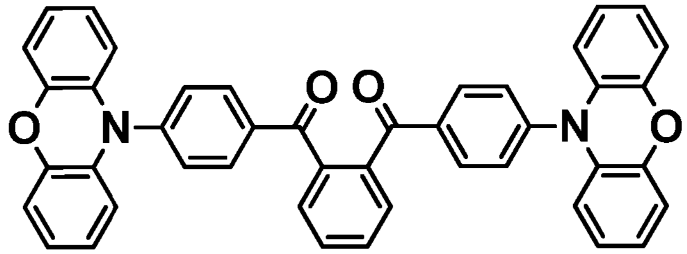

[Chem. 105]

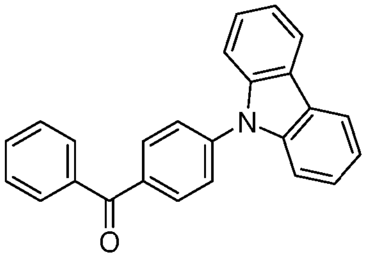

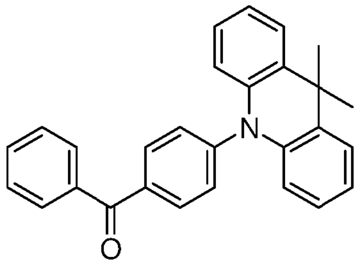

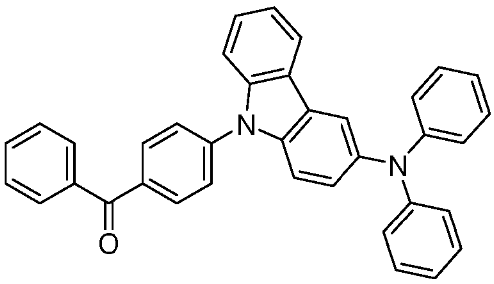

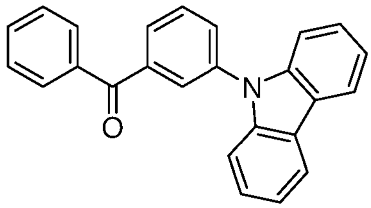

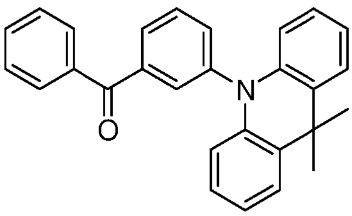

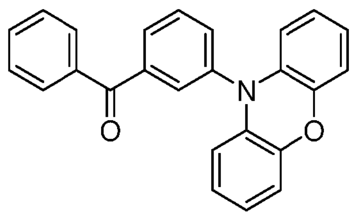

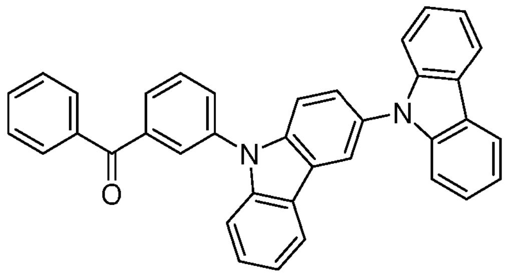

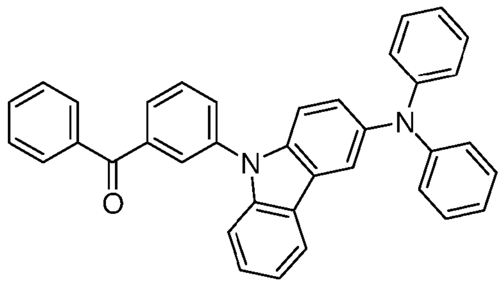

[Chem. 106]

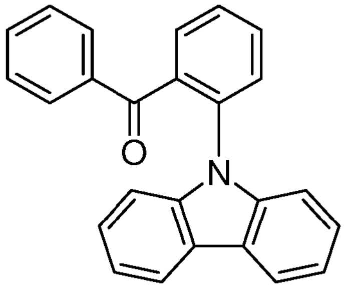

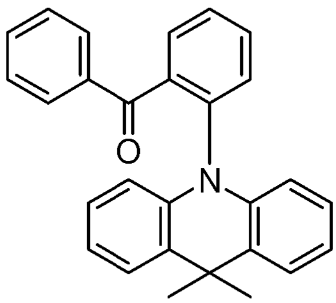

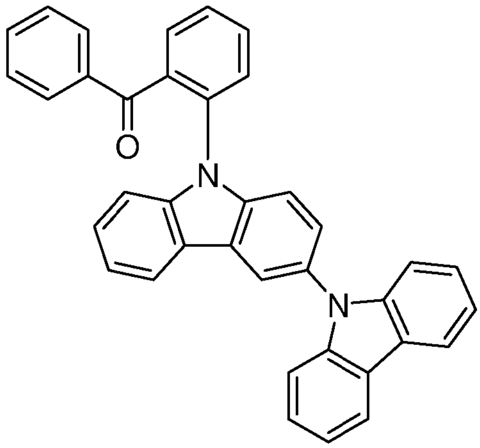

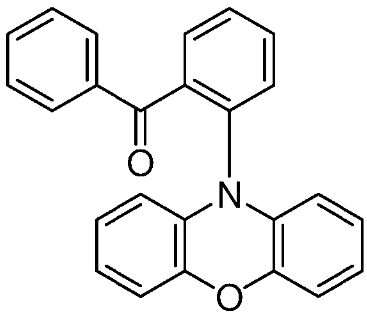

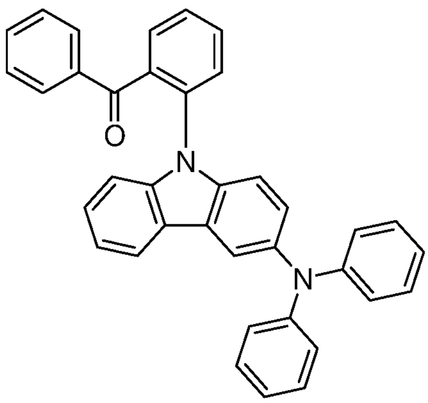

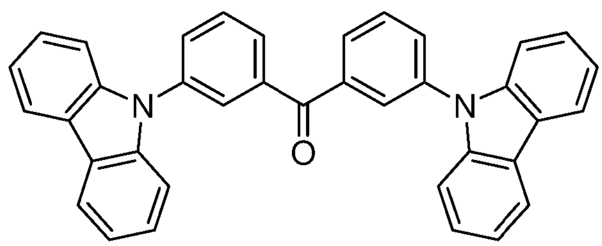

[Chem. 107]

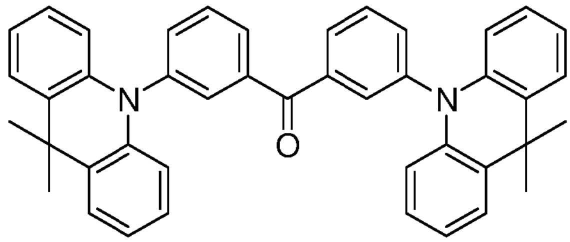

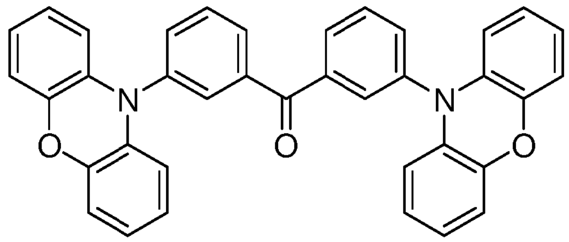

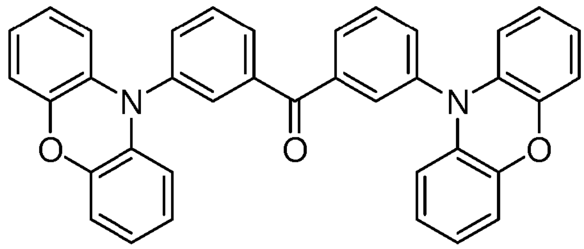

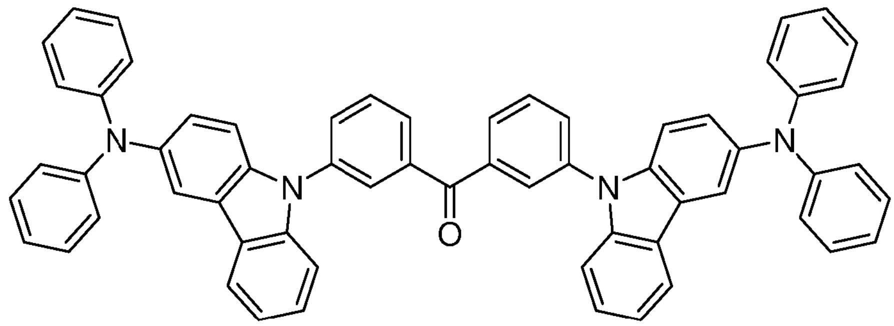

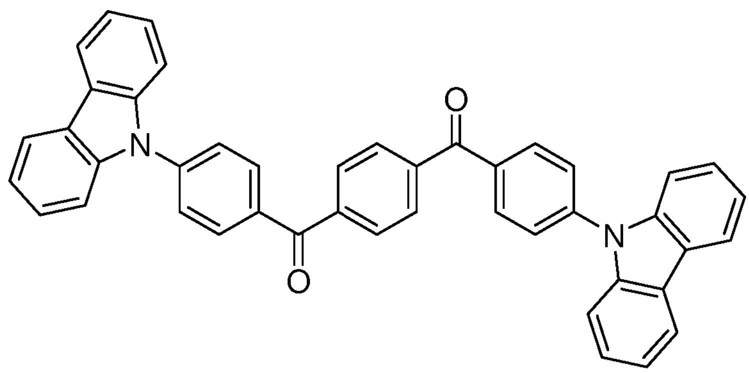

[Chem. 108]

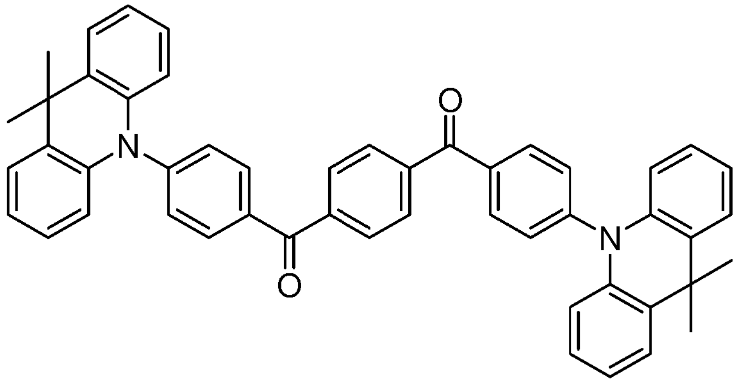

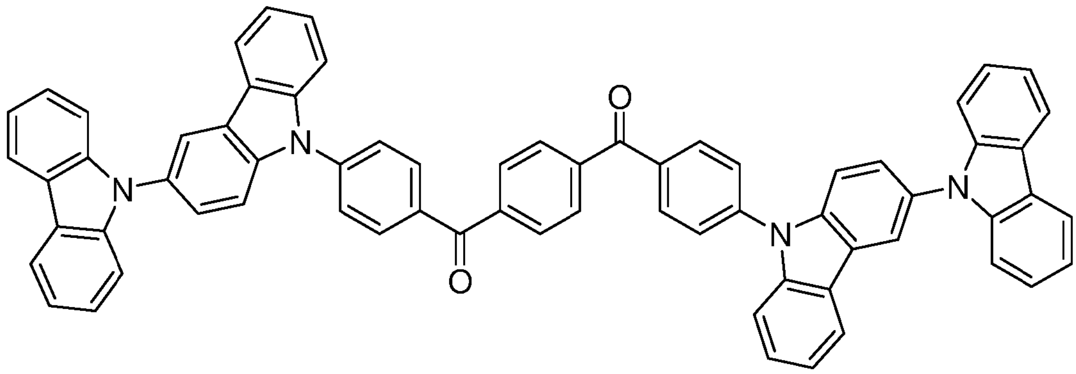

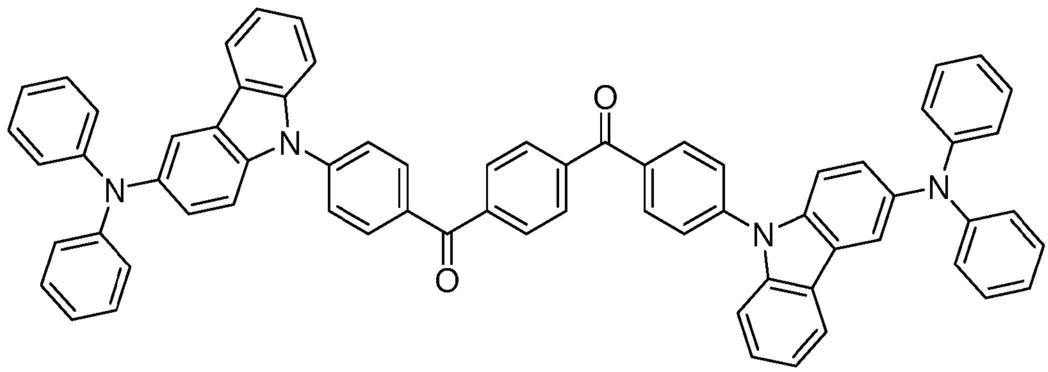

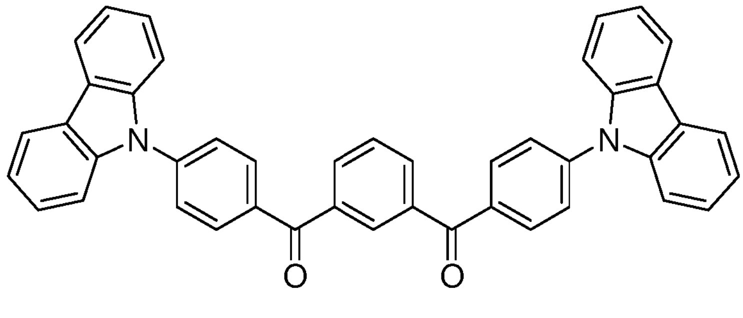

[Chem. 109]

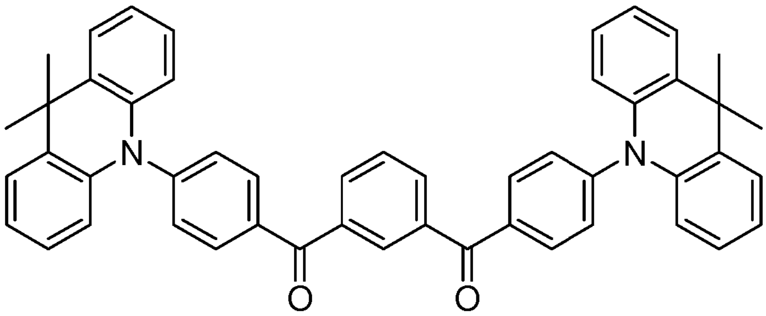

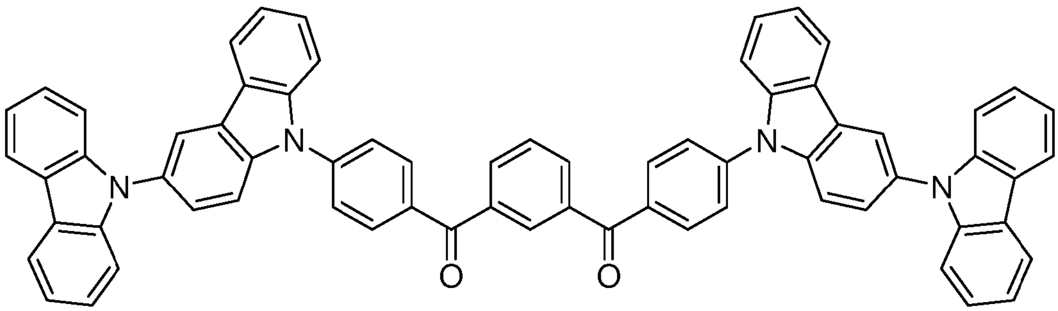

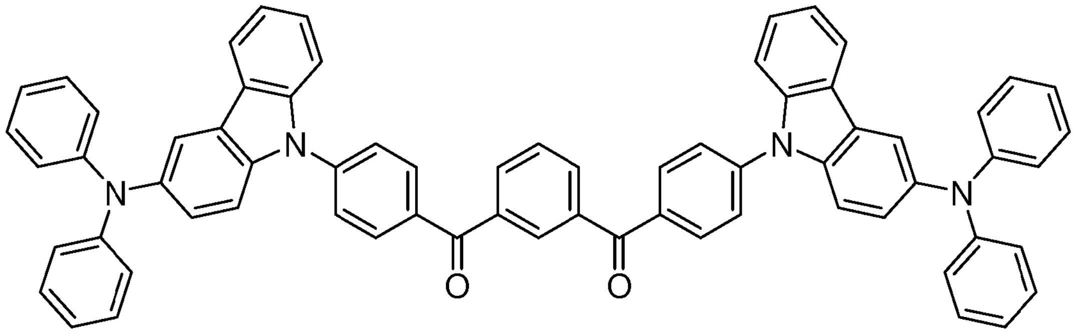

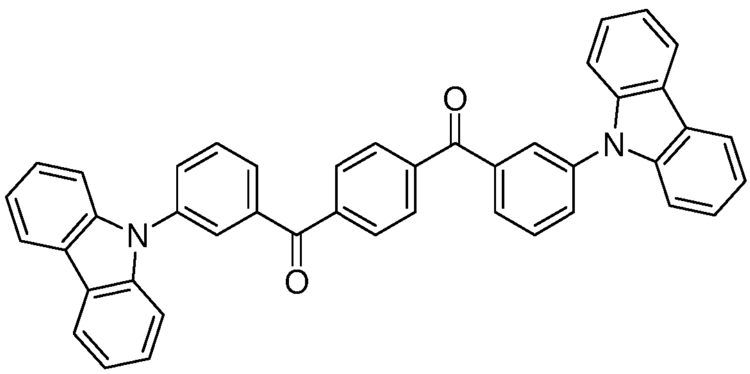

[Chem. 110]

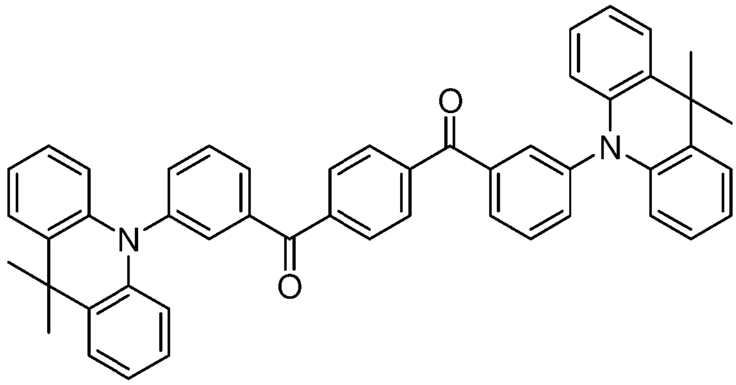

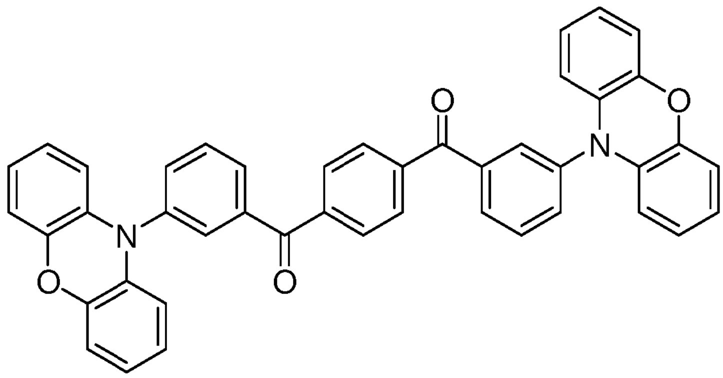

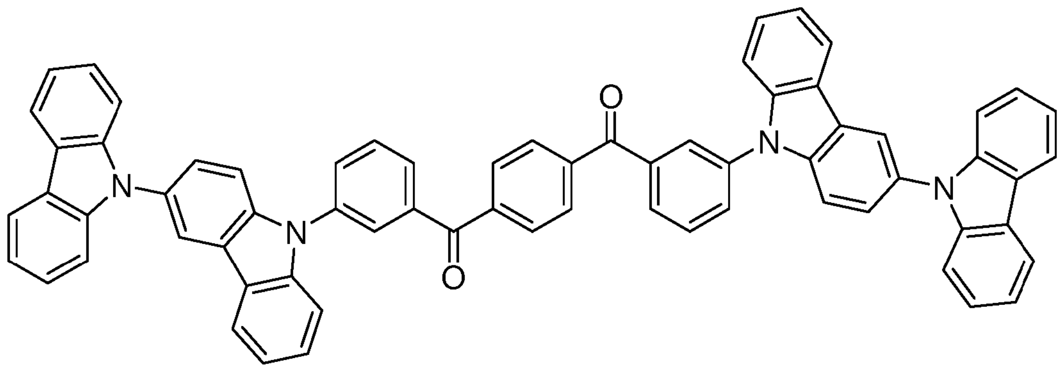

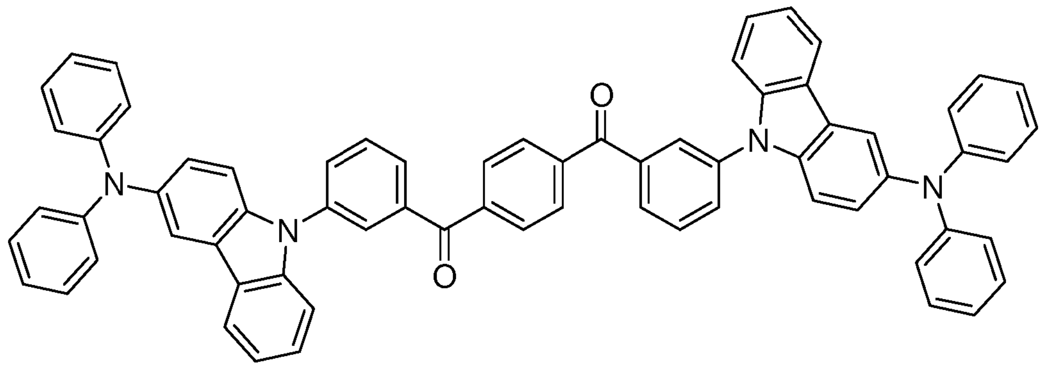

[0139]

[Chem. 111]

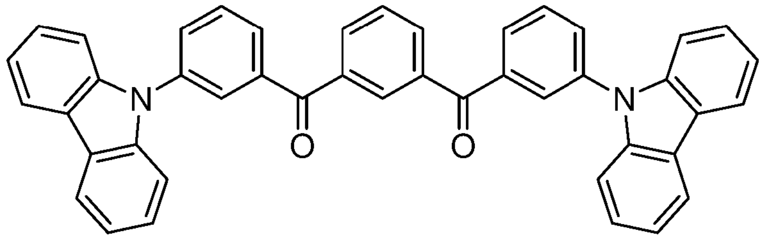

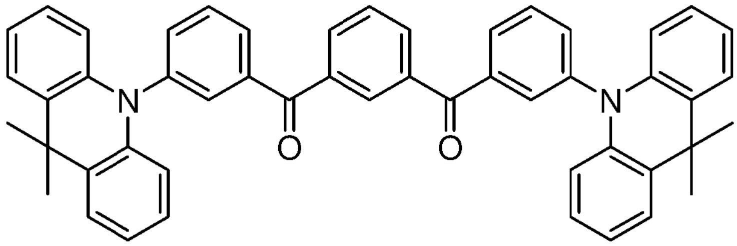

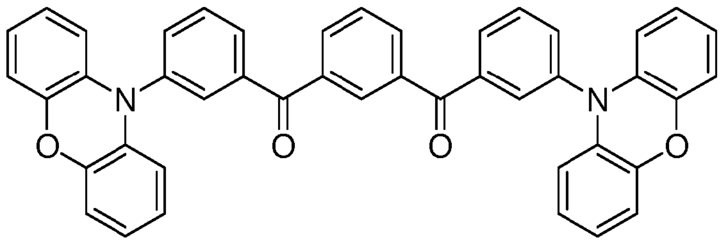

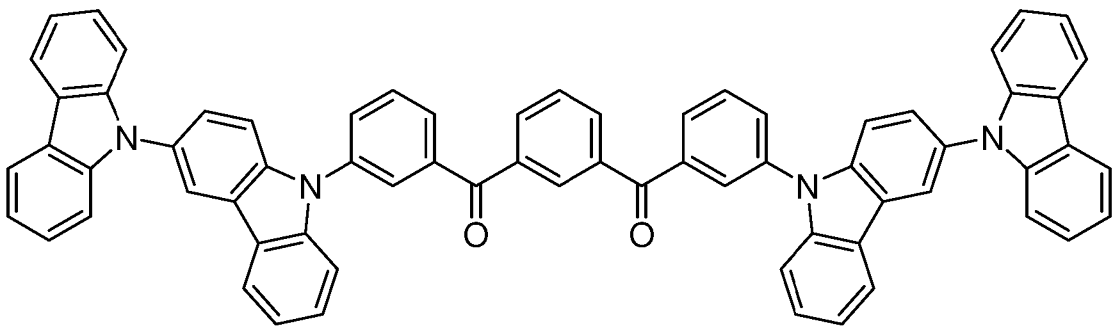

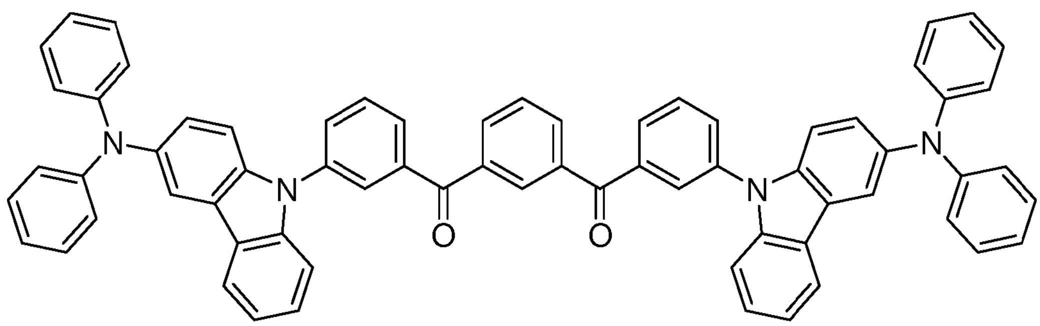

[Chem. 112]

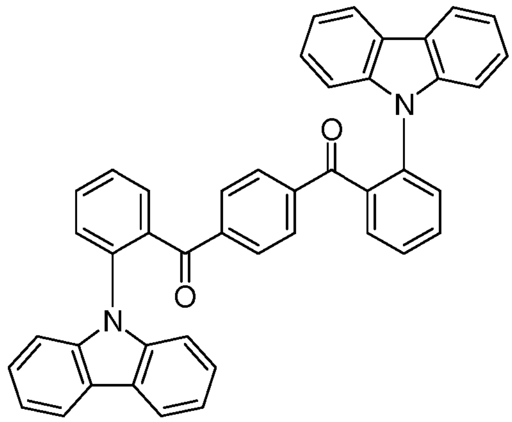

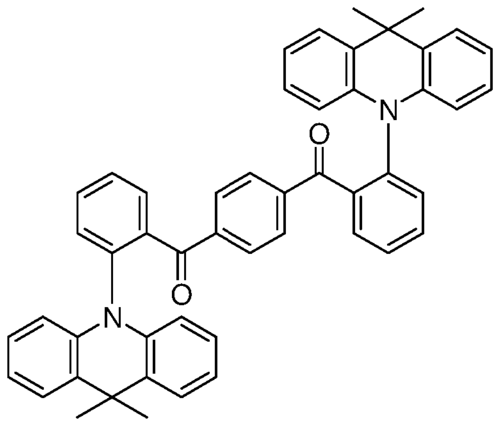

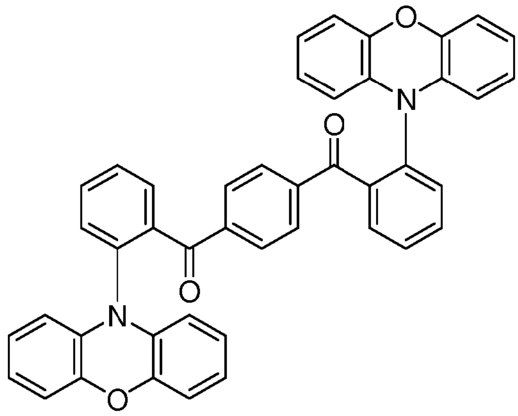

[Chem. 113]

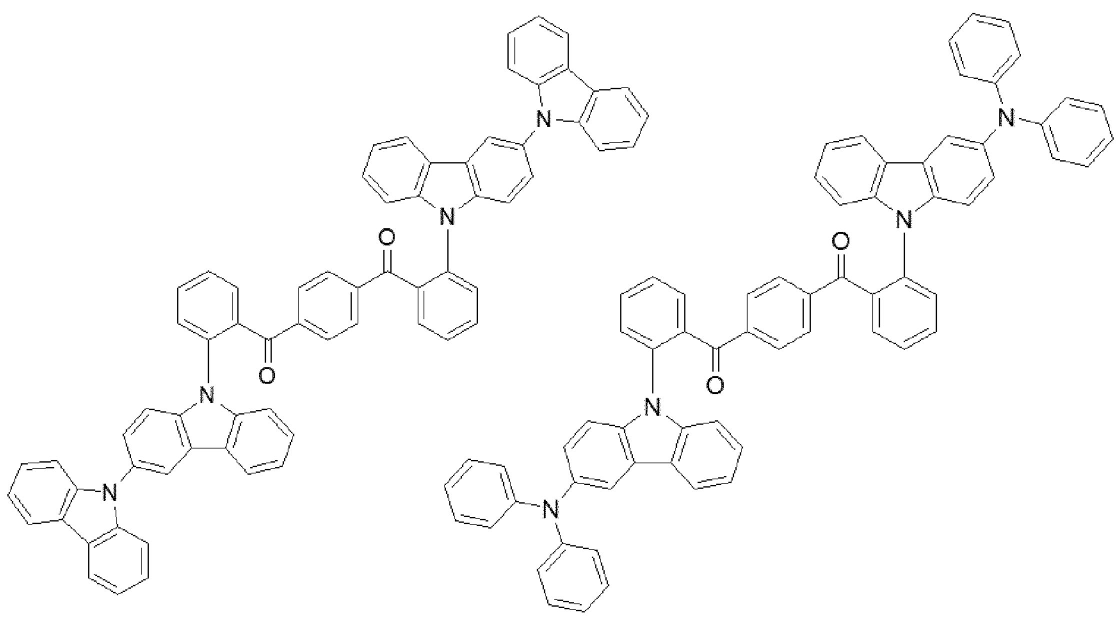

[Chem. 114]

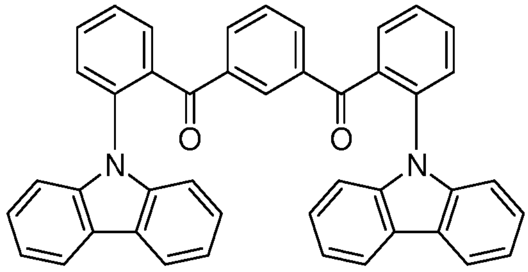

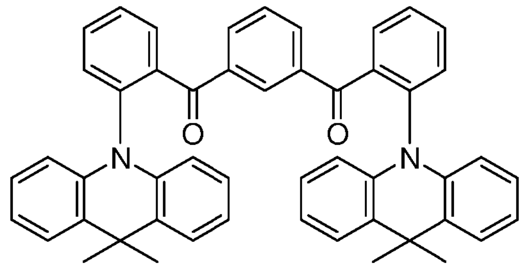

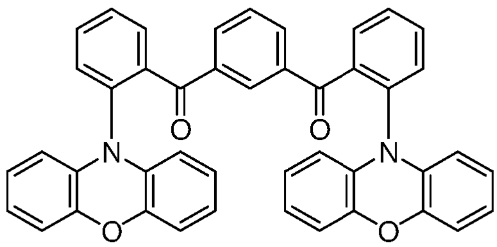

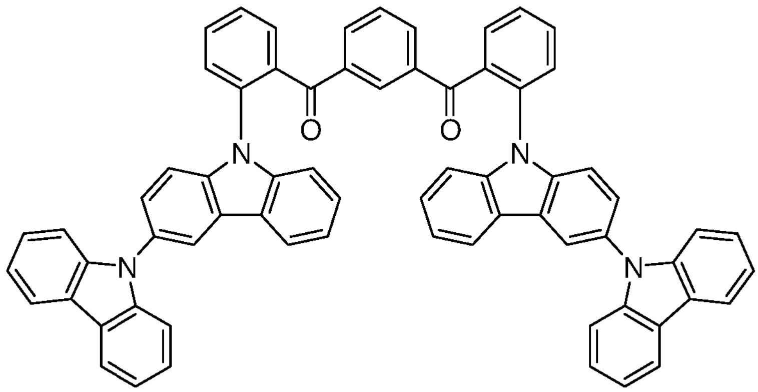

[Chem. 115]

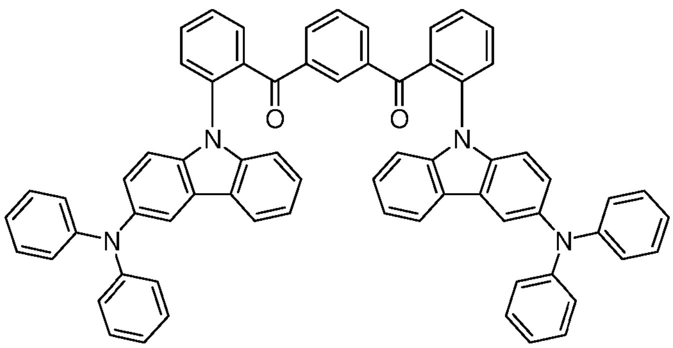

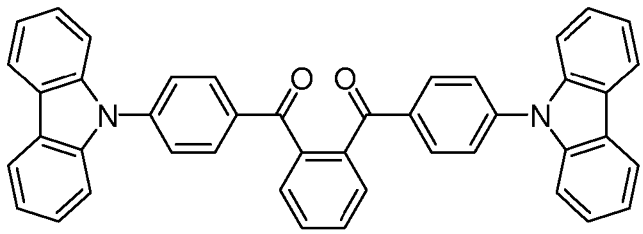

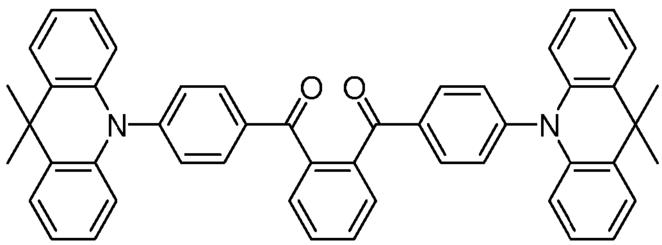

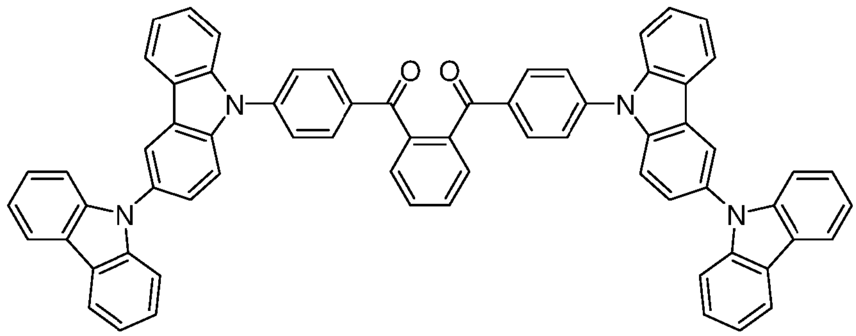

[Chem. 116]

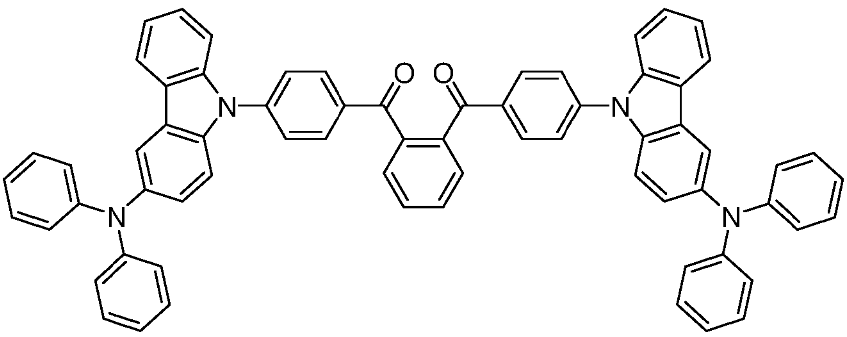

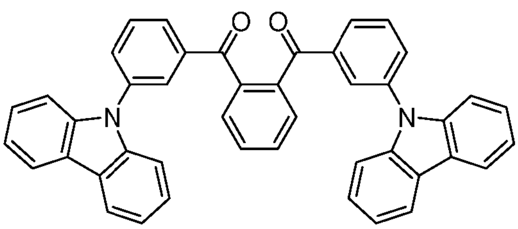

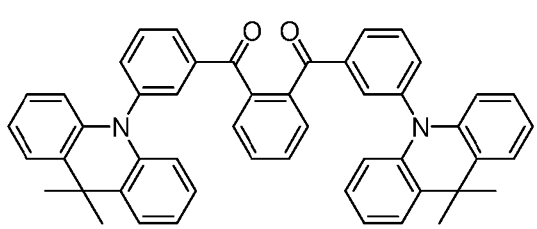

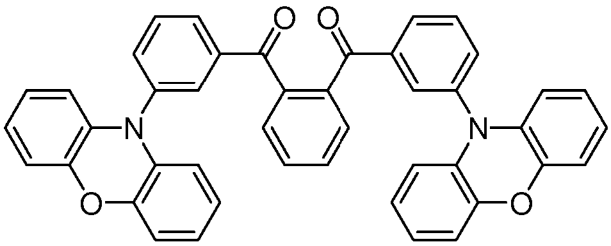

[Chem. 117]

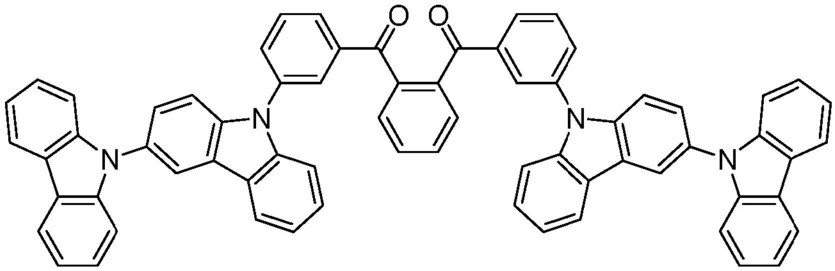

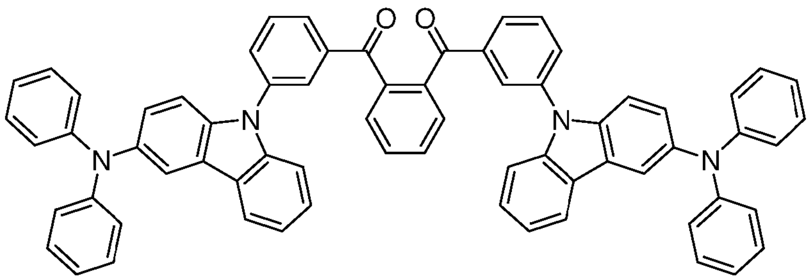

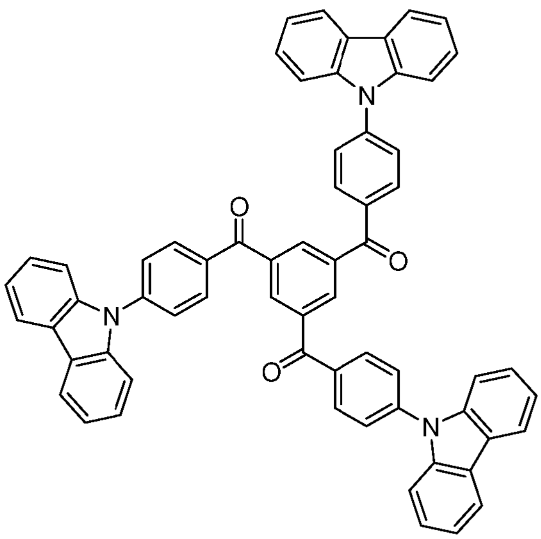

[Chem. 118]

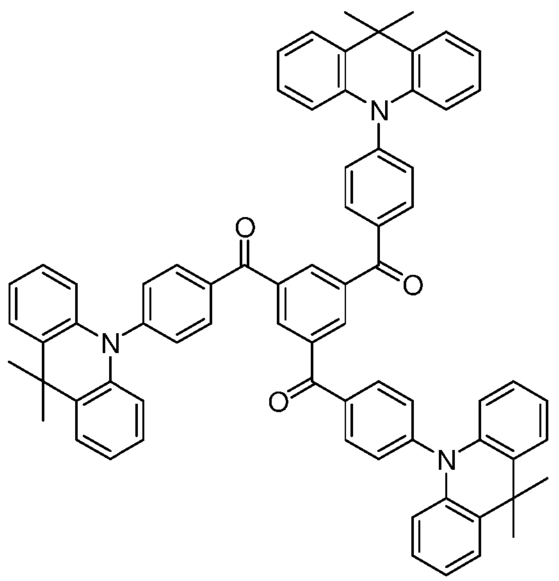

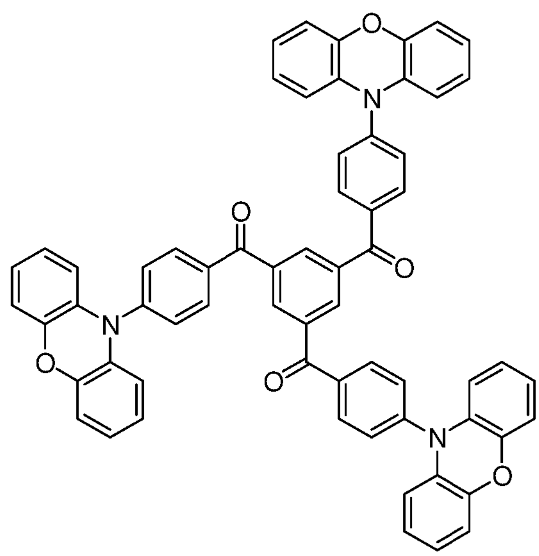

[Chem. 119]

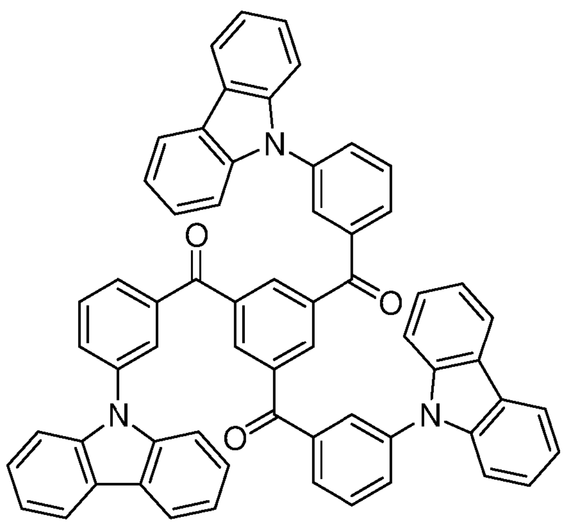

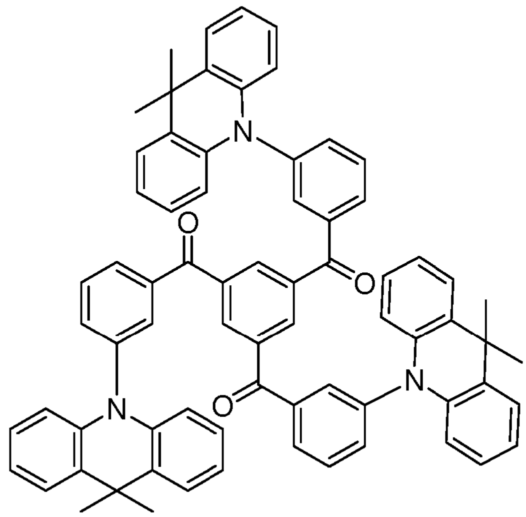

[Chem. 120]

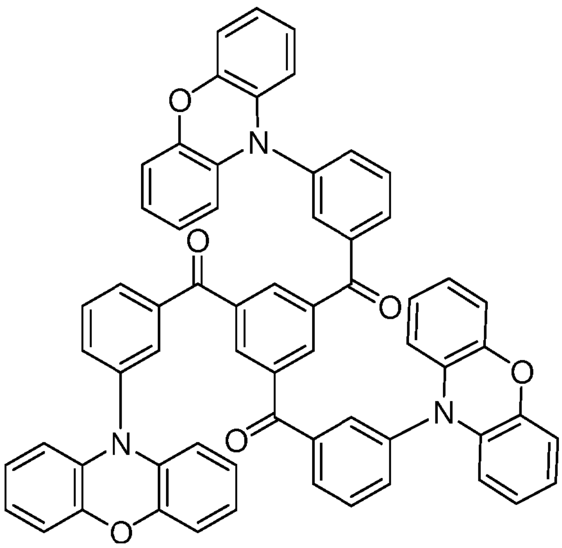

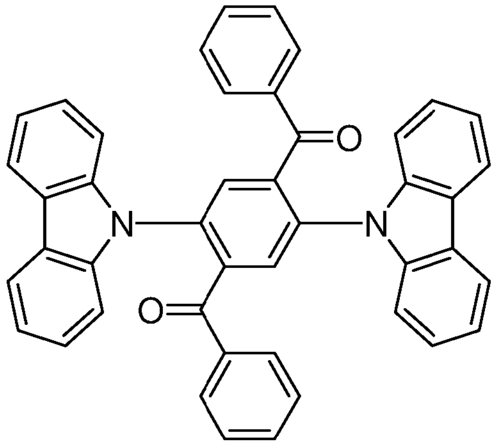

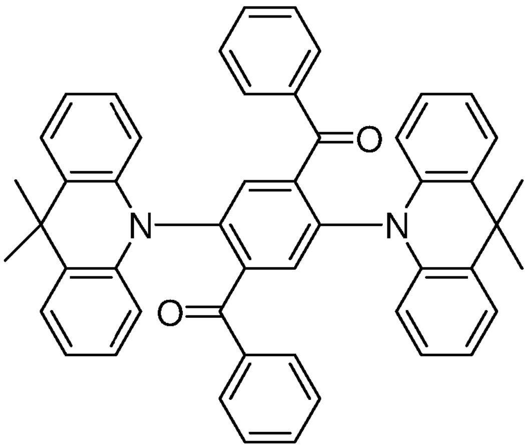

[Chem. 121]

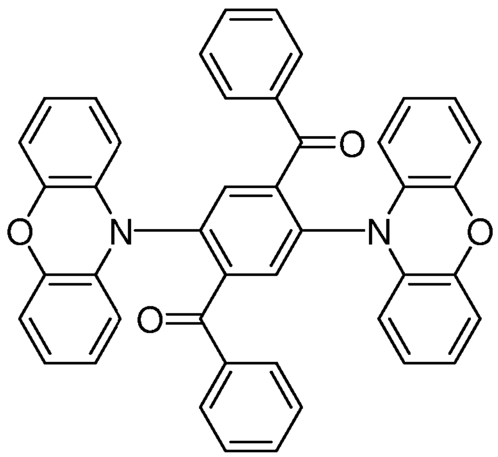

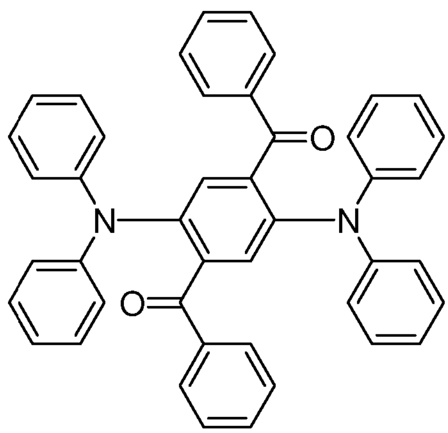

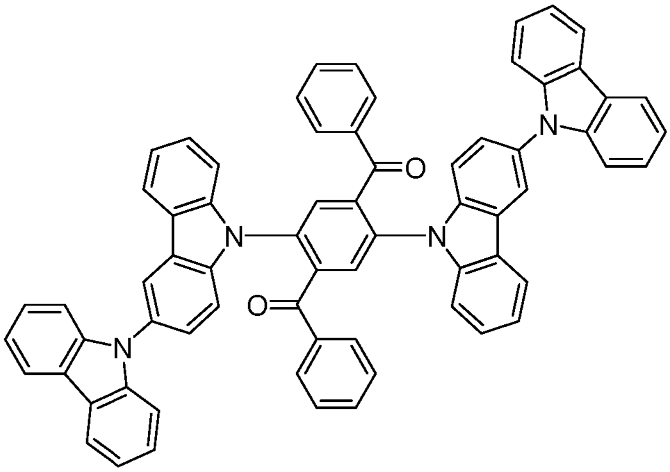

[Chem. 122]

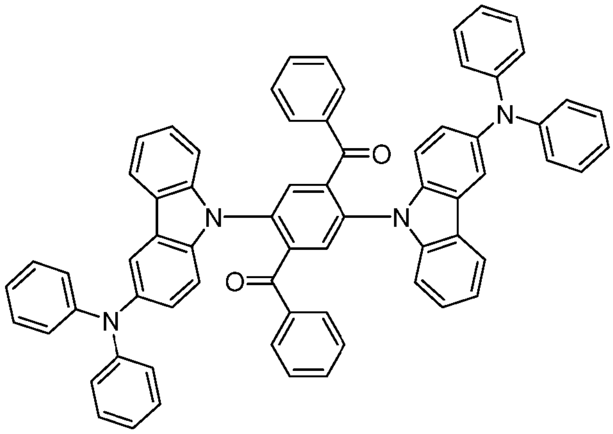

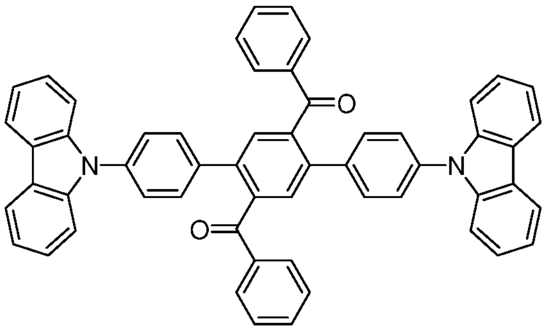

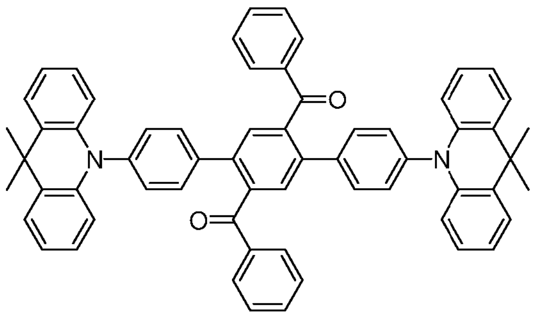

[Chem. 123]

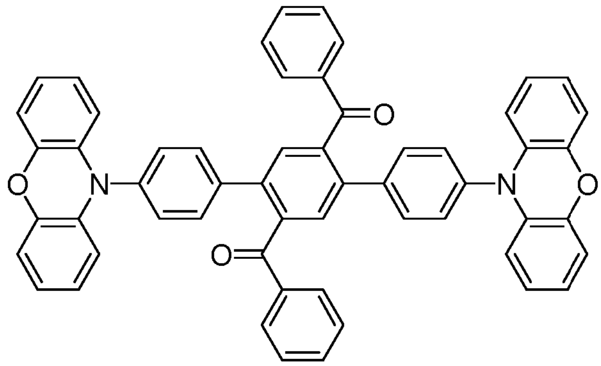

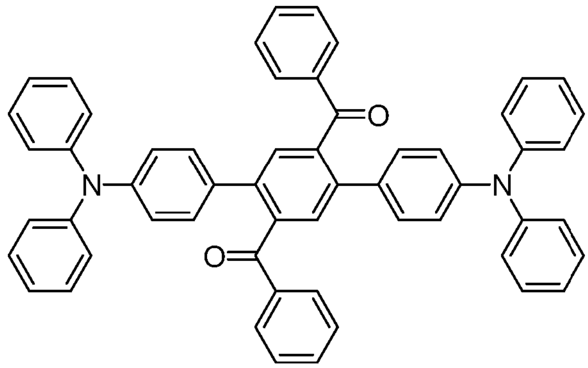

Examples of the preferred delayed fluorescent material include the following compounds.

[1] A compound represented by the following general formula (281):

[Chem. 124]

General Formula (281)

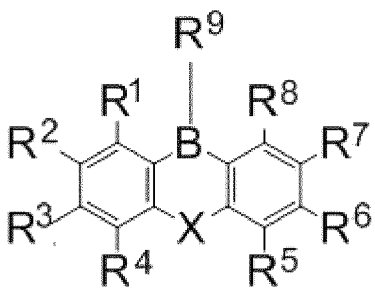

In the general formula (281), X represents an oxygen atom or a sulfur atom. $R^1$ to $R^8$ each independently represent a hydrogen atom or a substituent, provided that at least one of $R^1$ to $R^8$ represents a group represented by any one of the following general formulae (282) to (287). $R^1$ and $R^2$, $R^2$ and $R^3$, $R^3$ and $R^4$, $R^5$ and $R^6$, $R^6$ and $R^7$, $R^7$ and $R^8$, $R^8$ and $R^9$, and $R^9$ and $R^1$ may be bonded to each other to form a cyclic structure. $R^9$ represents a substituent, provided that when $R^9$ contains an atom that contains a lone electron pair without forming a single bond to the boron atom, the atom may form a cyclic structure through a coordination bond with the boron atom.

[Chem. 125]

General Formula (282)

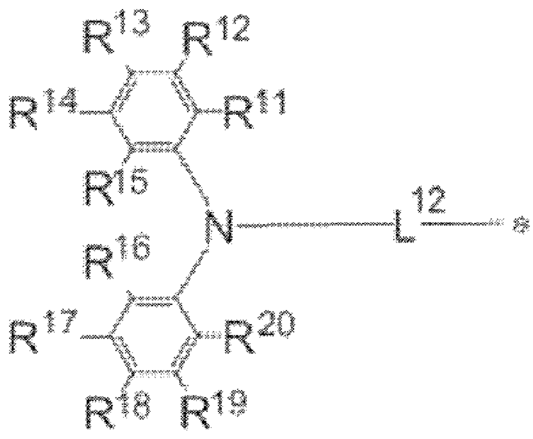

General Formula (283)

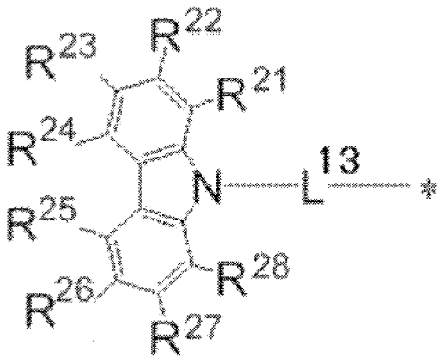

General Formula (284)

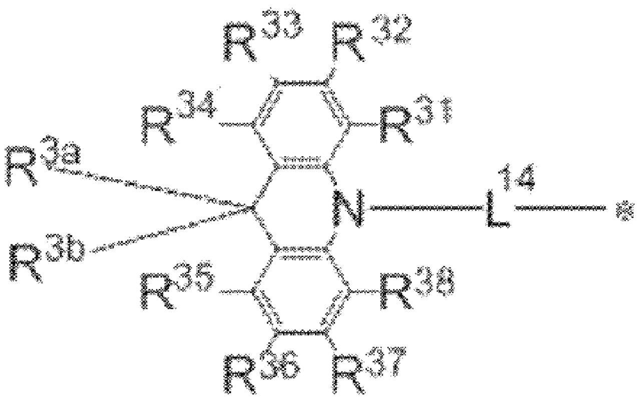

General Formula (285)

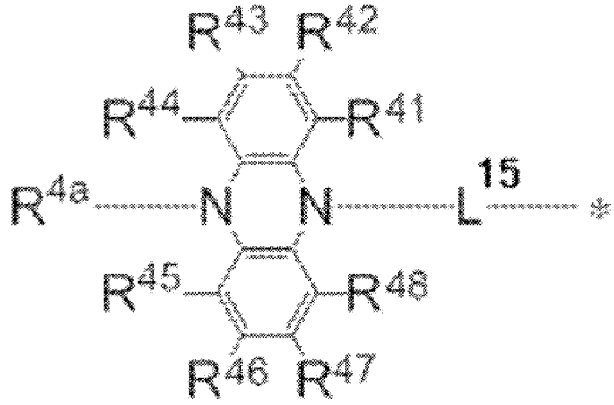

General Formula (286)

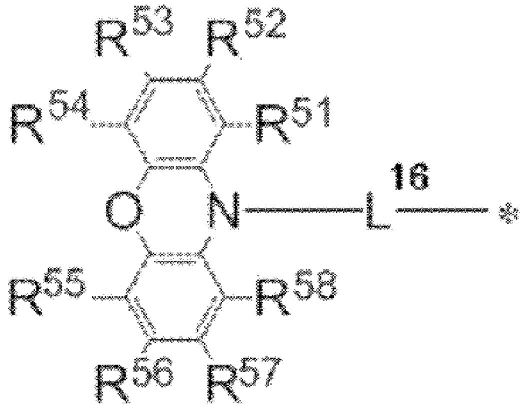

General Formula (287)

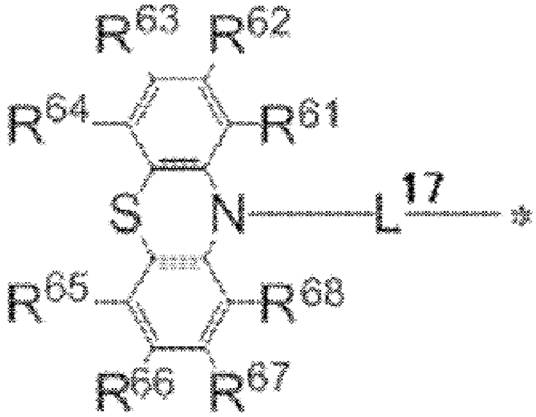

In the general formulae (282) to (287), $L^{12}$ to $L^{17}$ each independently represent a single bond or a divalent linking group; * represents the position bonded to the benzene ring in the general formula (281). $R^{11}$ to $R^{20}$, $R^{21}$ to $R^{28}$, $R^{31}$ to $R^{38}$, $R^{3a}$, $R^{3b}$, $R^{41}$ to $R^{48}$, $R^{4a}$, $R^{51}$ to $R^{58}$, $R^{61}$ to $R^{68}$ each independently represent a hydrogen atom or a substituent. $R^{11}$ and $R^{12}$, $R^{12}$ and $R^{13}$, $R^{13}$ and $R^{14}$, $R^{14}$ and $R^{15}$, $R^{16}$ and $R^{17}$, $R^{17}$ and $R^{18}$, $R^{18}$ and $R^{19}$, $R^{19}$ and $R^{20}$, $R^{21}$ and $R^{22}$, $R^{22}$ and $R^{23}$, $R^{23}$ and $R^{24}$, $R^{24}$ and $R^{25}$, $R^{25}$ and $R^{26}$, $R^{26}$ and $R^{27}$, $R^{27}$ and $R^{28}$, $R^{31}$ and $R^{32}$, $R^{32}$ and $R^{33}$, $R^{33}$ and $R^{34}$, $R^{35}$ and $R^{36}$, $R^{36}$ and $R^{37}$, $R^{37}$ and $R^{38}$, $R^{3a}$ and $R^{3b}$, $R^{41}$ and $R^{42}$, $R^{42}$ and $R^{43}$, $R^{43}$ and $R^{44}$, $R^{45}$ and $R^{46}$, $R^{46}$ and $R^{47}$, $R^{47}$ and $R^{48}$, $R^{51}$ and $R^{52}$, $R^{52}$ and $R^{53}$, $R^{53}$ and $R^{54}$, $R^{55}$ and $R^{56}$, $R^{56}$ and

$R^{57}$, $R^{57}$ and $R^{58}$, $R^{61}$ and $R^{62}$, $R^{62}$ and $R^{63}$, $R^{63}$ and $R^{64}$, $R^{65}$ and $R^{66}$, $R^{66}$ and $R^{67}$, and $R^{67}$ and $R^{68}$ each may be bonded to each other to form a cyclic structure.

[2] The compound according to [1], wherein in the general formula (281), at least one of $R^1$ to $R^8$ represents a group represented by any one of the general formulae (283) to (287).

[3] The compound according to [1] or [2], wherein in the case where at least one of $R^1$ to $R^8$ in the general formula (281) represents a group represented by the general formula (283), at least one of $R^{21}$ to $R^{28}$ in the general formula (283) represents a substituent.

[4] The compound according to any one of [1] to [3], wherein in the general formula (281), at least one of $R^2$, $R^3$, $R^6$, and $R^7$ represents a group represented by any one of the general formulae (282) to (287).

[5] The compound according to [4], wherein in the general formula (281), at least one of $R^3$ and $R^6$ represents a group represented by any one of the general formulae (282) to (287).

[6] The compound according to [5], wherein in the general formula (281), $R^3$ and $R^6$ each independently represent a group represented by any one of the general formulae (282) to (287).

[7] The compound according to any one of [1] to [6], wherein at least one of $R^{11}$ to $R^{20}$ in the general formula (282), at least one of $R^{21}$ to $R^{28}$ in the general formula (283), at least one of $R^{31}$ to $R^{38}$ and at least one of $R^{3a}$ and $R^{3b}$ in the general formula (284), at least one of $R^{41}$ to $R^{48}$ in the general formula (285), at least one of $R^{51}$ to $R^{58}$ in the general formula (286), and at least one of $R^{61}$ to $R^{68}$ in the general formula (287) each represent a substituent.

[8] The compound according to [7], wherein at least one of $R^{13}$ and $R^{18}$ in the general formula (282), at least one of $R^{23}$ and $R^{26}$ in the general formula (283), at least one of $R^{33}$ and $R^{36}$ and at least one of $R^{3a}$ and $R^{3b}$ in the general formula (284), at least one of $R^{43}$ and $R^{46}$ in the general formula (285), at least one of $R^{53}$ and $R^{56}$ in the general formula (286), and at least one of $R^{63}$ and $R^{66}$ in the general formula (287) each represent a substituent.

[9] The compound according to [8], wherein at least one of $R^{13}$ and $R^{18}$ in the general formula (282), at least one of $R^{23}$ and $R^{26}$ in the general formula (283), at least one of $R^{33}$ and $R^{36}$ and at least one of $R^{3a}$ and $R^{3b}$ in the general formula (284), at least one of $R^{43}$ and $R^{46}$ in the general formula (285), at least one of $R^{53}$ and $R^{56}$ in the general formula (286), and at least one of $R^{63}$ and $R^{66}$ in the general formula (287) each represent a group represented by any one of the general formulae (282) to (287).

[10] The compound according to any one of [1] to [9], wherein in the general formulae (282) to (287), $L^{12}$ to $L^{17}$ each represent a single bond.

[11] The compound according to any one of [1] to [10], wherein in the general formula (281), X represents an oxygen atom.

[12] The compound according to any one of [1] to [11], wherein in the general formula (281), $R^9$ represents a group represented by the following general formula (a): [Chem. 126]

General Formula (a)

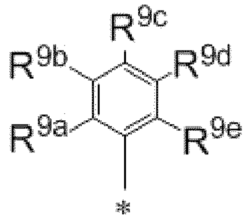

In the general formula (a), * represents the position bonded to the boron atom in the general formula (281). $R^{9a}$, $R^{9b}$, $R^{9c}$, $R^{9d}$, and $R^{9e}$ each independently represent a hydrogen atom or a substituent. $R^{9a}$ and $R^{9b}$, $R^{9b}$ and $R^{9c}$, $R^{9c}$ and $R^{9d}$, and $R^{9d}$ and $R^{9e}$ each may be bonded to each other to form a cyclic structure.

[13] The compound according to [12], wherein in the general formula (a), $R^{9a}$ and $R^{9b}$ each represent a substituent.

[14] The compound according to any one of [1] to [13], wherein in the general formula (281), at least one of $R^1$ to $R^8$ represents a group represented by the general formula (284).

[15] The compound according to any one of [1] to [4] and [7] to [14], wherein in the general formula (281), $R^3$ and $R^6$, or $R^2$ and $R^7$ each represent a group represented by the general formula (284).

[16] The compound according to [14] or [15], wherein in the general formula (284), $R^{3a}$ and $R^{3b}$ each represent a substituent.

[17] The compound according to any one of [14] to [16], wherein the substituent is an alkyl group having from 1 to 15 carbon atoms or a phenyl group.

[18] The compound according to any one of [14] to [16], wherein in the general formula (284), $R^{3a}$ and $R^{3b}$ are bonded to each other to form a cyclic structure.

Examples of the compound include the following compounds.

[Chem. 127]

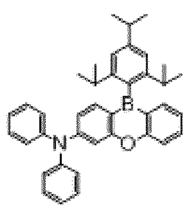
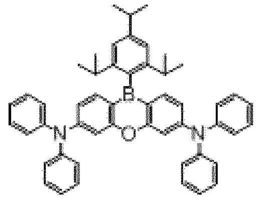
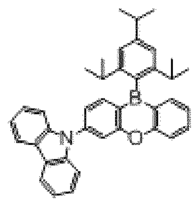
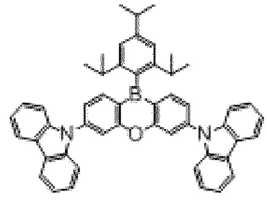
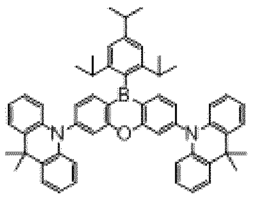
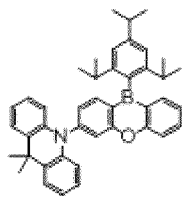
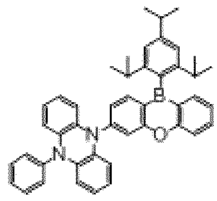
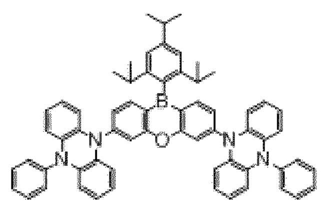
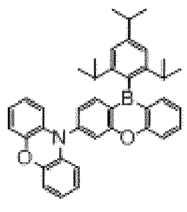
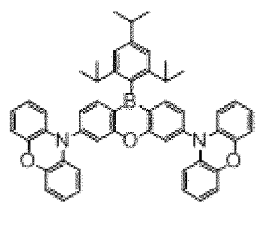
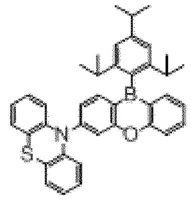
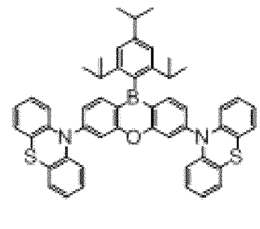
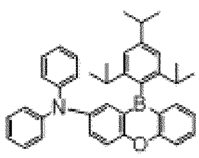
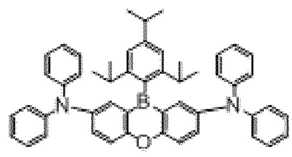
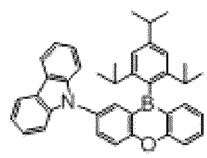
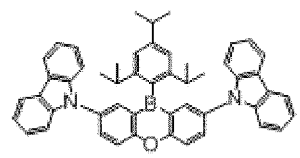
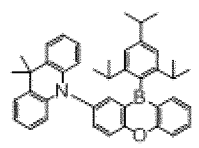
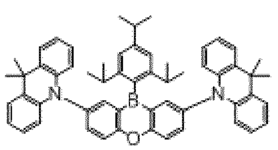
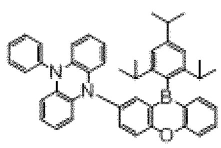
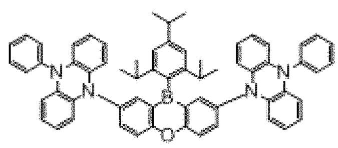
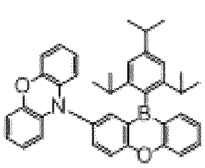
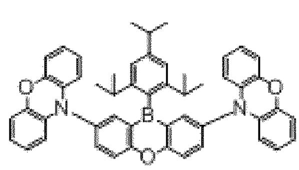
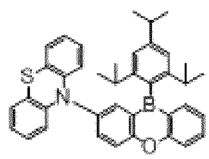
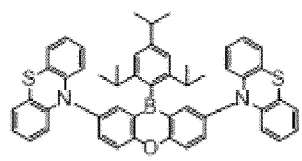

[Chem. 128]

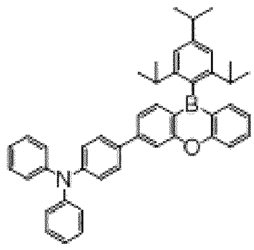
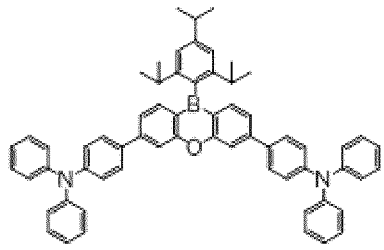
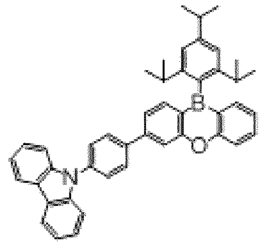
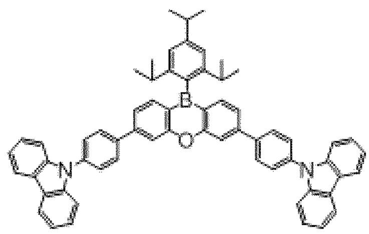
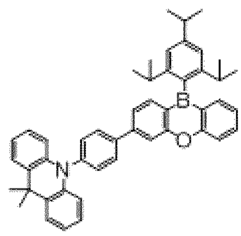
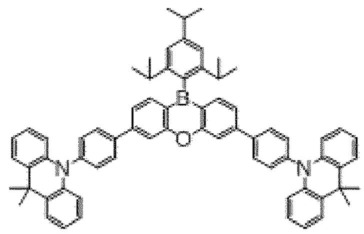
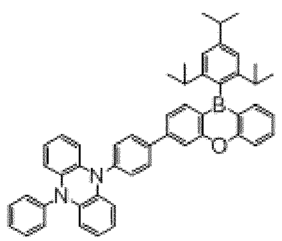
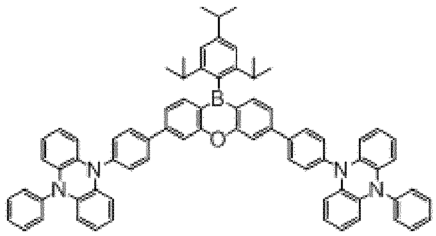
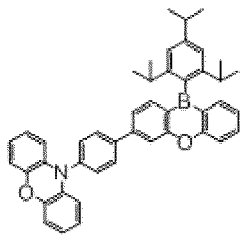
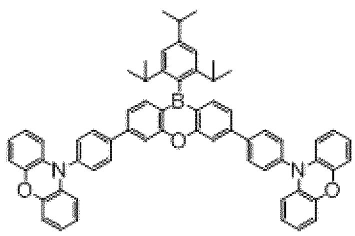
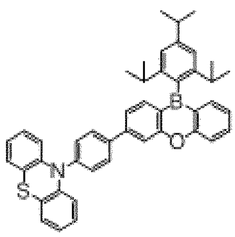
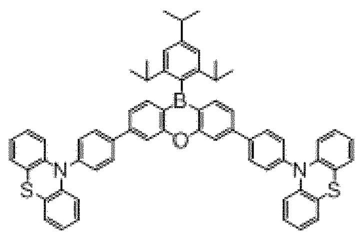

[Chem. 129]

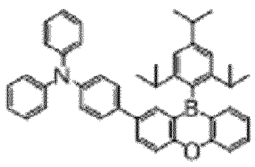
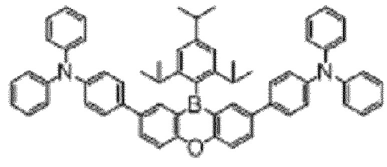
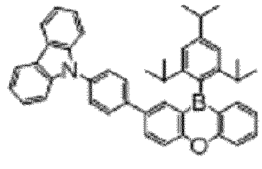
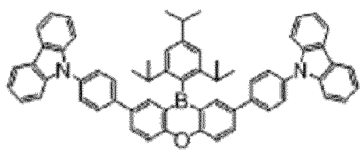
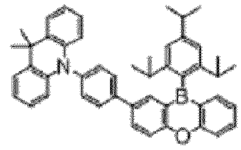
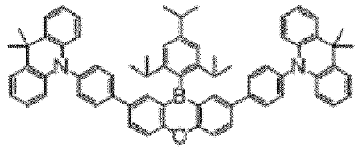
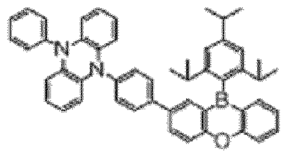
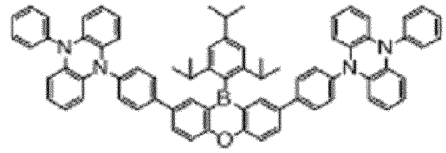
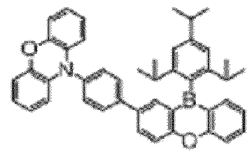
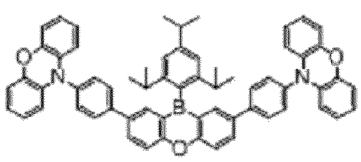
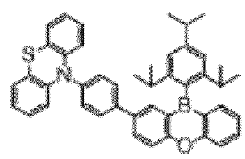
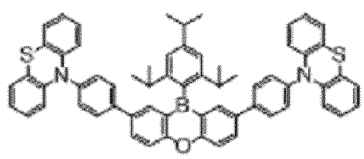
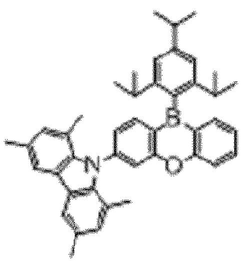
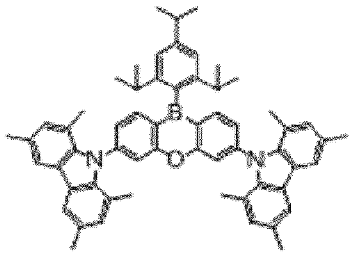
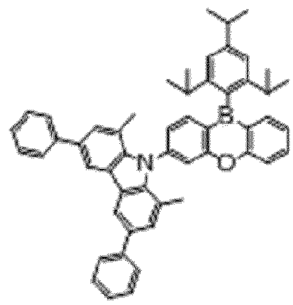

[Chem. 130]

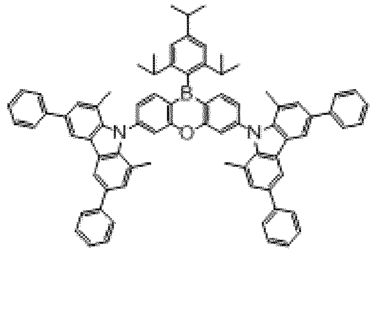
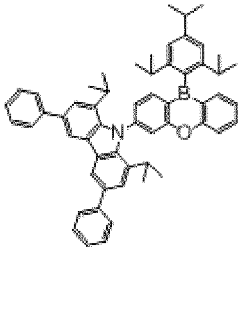
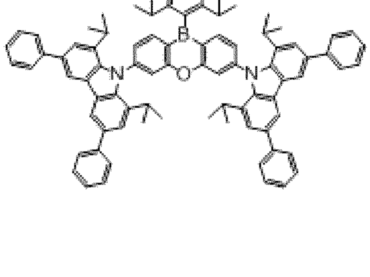

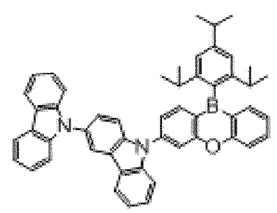
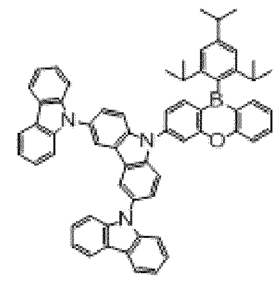
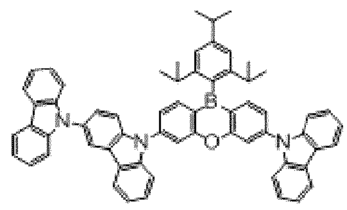

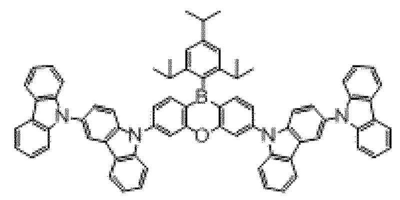
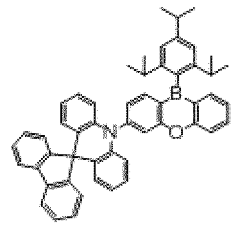
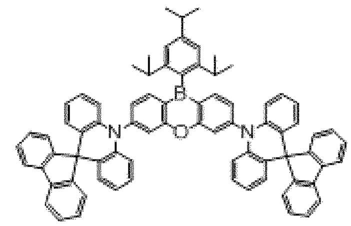

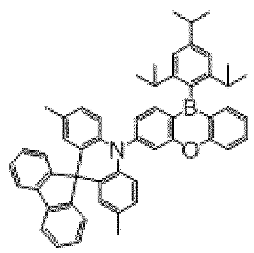
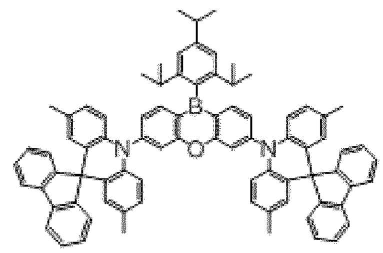
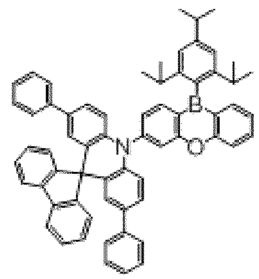

[Chem.131]

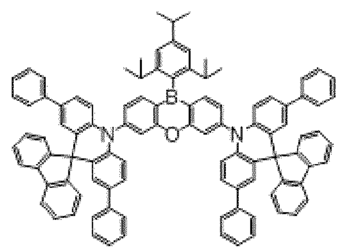
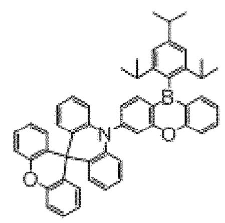
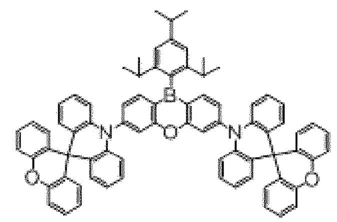

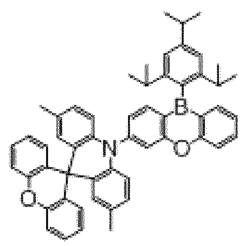
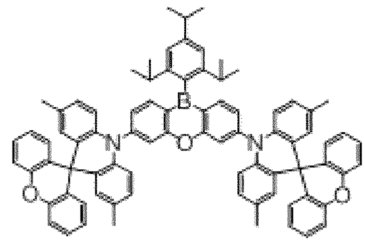
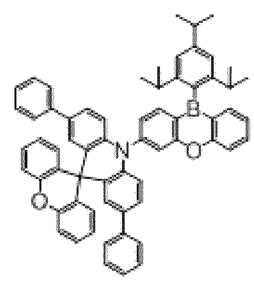

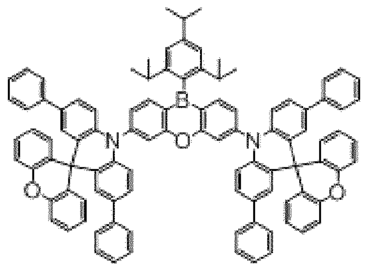
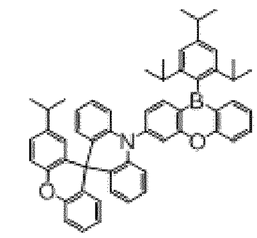
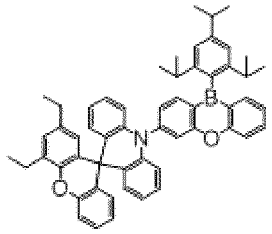

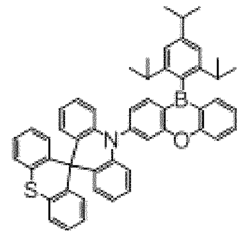
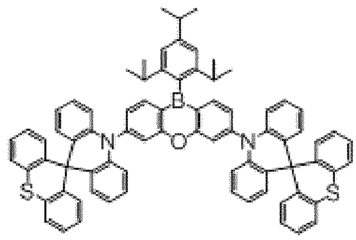
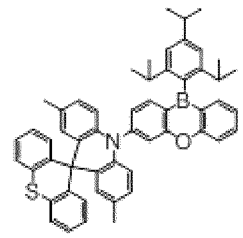

[Chem. 132]

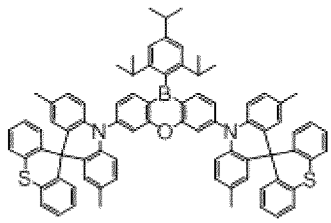
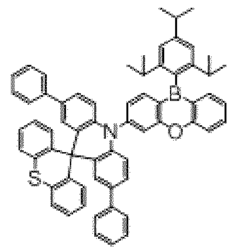
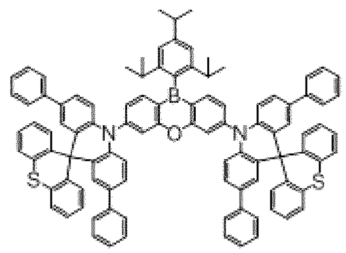

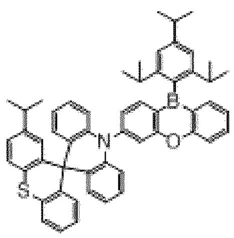
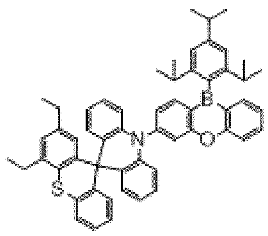
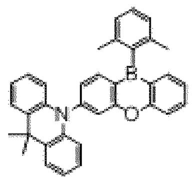
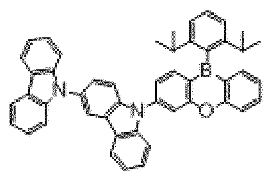

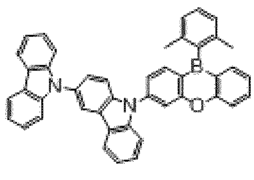
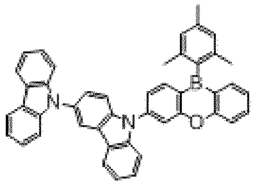
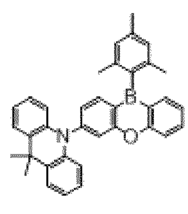
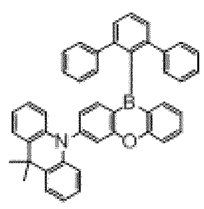

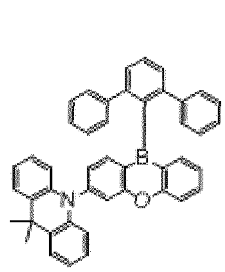
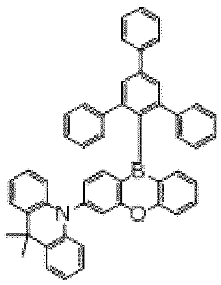
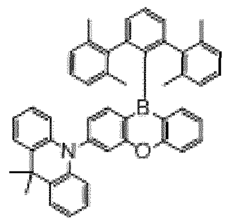
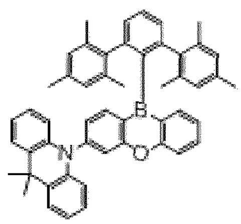

[Chem. 133]

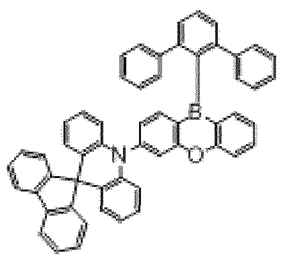
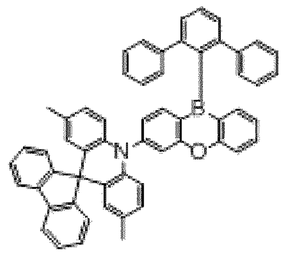
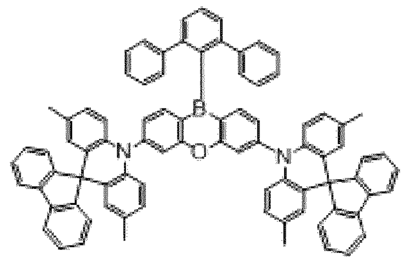

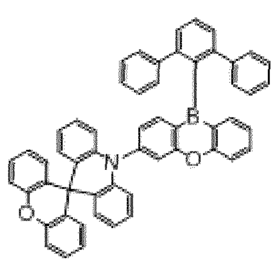
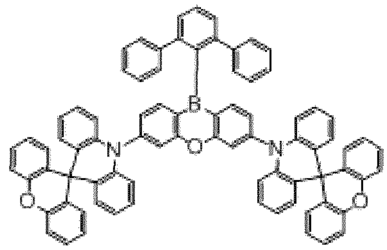
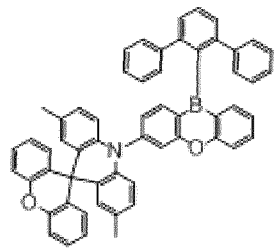

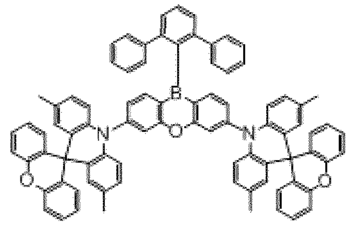
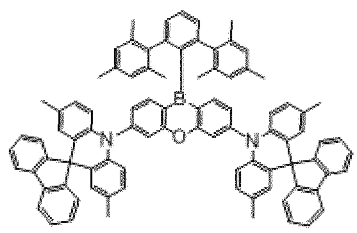
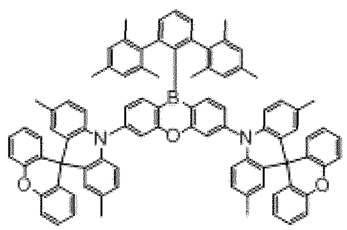

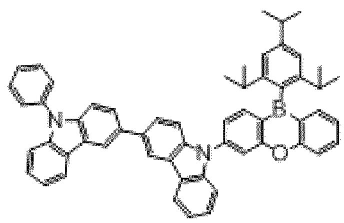
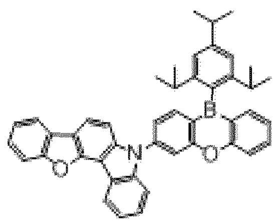
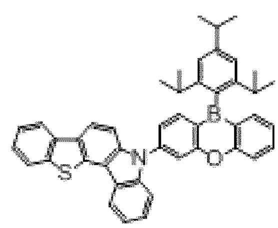

[Chem. 134]

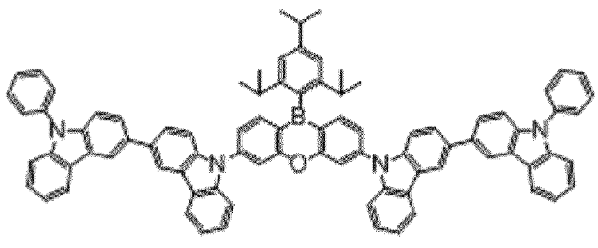
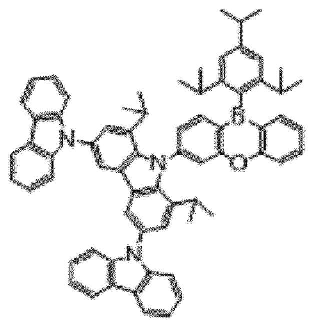

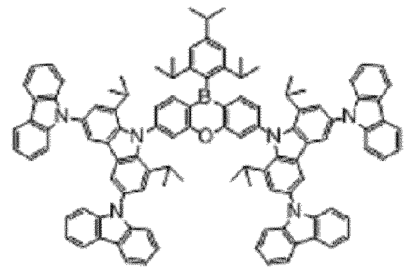
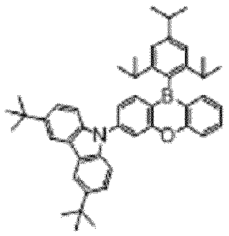
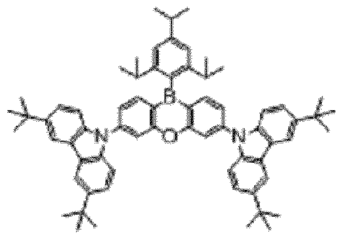

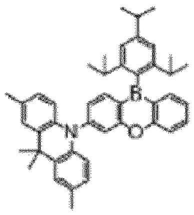
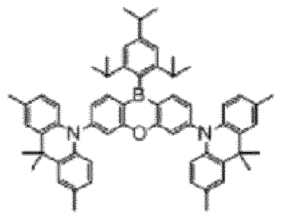
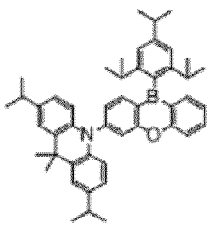
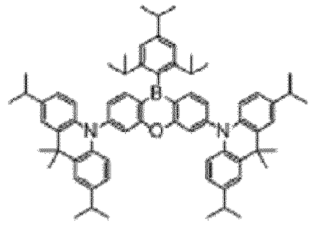

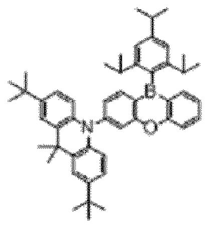
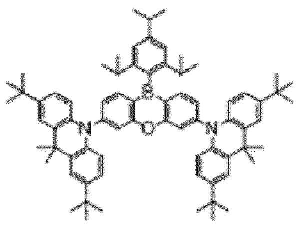
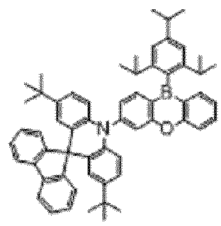
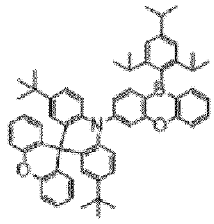

[Chem. 135]

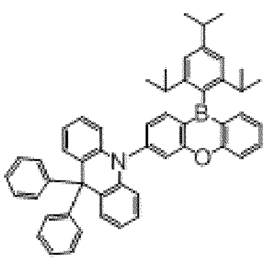
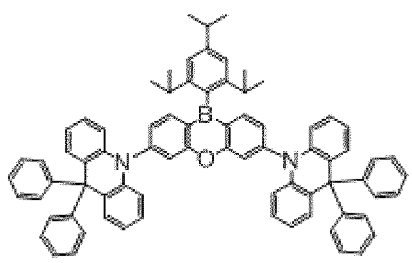
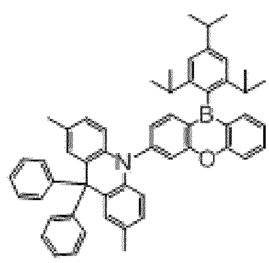

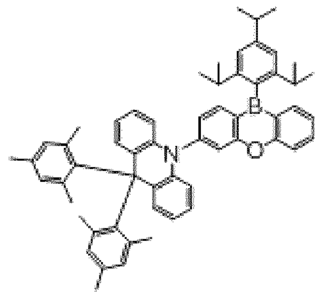
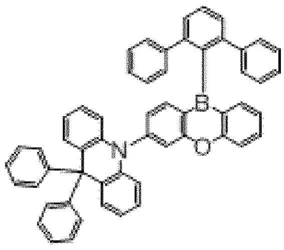
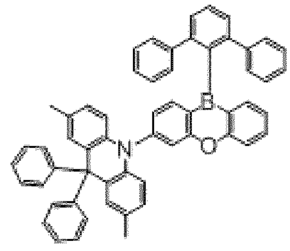

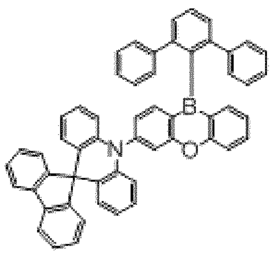
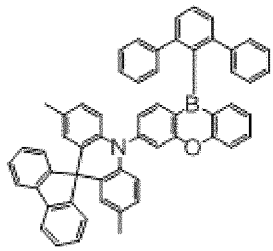
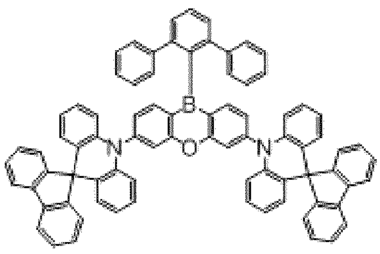

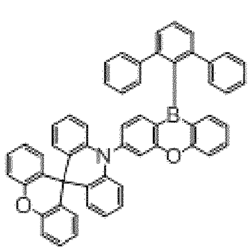
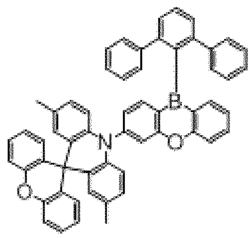
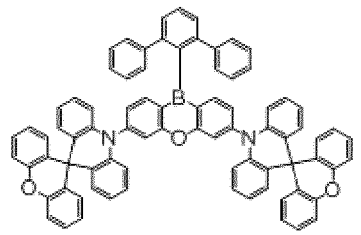

[Chem. 136]

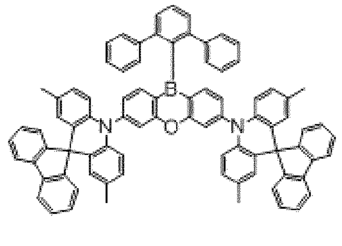
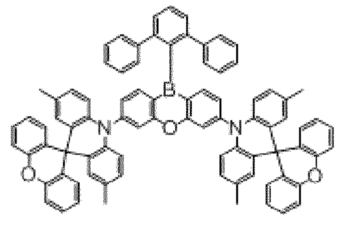
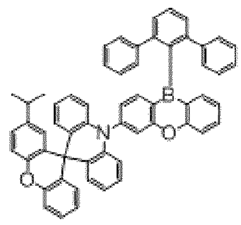

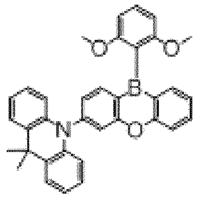
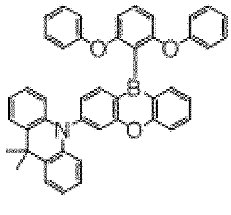
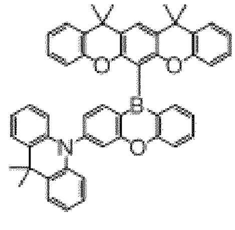
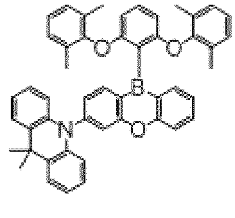

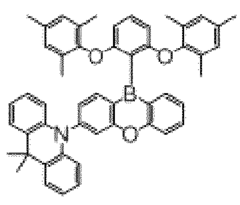
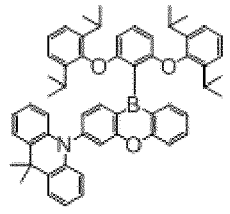
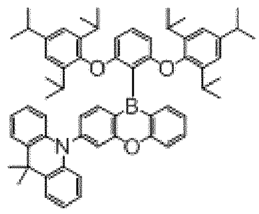
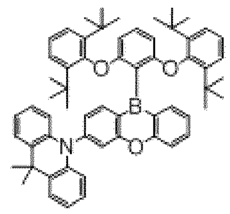

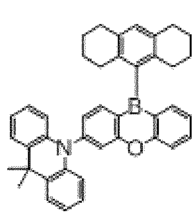
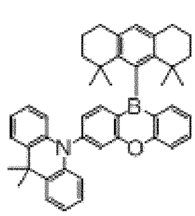
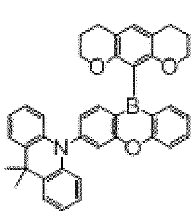
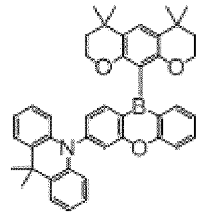

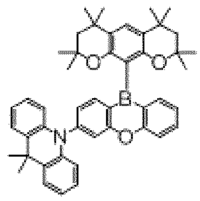
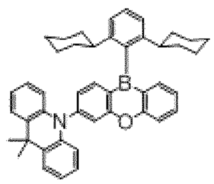
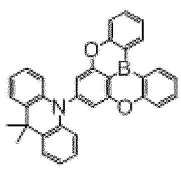
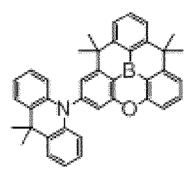
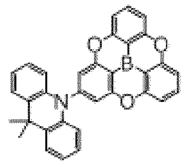

[Chem. 137]

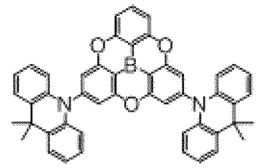
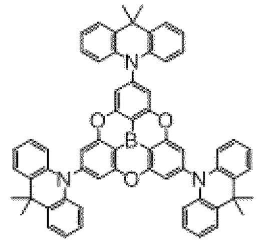
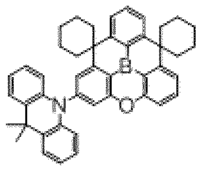
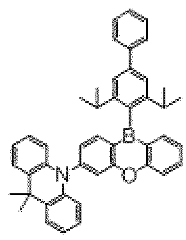

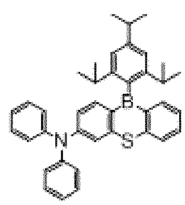
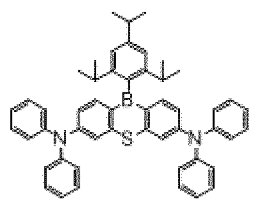
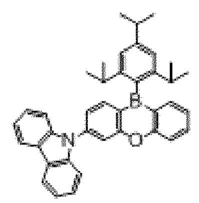
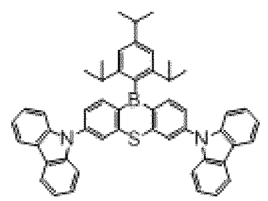

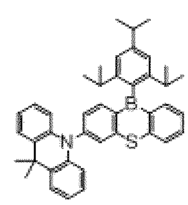
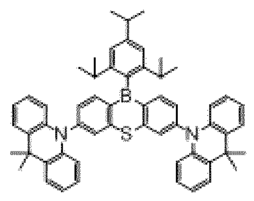
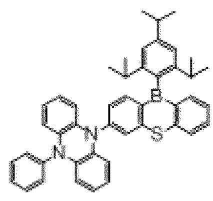
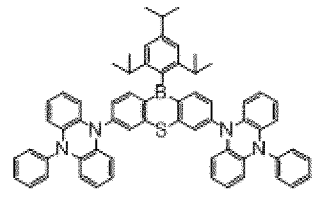

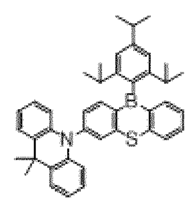
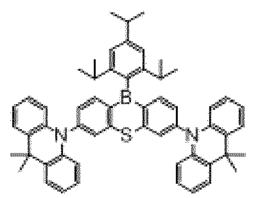
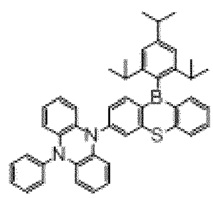
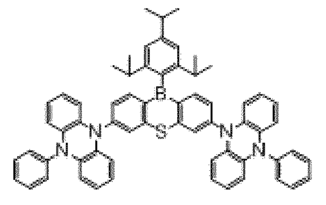

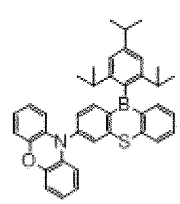
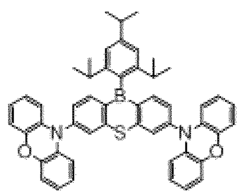
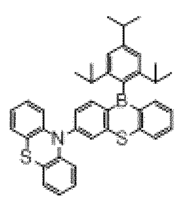
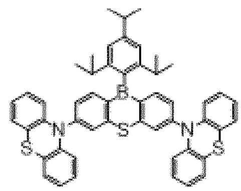

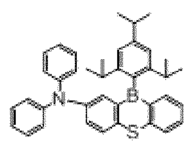
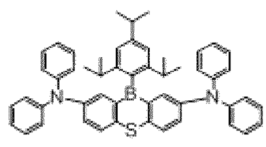
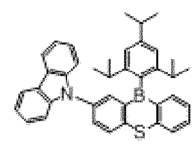
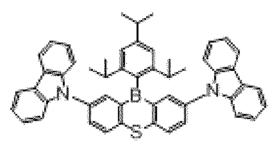

[Chem. 138]

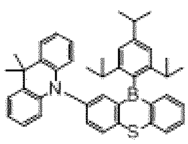
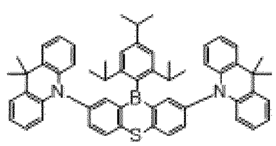
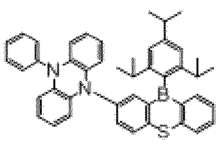
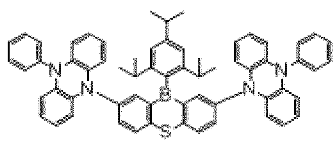

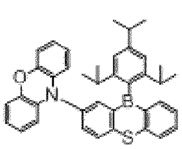
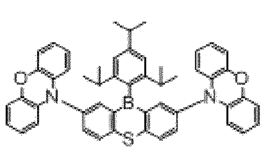
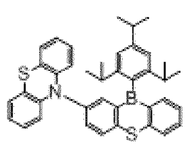
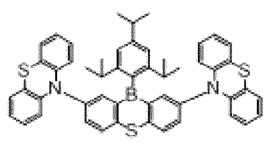

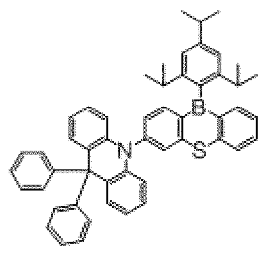

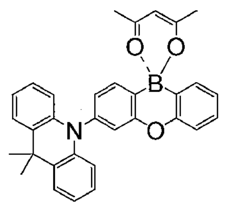

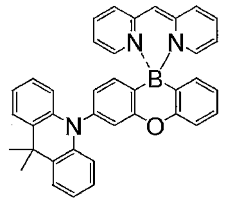

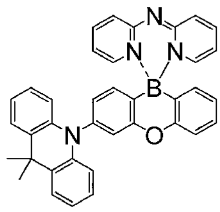

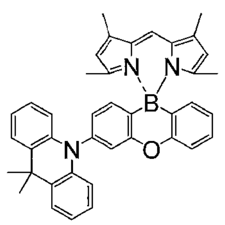

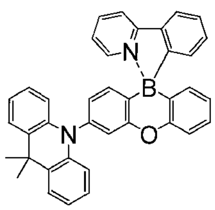

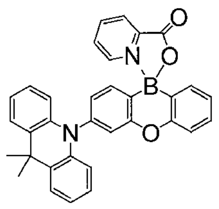

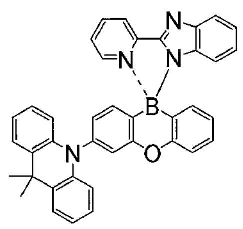

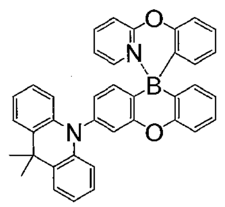

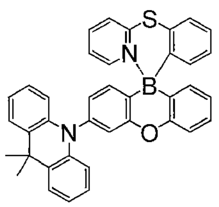

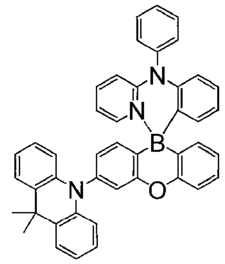

[Chem. 139]

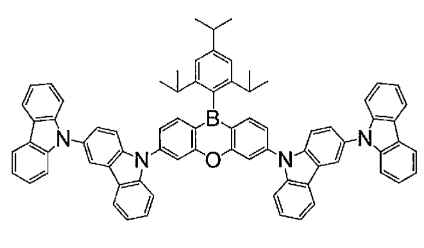

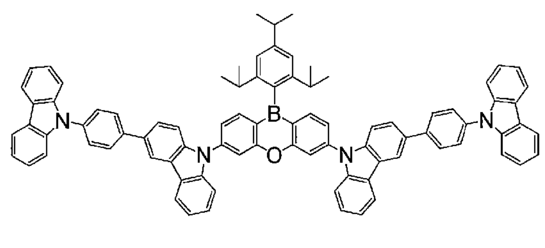

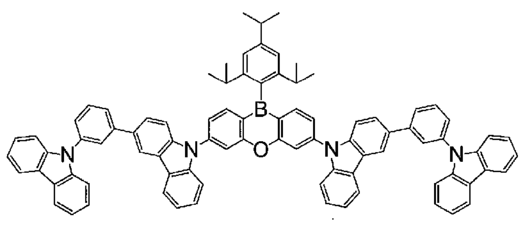

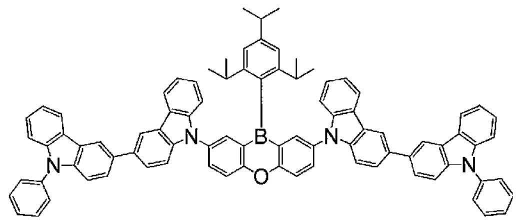

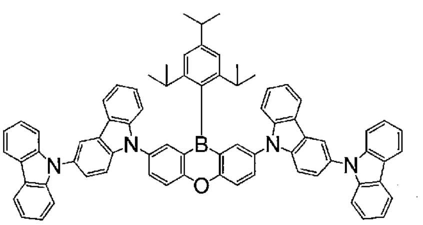

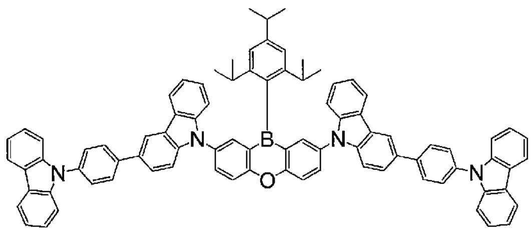

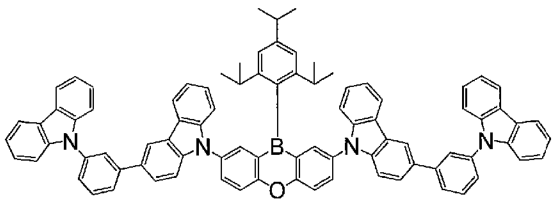

Examples of the preferred delayed fluorescent material include the following compounds.

[1] A compound represented by the following general formula (291):

[Chem. 140]

General Formula (291)

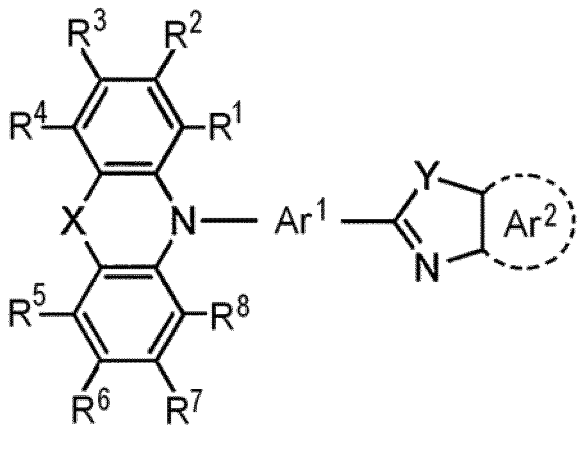

In the general formula (291), X represents O, S, N-$R^{11}$, C=O, C($R^{12}$)($R^{13}$), or Si($R^{14}$)($R^{15}$); Y represents O, S, or N-$R^{16}$. $Ar^1$ represents a substituted or unsubstituted arylene group; $Ar^2$ represents an aromatic ring or a heteroaromatic ring. $R^1$ to $R^8$ and $R^{11}$ to $R^{16}$ each independently represent a hydrogen atom or a substituent. $R^1$ and $R^2$, $R^2$ and $R^3$, $R^3$ and $R^4$, $R^5$ and $R^6$, $R^6$ and $R^7$, and Rand $R^8$ each may be bonded to each other to form a cyclic structure.

[2] The compound according to [1], wherein the compound represented by the general formula (291) is a compound represented by the following general formula (292):

[Chem. 141]

General Formula (292)

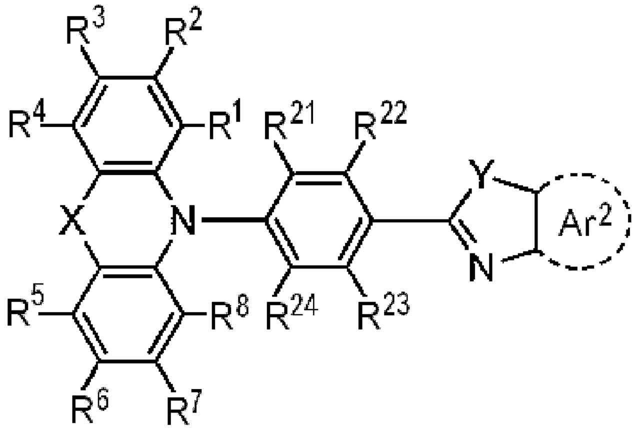

In the general formula (292), X represents O, S, N-$R^{11}$, C=O, C($R^{12}$)($R^{13}$), or Si($R^{14}$)($R^{15}$); Y represents O, S, or N-$R^{16}$. $Ar^2$ represents an aromatic ring or a heteroaromatic ring. $R^1$ to $R^8$, $R^{11}$ to $R^{16}$, and $R^{21}$ to $R^{24}$ each independently represent a hydrogen atom or a substituent. $R^1$ and $R^2$, $R^2$ and $R^3$, $R^3$ and $R^4$, $R^5$ and $R^6$, $R^6$ and $R^7$, $R^7$ and $R^8$, $R^{21}$ and $R^{22}$, and $R^{23}$ and $R^{24}$ each may be bonded to each other to form a cyclic structure.

[3] The compound according to [1], wherein the compound represented by the general formula (291) is a compound represented by the following general formula (293):

[Chem. 142]

General Formula (293)

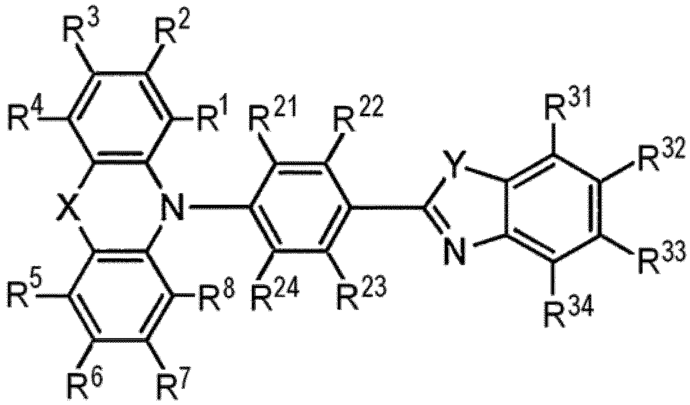

In the general formula (293), X represents O, S, N-$R^{11}$, C=O, C($R^{12}$)($R^{13}$), or Si($R^{14}$)($R^{15}$); Y represents O, S, or N-$R^{16}$. $R^1$ to $R^8$, $R^{11}$ to $R^{16}$, $R^{21}$ to $R^{24}$, and $R^{31}$ to $R^{34}$ each independently represent a hydrogen atom or a substituent. $R^1$ and $R^2$, $R^2$ and $R^3$, $R^3$ and $R^4$, $R^5$ and $R^6$, $R^6$ and $R^7$, $R^7$ and $R^8$, $R^{21}$ and $R^{22}$, $R^{23}$ and $R^{24}$, $R^{31}$ and $R^{32}$, $R^{32}$ and $R^{33}$, and $R^{33}$ and $R^{34}$ each may be bonded to each other to form a cyclic structure.

[4] The compound according to any one of [1] to [3], wherein X represents O or S.

[5] The compound according to any one of [1] to [4], wherein Y represents O, S, or N-$R^{16}$, and $R^{16}$ represents a substituted or unsubstituted aryl group.

[6] The compound according to any one of [1] to [5], wherein $R^1$ to $R^8$ each independently represent a hydrogen atom, a fluorine atom, a chlorine atom, a cyano group, a substituted or unsubstituted alkyl group having from 1 to 10 carbon atoms, a substituted or unsubstituted alkoxy group having from 1 to 10 carbon atoms, a substituted or unsubstituted dialkylamino group having from 1 to 10 carbon atoms, a substituted or unsubstituted diarylamino group having from 12 to 40 carbon atoms, a substituted or unsubstituted aryl group having from 6 to 15 carbon atoms, or a substituted or unsubstituted heteroaryl group having from 3 to 12 carbon atoms.

Examples of the compound include the following compounds.

[Chem. 143]

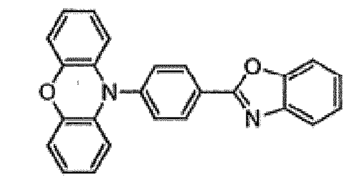
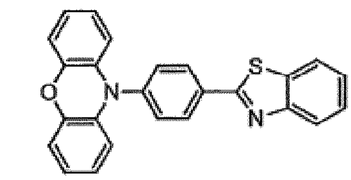
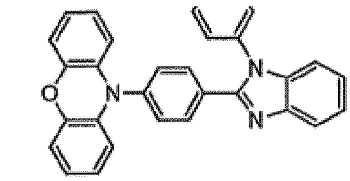
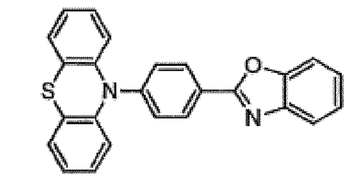
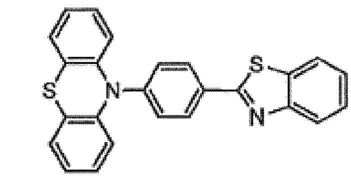
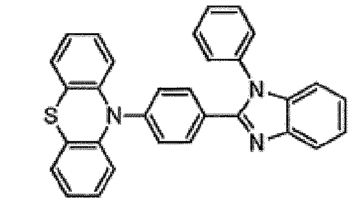
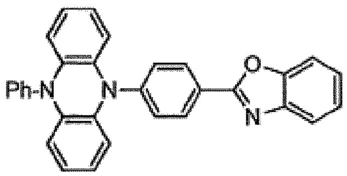
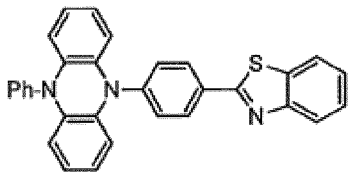
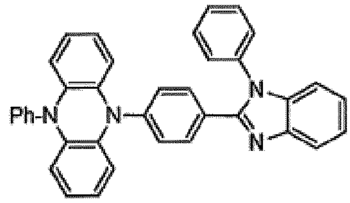
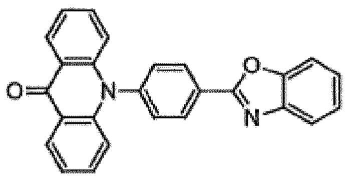
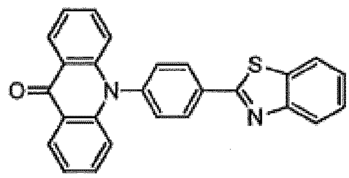
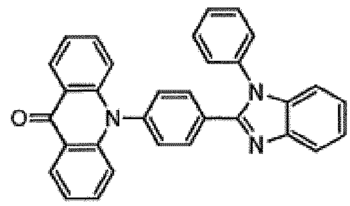
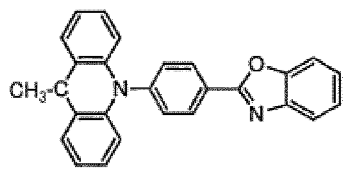
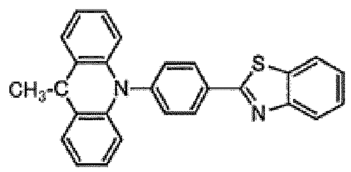
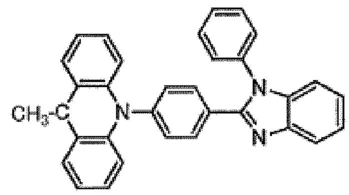
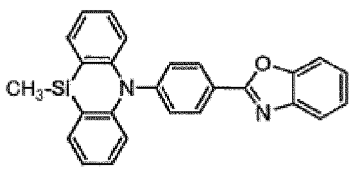
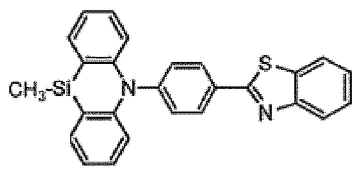
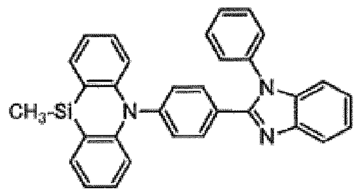

Examples of the preferred delayed fluorescent material include the following compounds.

[1] A compound represented by the following general formula (301):

General Formula (301) $(D)_n - A$

In the general formula (301), D represents a group represented by the following general formula (302); A represents an n-valent group containing a structure represented by the following general formula (303). n represents an integer of from 1 to 8.

[Chem. 144]

General Formula (302)

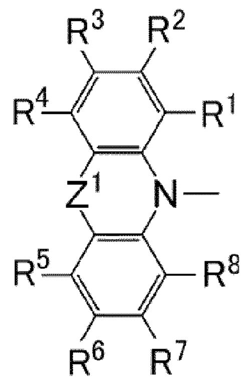

In the general formula (302), $Z^1$ represents O, S, C=O, $C(R^{21})(R^{22})$, $Si(R^{23})(R^{24})$, $N-Ar^3$, or a single bond; $R^{21}$ to $R^{24}$ each independently represent an alkyl group having from 1 to 8 carbon atoms; $Ar^3$ represents a substituted or unsubstituted aryl group. $R^1$ to $R^8$ each independently represent a hydrogen atom or a substituent. $R^1$ and $R^2$, $R^2$ and $R^3$, $R^3$ and $R^4$, $R^5$ and $R^6$, $R^6$ and $R^7$, and $R^7$ and $R^8$ may be bonded to each other to form a cyclic structure. When $Z^1$ represents a single bond, at least one of $R^1$ to $R^8$ represents a substituted or unsubstituted diarylamino group.

[Chem. 145]

General Formula (303)

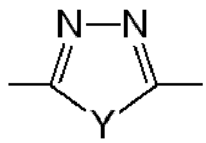

In the general formula (303), Y represents O, S, or $N-Ar^4$; and $Ar^4$ represents a substituted or unsubstituted aryl group.

[2] The compound according to [1], wherein in the general formula (302), $Z^1$ represents O, S, C=O, $C(R^{21})(R^{22})$, $Si(R^{23})(R^{24})$, or a single bond.

[3] The compound according to [1], wherein in the general formula (302), $Z^1$ represents $N-Ar^3$.

[4] The compound according to any one of [1] to [3], wherein in the general formula (301), A represents a group having a structure represented by the following general formula (304):

[Chem. 146]

General Formula (304)

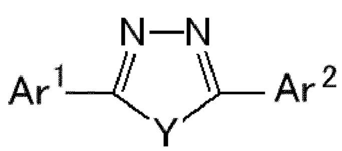

In the general formula (304), Y represents O, S, or $N-Ar^4$; and $Ar^1$ and $Ar^2$ each independently represent a substituted or unsubstituted aromatic group.

[5] The compound according to any one of [1] to [4], wherein in the general formula (301), n represents an integer of from 1 to 4.

[6] The compound according to any one of [1] to [3], wherein the compound is represented by the following general formula (305):

[Chem. 147]

General Formula (305)

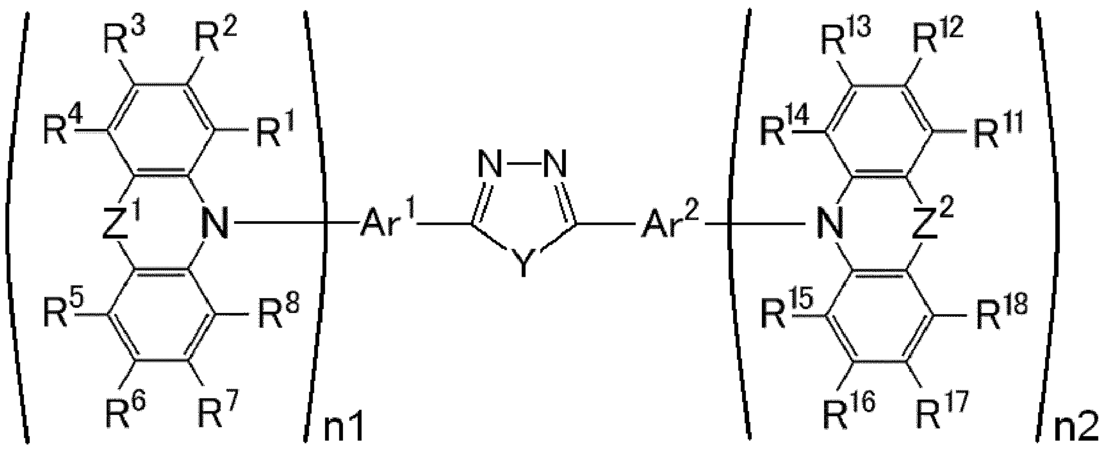

In the general formula (305), $Z^1$ and $Z^2$ each independently represent O, S, C=O, $C(R^{21})(R^{22})$, $Si(R^{23})(R^{24})$, N-$Ar^3$, or a single bond; $R^{21}$ to $R^{24}$ each independently represent an alkyl group having from 1 to 8 carbon atoms; $Ar^3$ represents a substituted or unsubstituted aryl group. $Ar^1$ and $Ar^2$ each independently represent a substituted or unsubstituted aromatic group. Y represents O, S, or N-$Ar^4$; $Ar^4$ represents a substituted or unsubstituted aryl group. $R^1$ to $R^8$ and $R^{11}$ to $R^{18}$ each independently represent a hydrogen atom or a substituent. $R^1$ and $R^2$, $R^2$ and $R^3$, $R^3$ and $R^4$, $R^5$ and $R^6$, $R^6$ and $R^7$, $R^7$ and $R^8$, $R^{11}$ and $R^{12}$, $R^{12}$ and $R^{13}$, $R^{13}$ and $R^{14}$, $R^{15}$ and $R^{16}$, $R^{16}$ and $R^{17}$, and $R^{17}$ and $R^{18}$ each may be bonded to each other to form a cyclic structure, provided that when $Z^1$ represents a single bond, at least one of $R^1$ to $R^8$ represents a substituted or unsubstituted diarylamino group, and when $Z^2$ represents a single bond, at least one of $R^{11}$ to $R^{18}$ represents a substituted or unsubstituted diarylamino group. n1 and n2 each independently represent an integer of from 0 to 8, provided that the sum of n1 and n2 is from 1 to 8.

[7] The compound according to [6], wherein in the general formula (305), $Z^1$ and $Z^2$ each independently represent O, S, N-$Ar^3$, or a single bond.

[8] The compound according to [6] or [7], wherein in the general formula (305), Y represents O or N-$Ar^4$.

[9] The compound according to any one of [1] to [3], wherein the compound is represented by the following general formula (306):

[Chem. 148]

General Formula (306)

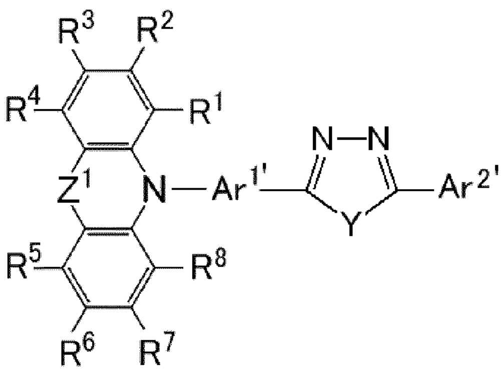

In the general formula (306), $Z^1$ represents O, S, C=O, $C(R^{21})(R^{22})$, $Si(R^{23})(R^{24})$, N-$Ar^3$, or a single bond; $R^{21}$ to $R^{24}$ each independently represent an alkyl group having from 1 to 8 carbon atoms; $Ar^3$ represents a substituted or unsubstituted aryl group. $Ar^{1'}$ represents a substituted or unsubstituted arylene group. $Ar^{2'}$ represents a substituted or unsubstituted aryl group. Y represents O, S, or N-$Ar^4$; $Ar^4$ represents a substituted or unsubstituted aryl group. $R^1$ to $R^8$ each independently represent a hydrogen atom or a substituent. $R^1$ and $R^2$, $R^2$ and $R^3$, $R^3$ and $R^4$, $R^5$ and

$R^6$, $R^6$ and $R^7$, and $R^7$ and $R^8$ each may be bonded to each other to form a cyclic structure, provided that when $Z^1$ represents a single bond, at least one of $R^1$ to $R^8$ represents a substituted or unsubstituted diarylamino group.
[10] The compound according to any one of [1] to [3], wherein the compound is represented by the following general formula (307):

[Chem. 149]

General Formula (307)

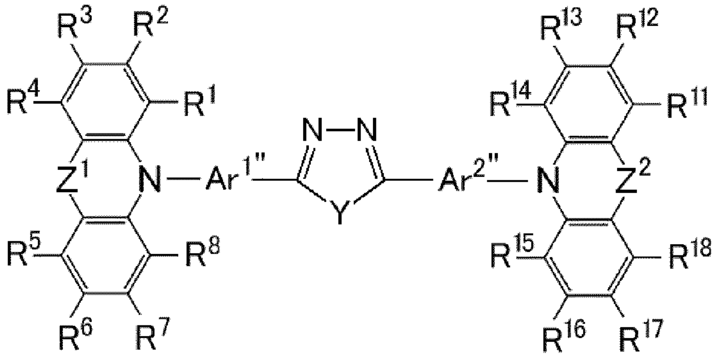

In the general formula (307), $Z^1$ and $Z^2$ each independently represent O, S, C=O, $C(R^{21})(R^{22})$, $Si(R^{23})(R^{24})$, $N-Ar^3$, or a single bond; $R^{21}$ to $R^{24}$ each independently represent an alkyl group having from 1 to 8 carbon atoms; $Ar^3$ represents a substituted or unsubstituted aryl group. $Ar^{1''}$ and $Ar^{2''}$ each independently represent a substituted or unsubstituted arylene group. Y represents O, S, or $N-Ar^4$; $Ar^4$ represents a substituted or unsubstituted aryl group. $R^1$ to $R^8$ and $R^{11}$ to $R^{18}$ each independently represent a hydrogen atom or a substituent. $R^1$ and $R^2$, $R^2$ and $R^3$, $R^3$ and $R^4$, $R^5$ and $R^6$, $R^6$ and $R^7$, $R^7$ and $R^8$, $R^{11}$ and $R^{12}$, $R^{12}$ and $R^{13}$, $R^{13}$ and $R^{14}$, $R^{15}$ and $R^{16}$, $R^{16}$ and $R^{17}$, and $R^{17}$ and $R^{18}$ each may be bonded to each other to form a cyclic structure, provided that when $Z^1$ represents a single bond, at least one of $R^1$ to $R^8$ represents a substituted or unsubstituted diarylamino group, and when $Z^2$ represents a single bond, at least one of $R^{11}$ to $R^{18}$ represents a substituted or unsubstituted diarylamino group.
[11] The compound according to [10], wherein in the general formula (307), $Z^1$ and $Z^2$ are the same as each other, $Ar^{1''}$ and $Ar^{2''}$ are the same as each other, $R^1$ and $R^{14}$ are the same as each other, $R^2$ and $R^{13}$ are the same as each other, $R^3$ and $R^{12}$ are the same as each other, $R^4$ and $R^{11}$ are the same as each other, $R^5$ and $R^{18}$ are the same as each other, $R^6$ and $R^{17}$ are the same as each other, $R^7$ and $R^{16}$ are the same as each other, and $R^8$ and $R^{15}$ are the same as each other.
[12] The compound according to [10] or [11], wherein in the general formula (307), $Z^1$ and $Z^2$ each independently represent O, S, or $N-Ar^3$.

Examples of the compound include the following compounds.

[Chem. 150]

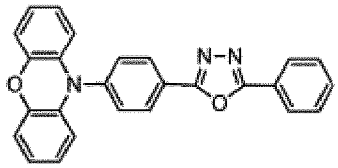

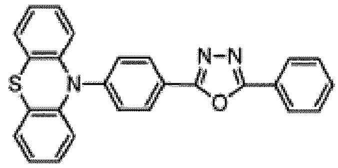

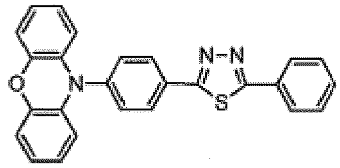

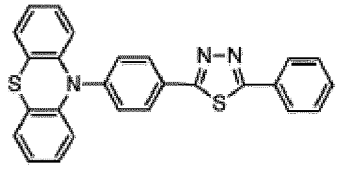

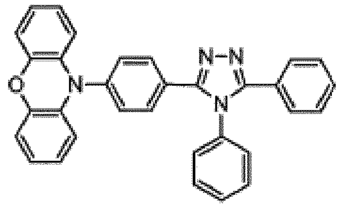

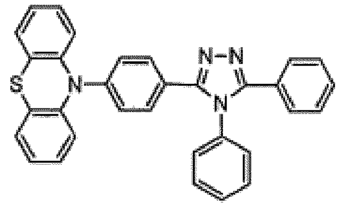

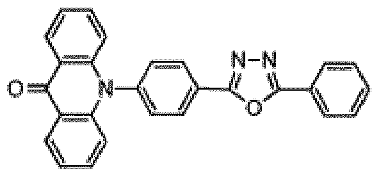

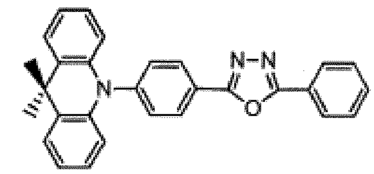

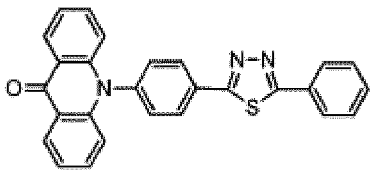

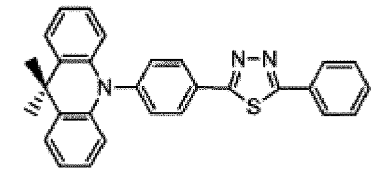

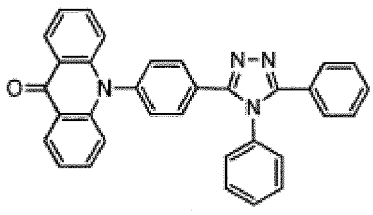

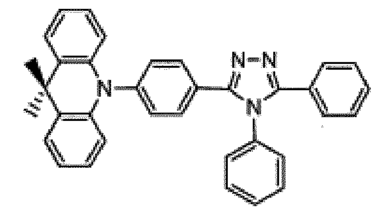

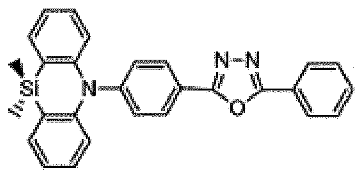

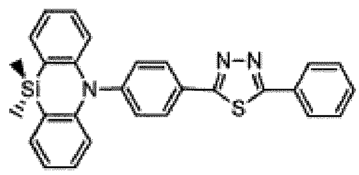

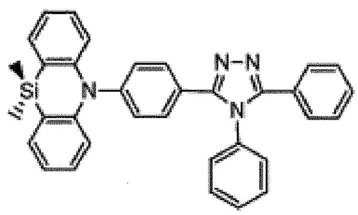

[Chem. 151]

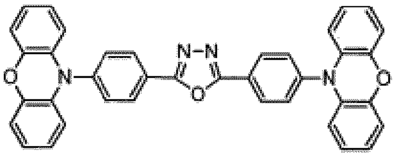
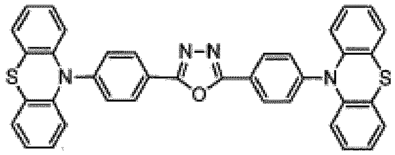
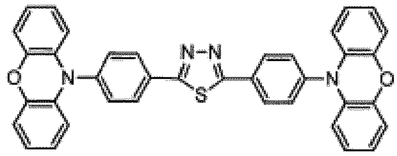
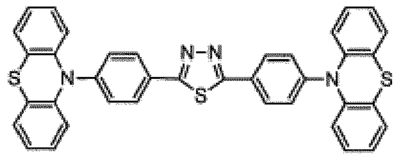
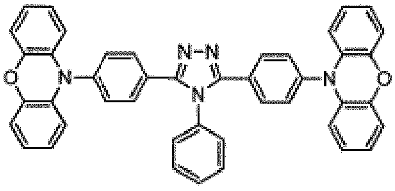
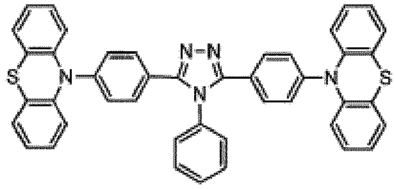
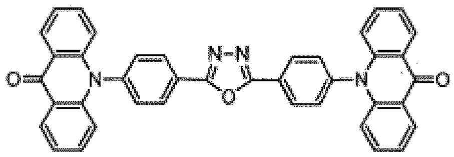
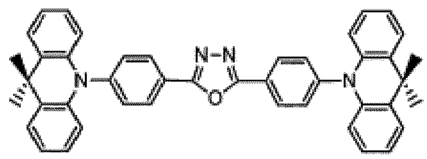
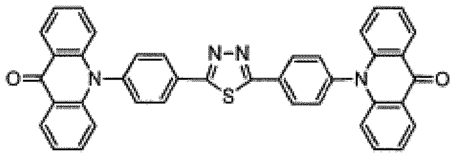
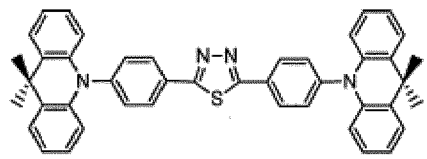
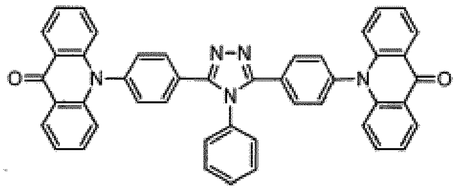
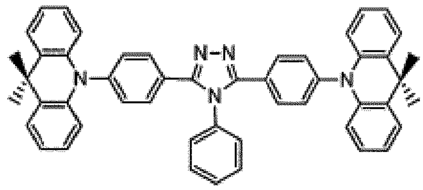
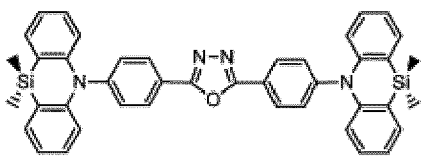
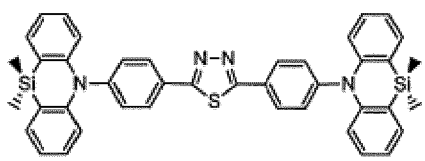
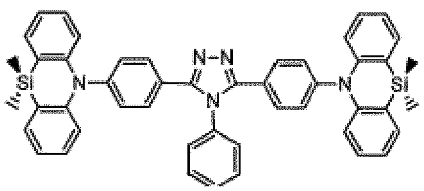

[Chem. 152]

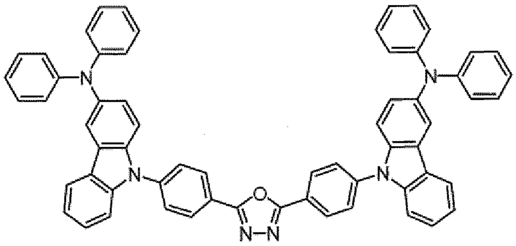

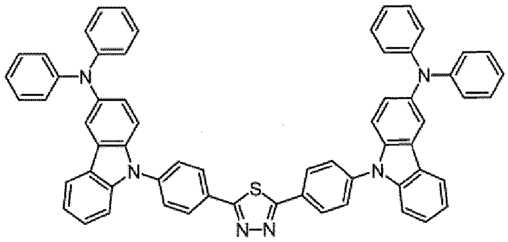

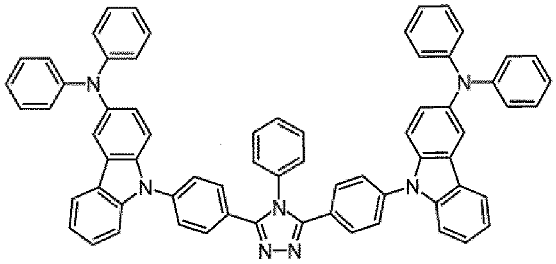

[Chem. 153]

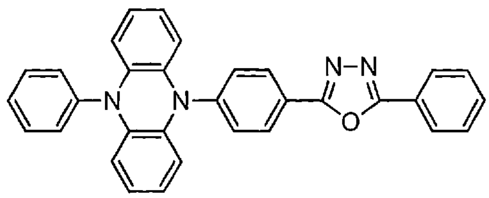

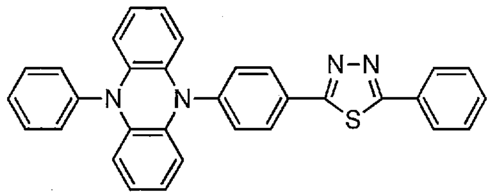

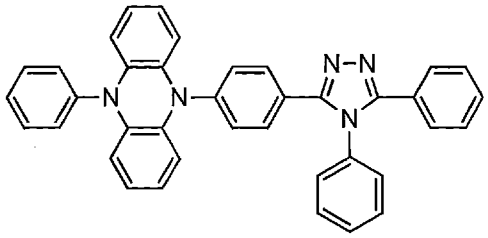

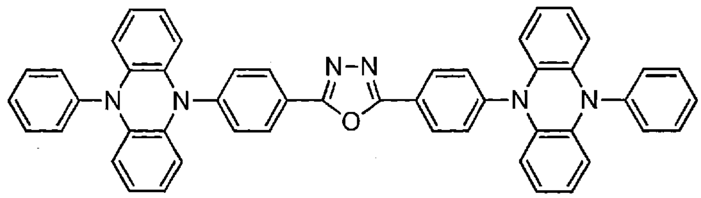

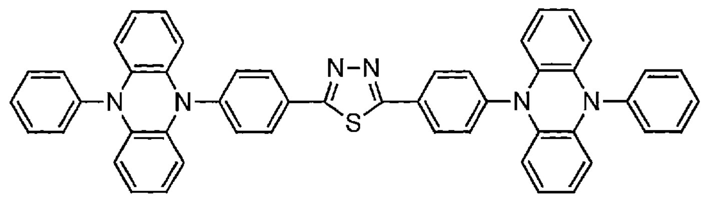

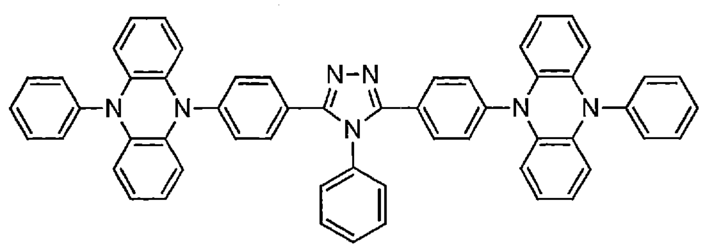

The molecular weight of the delayed fluorescent material is preferably 1,500 or less, more preferably 1,200 or less, further preferably 1,000 or less, and still further preferably 800 or less, for example, in the case where a light emitting layer containing the delayed fluorescent material is intended to be formed as a film by a vapor deposition method. The lower limit of the molecular weight, for example, of the delayed fluorescent material represented by the above-mentioned general formulae is the molecular weight of the smallest compound represented by these general formulae.

In the case where the light emitting layer is formed by a coating method, the material that has a relatively large molecular weight may also be preferably used irrespective of the molecular weight thereof.

(Host Material)

The host material is an organic compound having a lowest excited singlet energy that is larger than that of the delayed fluorescent material and the light-emitting material, and has a function of assuming the transfer of carriers and

a function of confining the energy of the light-emitting material within the material. Accordingly, the light-emitting material can efficiently convert the energy formed through recombination of holes and electrons in the molecule and the energy received from the host material and the delayed fluorescent material to light emission, and thus an organic light-emitting device having a high light emission efficiency can be realized.

The host material is preferably such an organic compound that has a hole transporting function and an electron transporting function, prevents the light emission from having a longer wavelength, and has a high glass transition temperature. Examples of the preferred compound capable of being used as the host material are shown below. In the structural formulae of the example compounds, R and $R_1$ to $R_{10}$ each independently represent a hydrogen atom or a substituent, and n represents an integer of from 3 to 5.

[Chem. 154]

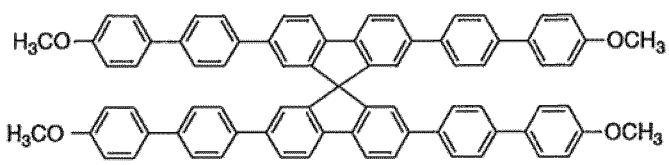

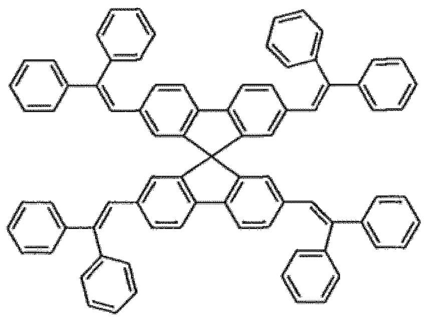

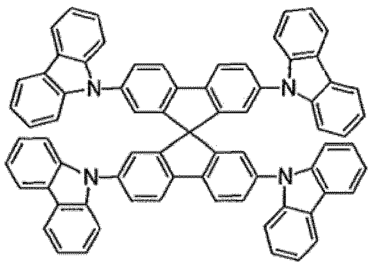

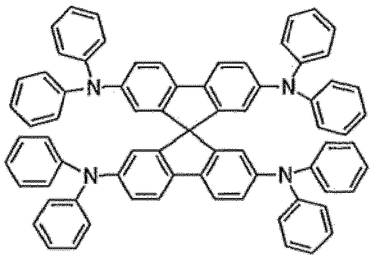

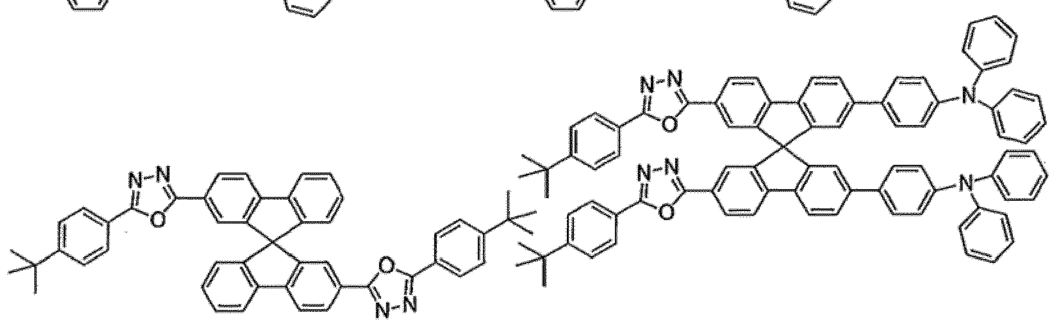

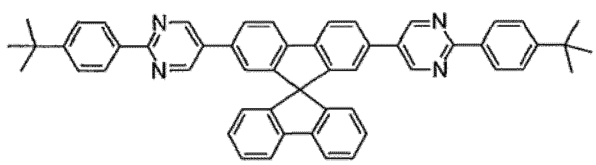

[Chem. 155]

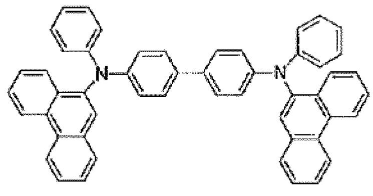

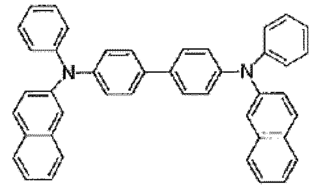

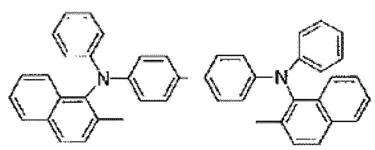

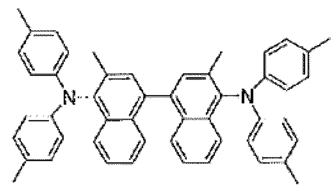

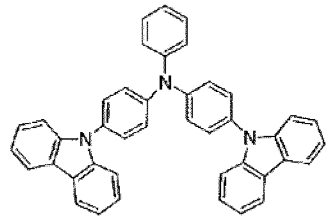

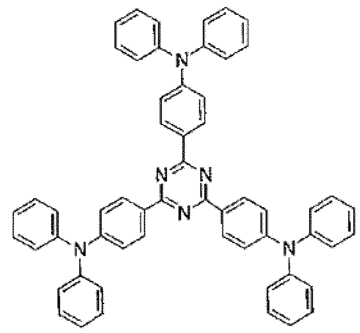

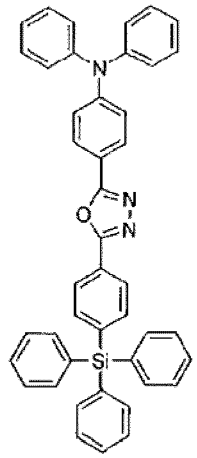

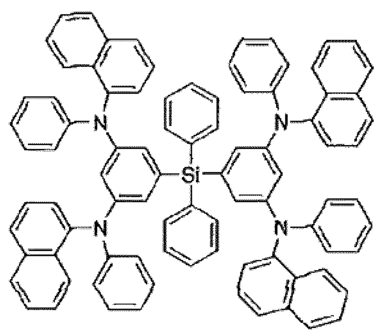

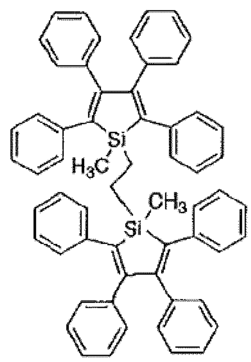

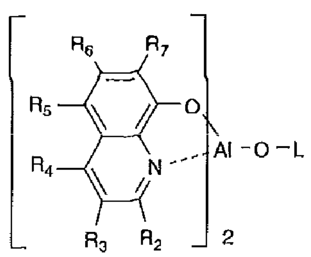

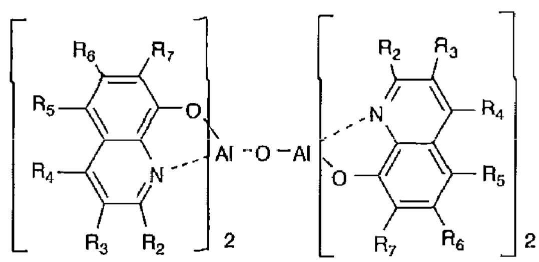

[Chem. 156]

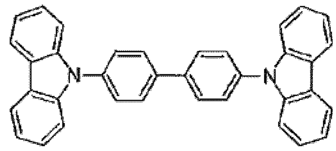

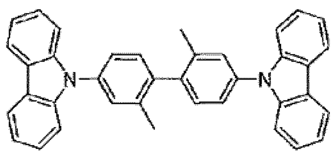

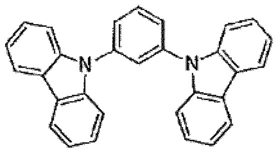

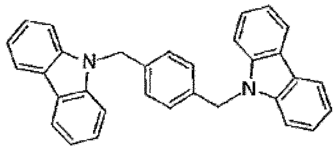

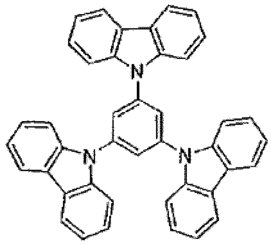

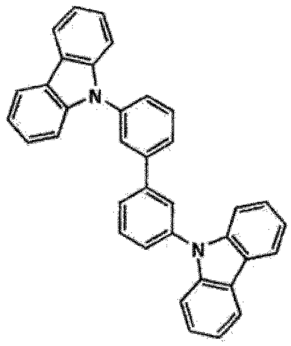

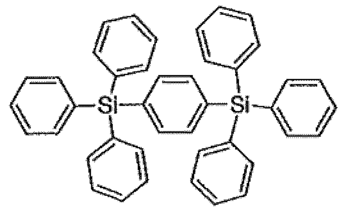

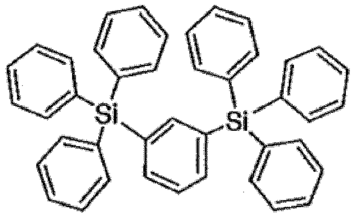

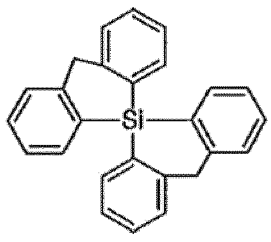

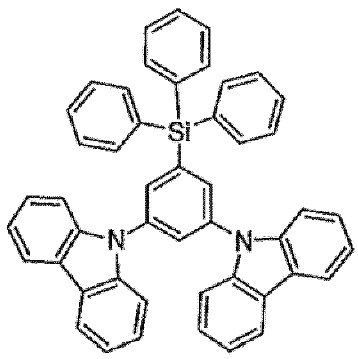

[Chem. 157]

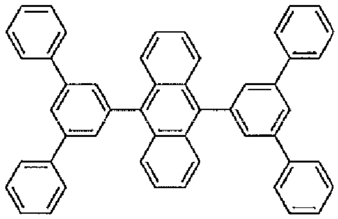
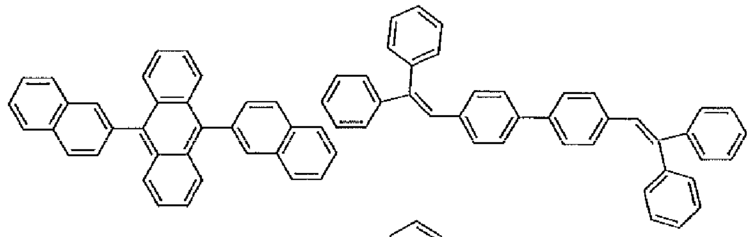

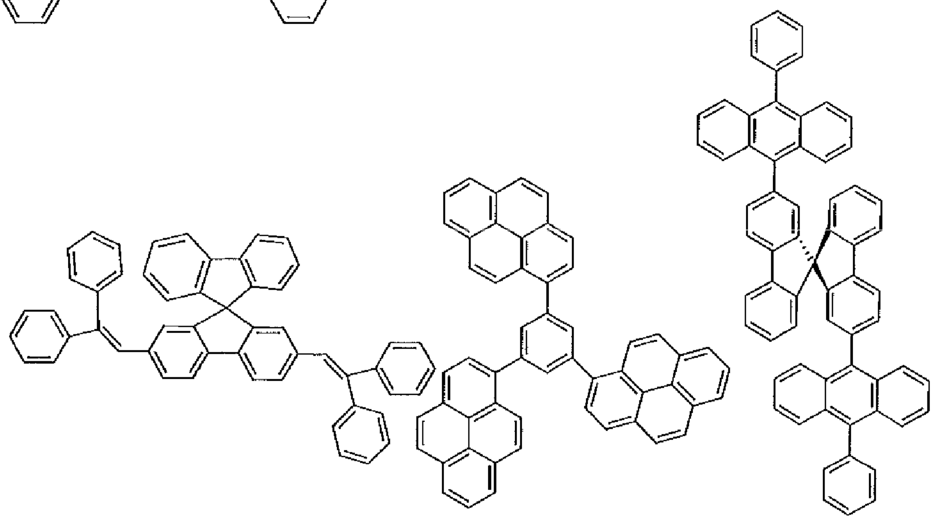

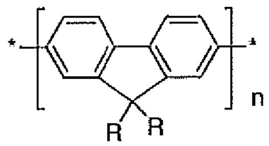
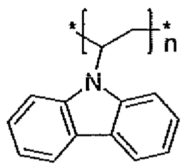
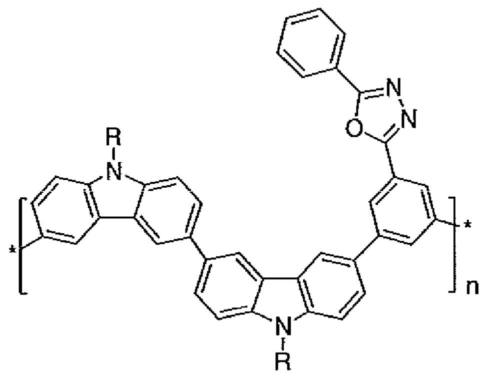

[Chem. 158]

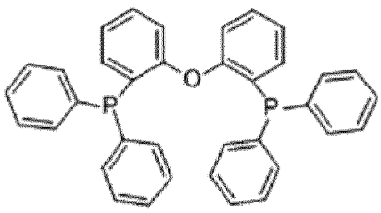

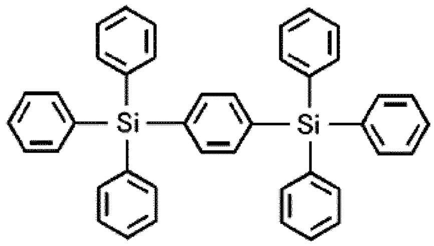

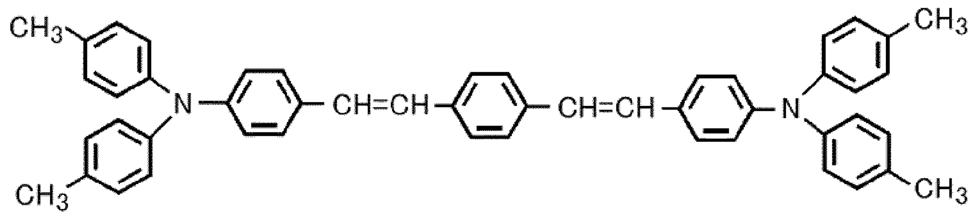

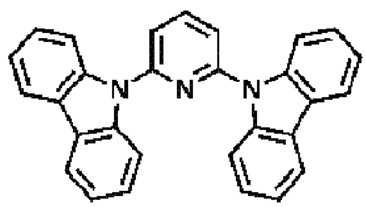

(Light-Emitting Material)

The light-emitting material is a luminescent material having a lowest excited singlet energy that is smaller than that of host material and the delayed fluorescent material. The light-emitting material receives energy from the host material and the delayed fluorescent material that are in an excited singlet state, and from the delayed fluorescent material that has become an excited singlet state from an excited triplet state through reverse intersystem crossing, thereby to transfer to an excited singlet state, and thereafter returns to the ground state thereof to emit light. The light-emitting material is not specifically limited so far as it emits light after having received energy from the host material and the delayed fluorescent material, but is preferably one capable of emitting fluorescence when returning back to the ground energy level from the lowest excited singlet energy level thereof. The light to be emitted may include fluorescence and, in addition thereto, delayed fluorescence and phosphorescence. In addition, the light-emitting material is a laser dye that gives amplified spontaneous emission (ASE). When a laser dye is used as the light-emitting material, the organic light-emitting device functions as an organic semiconductor laser. The organic semiconductor laser to which the present invention is applied has a low threshold energy and a low threshold current density necessary for ASE, and therefore can have excellent ASE characteristics.

Two or more kinds of light-emitting materials can be used so far as they satisfy the relationship of the expression (1). For example, combined use of two or more kinds of light-emitting materials each having a different emission color enables emission of a light of a desired color.

Preferred compounds usable as the light-emitting material are shown below.

[Chem. 159]

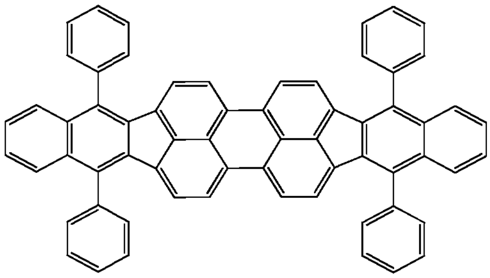

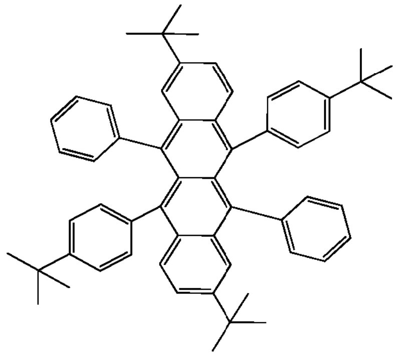

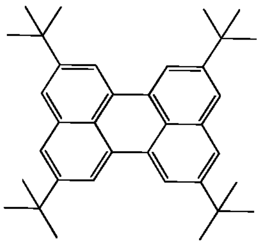

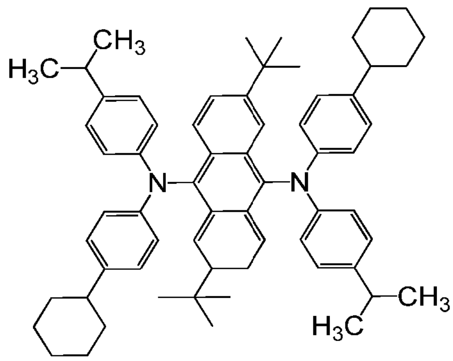

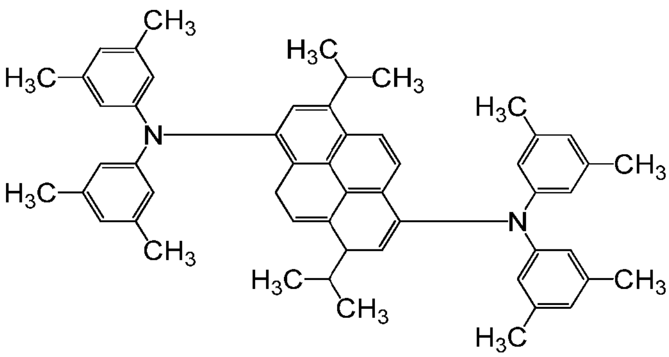

As the light-emitting material providing ASE, the following compound (C545T) is favorably used.

[Chem. 160]

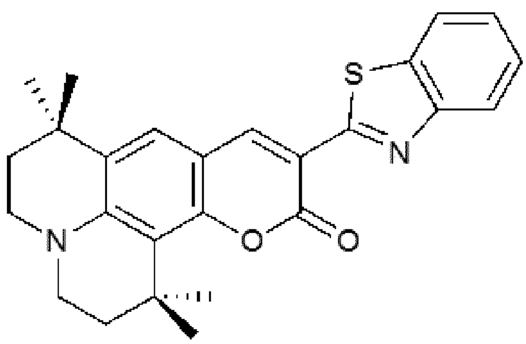

(Contents of Host Material, Delayed Fluorescent Material, Light-Emitting Material)

The content of each material contained in the light-emitting layer is not specifically limited, but the content of

the delayed fluorescent material is preferably smaller than that of the host material. With that, a higher light emission efficiency can be realized. Specifically, assuming that the total weight of the content W1 of the host material, the content W2 of the delayed fluorescent material, and the content W3 of the light-emitting material is 100% by weight, the content W1 of the host material is preferably 15% by weight or more and 99.9% by weight or less, the content W2 of the delayed fluorescent material is preferably 5.0% by weight or more and 50% by weight or less, and the content W3 of the light-emitting material is preferably 0.5% by weight or more and 5.0% by weight or less.

(Light-Emitting Layer)

The host material, the delayed fluorescent material and the light-emitting material constitute a light-emitting layer, for example, in a state where they are mixed in one and the same layer. The light-emitting layer may have a single-layer configuration or may have a multilayer configuration formed of plural layers that differ in point of the compositional ratio of the constituent materials and of the thickness. Having a multilayer configuration, the light-emitting layer may have diversified characteristics of driving voltage, external quantum efficiency, etc., and the characteristics of the organic light-emitting device can be therefore optimized in accordance with the use thereof. In one example of the multilayer light-emitting layer, the content of the delayed fluorescent material may be varied in each layer. Specifically, one preferred example of the light-emitting layer is a three-layered light-emitting layer having an interlayer and an upper layer and a lower layer arranged up and down the interlayer, in which the concentration of the delayed fluorescent material in the interlayer is lower than the concentration of the delayed fluorescent material in the upper layer and the lower layer.

The light-emitting layer may be formed of the host material, the delayed fluorescent material and the light-emitting material, or may contain any other organic material. Examples of the other organic material include a hole-transporting material, an electron-transporting material, etc. For the hole-transporting material and the electron-transporting material, those for use in the hole transport layer and the electron transport layer to be mentioned hereinunder may be referred to.

[Layer Configuration of Organic Light-Emitting Device]

The organic light-emitting device of the present invention is a photoexcitation-type organic semiconductor laser or a carrier injection-type organic semiconductor laser that uses a laser dye such as that mentioned above as the light-emitting material therein. When used in any system, the organic light-emitting device of the present invention can realize a high light emission efficiency. In particular, in the organic semiconductor laser of the present invention, the threshold energy and the threshold current density necessary for ASE can be reduced and excellent ASE characteristics can be realized.

The photoexcitation-type organic light-emitting device has a structure having at least a light-emitting layer formed on a substrate. The carrier injection-type organic light-emitting device has a structure having at least an anode, a cathode, and an organic layer between the anode and the cathode. The organic layer has at least the light-emitting layer containing the host material, the delayed fluorescent material and the light-emitting material satisfying the above-mentioned expression (1), and may be formed of the light-emitting layer alone, or may have one or more other organic layers in addition to the light-emitting layer. The other organic layers include a hole transport layer, a hole injection layer, an electron barrier layer, a hole barrier layer, an electron injection layer, an electron transport layer, an exciton barrier layer, etc. The hole transport layer may also be a hole injection transport layer having a hole injection function, and the electron transport layer may also be an electron injection transport layer having an electron injection function. A specific configuration example of the carrier injection-type organic light-emitting device is shown in Fig. 2. In Fig. 2, 1 is a substrate, 2 is an anode, 3 is a hole injection layer, 4 is a hole transport layer, 5 is a light-emitting layer, 6 is an electron transport layer, and 7 is a cathode.

In the following, members and layers of the carrier injection-type organic light-emitting device are described. For functions and specific examples of the host material constituting light-emitting layer, the delayed fluorescent material and the light-emitting material, the description given hereinabove may be referred to. In the following, the other members and layers are described. The description of the substrate and the light-emitting layer also applies to the substrate and the light-emitting layer of an organic photoluminescence device not falling under the present invention.

[Substrate]

The organic light-emitting device of the invention being an organic semiconductor laser is preferably supported by a substrate. The substrate is not particularly limited and may be those that have been commonly used in an organic light-emitting device, and examples thereof used include those formed of glass, transparent plastics, quartz and silicon.

[Anode]

The anode in the organic light-emitting device is preferably formed of, as an electrode material, a metal, an alloy or an electroconductive compound each having a large work function (4 eV or more), or a mixture thereof. Specific examples of the electrode material include a metal, such as Au, and an electroconductive transparent material, such as CuI, indium tin oxide (ITO), $SnO_2$ and ZnO. A material that is amorphous and is capable of forming a transparent electroconductive film, such as IDIXO ($In_2O_3$-ZnO), may also be used. The anode may be formed in such a manner that the electrode material is formed into a thin film by such a method as vapor deposition or sputtering, and the film is patterned into a desired pattern by a photolithography method, or in the case where the pattern may not require high accuracy (for example, approximately 100 μm or more), the pattern may be formed with a mask having a desired shape on vapor deposition or sputtering of the electrode material. In alternative, in the case where a material capable of being applied as a coating, such as an organic electroconductive compound, is used, a wet film forming method, such as a printing method and a coating method, may be used. In the case where emitted light is to be taken out through the anode, the anode preferably has a transmittance of more than 10%, and the anode preferably has a sheet resistance of several hundred Ohm per square or less. The thickness thereof may be generally selected from a range of from 10 to 1,000 nm, and preferably from 10 to 200 nm, while depending on the material used.

[Cathode]

The cathode is preferably formed of, as an electrode material, a metal (referred to as an electron injection metal), an alloy or an electroconductive compound each having a small work function (4 eV or less), or a mixture thereof. Specific examples of the electrode material include sodium, a sodium-potassium alloy, magnesium, lithium, a magnesium-copper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth metal. Among these, a mixture of an electron injection metal and a second metal that is a stable metal having a larger work function than the electron injection metal, for example, a magnesium-silver mixture, a magnesium-aluminum mixture, a magnesium-indium mixture, an aluminum-aluminum oxide ($Al_2O_3$) mixture, a lithium-aluminum mixture, and aluminum, are preferred from the standpoint of the electron injection property and the durability against oxidation and the like. The cathode may be produced by forming the electrode material into a thin film by such a method as vapor deposition or sputtering. The cathode preferably has a sheet resistance of several hundred Ohm per square or less, and the thickness thereof may be generally selected from a range of from 10 nm to 5 μm, and preferably from 50 to 200 nm. For transmitting the emitted light, any one of the anode and the cathode of the organic light-emitting device is preferably transparent or translucent, thereby enhancing the light emission luminance.

The cathode may be formed with the electroconductive transparent materials described for the anode, thereby forming a transparent or translucent cathode, and by applying the cathode, a device having an anode and a cathode, both of which have transmittance, may be produced.

[Injection Layer]

The injection layer is a layer that is provided between the electrode and the organic layer, for decreasing the driving voltage and enhancing the light emission luminance, and includes a hole injection layer and an electron injection layer, which may be provided between the anode and the light emitting layer or the hole transporting layer and between the cathode and the light emitting layer or the electron transport layer. The injection layer may be provided depending on necessity.

[Barrier Layer]

The barrier layer is a layer that is capable of inhibiting charges (electrons or holes) and/or excitons present in the light emitting layer from being diffused outside the light emitting layer. The electron barrier layer may be disposed between the light emitting layer and the hole transport layer, and inhibits electrons from passing through the light emitting layer toward the hole transport layer. Similarly, the hole barrier layer may be disposed between the light emitting layer and the electron transport layer, and inhibits holes from passing through the light emitting layer toward the electron transport layer. The barrier layer may also be used for inhibiting excitons from being diffused outside the light emitting layer. Thus, the electron barrier layer and the hole barrier layer each may also have a function as an exciton barrier layer. The term the electron barrier layer or the exciton barrier layer referred to herein is intended to include a layer that has both the functions of an electron barrier layer and an exciton barrier layer by one layer.

[Hole Barrier Layer]

The hole barrier layer has the function of an electron transporting layer in a broad sense. The hole barrier layer has a function of inhibiting holes from reaching the electron transport layer while transporting electrons, and thereby enhances the recombination probability of electrons and holes in the light emitting layer. As the material for the hole barrier layer, the materials for the electron transport layer described later may be used depending on necessity.

[Electron Barrier Layer]

The electron barrier layer has the function of transporting holes in a broad sense. The electron barrier layer has a function of inhibiting electrons from reaching the hole transport layer while transporting holes, and thereby enhances the recombination probability of electrons and holes in the light emitting layer.

[Exciton Barrier Layer]

The exciton barrier layer is a layer for inhibiting excitons generated through recombination of holes and electrons in the light emitting layer from being diffused to the charge transport layer, and the use of the layer inserted enables effective confinement of excitons in the light emitting layer, and thereby enhances the light emission efficiency of the device. The exciton barrier layer may be inserted adjacent to the light emitting layer on any of the side of the anode and the side of the cathode, and on both the sides. Specifically, in the case where the exciton barrier layer is present on the side of the anode, the layer may be inserted between the hole transport layer and the light emitting layer and adjacent to the light emitting layer, and in the case where the layer is inserted on the side of the cathode, the layer may be inserted between the light emitting layer and the cathode and adjacent to the light emitting layer. Between the anode and the exciton barrier layer that is adjacent to the light emitting layer on the side of the anode, a hole injection layer, an electron barrier layer and the like may be provided, and between the cathode and the exciton barrier layer that is adjacent to the light emitting layer on the side of the cathode, an electron injection layer, an electron transport layer, a hole barrier layer and the like may be provided. In the case where the barrier layer is provided, the material used for the barrier layer preferably has excited singlet energy and excited triplet energy, at least one of which is higher than the excited singlet energy and the excited triplet energy of the light emitting material, respectively.

[Hole Transport Layer]

The hole transport layer is formed of a hole transporting material having a function of transporting holes, and the hole transport layer may be provided as a single layer or plural layers.

The hole transporting material has one of injection or transporting property of holes and barrier property of electrons, and may be any of an organic material and an inorganic material. Examples of known hole transporting materials that may be used herein include a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer oligomer, particularly a thiophene oligomer. Among these, a porphyrin compound, an aromatic tertiary amine compound and a styrylamine compound are preferably used, and an aromatic tertiary amine compound is more preferably used.

Electron Transport Layer

The electron transporting layer is formed of a material having a function of transporting electrons, and the electron transporting layer may be provided as a single layer or plural layers.

The electron transporting material (which may also function as a hole barrier material in some cases) needs only to have a function of transporting electrons, which are injected from the cathode, to the light emitting layer. Examples of the electron transport layer that may be used herein include a nitro-substituted fluorene derivative, a diphenylquinone derivative, a thiopyran dioxide derivative, carbodiimide, a fluorenylidene methane derivative, anthraquinodimethane and anthrone derivatives, and an oxadiazole derivative. The electron transporting material used may be a thiadiazole derivative obtained by replacing the oxygen atom of the oxadiazole ring of the oxadiazole derivative by a sulfur atom, or a quinoxaline derivative having a quinoxaline ring, which is known as an electron attractive group. Furthermore, polymer materials having these materials introduced to the polymer chain or having these materials used as the main chain of the polymer may also be used.

In producing the organic light-emitting device, the method for forming the organic layers is not specifically

limited, and the layers may be formed by any of a dry process and a wet process.

Specific examples of preferred materials that may be used in the organic light-emitting device are shown below, but the materials that may be used in the invention are not construed as being limited to the example compounds. The compound that is shown as a material having a particular function may also be used as a material having another function. In the structural formulae of the example compounds, R, R' and $R_1$ to $R_{10}$ each independently represent a hydrogen atom or a substituent. X represents a carbon atom or a hetero atom to form the ring skeleton, n represents an integer of from 3 to 5, Y represents a substituent, and m represents an integer of 0 or more.

First, preferred examples of a compound that may be used as the hole injection material are shown below.

[Chem. 161]

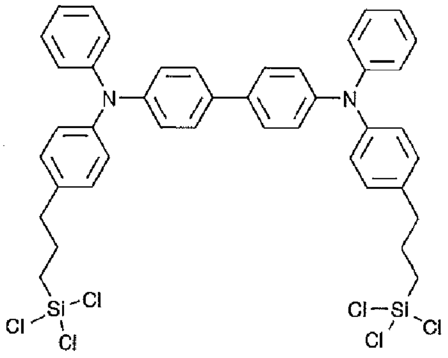

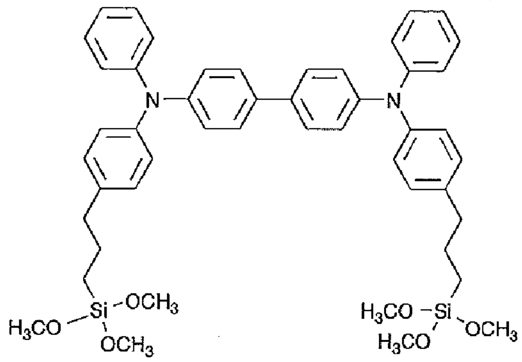

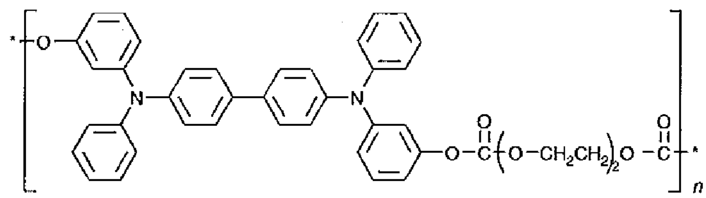

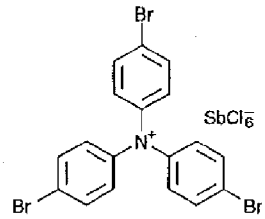

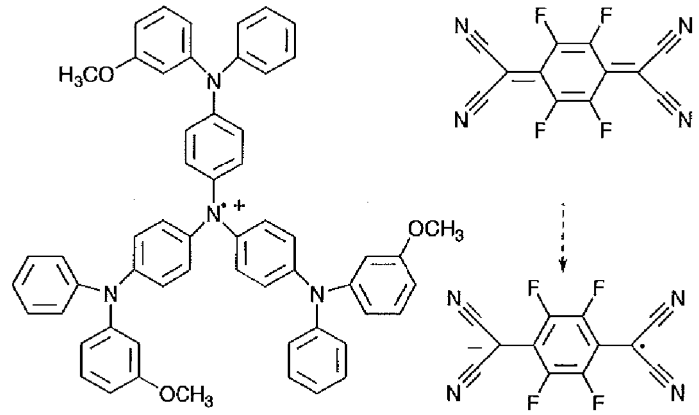

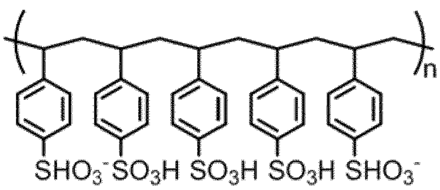

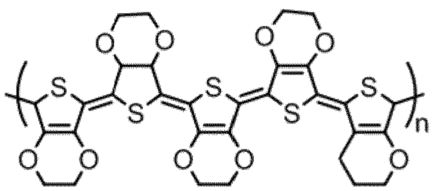

Next, preferred examples of a compound that may be used as the hole transporting material are shown below.

[Chem. 162]

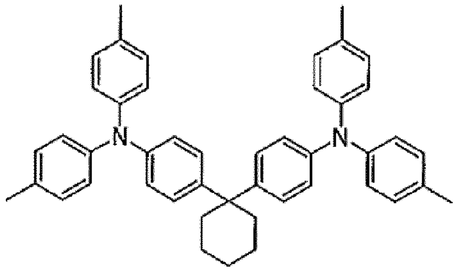

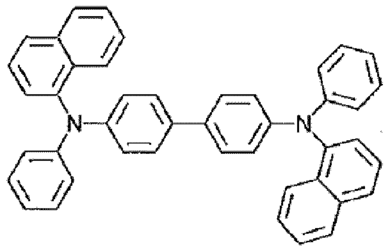

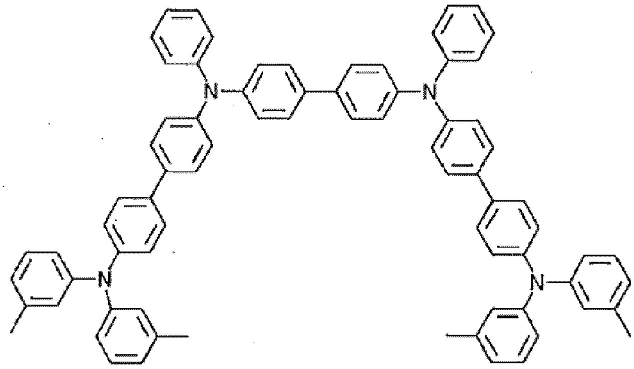

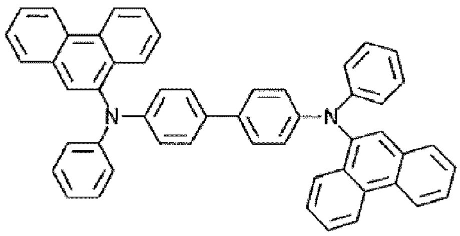

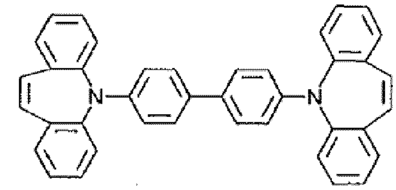

[Chem. 163]

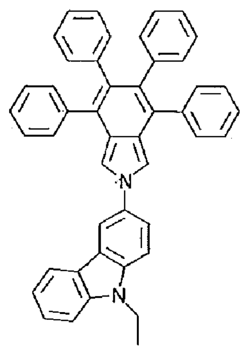

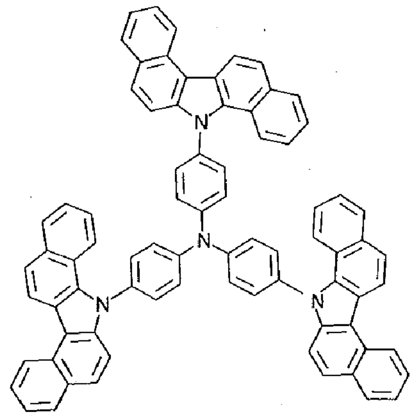

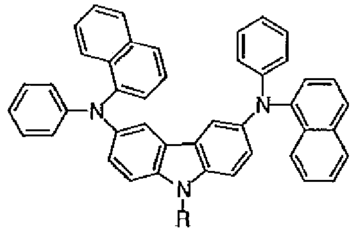

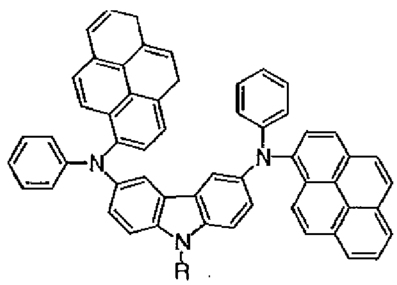

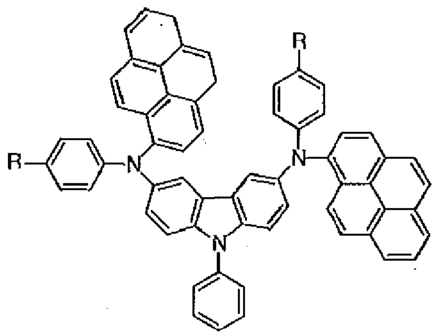

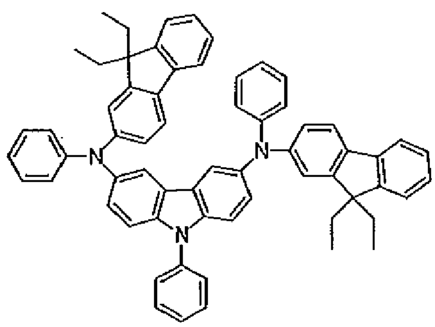

[Chem. 164]

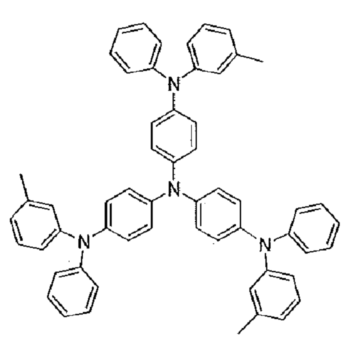

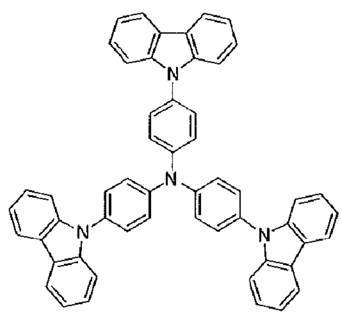

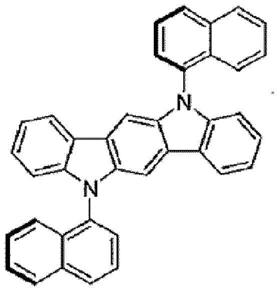

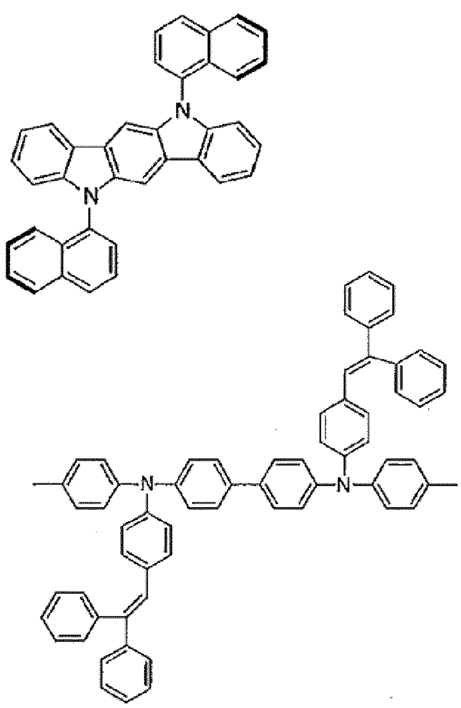

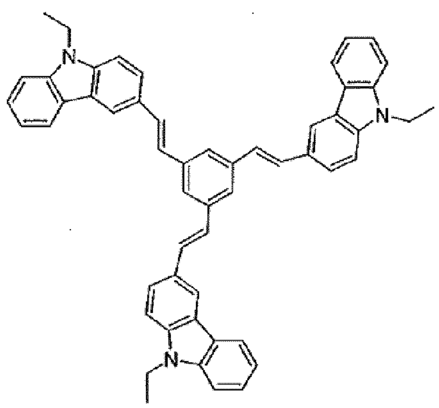

[Chem. 165]

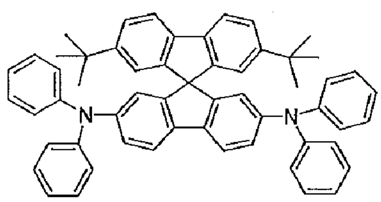

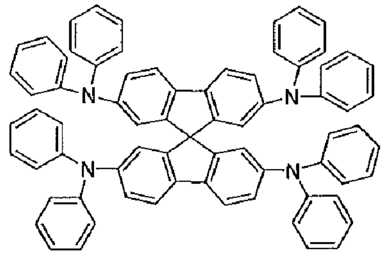

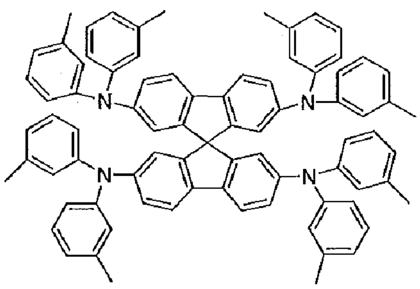

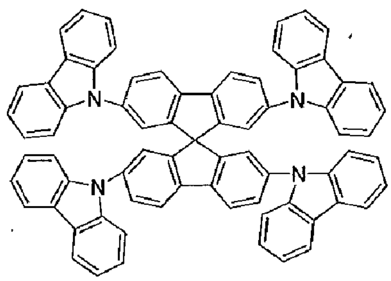

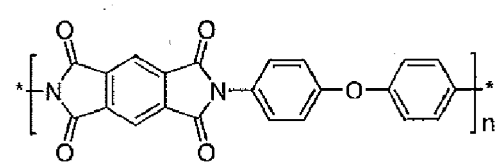

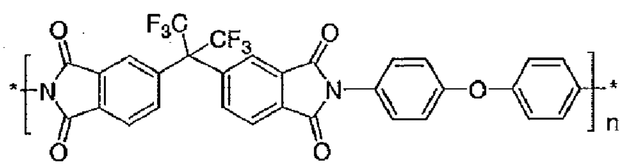

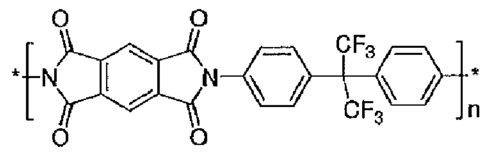

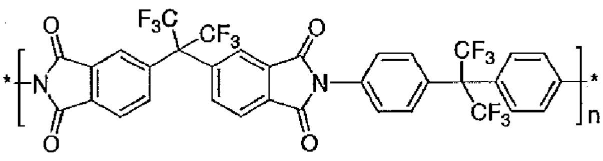

[Chem. 166]

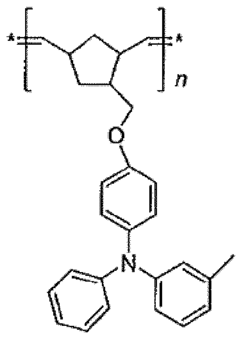

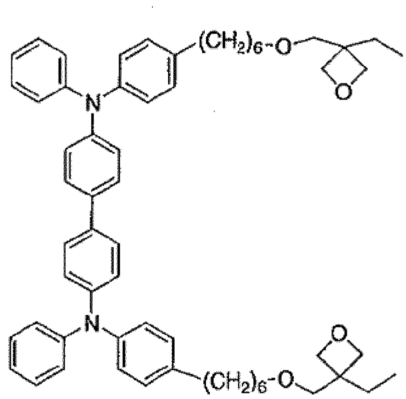

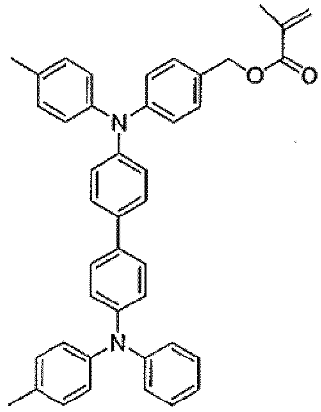

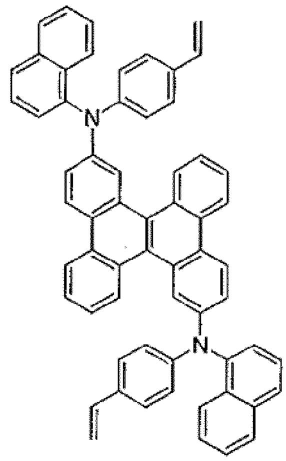

R=

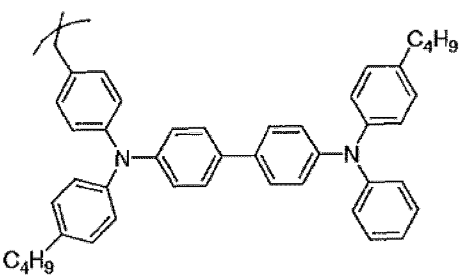

[Chem. 167]

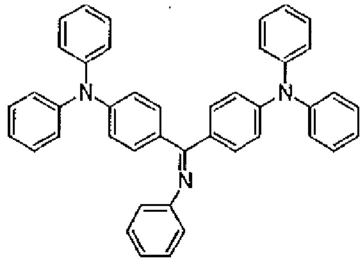

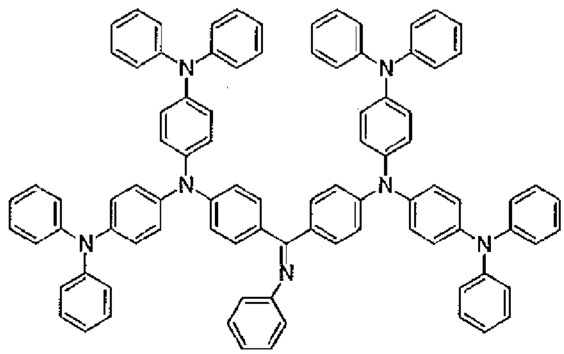

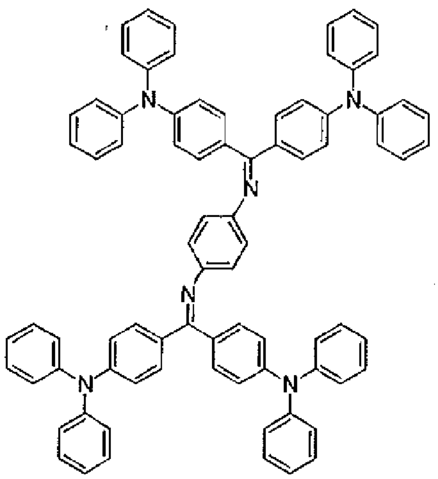

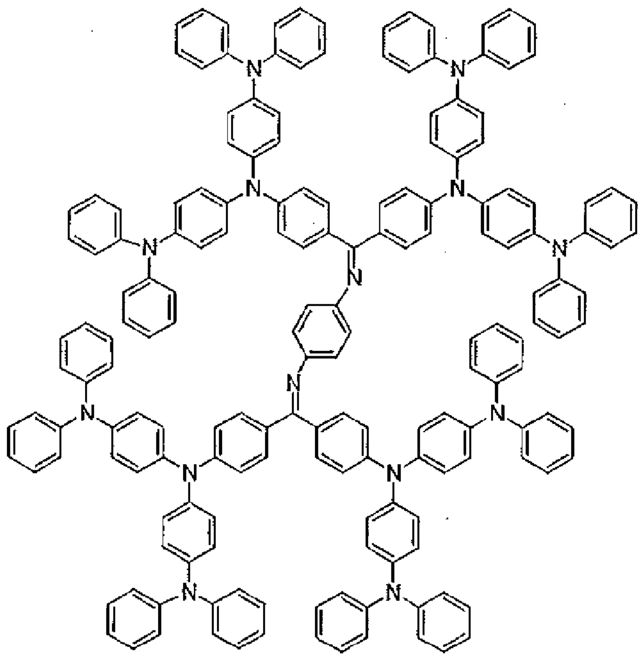

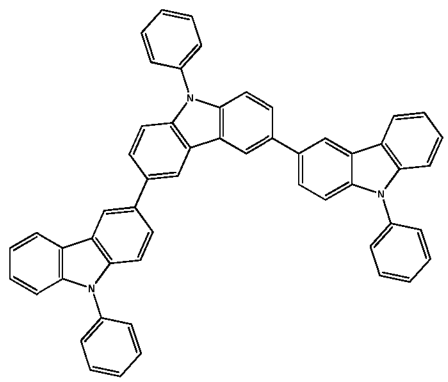

Next, preferred examples of a compound that may be used as the electron barrier material are shown below.

[Chem. 168]

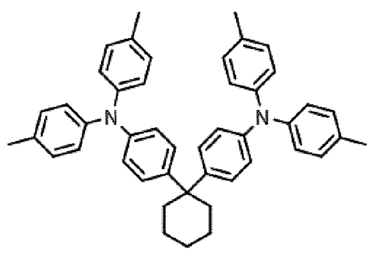

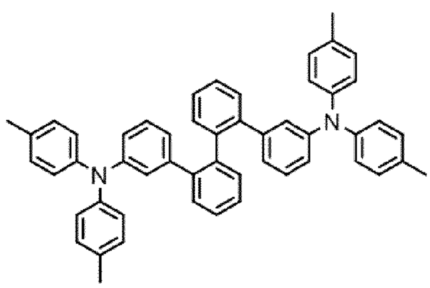

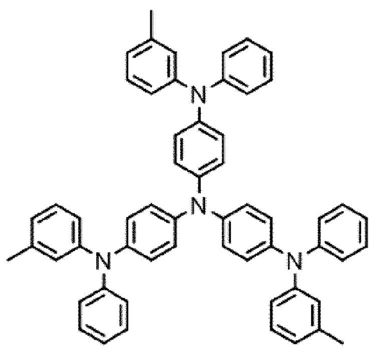 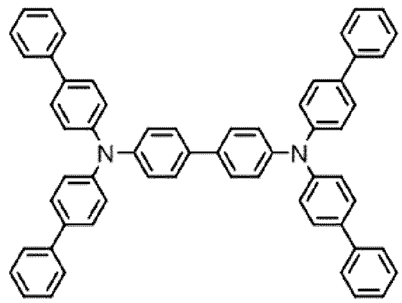

Next, preferred examples of a compound that may be used as the hole barrier material are shown below.

[Chem. 169]

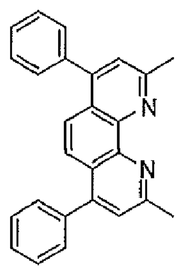 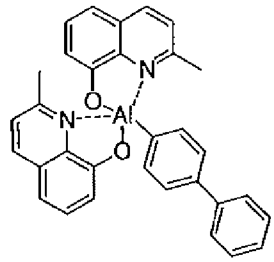 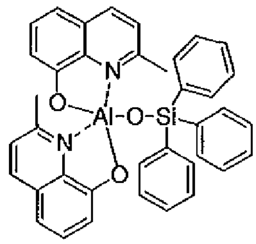 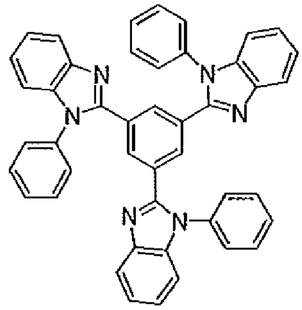

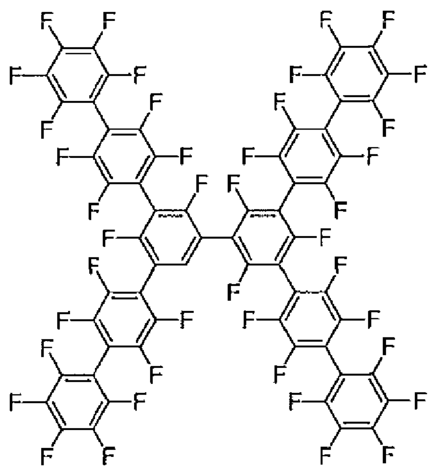 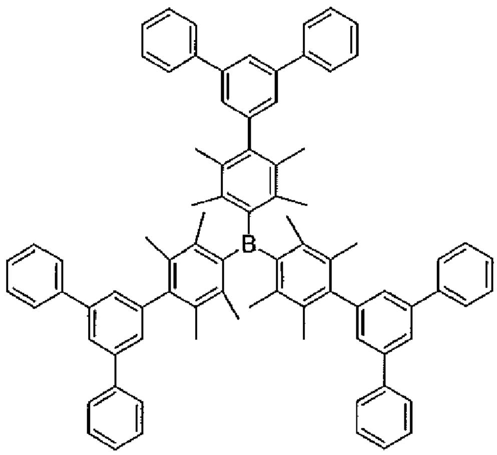

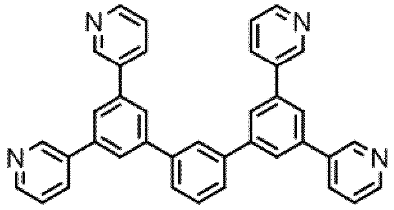
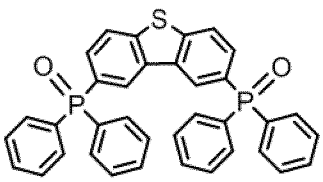
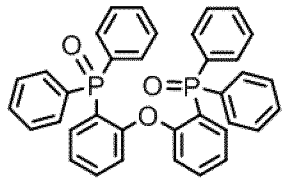

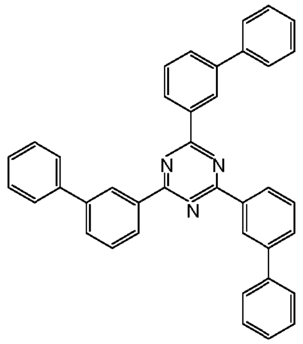

Next, preferred examples of a compound that may be used as the electron transporting material are shown below.

[Chem. 170]

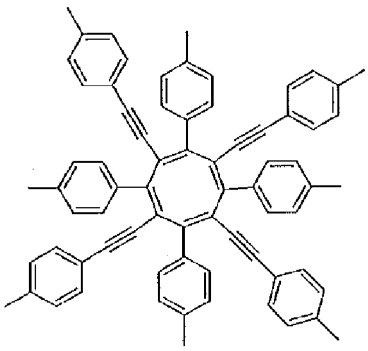
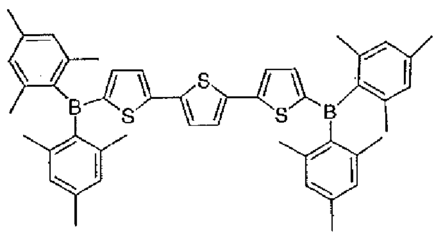

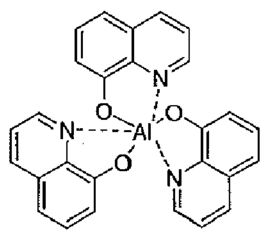
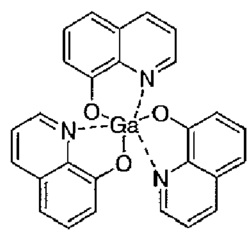
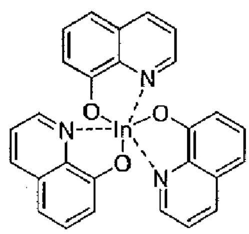

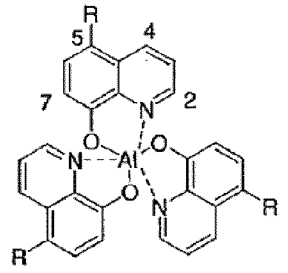

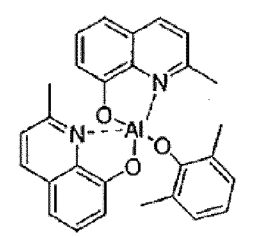

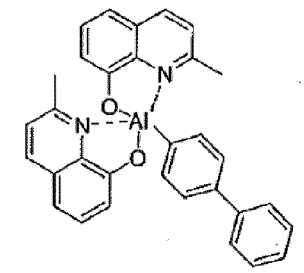

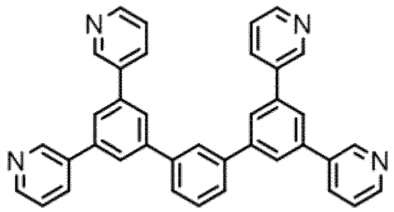

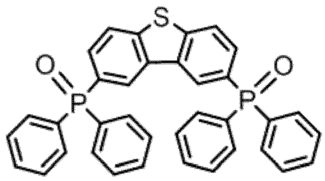

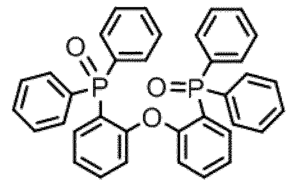

[Chem. 171]

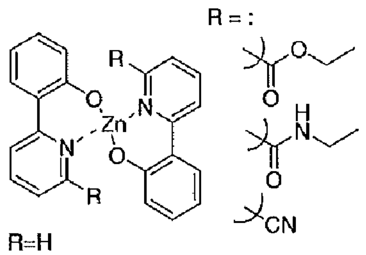

R = :

R=H

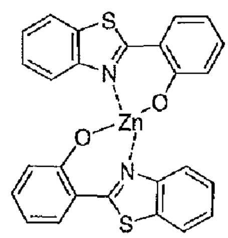

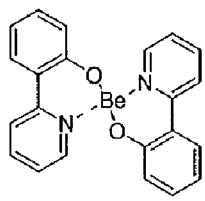

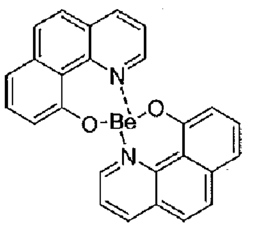

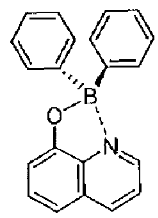

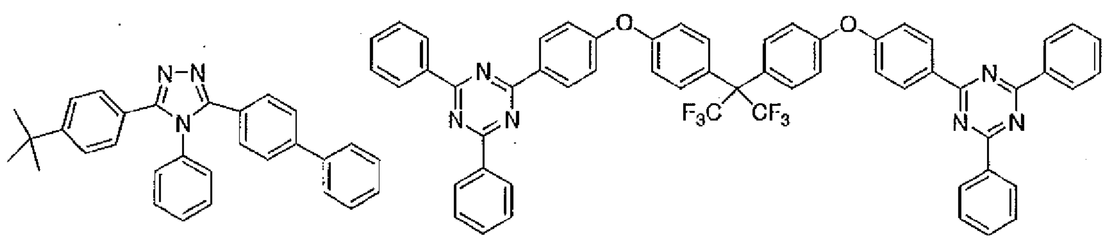

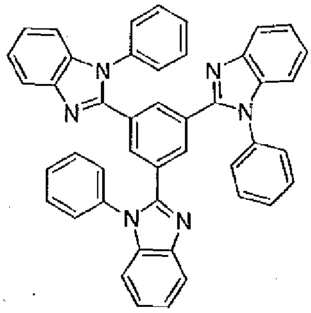

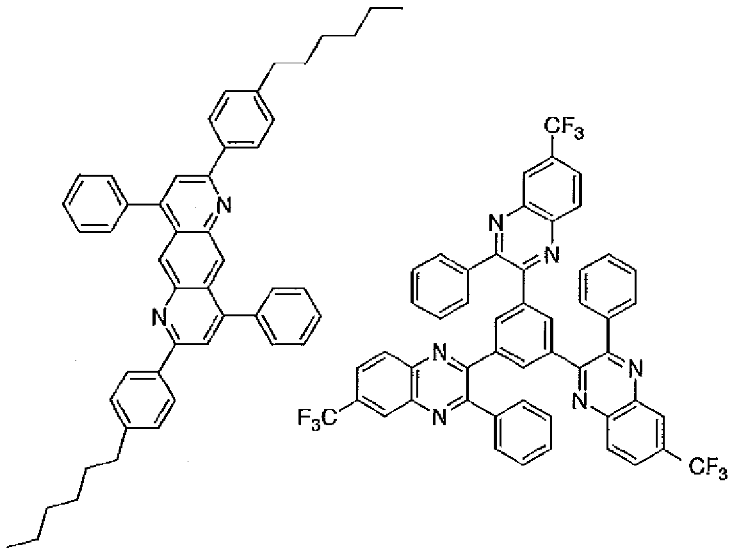

[Chem. 172]

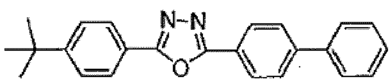

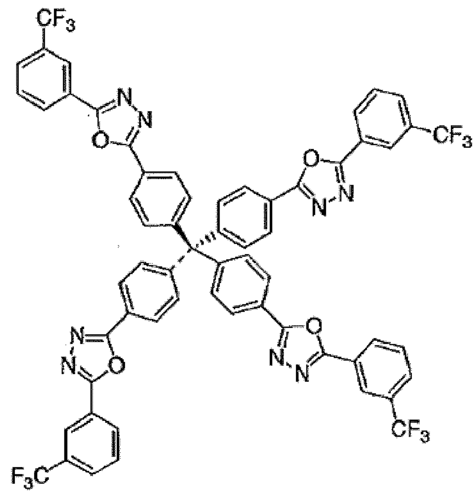

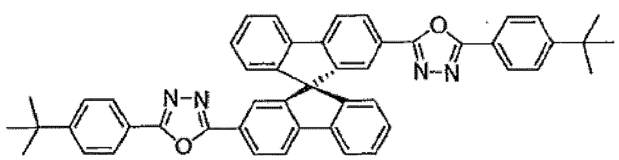

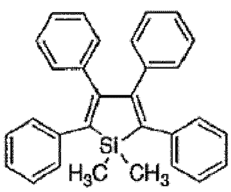

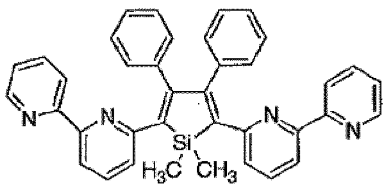

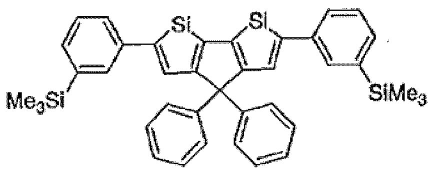

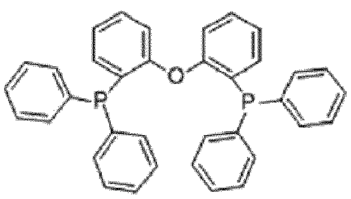

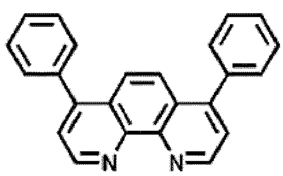

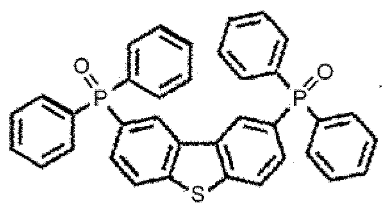

Next, preferred examples of a compound that may be used as the electron injection material are shown below.

[Chem. 173]

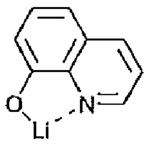
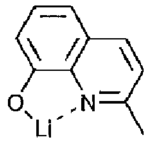
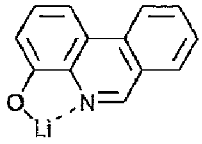
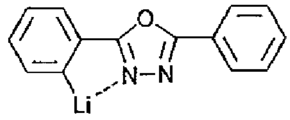

Preferred examples of a compound as a material that may be added are shown below. For example, the compound may be added as a stabilizing material.

[Chem. 174]

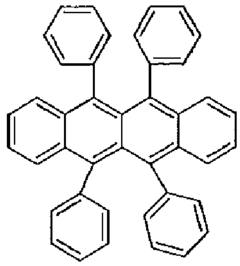
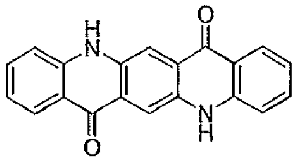
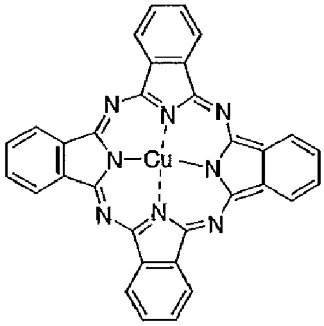
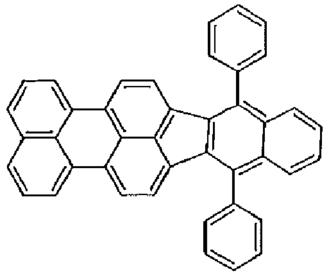
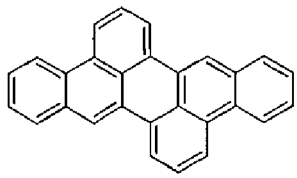

The organic light-emitting device thus produced by the aforementioned method emits light on application of an electric field between the anode and the cathode of the device. In this case, when the light emission is caused by excited singlet energy, light having a wavelength that corresponds to the energy level thereof may be confirmed as fluorescent light and delayed fluorescent light. When the light emission is caused by excited triplet energy, light having a wavelength that corresponds to the energy level thereof may be confirmed as phosphorescent light. The normal fluorescent light has a shorter light emission lifetime than the delayed fluorescent light, and thus the light emission lifetime may be distinguished between the fluorescent light and the delayed fluorescent light.

On the other hand, phosphorescent light is substantially not observed at room temperature since in an ordinary organic compound, such as the compound of the invention, the excited triplet energy is converted to heat or the like due to the instability thereof, and thus is immediately deactivated with a short lifetime. The excited triplet energy of the ordinary organic compound may be measured only by observing light emission under an extremely low temperature condition.

The organic light-emitting device of the invention may be applied to any of a single device, a structure with plural devices disposed in an array, and a structure having anodes and cathodes disposed in an X-Y matrix. According to the invention, an organic light-emitting device that is largely improved in light emission efficiency may be obtained, which contains a host material, a delayed fluorescent material and a light-emitting material and in which the relationship of the lowest excited singlet energy level $E_{S1}$ between the materials is specifically defined. The organic light-emitting device of the invention may be applied to a further wide range of purposes. In particular, the organic electroluminescent device of the invention may be applied to organic electroluminescent illumination and backlight which are highly demanded.

Examples

The features of the invention will be described more specifically with reference to examples below. The materials, processes, procedures and the like shown below may be appropriately modified. The light emission characteristics were evaluated by using High-performance UV/Vis/NIR Spectrophotometer (Lambda 950, produced by PerkinElmer, Co., Ltd.), Fluorescence Spectrophotometer (FluoroMax-4, produced by Horiba, Ltd.), Absolute PL Quantum Yield Measurement System (C11347, produced by Hamamatsu Photonics K.K.), Source Meter (2400 Series, produced by Keithley Instruments Inc.), Semiconductor Parameter Analyzer (E5273A, produced by Agilent Technologies, Inc.), Optical Power Meter (1930C, produced by Newport Corporation), Optic Spectrometer (USB2000, produced by Ocean Optics, Inc.), Spectroradiometer (SR-3, produced by Topcon Corporation), Streak Camera (Model C4334, produced by Hamamatsu Photonics K.K.) and Multichannel Detector (PMA-11, produced by Hamamatsu Photonics K.K.).

The lowest excited singlet energy level $E_{S1}$, the lowest excited triplet energy level $E_{T1}$, the rate constant kisc from a lowest excited singlet state to a lowest excited triplet state through intersystem crossing, and the rate constant $k_{RISC}$ from a lowest excited triplet state to a lowest excited singlet state through reverse intersystem crossing of the compounds used in Examples and Comparative Examples were measured in the following procedures. The energy difference $\Delta E_{st}$ between a lowest excited singlet state and a lowest excited triplet state at 77 K was obtained by measuring the difference between $E_{S1}$ and $E_{T1}$.

(1) Lowest Excited Singlet Energy Level $E_{S1}$

The compound to be measured was vapor-deposited on a Si substrate to produce a specimen, and the specimen was measured for a fluorescent spectrum at ordinary temperature (300 K). In the fluorescent spectrum, the ordinate is the light emission, and the abscissa is the wavelength. A tangent line was drawn for the downfalling part of the light emission spectrum on the short wavelength side, and the wavelength λedge (nm) of the intersection point of the tangent line and the abscissa was obtained. The wavelength value was converted to an energy value according to the following conversion expression to provide the singlet energy $E_{S1}$.

Conversion Expression

$$E_{S1}\ (\mathrm{eV}) = 1239.85/\lambda\mathrm{edge}$$

The light emission spectrum was measured with a nitrogen laser (MNL200, produced by Lasertechnik Berlin GmbH) as an excitation light source and Streak Camera (C4334, produced by Hamamatsu Photonics K.K.) as a detector.

(2) Lowest Excited Triplet Energy Level $E_{T1}$

The same specimen as used for the singlet energy $E_{S1}$ was cooled to 77 [K], the specimen for measuring phosphorescent light was irradiated with excitation light (337 nm), and the phosphorescence intensity was measured with a streak camera. A tangent line was drawn for the upstanding part of the phosphorescent spectrum on the short wavelength side, and the wavelength λedge (nm) of the intersection point of the tangent line and the abscissa was obtained. The wavelength value was converted to an energy value according to the following conversion expression to provide the triplet energy $E_{T1}$.

Conversion Expression

$$E_{T1}\ (\mathrm{eV}) = 1239.85/\lambda\mathrm{edge}$$

The tangent line for the upstanding part of the phosphorescent spectrum on the short wavelength side was drawn in the following manner. Over the range in the phosphorescent spectrum curve of from the short wavelength end to the maximum peak value closest to the short wavelength end among the maximum peak values of the spectrum, a tangent line was assumed while moving within the range toward the long wavelength side. The gradient of the tangent line was increased while the curve was standing up (i.e., the value of the ordinate was increased). The tangent line that was drawn at the point where the gradient thereof became maximum was designated as the tangent line for the upstanding part of the phosphorescent spectrum on the short wavelength side.

A maximum peak having a peak intensity that was 10% or less of the maximum peak point intensity of the spectrum was not included in the maximum peak values and thus was not designated as the maximum peak value

closest to the short wavelength end, and the tangent line that was drawn at the point where the gradient became maximum that was closest to the maximum peak value closest to the short wavelength end was designated as the tangent line for the upstanding part of the phosphorescent spectrum on the short wavelength side.

(3) Rate Constant of Intersystem Crossing kisc and Rate Constant of Reverse Intersystem Crossing $k_{RISC}$

The rate constant in intersystem crossing from a lowest excited singlet state to a lowest excited triplet state kisc and the rate constant in reverse intersystem crossing from a lowest excited triplet state to a lowest excited singlet state $k_{RISC}$ were obtained according to the following expressions (1) to (5).
[Math.]

$$\tau_{\mathrm{p}} = {}^{1}\!/_{k_{\mathrm{p}}} \;, \qquad (1)$$

$$\tau_{\mathrm{d}} = {}^{1}\!/_{k_{\mathrm{d}}} \,, \qquad (2)$$

$$k_{\mathrm{ISC}} = (1 - \phi_{\mathrm{prompt}})k_{\mathrm{p}}, \qquad (3)$$

$$k_{\mathrm{RISC}} = \frac{k_{\mathrm{p}}k_{\mathrm{d}}}{k_{\mathrm{ISC}}}\frac{\phi_{\mathrm{delayed}}}{\phi_{\mathrm{prompt}}}, \text{ and} \qquad (4)$$

$$(1 - \phi_{\mathrm{prompt}})\phi_{\mathrm{RISC}} = \phi_{\mathrm{delayed}}, \qquad (5)$$

In the above expressions, $\tau_p$ represents the transient decay time of a prompt fluorescent component, $\tau_d$ represents the transient decay time of a delayed fluorescent component, $\phi_{prompt}$ represents the quantum efficiency of a prompt fluorescent component, and $\phi_{delayed}$ represents the quantum efficiency of a delayed fluorescent component.

The transient decay time $\tau_p$ of a prompt fluorescent component and the transient decay time $\tau_d$ of a delayed fluorescent component can be measured with a streak camera.

The quantum efficiency $\phi_{prompt}$ of a prompt fluorescent component and the quantum efficiency $\phi_{delayed}$ of a delayed fluorescent component can be determined by measuring a total $\phi$PL using an absolute quantum yield measuring apparatus and then measuring the integral value of the time-resolved emission spectrum with a streak camera.

[Production and Evaluation of Photoexcitation-Type Organic Semiconductor Laser] (Example 1) Production of photoexcitation-type organic semiconductor laser using mCBP (host material), ACRXTN (delated fluorescent material) and C545T (light-emitting material)

The following compounds were prepared as materials of a light-emitting layer.

[Chem. 175]

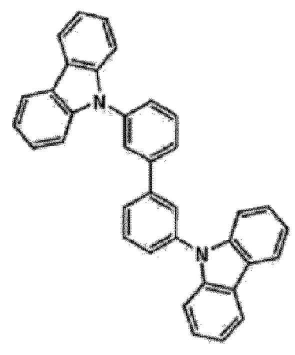

mCBP

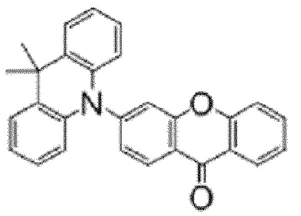

ACRXTN

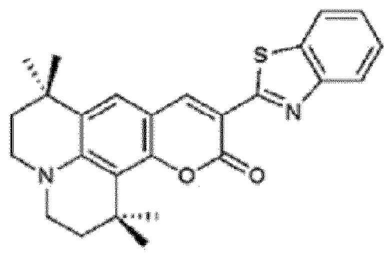

C545T

mCBP has a lowest excited singlet energy level $E_{S1}$(H) of 3.5 eV, ACRXTN has a lowest excited singlet energy level $E_{S1}$(F) of 2.76 eV, C545T has a lowest excited singlet energy level $E_{S1}$(D) of 2.7 eV. ACRXTN has an energy difference $\Delta E_{st}$ between a lowest excited singlet energy level $E_{S1}$ and a lowest excited triplet energy level $E_{T1}$ of 0.06 eV, a rate constant kisc from a lowest excited singlet state to a lowest excited triplet state through intersystem crossing of $2.0 \times 10^7$/s, and a rate constant $k_{RISC}$ from a lowest excited triplet state to a lowest excited singlet state through reverse intersystem crossing of $4.0 \times 10^5$/s.

mCBP, ACRXTN and C545T were co-deposited on a quartz substrate from different deposition sources under a condition of a vacuum degree of $5 \times 10^{-5}$ Pa or less according to a vapor deposition method to form thereon a thin film having a thickness of 100 nm in which the concentration of ACRXTN was 6.0% by weight and the concentration of C545T was 1% by weight, thereby producing a photoexcitation-type organic semiconductor laser.

(Comparative Example 1) Production of photoexcitation-type organic semiconductor laser using mCBP (host material) and C545T (light-emitting material)

A photoexcitation-type organic semiconductor laser having a thin film containing mCBP and C545T (1% by weight) was produced according to the same process as in Example 1 except that a vapor deposition source of ACRXTN was not used in forming the thin film.

The organic semiconductor lasers produced in Example 1 and Comparative Example 1 were evaluated for the characteristics thereof.

The organic semiconductor laser of Example 1 was irradiated with excitation light having a wavelength of 337 nm and a pulse width of 0.8 $\mu$s, and the time-dependent light intensity change thereof was measured with a streak camera. The result is shown in Fig. 3. The organic semiconductor laser of Comparative Example 1 was observed under the same condition for the time-dependent light intensity change thereof, and the result is shown in Fig. 4. As shown in Fig. 3, the organic semiconductor laser of Example 1 showed a prompt emission component and also showed a delayed emission component in a range of 2.0 to 10 (ts. These emission spectra were the same as the emission spectra of C545T. On the other hand, the organic semiconductor laser of Comparative Example 1 did not show a delayed emission component in the range of 2.0 to 10 $\mu$s. Only the organic semiconductor laser of Example 1 showed a delayed emission component, and this indicates that the excited triplet energy formed in ACRXTN transferred to C545T through reverse intersystem crossing. The photoluminescence quantum efficiency of the organic semiconductor laser of Example 1 was 86 $\pm$ 3%, and among this, the photoluminescence quantum yield of the prompt emission component was 74% and the photoluminescence quantum yield of the delayed emission component was 12%. From this, it is known that the excited singlet energy transferred from ACRXTN through reverse intersystem crossing contributed to 10% or more of the radiation-deactivated singlet excitons of C545T. The photoluminescence quantum efficiency of the organic semiconductor laser of Comparative Example 1 was 81 $\pm$ 3%.

Fig. 5 shows the emission spectrum of the organic semiconductor laser of Example 1 with 337-nm excitation light, and the emission peaks thereof at 535 nm measured with excitation energy of 0.5 $\mu J/cm^2$, 1.5 $\mu J/cm^2$, 2.9 $\mu J/cm^2$ and 5.8 $\mu J/cm^2$. Fig. 6 shows a relationship between the excitation energy and the half-value width FWHM of the emission peak of the organic semiconductor laser of Example 1; and Fig. 7 shows a relationship between the excitation energy and the emission peak intensity thereof. Fig. 6 also shows a relationship between the excitation energy and the half-value width FWHM of the emission peak of the organic semiconductor laser of Comparative Example 1, as measured under the same condition as in Example 1; and Fig. 8 shows a relationship between the excitation energy and the

emission peak intensity thereof. The emission peak and the emission peak intensity in Figs. 6 to 8 are the emission peak and the emission peak intensity, respectively, at 535 nm. Fig. 6 to Fig. 8 confirmed ASE in both the organic semiconductor lasers of Example 1 and Comparative Example 1, in which the half-value width FWHM of the emission peak rapidly decreased and the emission peak intensity rapidly increased at the excitation energy of 1.0 μJ/cm$^2$ or more. Of the organic semiconductor laser of Example 1, the threshold energy $E_{th}$ at which the emission peak intensity rapidly changed was 0.8 ± 0.3 μJ/cm$^2$, while the threshold energy $E_{th}$ of the organic semiconductor laser of Comparative Example 1 was 1.2 ± 0.3 μJ/cm$^2$ and was a large value. The results show that, in the organic semiconductor laser of Example 1 having an energy transfer mechanism via reverse intersystem crossing at ACRXTN, the singlet excitons of C545T effectively increased.

Further, the loss coefficient through a waveguide of the organic semiconductor laser of Example 1 was 11 ± 1/cm and that of the organic semiconductor laser of Comparative Example 1 was 10 ± 1/cm. From the results, it is known that the triplet excitons in ACRXTN do not have any negative influence on the optical amplification process of C545T.

(Comparative Example 2) Production and Evaluation of Photoexcitation-Type Organic Semiconductor Laser Using mCBP (host material), FIrpic (phosphor material) and C545T (light-emitting material)

A photoexcitation-type organic semiconductor laser having a thin film containing mCBP, FIrpic (6% by weight) and C545T (1% by weight) was produced according to the same process as in Example 1 except that FIrpic was used in place of ACRXTN in forming the thin film.

[Chem. 176]

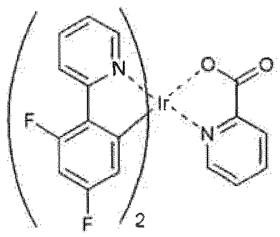

FIrpic

Fig. 9 shows the emission spectrum with 377-nm excitation light of the produced organic semiconductor laser, and Fig. 10 shows the relationship between the excitation energy and the emission peak intensity. As shown in Fig. 9, the organic semiconductor laser provided emission derived from C545T having a photoluminescence quantum efficiency of 80 ± 3% and a delayed emission component. It is presumed that the delayed emission component would be based on the formation of singlet excitons through transfer of the excited triplet energy of FIrpic to C545T. However, as shown in Fig. 10, in the organic semiconductor laser of Comparative Example 2, any rapid emission peak intensity change was not admitted even though the excitation energy was 100 μJ/cm$^2$ or more. This may be considered because singlet-triplet annihilation and triplet-triplet annihilation would have occurred so that the singlet excitons of C545T and the triplet excitons of FIrpic would have been therefore annihilated.

[Production and Evaluation of Current Injection-Type Organic Semiconductor Laser] (Example 2) Production of Carrier Injection-Type Organic Semiconductor Laser Using mCBP (host material), ACRXTN (delayed fluorescent material) and C545T (light-emitting material)

On a glass substrate having, as formed thereon, an anode of indium tin oxide (ITO) having a thickness of 110 nm, thin films were laminated under a vacuum degree of 5.0 × 10$^{-5}$ Pa or less according to a vacuum evaporation method. First, HATCN was formed to have a thickness of 10 nm on ITO, and Tris-PCz was further formed thereon to have a thickness of 20 nm. Next, C545T, ACRXTN and mCBP were co-deposited from different deposition sources, thereby forming a first light-emitting layer having a thickness of 20 mm, a second light-emitting layer having a thickness of 5 nm and a third light-emitting layer having a thickness of 15 nm. At this time, in the first light-emitting layer, the concentration of C545T was 1% by weight and the concentration of ACRXTN was 20% by weight, in the second light-emitting layer, the concentration of C545T was 1% by weight and the concentration of ACRXTN was 6% by weight, and in the third light-emitting layer, the concentration of C545T was 1% by weight and the concentration of ACRXTN was 20% by weight. Next, T2T was formed to have a thickness of 10 nm, and BPyTP2 was further formed thereon to have

a thickness of 20 nm. In addition, lithium fluoride (LiF) was deposited thereon in a thickness of 0.8 nm through vapor deposition, and then aluminum (Al) was deposited thereon in a thickness of 100 nm, thereby forming a cathode to give a carrier injection-type organic semiconductor laser.

(Comparative Example 3) Production of Current Injection-Type Organic Semiconductor Laser Using mCBP (host material) and C545T (light-emitting material)

A carrier injection-type organic semiconductor laser was produced in the same manner as in Example 2 except that one light-emitting layer having a thickness of 40 nm was formed through co-deposition of C545T and mCBP in place of forming the first light-emitting layer to the third light-emitting layer. Here, the concentration of C545T in the light-emitting layer was 1 % by weight.

The organic semiconductor lasers produced in Example 2 and Comparative Example 3 were evaluated for device characteristics.

Fig. 11 shows the emission spectrum of each organic semiconductor laser, Fig. 12 shows the voltage-current density characteristic, and Fig. 13 shows the current density-external quantum efficiency characteristic. The right-side scale indicates the increase rate in the external quantum efficiency of Example 2 relative to that of Comparative

Example 3.

Both the organic semiconductor laser of Example 2 and the organic semiconductor laser of Comparative Example 3 provided green emission resulting from C545T, but external quantum efficiency of the organic semiconductor laser of Example 2 provided was larger by 6 times or more than that of the organic semiconductor laser of Comparative Example 3. The increase rate in the external quantum efficiency in Example 2 was higher than that in Comparative Example 3 even in the high current range, and therefore it is presumed that the exciton annihilation would be suppressed in Example 2. The maximum internal quantum efficiency was calculated. The organic semiconductor laser of Example 2 was 33 to 50%, and the organic EL device of Comparative Example 2 was 5 to 7.5%. The data of maximum internal quantum efficiency correspond to 38 to 58%, and 6 to 9%, respectively, of the exciton formation efficiency. From the value of the exciton formation efficiency, it is known that, in the organic semiconductor laser of Example 2, the triplet excitons formed in ACRXTN greatly contribute toward the number of the singlet excitons in C545T.

The threshold current density was measured using the threshold energy $E_{th}$ and the maximum exciton formation efficiency. The value of the organic semiconductor laser of Example 2 was 186 to 280 A/cm$^2$, and the value of the organic semiconductor laser of Comparative Example 3 was 1.8 to 2.69 kA/cm$^2$. From this, it is known that, in the carrier injection-type system, addition of ACRXTN greatly reduced the threshold value because of optical amplification.

[Chem. 177]

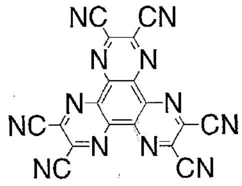

HATCN

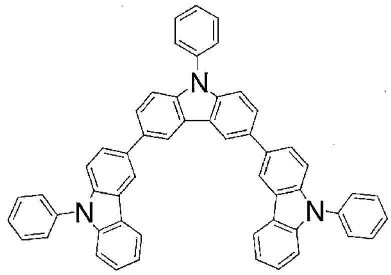

Tris-PCz

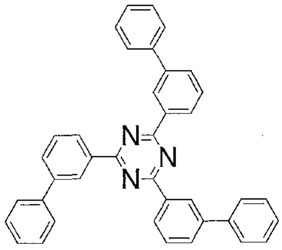

T2T

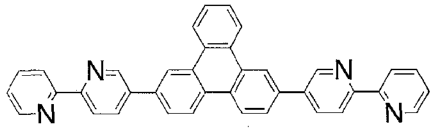

BPyTP2

Industrial Applicability

The organic light-emitting device of the present invention realizes a high light emission efficiency and is therefore applicable to various instruments as an organic semiconductor laser. Accordingly, the industrial applicability of the present invention is high.

Reference Signs List

1 Substrate
2 Anode
3 Hole Injection Layer
4 Hole Transport Layer
5 Light-Emitting Layer
6 Electron Transport Layer
7 Cathode

**Claims**

**1.** An organic light-emitting device containing a host material, a delayed fluorescent material and a light-emitting material satisfying the following expression (1):

$$\text{Expression (1)} \quad E_{S1}(H)>E_{S1}(F)>E_{S1}(D)$$

wherein $E_{S1}(H)$ represents a lowest excited singlet energy level of the host material, $E_{S1}(F)$ represents a lowest excited singlet energy level of the delayed fluorescent material, and $E_{S1}(D)$ represents a lowest excited singlet energy level of the light-emitting material,
wherein the organic light-emitting device is an organic semiconductor laser,
**characterized in that**
the delayed fluorescent material has an energy difference $\Delta E_{st}$ between a lowest excited singlet state and a lowest excited triplet state at 77 K of 0.3 eV or less,
energy from an excited singlet state of the host material transfers to the delayed fluorescent material and the

light-emitting material,
a triplet excited state of the delayed fluorescent material transfers to a singlet excited state of the delayed fluorescent material through reverse intersystem crossing, and
the excited singlet energy of the delayed fluorescent material transfers to the light-emitting material.

**2.** The organic light-emitting device according to claim 1, wherein the delayed fluorescent material has an energy difference $\Delta E_{st}$ between a lowest excited singlet state and a lowest excited triplet state at 77 K of 0.08 eV or less.

**3.** The organic light-emitting device according to any one of claims 1 or 2, wherein the delayed fluorescent material has a rate constant $k_{RISC}$ from a lowest excited triplet state to a lowest excited singlet state of $10^5$/s or more.

**4.** The organic light-emitting device according to any one of claims 1 to 3, wherein the light-emitting material radiates fluorescence when returning from a lowest excited singlet energy level to a ground energy level.

**5.** The organic light-emitting device according to any one of claims 1 to 4, wherein the light-emitting material radiates amplified spontaneous emission.

**6.** The organic light-emitting device according to any one of claims 1 to 5, wherein the content of the delayed fluorescent material is smaller than the content of the host material.

**7.** The organic light-emitting device according to any one of claims 1 to 6, containing two or more kinds of compounds as the light-emitting material.

**8.** The organic light-emitting device according to any one of claims 1 to 7, having a light-emitting layer that contains the host material, the delayed fluorescent material and the light-emitting material satisfying the expression (1).

**9.** The organic light-emitting device according to claim 8, wherein the light-emitting layer has a multilayer configuration of plural layers.

**10.** The organic light-emitting device according to claim 9, wherein the plural layers constituting the light-emitting layer each have a different content of the delayed fluorescent material therein.

**11.** The organic light-emitting device according to any one of claims 1 to 10, which is a photoexcitation-type organic semiconductor laser.

**12.** The organic light-emitting device according to claim 1, which is a carrier injection-type organic semiconductor laser.

**Patentansprüche**

**1.** Organische, Licht emittierende Vorrichtung, enthaltend ein Wirtsmaterial, ein verzögert fluoreszierendes Material und ein Licht emittierendes Material, welche den folgenden Ausdruck (1) erfüllt:

$$\text{Ausdruck (1)} \qquad E_{S1}(H) > E_{S1}(F) > E_{S1}(D)$$

wobei $E_{S1}(H)$ ein niedrigstes angeregtes Singulett-Energieniveau des Wirtsmaterials darstellt, $E_{S1}(F)$ ein niedrigstes angeregtes Singulett-Energieniveau des verzögert fluoreszierenden Materials darstellt und $E_{S1}(D)$ ein niedrigstes angeregtes Singulett-Energieniveau des Licht emittierenden Materials darstellt,
wobei die organische, Licht emittierende Vorrichtung ein organischer Halbleiter-Laser ist, **dadurch gekennzeichnet, dass**
das verzögert fluoreszierende Material eine Energiedifferenz $\Delta E_{st}$ zwischen einem niedrigsten angeregten Singulett-Zustand und einem niedrigsten angeregten Triplett-Zustand bei 77 K von 0,3 eV oder weniger aufweist,
Energie von einem angeregten Singulett-Zustand des Wirtsmaterials zu dem verzögert fluoreszierenden Material und dem Licht emittierenden Material übertragen wird,
ein Triplett-angeregter Zustand des verzögert fluoreszierenden Materials zu einem Singulettangeregten Zustand des verzögert fluoreszierenden Materials durch umgekehrte Kreuzung zwischen Systemen übertragen wird, und
die angeregte Singulett-Energie des verzögert fluoreszierenden Materials zum Licht emittierenden Material

übertragen wird.

2. Organische, Licht emittierende Vorrichtung nach Anspruch 1, wobei das verzögert fluoreszierende Material eine Energiedifferenz $\Delta E_{st}$ zwischen einem niedrigsten angeregten Singulett-Zustand und einem niedrigsten angeregten Triplett-Zustand bei 77 K von 0,08 eV oder weniger aufweist.

3. Organische, Licht emittierende Vorrichtung nach einem der Ansprüche 1 oder 2, wobei das verzögert fluoreszierende Material eine Geschwindigkeitskonstante $k_{RISC}$ von einem niedrigsten angeregten Triplett-Zustand zu einem niedrigsten angeregten Singulett-Zustand von $10^5$/s oder mehr aufweist.

4. Organische, Licht emittierende Vorrichtung nach einem der Ansprüche 1 bis 3, wobei das Licht emittierende Material fluoreszierend strahlt, wenn es von einem niedrigsten angeregten Singulett-Energieniveau zu einem Grundenergieniveau zurückkehrt.

5. Organische, Licht emittierende Vorrichtung nach einem der Ansprüche 1 bis 4, wobei das Licht emittierende Material verstärkte Spontanemission ausstrahlt.

6. Organische, Licht emittierende Vorrichtung nach einem der Ansprüche 1 bis 5, wobei der Gehalt an verzögert fluoreszierendem Material geringer ist als der Gehalt an Wirtsmaterial.

7. Organische, Licht emittierende Vorrichtung nach einem der Ansprüche 1 bis 6, die zwei oder mehr Arten von Verbindungen als das Licht emittierende Material enthält.

8. Organische, Licht emittierende Vorrichtung nach einem der Ansprüche 1 bis 7, die eine Licht emittierende Schicht aufweist, welche das Wirtsmaterial enthält, wobei das verzögert fluoreszierende Material und das Licht emittierende Material den Ausdruck (1) erfüllen.

9. Organische, Licht emittierende Vorrichtung nach Anspruch 8, wobei die Licht emittierende Schicht einen mehrschichtigen Aufbau aus mehreren Schichten aufweist.

10. Organische, Licht emittierende Vorrichtung nach Anspruch 9, wobei die mehreren Schichten, welche die Licht emittierende Schicht bilden, jeweils einen unterschiedlichen Gehalt an verzögert fluoreszierendem Material aufweisen.

11. Organische, Licht emittierende Vorrichtung nach einem der Ansprüche 1 bis 10, welche ein organischer Halbleiterlaser vom Photoanregungstyp ist.

12. Organische, Licht emittierende Vorrichtung nach Anspruch 1, welche ein organischer Halbleiterlaser vom Trägerinjektionstyp ist.

**Revendications**

1. Dispositif électroluminescent organique contenant un matériau hôte, un matériau à fluorescence retardée et un matériau électroluminescent satisfaisant à l'expression (1) suivante :

$$\text{Expression (1) } E_{SI}(H)>E_{SI}(F)>E_{SI}(D)$$

sachant que $E_{SI}(H)$ représente un niveau d'énergie de singulet excité le plus bas du matériau hôte, $E_{SI}(F)$ représente un niveau d'énergie de singulet excité le plus bas du matériau à fluorescence retardée, et $E_{SI}(D)$ représente un niveau d'énergie de singulet excité le plus bas du matériau électroluminescent,
sachant que le dispositif électroluminescent organique est un laser à semiconducteur organique,
**caractérisé en ce que**
le matériau à fluorescence retardée présente une différence d'énergie $\Delta E_{st}$ entre un état de singulet excité le plus bas et un état de triplet excité le plus bas à 77 K de 0,3 eV ou moins,
l'énergie provenant d'un état de singulet excité du matériau hôte est transférée au matériau à fluorescence retardée et au matériau électroluminescent,

un état de triplet excité du matériau à fluorescence retardée est transféré à un état de singulet excité du matériau à fluorescence retardée par croisement intersystème inverse, et
l'énergie de singulet excité du matériau à fluorescence retardée est transférée au matériau électroluminescent.

**2.** Le dispositif électroluminescent organique selon la revendication 1, sachant que le matériau à fluorescence retardée présente une différence d'énergie $\Delta E_{st}$ entre un état de singulet excité le plus bas et un état de triplet excité le plus bas à 77 K de 0,08 eV ou moins.

**3.** Le dispositif électroluminescent organique selon l'une quelconque des revendications 1 ou 2, sachant que le matériau à fluorescence retardée présente une constante cinétique $k_{RISC}$ d'un état de triplet excité le plus bas à un état de singulet excité le plus bas de $10^5$/s ou plus.

**4.** Le dispositif électroluminescent organique selon l'une quelconque des revendications 1 à 3, sachant que le matériau électroluminescent irradie de la fluorescence lorsqu'il repasse d'un niveau d'énergie de singulet excité le plus bas à un niveau d'énergie fondamental.

**5.** Le dispositif électroluminescent organique selon l'une quelconque des revendications 1 à 4, sachant que le matériau électroluminescent irradie une émission spontanée amplifiée.

**6.** Le dispositif électroluminescent organique selon l'une quelconque des revendications 1 à 5, sachant que la teneur en le matériau à fluorescence retardée est inférieure à la teneur en le matériau hôte.

**7.** Le dispositif électroluminescent organique selon l'une quelconque des revendications 1 à 6, contenant deux ou plus de deux types de composés en tant que le matériau électroluminescent.

**8.** Le dispositif électroluminescent organique selon l'une quelconque des revendications 1 à 7, comportant une couche électroluminescente qui contient le matériau hôte, le matériau à fluorescence retardée et le matériau électroluminescent satisfaisant à l'expression (1).

**9.** Le dispositif électroluminescent organique selon la revendication 8, sachant que la couche électroluminescente présente une configuration multicouche de couches multiples.

**10.** Le dispositif électroluminescent organique selon la revendication 9, sachant que les couches multiples constituant la couche électroluminescente comportent chacune une teneur différente en le matériau à fluorescence retardée.

**11.** Le dispositif électroluminescent organique selon l'une quelconque des revendications 1 à 10, lequel est un laser à semiconducteur organique de type à photoexcitation.

**12.** Le dispositif électroluminescent organique selon la revendication 1, lequel est un laser à semiconducteur organique de type à injection de porteurs.

Fig. 1

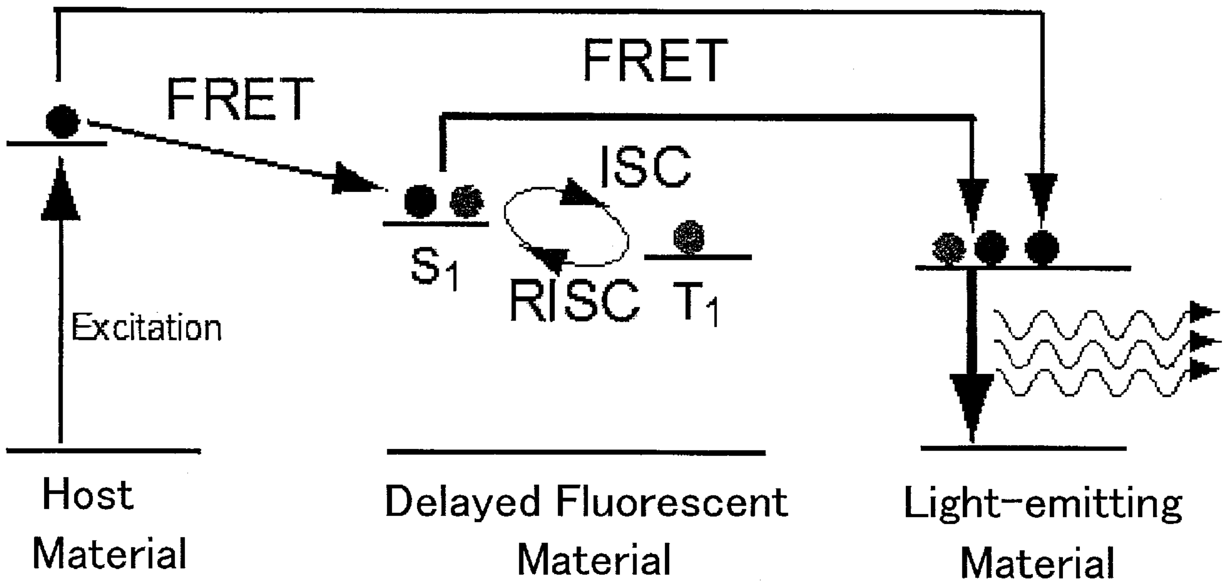
FRET
FRET
ISC
S1
RISC
T1
Excitation
Host Material
Delayed Fluorescent Material
Light-emitting Material

Fig. 2

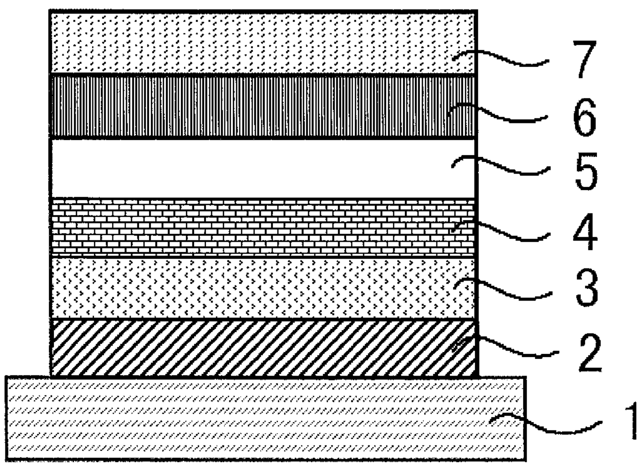
7
6
5
4
3
2
1

Fig. 3

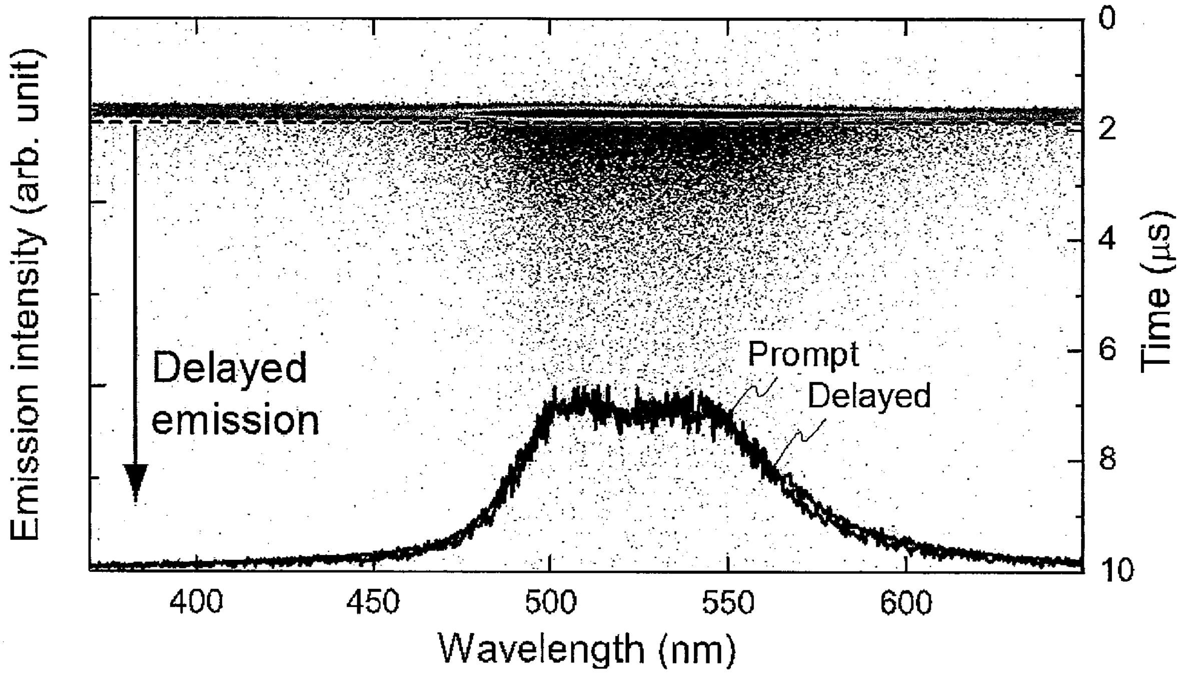
Emission intensity (arb. unit)
Delayed
emission
Prompt
Delayed
400
450
500
550
600
Wavelength (nm)
0
2
4
6
8
10
Time (μs)

Fig. 4

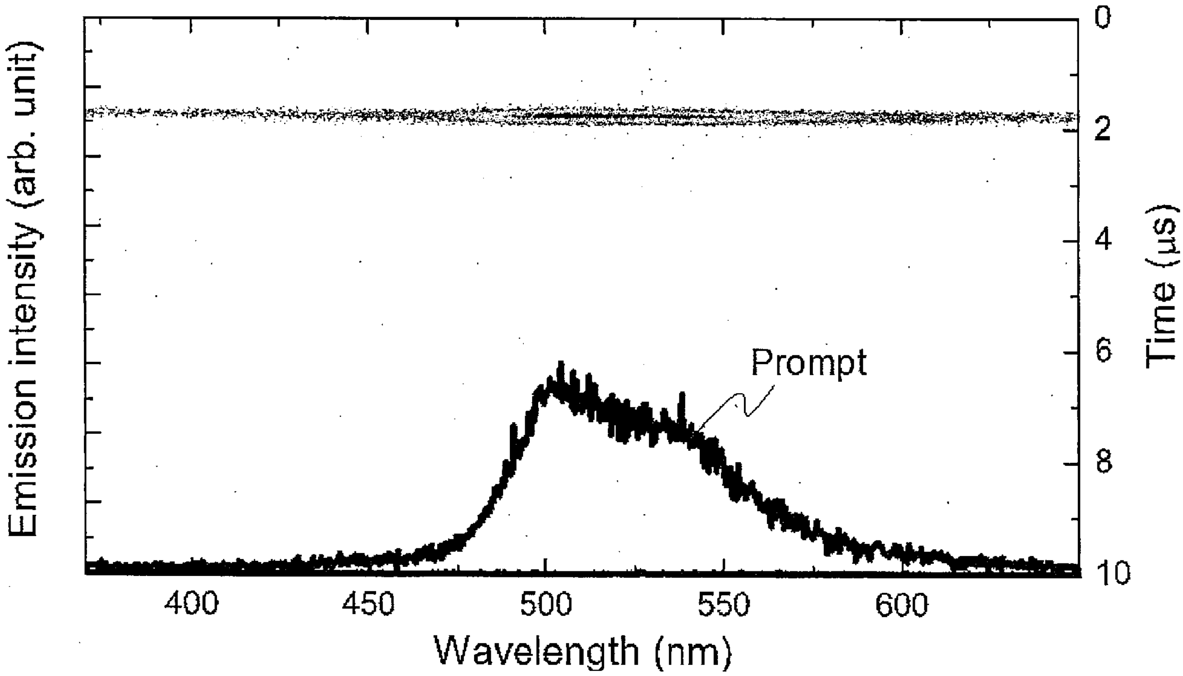
Emission intensity (arb. unit)
Prompt
400
450
500
550
600
Wavelength (nm)
0
2
4
6
8
10
Time (μs)

Fig. 5

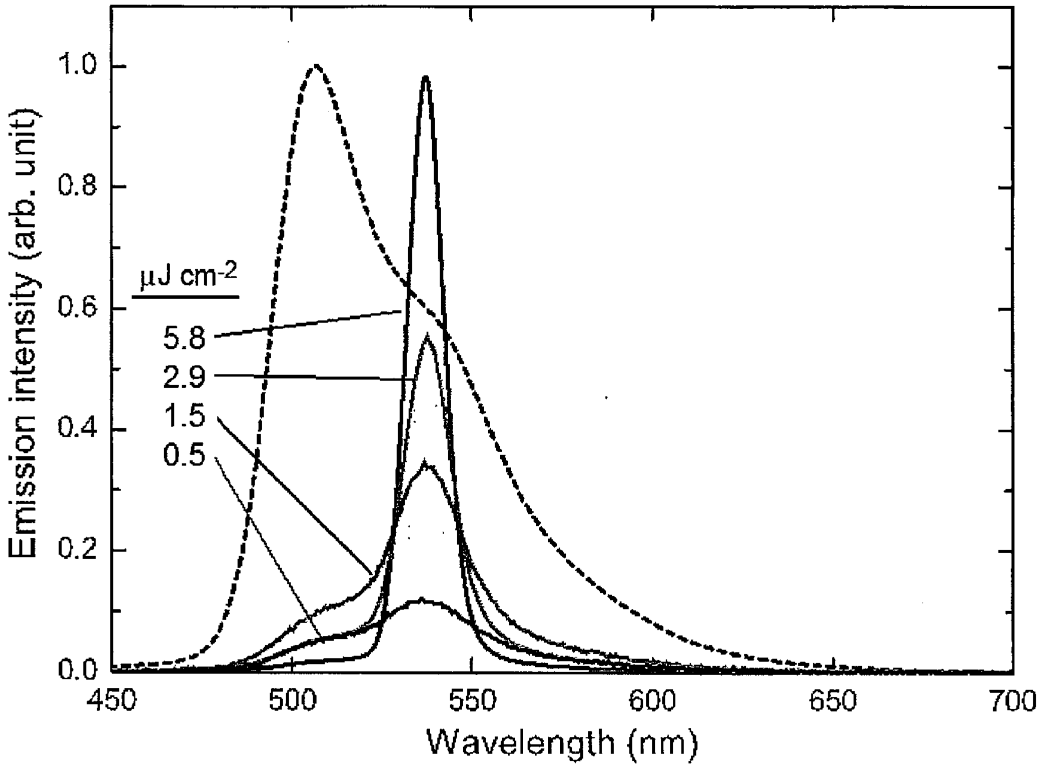
Emission intensity (arb. unit)
1.0
0.8
0.6
0.4
0.2
0.0
μJ cm-2
5.8
2.9
1.5
0.5
450
500
550
600
650
700
Wavelength (nm)

Fig. 6

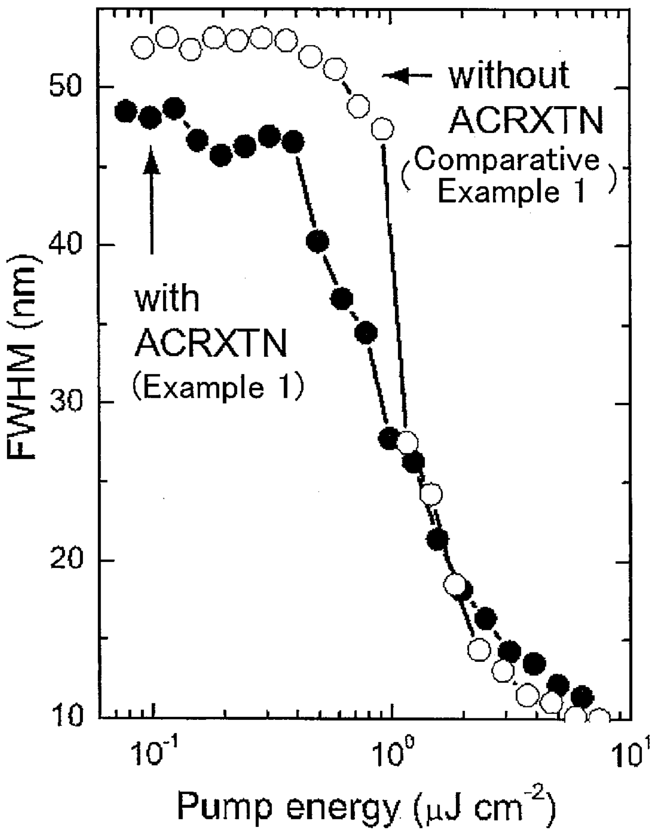
FWHM (nm)
50
40
30
20
10
without ACRXTN (Comparative Example 1)
with ACRXTN (Example 1)
10-1
100
101
Pump energy (μJ cm-2)

Fig. 7

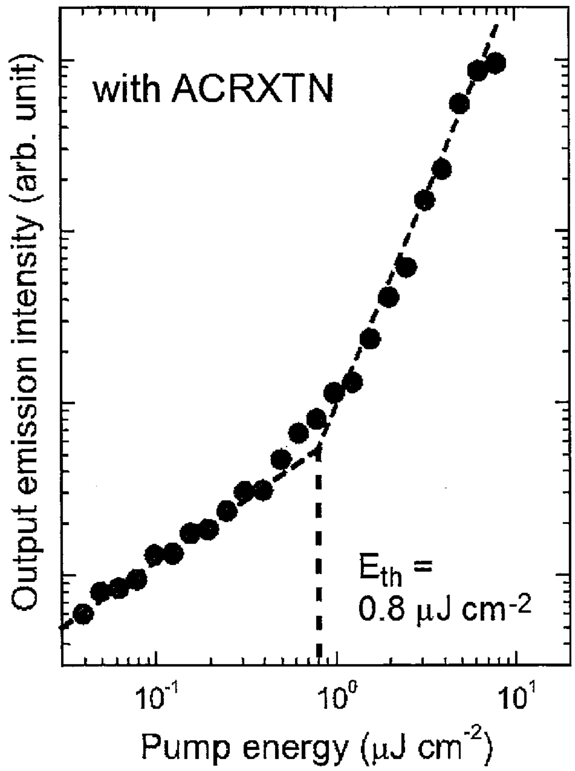
with ACRXTN
Output emission intensity (arb. unit)
$E_{th}$ =
0.8 μJ cm-2
10-1
100
101
Pump energy (μJ cm-2)

Fig. 8

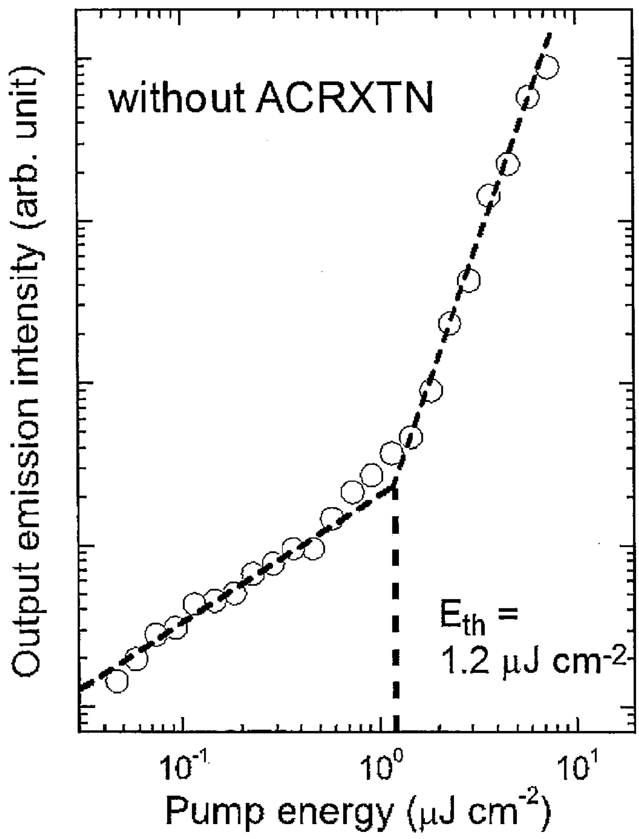
without ACRXTN
Output emission intensity (arb. unit)
$E_{th}$ =
1.2 μJ cm-2
10-1
100
101
Pump energy (μJ cm-2)

Fig. 9

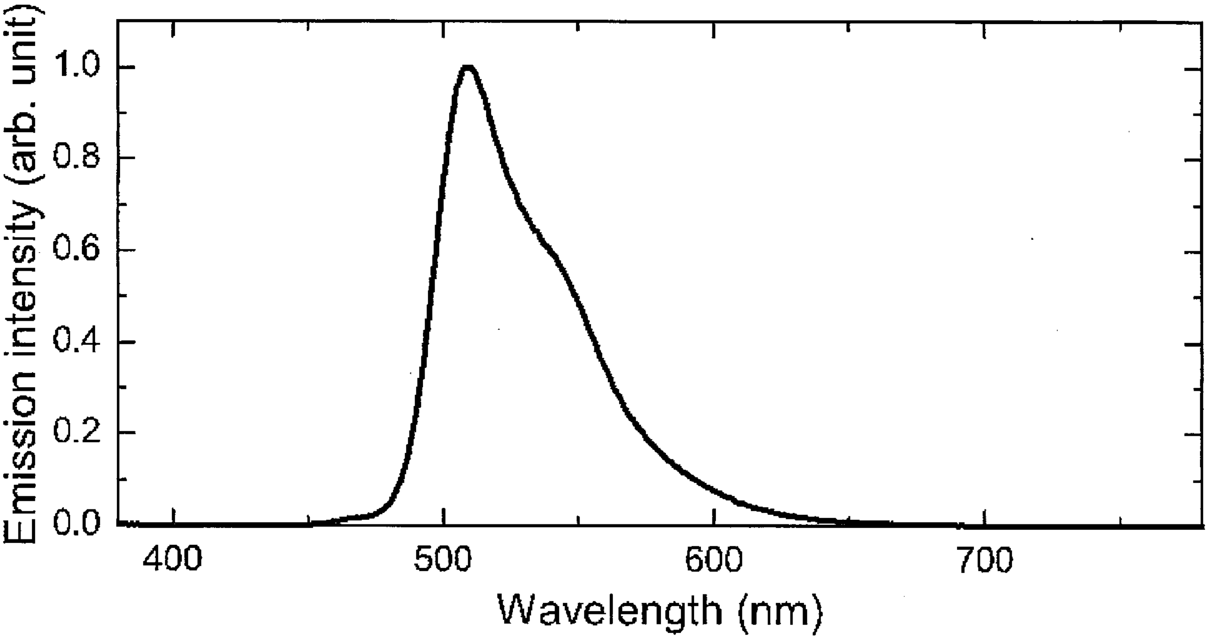
Emission intensity (arb. unit)
1.0
0.8
0.6
0.4
0.2
0.0
400
500
600
700
Wavelength (nm)

Fig. 10

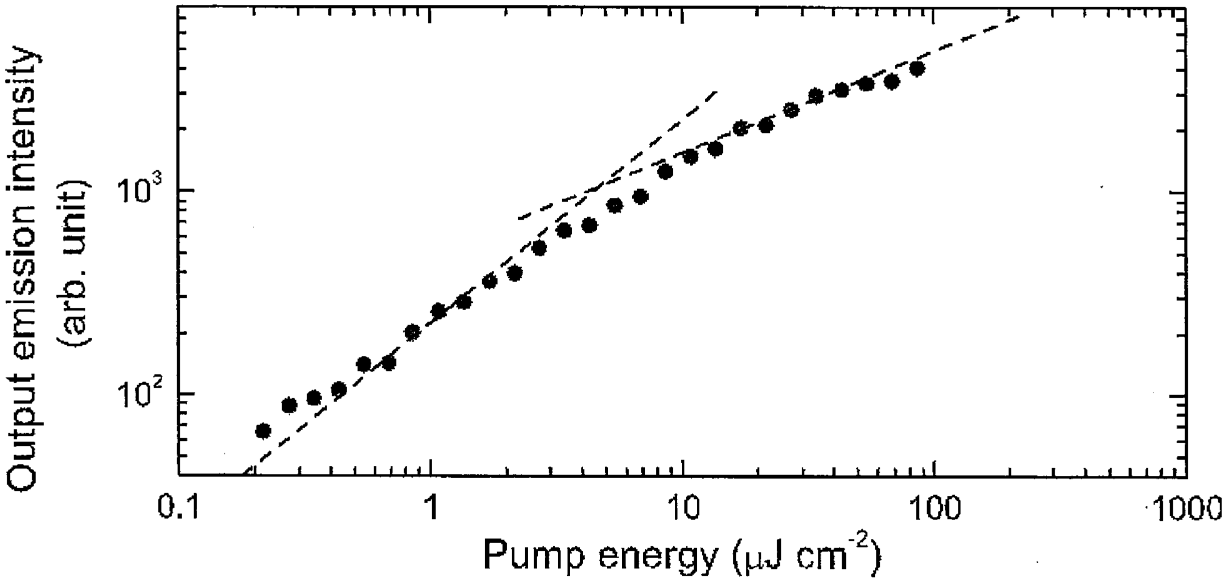
Output emission intensity
(arb. unit)
10³
10²
0.1
1
10
100
1000
Pump energy (μJ cm⁻²)

Fig. 11

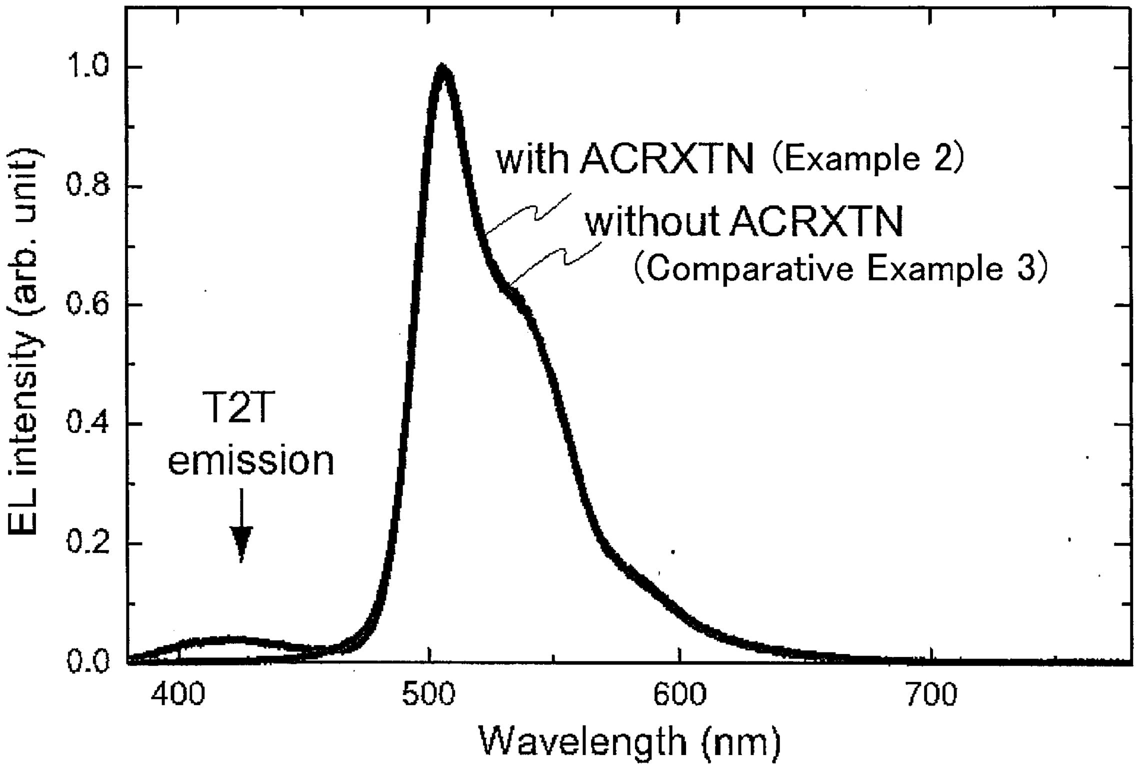

1.0
0.8
0.6
0.4
0.2
0.0
EL intensity (arb. unit)
with ACRXTN (Example 2)
without ACRXTN
(Comparative Example 3)
T2T
emission
400
500
600
700
Wavelength (nm)


Fig. 12

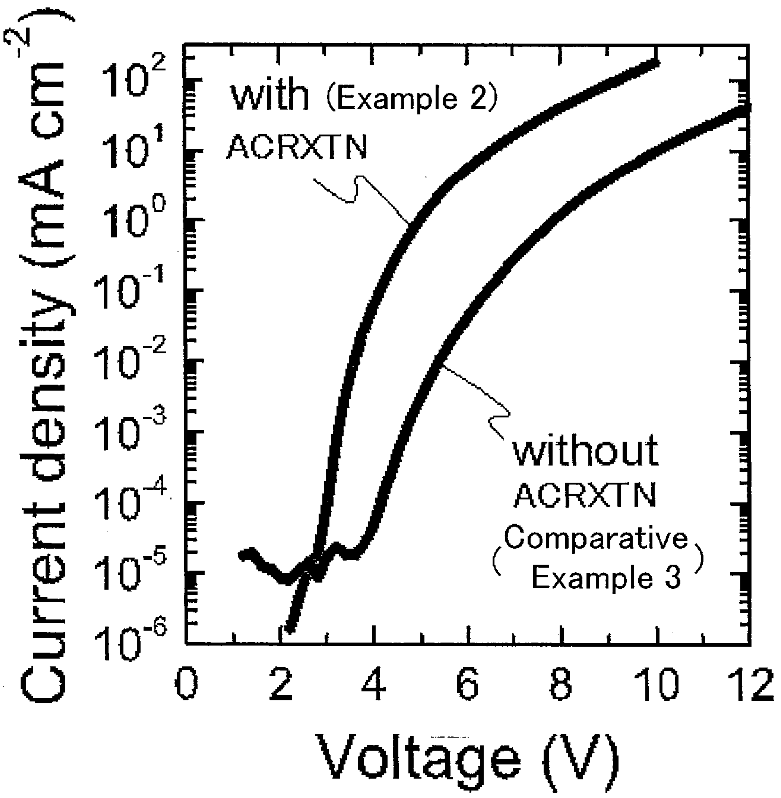

Current density (mA cm-2)
10^2
10^1
10^0
10^-1
10^-2
10^-3
10^-4
10^-5
10^-6
with (Example 2)
ACRXTN
without
ACRXTN
(Comparative Example 3)
0
2
4
6
8
10
12
Voltage (V)

Fig. 13

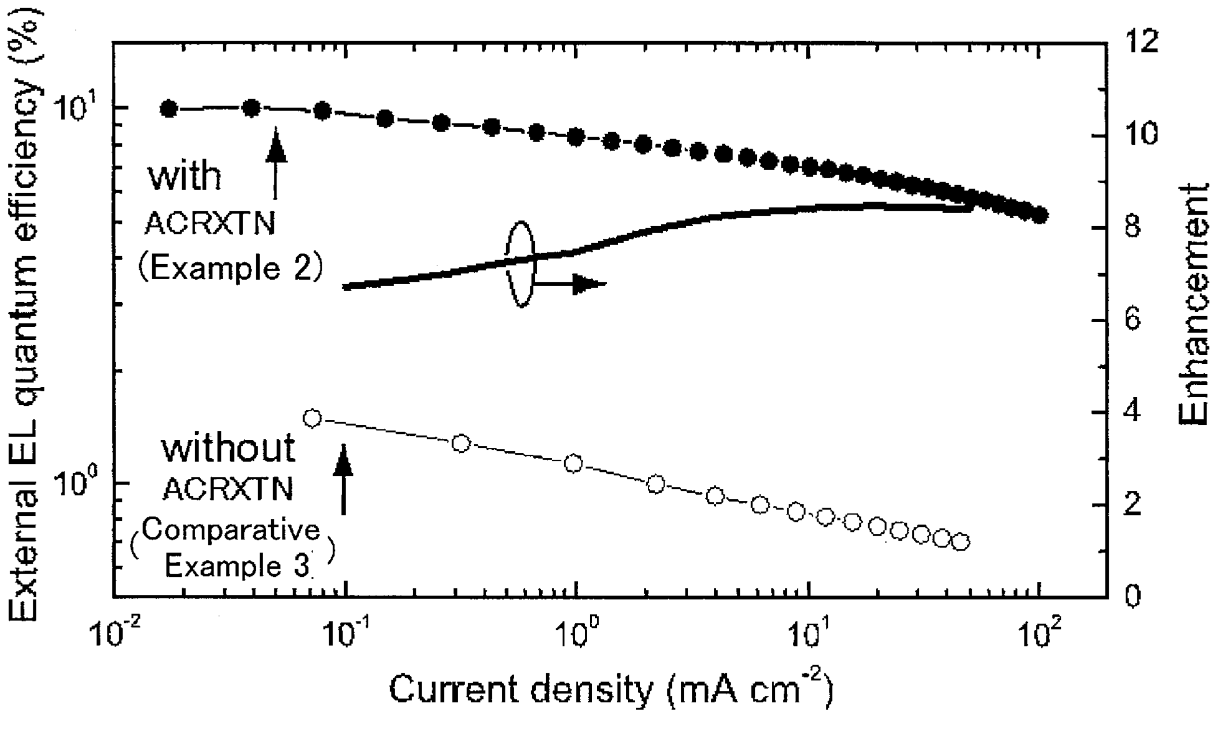
External EL quantum efficiency (%)
$10^1$
$10^0$
with
ACRXTN
(Example 2)
without
ACRXTN
(Comparative Example 3)
$10^{-2}$
$10^{-1}$
$10^0$
$10^1$
$10^2$
Current density (mA $cm^{-2}$)
12
10
8
6
4
2
0
Enhancement

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 102012021174 A1
- JP 2006265172 A
- US 8654806 B


### Non-patent literature cited in the description


- **TETSUO NOZAWA.** *Kyushu University Claims OLED Breakthrough,* 01 June 2014, 1-5